# EUROPEAN PATENT SPECIFICATION

(11) **EP 1 254 260 B1**
(45) Date of publication and mention of the grant of the patent: **30.08.2006**
(21) Application number: 01902063.5
(22) Date of filing: 12.01.2001
(51) Int. Cl.: C12Q 1/68, A61K 48/00, C12N 15/00, A01K 67/027, C07K 14/46

(54) **METHODS FOR DIAGNOSING AND TREATING HEART DISEASE**
METHODEN ZUR DIAGNOSE UND BEHANDLUNG VON HERZKRANKHEITEN
METHODES POUR DIAGNOSTIQUER ET TRAITER UNE MALADIE DU COEUR

(30) Priority: 12.01.2000 US 175787 P
(43) Date of publication of application: 06.11.2002
(73) Proprietor: THE GENERAL HOSPITAL CORPORATION, Boston, MA 02114 (US)
(72) Inventor: FISHMAN, Mark, C., Newton Center, MA 02459 (US); XU, Xiaolei, Brookline, MA 02446 (US)
(74) Representative: Bösl, Raphael Konrad
(86) International application number: PCT/US2001/001212
(87) International publication number: WO 2001/051666

(56) References cited:
- STAINIER DIDIER Y R ET AL: "Mutations affecting the formation and function of the cardiovascular system in the zebrafish embryo." DEVELOPMENT (CAMBRIDGE), vol. 123, no. 1, 1996, pages 285-292, XP002241860 ISSN: 0950-1991
- MUELLER SEITZ M ET AL: "Chromosomal Localization of the Mouse Titin Gene and Its Relation to "Muscular Dystrophy with Myositis" and Nebulin Genes on Chromosome 2." GENOMICS, vol. 18, no. 3, 1993, pages 559-561, XP009010992 ISSN: 0888-7543
- GARVEY SEAN M ET AL: "The muscular dystrophy with myositis (mdm) mouse mutation disrupts a skeletal muscle-specific domain of titin." GENOMICS. UNITED STATES FEB 2002, vol. 79, no. 2, February 2002 (2002-02), pages 146-149, XP002241861 ISSN: 0888-7543
- HEIN S ET AL: "Altered expression of titin and contractile proteins in failing human myocardium." JOURNAL OF MOLECULAR AND CELLULAR CARDIOLOGY. ENGLAND OCT 1994, vol. 26, no. 10, October 1994 (1994-10), pages 1291-1306, XP002241862 ISSN: 0022-2828
- FISHMAN M C: "Zebrafish genetics: the enigma of arrival." PROCEEDINGS OF THE NATIONAL ACADEMY OF SCIENCES OF THE UNITED STATES OF AMERICA. UNITED STATES 14 SEP 1999, vol. 96, no. 19, 14 September 1999 (1999-09-14), pages 10554-10556, XP002241863 ISSN: 0027-8424
- DRIEVER W ET AL: "A genetic screen for mutations affecting embryogenesis in zebrafish." DEVELOPMENT (CAMBRIDGE, ENGLAND) ENGLAND DEC 1996, vol. 123, December 1996 (1996-12), pages 37-46, XP002241864 ISSN: 0950-1991
- XU XIAOLEI ET AL: "Cardiomyopathy in zebrafish due to mutation in an alternatively spliced exon of titin." NATURE GENETICS. UNITED STATES FEB 2002, vol. 30, no. 2, February 2002 (2002-02), pages 205-209, XP001152521 ISSN: 1061-4036
- SATOH M.: 'Structural analysis of the titin gene in hypertrophic cardiomyopathy: Identification of a novel disease gene' BIOCHEMICAL AND BIOPHYSICAL RESEARCH COMMUNICATIONS vol. 262, August 1999, pages 411 - 417, XP002940257
- SIU B.L.: 'Familial dilated cardiomyopathy locus maps to chromosome 2q31' CIRCULATION vol. 99, 02 March 1999, pages 1022 - 1026, XP002940256

## Description

### Field of the Invention

This invention relates to methods for diagnosing and treating heart disease.

### Background of the Invention

Heart disease is a general term used to describe many different heart conditions. For example, coronary artery disease, which is the most common heart disease, is characterized by constriction or narrowing of the arteries supplying the heart with oxygen-rich blood, and can lead to myocardial infarction, which is the death of a portion of the heart muscle. Heart failure is a condition resulting from the inability of the heart to pump an adequate amount of blood through the body. Heart failure is not a sudden, abrupt stop of heart activity, but, rather, typically develops slowly over many years, as the heart gradually loses its ability to pump blood efficiently. Risk factors for heart failure include coronary artery disease, hypertension, valvular heart disease, cardiomyopathy, disease of the heart muscle, obesity, diabetes, and a family history of heart failure.

### Summary of the Invention

The invention provides diagnostic, drug screening, and therapeutic methods based on the observation that mutation of the *titin* gene leads to a phenotype in zebrafish that is similar to mammalian heart failure.

In one aspect, the invention provides a method of determining whether a test subject *(e.g.,* a mammal, such as a human) has, or is at risk of developing, a titin-reiated disease or condition *(e.g.,* heart failure). This method involves analyzing a nucleic acid molecule of a sample from the test subject to determine whether the test subject has a mutation (for example, a mutation in a cardiac-specific exon, such as the N2B exon; *e.g.,* the *pickwick* mutation; see below) in a *titin* gene. The presence of such a mutation is an indication that the test subject has, or is at risk of developing, a titin-related disease. This method can further involve using nucleic acid molecule primers specific for the *titin* gene for nucleic acid molecule amplification of the *titin* gene by the polymerase chain reaction, or sequencing *titin* nucleic acid molecules from the test subject.

In another aspect, the invention provides a screening method for identifying a compound that can be used to treat or to prevent heart failure. This method involves contacting an organism *(e.g.,* a zebrafish) having a *titin* mutation (for example, a mutation in a cardiac-specific exon, such as the N2B exon; *e.g.,* the *pickwick* mutation) and a phenotype characteristic of heart failure with the compound, and determining the effect of the compound on the phenotype. Detection of an improvement in the phenotype indicates the identification of a compound that can be used to treat or to prevent heart failure.

In another aspect, the invention provides a method of treating or preventing heart disease, such as heart failure, in a patient. This method involves administering to the patient a compound identified using the screening method described above. A patient treated using this method can have a mutation in the *titin* gene.

In a further aspect, the invention provides a non-human animal *(e.g.,* a zebrafish or a mouse) that has a mutation in a *titin* gene. The mutation can be, for example, in a cardiac-specific exon of the *titin* gene, such as the N2B exon, and can result in production of a truncated titin product, for example, by the introduction of a stop codon.

By "polypeptide" or "polypeptide fragment" is meant a chain of two or more amino acids, regardless of any post-translational modification *(e.g.,* glycosylation or phosphorylation), constituting all or part of a naturally or non-naturally occurring polypeptide. By "post-translational modification" is meant any change to a polypeptide or polypeptide fragment during or after synthesis. Post-translational modifications can be produced naturally (such as during synthesis within a cell) or generated artificially (such as by recombinant or chemical means). A "protein" can be made up of one or more polypeptides.

By "titin," "titin protein," or "titin polypeptide" is meant a polypeptide that has at least 45%, preferably at least 60%, more preferably at least 75%, and most preferably at least 90% amino acid sequence identity to the sequence of the human (see below) or the zebrafish titin polypeptides. Polypeptide products from splice variants of *titin* gene sequences and *titin* genes containing mutations are also included in this definition. A titin polypeptide as defined herein plays a role in heart development, modeling, structure, and contractility. It can be used as a marker of heart disease, such as heart failure.

By a *"titin* nucleic acid molecule" is meant a nucleic acid molecule, such as a genomic DNA, cDNA, or RNA *(e.g.,* mRNA) molecule, that encodes titin, a titin protein, a titin polypeptide, or a portion thereof, as defined above.

The term "identity" is used herein to describe the relationship of the sequence of a particular nucleic acid molecule or polypeptide to the sequence of a reference molecule of the same type. For example, if a polypeptide or nucleic acid molecule has the same amino acid or nucleotide residue at a given position, compared to a reference molecule to which it is aligned, there is said to be "identity" at that position. The level of sequence identity of a nucleic acid molecule or a polypeptide to a reference molecule is typically measured using sequence analysis software with the default parameters specified therein, such as the introduction of gaps to achieve an optimal alignment *(e.g.,* Sequence Analysis Software Package of the Genetics Computer Group, University of Wisconsin Biotechnology Center, 1710 University Avenue, Madison, WI 53705, BLAST, or PILEUP/PRETTYBOX programs). These software programs match identical or similar sequences by assigning degrees of identity to various substitutions, deletions, or other modifications. Conservative substitutions typically include substitutions within the following groups: glycine, alanine, valine, isoleucine, and leucine; aspartic acid, glutamic acid, asparagine, and glutamine; serine and threonine; lysine and arginine; and phenylalanine and tyrosine.

A nucleic acid molecule or polypeptide is said to be "substantially identical" to a reference molecule if it exhibits, over its entire length, at least 51 %, preferably at least 55%, 60%, or 65%, and most preferably 75%, 85%, 90%, or 95% identity to the sequence of the reference molecule. For polypeptides, the length of comparison sequences is at least 16 amino acids, preferably at least 20 amino acids, more preferably at least 25 amino acids, and most preferably at least 35 amino acids. For nucleic acid molecules, the length of comparison sequences is at least 50 nucleotides, preferably at least 60 nucleotides, more preferably at least 75 nucleotides, and most preferably at least 110 nucleotides.

A *titin* nucleic acid molecule or titin polypeptide is "analyzed" or subject to "analysis" if a test procedure is carried out on it that allows the determination of its biological activity or whether it is wild type or mutated. For example, one can analyze the *titin* genes of an animal *(e.g.,* a human or a zebrafish) by amplifying genomic DNA of the animal using the polymerase chain reaction, and then determining whether the amplified DNA contains a mutation, for example, the *pickwick* mutation, by, *e.g.,* nucleotide sequence or restriction fragment analysis.

By "probe" or "primer" is meant a single-stranded DNA or RNA molecule of defined sequence that can base pair to a second DNA or RNA molecule that contains a complementary sequence ("target"). The stability of the resulting hybrid depends upon the extent of the base pairing that occurs. This stability is affected by parameters such as the degree of complementarity between the probe and target molecule, and the degree of stringency of the hybridization conditions. The degree of hybridization stringency is affected by parameters such as the temperature, salt concentration, and concentration of organic molecules, such as formamide, and is determined by methods that are well known to those skilled in the art. Probes or primers specific for *titin* nucleic acid molecules, preferably, have greater than 45% sequence identity, more preferably at least 55-75% sequence identity, still more preferably at least 75-85% sequence identity, yet more preferably at least 85-99% sequence identity, and most preferably 100% sequence identity to the sequences of human (see below) or zebrafish *titin.*

Probes can be detectably-labeled, either radioactively or non-radioactively, by methods that are well-known to those skilled in the art. Probes can be used for methods involving nucleic acid hybridization, such as nucleic acid sequencing, nucleic acid amplification by the polymerase chain reaction, single stranded conformational polymorphism (SSCP) analysis, restriction fragment polymorphism (RFLP) analysis, Southern hybridization, northern *hybridization, in situ* hybridization, electrophoretic mobility shift assay (EMSA), and other methods that are well known to those skilled in the art.

A molecule, *e.g.,* an oligonucleotide probe or primer, a gene or fragment thereof, a cDNA molecule, a polypeptide, or an antibody, can be said to be "detectably-labeled" if it is marked in such a way that its presence can be directly identified in a sample. Methods for detectably-labeling molecules are well known in the art and include, without limitation, radioactive labeling *(e.g.**,*** with an isotope, such as ³²P or ³⁵S) and nonradioactive labeling *(e.g.,* with a fluorescent label, such as fluorescein).

By a "substantially pure polypeptide" is meant a polypeptide (or a fragment thereof) that has been separated from proteins and organic molecules that naturally accompany it. Typically, a polypeptide is substantially pure when it is at least 60%, by weight, free from the proteins and naturally-occurring organic molecules with which it is naturally associated. Preferably, the polypeptide is a titin polypeptide that is at least 75%, more preferably at least 90%, and most preferably at least 99%, by weight, pure. A substantially pure titin polypeptide can be obtained, for example, by extraction from a natural source *(e.g.,* isolated heart tissue), by expression of a recombinant nucleic acid molecule encoding a titin polypeptide, or by chemical synthesis. Purity can be measured by any appropriate method, *e.g.,* by column chromatography, polyacrylamide gel electrophoresis, or HPLC analysis.

A polypeptide is substantially free of naturally associated components when it is separated from those proteins and organic molecules that accompany it in its natural state. Thus, a protein that is chemically synthesized or produced in a cellular system different from the cell in which it is naturally produced is substantially free from its naturally associated components. Accordingly, substantially pure polypeptides not only include those derived from eukaryotic organisms, but also those synthesized in *E*. *coli* or other prokaryotes.

An antibody is said to "specifically bind" to a polypeptide if it recognizes and binds to the polypeptide *(e.g.,* a titin polypeptide), but does not substantially recognize and bind to other molecules *(e.g.,* non-titin related polypeptides) in a sample, *e.g.,* a biological sample that naturally includes the polypeptide.

By "high stringency conditions" is meant conditions that allow hybridization comparable with the hybridization that occurs using a DNA probe of at least 500 nucleotides in length, in a buffer containing 0.5 M NaHPO₄, pH 7.2, 7% SDS, 1 mM EDTA, and 1% BSA (fraction V), at a temperature of 65°C, or a buffer containing 48% formamide, 4.8 x SSC, 0.2 M Tris-Cl, pH 7.6, 1 x Denhardt's solution, 10% dextran sulfate, and 0.1 % SDS, at a temperature of 42°C. (These are typical conditions for high stringency northern or Southern hybridizations.) High stringency hybridization is also relied upon for the success of numerous techniques routinely performed by molecular biologists, such as high stringency PCR, DNA sequencing, single strand conformational polymorphism analysis, and *in situ* hybridization. In contrast to northern and Southern hybridizations, these techniques are usually performed with relatively short probes *(e.g.,* usually 16 nucleotides or longer for PCR or sequencing and 40 nucleotides or longer for *in situ* hybridization). The high stringency conditions used in these techniques are well known to those skilled in the art of molecular biology, and examples of them can be found, for example, in Ausubel *et al., Current Protocols in Molecular Biology,* John Wiley & Sons, New York, NY, 1998, which is hereby incorporated by reference.

By "sample" is meant a tissue biopsy, amniotic fluid, cell, blood, serum, urine, stool, or other specimen obtained from a patient or test subject. The sample can be analyzed to detect a mutation in a *titin* gene, or expression levels of a *titin* gene, by methods that are known in the art. For example, methods such as sequencing, single-strand conformational polymorphism (SSCP) analysis, or restriction fragment length polymorphism (RFLP) analysis of PCR products derived from a patient sample can be used to detect a mutation in a *titin* gene; ELISA can be used to measure levels of titin polypeptide; and PCR can be used to measure the level of a *titin* nucleic acid molecule.

By "titin-related disease" or "titin-related condition" is meant a disease or condition that results from inappropriately high or low expression of a *titin* gene, or a mutation in a *titin* gene that alters the biological activity of a *titin* nucleic acid molecule or polypeptide. Titin-related diseases and conditions can arise in any tissue in which titin is expressed during prenatal or post-natal life. Titin-related diseases and conditions can include heart diseases, such as heart failure. Specific examples of different types of heart failure are provided below.

The invention provides several advantages. For example, using the diagnostic methods of the invention, it is possible to detect an increased likelihood of heart disease, such as heart failure, in a patient, so that appropriate intervention can be instituted before any symptoms occur. This may be useful, for example, with patients in high-risk groups for heart failure (see above). Also, the diagnostic methods of the invention facilitate determination of the etiology of an existing heart condition, such as heart failure, in a patient, so that an appropriate approach to treatment can be selected. In addition, the screening methods of the invention can be used to identify compounds that can be used to treat or to prevent heart conditions, such as heart failure.

Other features and advantages of the invention will be apparent from the following detailed description and the claims.

### Brief Description of the Drawing

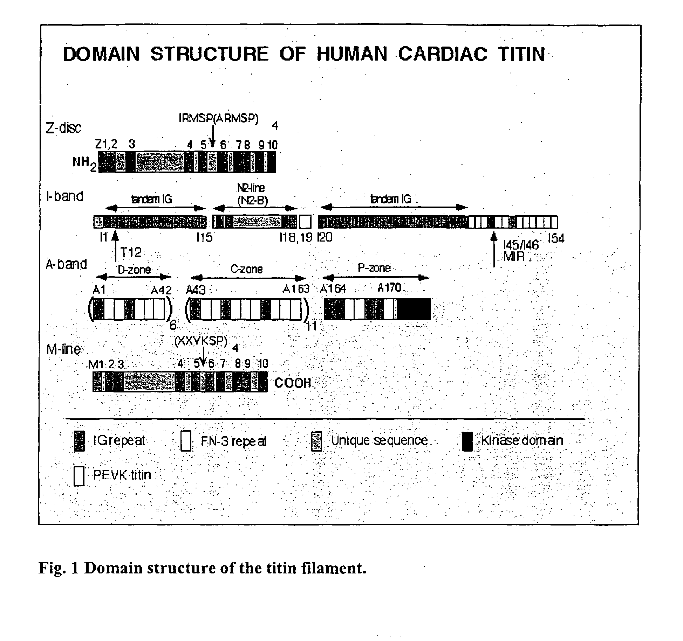

Fig. 1 is a schematic representation of the domain structure of the human titin filament. The nucleotide and amino acid sequences of human titin are provided in SEQ ID NOs:1 and 2, respectively. The modular architecture of cardiac titin as predicted by its full-length cDNA is shown.

A total of 244 copies of 100 residue repeats, as indicated by vertical rectangles, are contained in the molecule. One hundred and twelve of these belong to the Ig domain, and 132 belong to the FN3 superfamily. The titin kinase domain, as well as the PEVK element N2-B 163-residue variant are also shown. Within the A-band , the D-zone contains six tandem repeats of the seven domains shown (A1 through A42), and the C-zone contains 11 tandem repeats of the 11 domains shown (A43 through A163). The positions of the tandemly repeated RMSP and VKSP motifs in the Z-disc and M-line region are also shown

### Detailed Description

The invention provides methods of diagnosing heart disease, screening methods for identifying compounds that can be used to treat or to prevent heart disease, and methods of treating or preventing heart disease using these compounds. The invention also provides animal model systems that can be used in the screening methods of the invention.

In particular, we have discovered that a mutation (the *pickwick* mutation) in the *titin* gene is associated with heart disease, such as heart failure. Titin, which is also known as "connectin," is the largest known single-chain protein, having a molecular weight of about 3,000 kDa. Titin is a structural protein, and plays a central role in the assembly and elasticity of vertebrate skeletal and cardiac muscle. Thus, the diagnostic methods of the invention involve detection of mutations in the *titin* gene, while the compound identification methods of the invention involve screening for compounds that affect the phenotype of *titin* mutants or other models of heart disease, such as heart failure. Compounds identified in this manner can be used in methods to treat or to prevent heart disease (e.g., heart failure). The diagnostic, screening, and therapeutic methods of the invention, as well as the animal model systems of the invention, are described further, as follows.

### Diagnostic Methods

*Titin* nucleic acid molecules, polypeptides, and antibodies can be used in methods to diagnose or to monitor diseases and conditions involving mutations in, or inappropriate expression of, *titin* genes. As discussed further below, the *pickwick* mutation in zebrafish, which is present in the *titin* gene, is characterized by a phenotype that is similar to that of heart failure in humans. Thus, detection of abnormalities in *titin* genes or their expression can be used in methods to diagnose, or to monitor treatment or development of, human heart disease, such as heart failure. For use as references, the human cardiac *titin* cDNA sequence can be found at: http://www.embl-heidelberg.de/ExternalInfo/Titin/cardiacseq.html (SEQ ID NO:1), while the corresponding protein sequence can be found at: http://www.embl-heidelberg.de/ExternalInfo/Titin/cardiacpep.html (SEQ ID NO:2).

As noted above, the diagnostic methods of the invention can be used, for example, with patients that have heart failure, in an effort to determine its etiology and, thus, to facilitate selection of an appropriate course of treatment. The diagnostic methods can also be used with patients that have not yet developed heart failure, but who are at risk of developing such a disease, or with patients that are at an early stage of developing such a disease. Also, the diagnostic methods of the invention can be used in prenatal genetic screening, for example, to identify parents who may be carriers of a recessive *titin* mutation.

Examples of heart failure that can be diagnosed (and treated) using the methods of the invention include congestive heart failure, which is characterized by fluid in the lungs or body, resulting from failure of the heart in acting as a pump; right sided heart failure (right ventricular), which is characterized by failure of the pumping action of the right ventricle, resulting in swelling of the body, especially the legs and abdomen; left sided heart failure (left ventricular), which is caused by failure of the pumping action of the left side of the heart, resulting in congestion of the lungs; forward heart failure, which is characterized by the inability of the heart to pump blood forward at a sufficient rate to meet the oxygen needs of the body at rest or during exercise; backward heart failure, which is characterized by the ability of the heart to meet the needs of the body only if heart filling pressures are abnormally high; low-output, which is characterized by failure to maintain blood output; and high-output, which is characterized by heart failure symptoms, even when cardiac output is high.

Titin may also play a role in cardiovascular diseases other than heart failure, such as coronary artery disease or conditions associated with valve formation defects, and, thus, detection of abnormalities in *titin* genes or their expression can be used in methods to diagnose and monitor these conditions as well. The methods of the invention can be used to diagnose (or to treat) the disorders described herein in any mammal, for example, humans, domestic pets, or livestock.

Titin abnormalities that can be detected using the diagnostic methods of the invention include those characterized by, for example, (i) abnormal titin polypeptides, (ii) *titin* genes containing mutations that result in the production of such polypeptides, and (iii) *titin* mutations that result in production of abnormal amounts of *titin*. Detection of such abnormalities, thus, can be used in methods to diagnose human heart disease, such as heart failure. Exemplary of the *titin* mutations that can be detected using the methods of the invention is the *pickwick* mutation (see below).

Detection of *titin* mutations can be carried out using any diagnostic technique. For example, a biological sample obtained from a patient can be analyzed for one or more mutations in *titin* nucleic acid molecules *(e.g.,* the *pickwick* mutation) using a mismatch detection approach. Generally, this approach involves polymerase chain reaction (PCR) amplification of nucleic acid molecules from a patient sample, followed by identification of a mutation *(i.e.,* a mismatch) by detection of altered hybridization, aberrant electrophoretic gel migration, binding, or cleavage mediated by mismatch binding proteins, or by direct nucleic acid molecule sequencing. Any of these techniques can be used to facilitate detection of mutant *titin* genes, and each is well known in the art. Examples of these techniques are described by Orita *et al.* (Proc. Natl. Acad. Sci. U.S.A. 86:2766-2770, 1989) and Sheffield *et al.* (Proc. Natl. Acad. Sci. U.S.A. 86:232-236, 1989).

Mutation detection assays also provide an opportunity to diagnose a titin-mediated predisposition to heart disease before the onset of symptoms. For example, a patient heterozygous for a *titin* mutation that suppresses normal titin biological activity or expression may show no clinical symptoms of a titin-related disease, and yet possess a higher than normal probability of developing heart disease, such as heart failure. Given such a diagnosis, a patient can take precautions to minimize exposure to adverse environmental factors, and can carefully monitor their medical condition, for example, through frequent physical examinations. As mentioned above, this type of diagnostic approach can also be used to detect *titin* mutations in prenatal screens.

The *titin* diagnostic assays described above can be carried out using any biological sample (for example, a muscle tissue sample) in which titin is normally expressed. Because of the limited number of tissues in which titin is expressed, as well as the relative difficulties involved in obtaining samples of these tissues, it may be preferable to detect mutant *titin* genes in another, more easily obtained sample type, such as blood or amniotic fluid samples using, for example, mismatch detection techniques. Preferably, the DNA in such a sample is subjected to PCR amplification prior to analysis.

Levels of titin expression in a patient sample can be determined by using any of a number of standard techniques that are well known in the art. For example, titin expression in a biological sample *(e.g.*, a blood or tissue sample, or amniotic fluid) from a patient can be monitored by standard northern blot analysis or by quantitative PCR (see, e.g., Ausubel *et al., supra; PCR Technology: Principles and Applications for DNA Amplification,* H.A. Ehrlich, Ed., Stockton Press, NY; Yap *et al.,* Nucl. Acids. Res. 19:4294, 1991).

In yet another diagnostic approach of the invention, an immunoassay is used to detect or to monitor titin protein levels in a biological sample. Titin-specific polyclonal or monoclonal antibodies can be used in any standard immunoassay format *(e.g.,* ELISA, Western blot, or RIA; see, *e.g.,* Ausubel *et al., supra)* to measure titin polypeptide levels. These levels are compared to wild-type titin levels. For example, a decrease in titin production may be indicative of a condition or a predisposition to a condition involving insufficient titin biological activity.

Immunohistochemical techniques can also be utilized for titin detection. For example, a tissue sample can be obtained from a patient, sectioned, and stained for the presence of titin using an anti-titin antibody and any standard detection system *(e.g.,* one that includes a secondary antibody conjugated to horseradish peroxidase). General guidance regarding such techniques can be found in, *e.g.,* Bancroft *et al., Theory and Practice of Histological Techniques,* Churchill Livingstone, 1982, and in Ausubel *et al., supra.*

### Identification of Molecules That Can Be Used to Treat or to Prevent Heart Failure

Identification of a mutation in *titin* as resulting in a phenotype that is related to heart failure facilitates the identification of molecules *(e.g.,* small organic molecules, peptides, or nucleic acid molecules) that can be used to treat or to prevent heart failure. The effects of candidate compounds on heart failure can be investigated using, for example, the zebrafish system. The zebrafish, *Danio rerio,* is a convenient organism to use in genetic analysis of vascular development. In addition to its short generation time and fecundity, it has an accessible and transparent embryo, allowing direct observation of blood vessel function from the earliest stages. As discussed further below, zebrafish and other animals having mutations in the *titin* gene, which can be used in these methods, are also included in the invention.

In one example of the screening methods of the invention, a zebrafish having a mutation in the *titin* gene *(e.g.,* a zebrafish having the *pickwick* mutation; see below) is contacted with a candidate compound, and the effect of the compound on the development of a heart abnormality that is characteristic of heart failure, or on the status of such an existing heart abnormality, is monitored, relative to an untreated, identically mutant control. As discussed further below, zebrafish having the *pickwick* mutation are characterized by, for example, reduction of peak systolic pressures, stretched and thin myocardium, excess cardiac jelly, absent A-V cushions, and an obstructed ventricular outflow tract. Thus, these characteristics (in addition to other characteristics of heart failure) can be monitored using the screening methods of the invention.

After a compound has been shown to have a desired effect in the zebrafish system, it can be tested in other models of heart disease, for example, in mice or other animals having a mutation in the *titin* gene. Alternatively, testing in such animal model systems can be carried out in the absence of zebrafish testing.

Candidate compounds can be purified (or substantially purified) molecules or can be one component of a mixture of compounds *(e.g.*, an extract or supernatant obtained from cells; Ausubel *et al., supra).* In a mixed compound assay, the effect on a phenotype of heart failure is tested against progressively smaller subsets of the candidate compound pool *(e.g.,* produced by standard purification techniques, *e.g.,* HPLC or FPLC) until a single compound or minimal compound mixture is demonstrated to have the desired effect.

Test compounds that can be screened in the methods described above can be chemicals that are naturally occurring or artificially derived. Such compounds can include, for example, polypeptides, synthesized organic molecules, naturally occurring organic molecules, nucleic acid molecules, and components thereof. Candidate compound can be found, for example, in a cell extract, mammalian serum, or growth medium in which mammalian cells have been cultured.

In general, novel drugs for prevention or treatment of mutant titin-related heart diseases can be identified from large libraries of both natural products, synthetic (or semi-synthetic) extracts, and chemical libraries using methods that are well known in the art. Those skilled in the field of drug discovery and development will understand that the precise source of test extracts or compounds is not critical to the screening methods of the invention. Accordingly, virtually any number of chemical extracts or compounds can be screened using these methods. Examples of such extracts or compounds include, but are not limited to, plant-, fungal-, prokaryotic-, or animal-based extracts, fermentation broths, and synthetic compounds, as well as modifications of existing compounds.

Numerous methods are also available for generating random or directed synthesis *(e.g.,* semi-synthesis or total synthesis) of any number of chemical compounds, including, but not limited to, saccharide-, lipid-, peptide-, and nucleic acid molecule-based compounds. Synthetic compound libraries are commercially available from Brandon Associates (Merrimack, NH) and Aldrich Chemical (Milwaukee, WI). Alternatively, libraries of natural compounds in the form of bacterial, fungal, plant, and animal extracts are commercially available from a number of sources, including Biotics (Sussex, UK), Xenova (Slough, UK), Harbor Branch Oceanographic Institute (Ft. Pierce, FL), and PharmaMar, U.S.A. (Cambridge, MA). In addition, natural and synthetically produced libraries can be produced, if desired, according to methods known in the art, *e.g.,* by standard extraction and fractionation. Furthermore, if desired, any library or compound can be readily modified using standard chemical, physical, or biochemical methods.

In addition, those skilled in the art of drug discovery and development readily understand that methods for dereplication *(e.g.,* taxonomic dereplication, biological dereplication, and chemical dereplication, or any combination thereof) or the elimination of replicates or repeats of materials already known for their therapeutic activities for heart failure can be employed whenever possible.

When a crude extract is found to have an effect on the development or persistence of heart failure, further fractionation of the positive lead extract can be carried out to isolate chemical constituents responsible for the observed effect. Thus, the goal of the extraction, fractionation, and purification process is the careful characterization and identification of a chemical entity within the crude extract having a desired activity. The same assays described herein for the detection of activities in mixtures of compounds can be used to purify the active component and to test derivatives of these compounds. Methods of fractionation and purification of such heterogeneous extracts are well known in the art. If desired, compounds shown to be useful agents for treatment can be chemically modified according to methods known in the art.

### Treatment or Prevention of Heart Failure

Compounds identified using the screening methods described above can be used to treat patients that have or are at risk of developing heart disease, such as heart failure. Such treatment may be required only for a short period of time, or may, in some form, be required throughout a patient's lifetime. Any continued need for treatment, however, can be determined using, for example, the diagnostic methods described above. In considering various therapies, it is understood that such therapies are, preferably, targeted to the affected or potentially affected organ, that is, the heart.

Treatment or prevention of diseases resulting from a mutated *titin* gene can be accomplished, for example, by modulating the function of a mutant titin protein, delivering normal titin protein to the appropriate cells, altering the levels of normal or mutant titin protein, replacing a mutant *titin* gene with a normal *titin* gene or, administering a normal *titin* gene. It is also possible to correct a *titin* defect by modifying the physiological pathway *(e.g.,* a signal transduction pathway) in which the titin protein participates.

In a patient diagnosed as heterozygous for a *titin* mutation, or as susceptible to *titin* mutations or aberrant titin expression (even if those mutations or expression patterns do not yet result in alterations in titin expression or biological activity), any of the above-described therapies can be administered before the occurrence of the disease phenotype. In particular, compounds shown to modulate titin expression or to have an effect on the phenotype *of titin* mutants can be administered to patients diagnosed with potential or actual heart disease by any standard dosage and route of administration.

Any appropriate route of administration can be employed to administer a compound found to be effective in treating or preventing heart failure, according to the invention. For example, administration can be parenteral, intravenous, intra-arterial, subcutaneous, intramuscular, intraventricular, intracapsular, intraspinal, intracisternal, intraperitoneal, intranasal, aerosol, or oral. However, as noted above, preferably, the administration is local to the afflicted tissue, that is, the heart. Therapeutic formulations can be in the form of liquid solutions or suspensions; for oral administration, formulations can be in the form of tablets or capsules; and for intranasal formulations, in the form of powders, nasal drops, or aerosols.

A therapeutic compound of the invention can be administered within a pharmaceutically acceptable diluent, carrier, or excipient, in unit dosage form. Administration can begin before or after the patient is symptomatic. Methods that are well known in the art for making formulations are found, for example, in *Remington's Pharmaceutical Sciences,* (18^{th} edition), ed. A. Gennaro, 1990, Mack Publishing Company, Easton, PA. Formulations for parenteral administration can, for example, contain excipients; sterile water; or saline; polyalkylene glycols, such as polyethylene glycol; oils of vegetable origin; or hydrogenated napthalenes. Biocompatible, biodegradable lactide polymer, lactide/glycolide copolymer, or polyoxyethylene-polyoxypropylene copolymers can be used to control the release of the compounds. Other potentially useful parenteral delivery systems for compounds identified using the methods of the invention include ethylene-vinyl acetate copolymer particles, osmotic pumps, implantable infusion systems, and liposomes. Formulations for inhalation can contain excipients, for example, lactose, or can be aqueous solutions containing, for example, polyoxyethylene-9-lauryl ether, glycocholate, and deoxycholate, or can be oily solutions for administration in the form of nasal drops, or as a gel.

*Titin* nucleic acid molecules and polypeptides can also be used in tissue engineering, for example, in the manufacture of artificial or partially artificial hearts. As mentioned above, titin plays a role in cardiovascular elasticity, integrity, and contractility. Thus, a *titin* nucleic acid molecule or polypeptide, as described above, can be used to impart such characteristics on an artificial or partially artificial heart.

### Animal Model Systems

The invention also provides animal model systems for use in carrying out the screening methods described above. Examples of these model systems include zebrafish and other animals, such as mice, that have mutations in a *titin* gene. For example, a zebrafish model that can be used in the invention can include a mutation that results in a lack of titin production or production of a truncated *(e.g.,* by introduction of a stop codon) or otherwise altered titin gene product. The mutation can, for example, result in the presence of a stop codon in a cardiac-specific exon, such as the N2B exon *(e.g.,* in the IS3 region; see below). The mutation can be in a region encoding the I band, resulting in the production of a protein in which the I band is truncated and the A band and M line are absent, or can be in a region encoding another portion of the molecule, such as the A band or M line region. As a specific example, a zebrafish having the *pickwick* mutation can be used.

### Experimental Results

During a large-scale mutagenesis screening of the zebrafish genome, a group of mutations were identified that affect cardiac contractility. One of these mutations, called *pickwick (pik),* dramatically reduces the function of both chambers, causing a recessive, lethal form of heart failure in the zebrafish embryo.

Direct *in vivo* recording of ventricular pressures by the null balance feedback system shows that *pickwick* causes a 5.8 fold reduction of peak systolic pressures, compared to age-matched controls (0.084+/- 0.008 vs. 0.49+/- 0.06 mm Hg). Morphological analysis of *pickwick* revealed a stretched and thin myocardium, excess cardiac jelly, absent A-V cushions, and an obstructed ventricular outflow tract. Extensive ultrastructural defects were found by transmission electron microscopy, affecting the assembly of Z-discs and the organization of myofilaments. Reciprocal blastomere transplants identified *pickwick* as a cell autonomously acting mutation of the myoblast lineage.

A positional cloning approach was adopted for gene identification. The *pickwick* mutation has been assigned to a small chromosomal interval, which is covered by BAC clones. The *titin* gene spans this interval, and thus the *pickwick* phenotype is due to a mutation in the *titin* gene. In particular, the *pickwick* gene was mapped to linkage group (LG) 9 by bulk segregant analysis using the *pikm242* allele, which is one of five cardiac specific alleles *(pikm242, pikm171, pikm740, pikm186, pikmnm2).* A panel of Z-markers in the linkage group was tested for simple sequence length polymorphisms (SSLPs) using 931 homozygous mutant embryos. Markers Z8363 and Z26463 were shown to flank the *pickwick* locus, defining a 1.2 cM interval containing the gene. It is estimated that 1 cM corresponds to 500-600 kb DNA in the zebrafish genome (Postlethwait *et al.,* Science 264:699-703, 1994). We thus initiated chromosomal walking from the Z8363 marker, which is 0.7 cM from the mutation (12 recombinants out of 1750 meioses).

A positive YAC clone (YAC5) was identified that had a T7 end that is highly homologous to human *titin* coding sequences. As the *titin* genomic region was estimated to be over 300 kb in humans, we decided to identify BAC clones based on sequence information of the zebrafish *titin* EST clones. A physical contig was constructed based on these sequences, which covers the whole *titin* genomic area. Single stranded conformational polymorphisms (SSCPs) were developed from the ends and internal sequences of these clones were used for fine recombinational mapping. One SSCP marker inside the *titin* genomic area (B9F2) picked up one recombinant from the Z26463 side. The other four SSCP markers inside the *titin* genomic area (B4SP1, B2T7, B7SP, and B6SP) picked up zero recombinants out of 1750 meioses. These genetic data indicated that the *pickwick* locus is very close to or within the *titin* genomic region.

As the *titin* cDNA alone is around 82 kb, rescuing the phenotype by RNA injection could prove to be quite difficult. However, we found evidence that the *pickwick* locus is within the *titin* gene by identification of point mutations in one of the *pickwick* alleles. As most of the alleles of *pickwick* have a cardiac-specific phenotype, we presumed that the point mutation is located in a cardiac-specific exon. We focused on the N2B domain in the I-band of titin, as all of the cardiac isoforms are N2B domain based. The zebrafish N2B domain was cloned by RT-PCR. It is a 4.3 kb cDNA encoding infrastructures similar to those in humans and mice, and contains 4 IG repeats and three unique sequences, including longer IS3 and shorter IS1.

N2B domains from *pickwick* mutant embryos were then cloned. RNA mismatch analysis was performed to identify the location of the point mutation. One mismatch between the PCR products from *pikm17*1 *and pikm242* was identified. Sequencing of the PCR product resulted in the identification of a T-> G transition in the *pikm171* allele. This mutation resulted in a change of leucine in the IS3 fragment of N2B domain (N2B-IS3) into a stop codon. The mutation was confirmed in all of the seven homozygous *pikm171* mutant embryos, but none of the four *pikm242* homozygous embryos. A truncated version of titin is predicted to be in the *pikm171* mutant, only as a cardiac specific isoform. It should contain the Z-disc and part of the 1-band and be sized around 4,000 amino acids, based on comparison to the homologous human titin sequence, which has a full length of 27,000 amino acids. The identification of a non-sense mutation in the cardiac specific N2B domain in *pikm 171* allele confirmed the hypothesis that *titin* is the *pickwick* gene.

Titin was expressed in the zebrafish embryo during the period when the *pickwick* phenotype was first detected. Whole mount *in situ* hybridization analysis indicated that *titin* was expressed strongly in both the heart and the somites at 24 hpf. *Titin* mRNA expression in the heart is normal in *pikm171.* We confirmed the notion that N2B is a cardiac-specific exon in zebrafish by labeling a probe in the IS3 domain for the whole *mount in situ* hybridization.

The identification of a point mutation in the N2B domain thus establishes *pickwick* as the first *in vivo* vertebrate system to study the functions of titin in the heart. If titin functions as a spring, as proposed, it is expected that the contraction will be much weaker. This is exactly what we observed in pickwick mutant embryos. If titin functions as a template during the sarcomere assembly, a "silent heart" phenotype would be expected in titin null mutation. According to the current model of the myofibrillogenesis (Dabiri *et al.,* Proc. Natl. Acad. Sci. U.S.A. 94:9493-9498, 1997), the thick element and/or the sarcomere could not assemble into a beating machine. In contrast, the hearts in the homozygous *pikm171* mutant embryos and all of the other *pickwick* alleles still beat, despite being weaker.

The mutation in *pikm 171* predicts a truncated protein in which most of the elastic I-band is deleted and the C-terminal A-band and M-line regions eliminated. It thus could be considered as a null mutation in terms of function as a spring and a potential dominant negative mutation in terms of its function as a template for sarcomere assembly (Turnacioglu *et al.,* Mol. Biol. Cell 8:705-717, 1997). The observation of the weak beating in the *pickwick* mutant embryos suggested the existence of primary contractile machinery without titin. Indeed, thick and thin elements can be detected in the ventricular myocardium cells. They have the capacity to assemble into a functional beating structure in the absence of titin.

We thus have carried out a detailed physiological and morphological analysis *of pickwick,* a zebrafish heart function mutation that reduces the contractility of both chambers. Several pieces of evidence pointed out that *titin* is *the pickwick* gene. Genetic analysis linked the *pickwick* locus closely to the *titin* genomic area. The identification of the *pikmVO62H, a pickwick* allele that has an additional somite phenotype, can be explained by the titin hypothesis. The point mutation is expected to be in the common exons that are shared between the cardiac and somatic isoforms of titin. Evidence confirming this hypothesis came from the identification of a point mutation in the cardiac specific N2B domain of titin in one of the *pickwick* alleles, *pikm171.* We went on to show that sarcomere structure is disrupted in the myocardium cells of *pikml 71,* but not the somatic muscle cells. The expression pattern of titin is consistent with this phenotype. Strong expression in both cardiac and skeletal muscles was detected at the onset of the *pickwick* phenotype.

Thus, our observation in zebrafish is in consistent with the notion that titin functions as a spring during the muscle contraction. As titin is a sarcomere structure protein, it is conceivable that myocardium is affected cell-autonomously in *pickwick* mutant embryos. The thin and stretched morphology could be due to the mechanic tension generated from the failure to form higher-order sarcomere structure and the loss of spring. The mechanic tension may also be the reason for the separation between the myocardium cells and endocardium cells, generating the excess cardiac jelly. However, there is a possibility that the differentiation program of the myocardium was affected in the *titin* mutation. The valve formation phenotype in *pickwick* mutant embryos could be a secondary defect. It has been suggested that the process of endothelial invasion during valve formation is under control of a localized myocardial signal. (For a review, see Fishman *et al.,* Development 124:2099-2117, 1997.) The physical distance between myocardium and endocardium and/or the stalked differential program in the myocardium could be the reason that prevents a normal cushion formation.

### Material and Methods

### Zebrafish strains and maintenance

Zebrafish were maintained and staged as described. *pikm242, pilan171, pikn740, pikn186* were generated in a screen on the AB background (Stainier *et al.,* Development 123:285-292, 1996). *pikm V062H* and *pikmnml2* were generated in a screen on the TL background. Mapping strains were constructed by crossing *pikm242* into india strain. *pikm171* embryos used in expression analysis and EM were obtained from pair wise matings of *pikm171*/*TL* heterozygotes.

### In situ hybridization

Whole *mount in situ* hybridization was performed as described (Thisse *et al.,* Development 119:1203-1215, 1993). T5 probe was generated by digestion of the EST clone AI629069 (Research Genetics). The N2B and N2A probe were generated through PCR with a tagged T7 promoter. The primer pairs are:
P238F: 5'-AGGGACACTCAGAGACCATAG (SEQ ID NO:3); and P3785RT: 5'- TAATACGACTCACTATAGGGGTCTGAGGATACTCGCCTTC(SEQ ID NO:4).

### Mapping of pickwick

Linkage was established using DNA from 16 homozygous mutations and 16 heterozygous or wild type pick pikm242/indian embryos in bulk segregation analysis (Michelmore *et al.,* Proc. Natl. Acad. Sci. U.S.A. 88:9828-9832, 1991). Z-markers (simple sequence length polymorphisms, SSLP) were developed in this lab (Knapik *et al.,* Nat. Genet. 18:338-343, 1998; Shimoda *et al.,* Genomics 58:219-232, 1999). Genotyping products were resolved in 6% PAGE gels.

### Chromosomal walking

YAC and BAC clones were screened by PCR as pools of clones (Research Genetics) according to the manufacturer's instruction. YAC ends were cloned by plasmid rescue (Zhong *et al.,* Genomics 48:136-138, 1998). Chimeric ends were determined by the RH panel (Geisler *et al.,* Nat. Genet. 23:86-89, 1999). BAC DNA was extracted using the QIAgene kit and the end sequences were determined by direct sequencing. BLAST-X was performed to search for homologous sequence in Genebank. EST clones were generated by Washington University zebrafish EST project and obtained from Research Genetics. Oligonucleotides derived from the end sequences of YAC and BAC clones were used in standard PCR reactions to determine clone overlap. Primer pair B9F2 was derived from a fragment that was generated by digesting BAC5 with BamHI and then subcloning into the pUC19 vector. This clone can be hit by a primer pair derived form the T7 end of BAC7. Single-strand conformation polymorphisms (SSCP) were tested on 6% MDE acrylamide (FMC Bioproducts) gels at 40°C.

### Cloning of the zebrafish N2B domain

Long RT-PCR was performed to amplify the N2B domain from adult zebrafish heart mRNA extracts. mRNA was extracted from pools of ten zebrafish adult hearts using TRIzole reagent (GibcolBRL), as described. The cDNA was synthesized using SuperScripTMII RNase H-Reverse Transcriptase (GibcoBRL) and then treated with RNase H. The primer is derived from EST3: I19R1:
5'- TTTGAACCACTTGAAGGTCACACCAGG (SEQ ID NO:5).
Long-PCR was performed using Expand 20kbPlus PCR System (Roch) as described. The primer pairs are:
I14F1: 5'- GCTAAGAATGACTATGGAGTTGCCACAAGC (SEQ ID NO:6)
I19R2: 5'- TGAACCACTTGAAGGTCACACCAGGAG (SEQ ID NO:7)

The 4.6 kb product was subcloned using TOPO TA Cloning Kit (Invitrogen) as described. The sequence was determined by primer walking. Forty eight reads were aligned by Phred/Phrad software to get a large contig that contains only one reading frame with around three fold coverage.

To amplify the N2B region from the homozygous mutant zebrafish embryos, three overlapping primer pairs were designed according to the adult N2B sequence. The contamination from the skeletal muscle specific titin isoform was thus eliminated. mRNA was extracted from pools of ten zebrafish day 2 embryos using TRIzole reagent (GibcoBRL) as described. Sequencing results indicated that the embryonic heart titin contain one less IG domain in the area.

### Identification of the point mutation

N2B domains from the homozygous mutant embryos were further amplified by primer pairs that generate six overlapping PCR products sized between 0.6 kb and about 1 kb. RNA mismatch analysis was performed using MutationScreenerTM (Ambion) according to the manufacture's protocol. A mismatch was identified between *pikm171* and *pikm242* using the primer pairs:
P238FT:
5'-TAATACGACTCACTATAGGGAGGGACACTCAGAGACCATAG (SEQ ID NO: 8)
P3785RT: 5'-TAATACGACTCACTATAGGGGTCTGAGGATACTCGCCTTC (SEQ ID NO:9). Sequencing results indicate a T->G non-sense mutation in the cDNA from homozygous *pikm171* embryos.
Genomic sequence in this region was amplified using the primer pair:
P238F: 5'-AGGGACACTCAGAGACCATAG (SEQ ID NO:10)
P341R: 5'- GGCAATGTTACTCTCTGTTGAG (SEQ ID NO:11) and sent out directly for sequencing after purification using the Geneclean Spin Kit (BIO101).

### Electron Microscopy

Forty eight hour embryos were fixed overnight at 4°C in 1.5% glutaraldehyde, 1% paraformaldehyde, 70 mM NaPO₄pH 7.2, 3% Sucrose. They were then washed 3 times for 5 minutes each in 0.1M cacodylate buffer, pH 7.4.

## Claims

1. *An in vitro* method of determining whether a test subject has, or is at risk of developing, a heart failure, said method comprising analyzing a nucleic acid molecule or protein of a sample from the test subject to determine whether the test subject has a mutation in the N2-B exon of a *titin* gene, wherein the mutation results in the production of an abnormal *titin* polypeptide or in the production of an abnormal amount of the *titin* polypeptide, and wherein the presence of said mutation is an indication that said test subject has, or is at risk of developing, a heart failure.

2. The *in vitro* method of claim 1, wherein the abnormal titin polypeptide is a truncated *titin* polypeptide.

3. The *in vitro* method of claim 1, further comprising the step of using nucleic acid molecule primers specific for the *titin* gene for nucleic acid molecule amplification of the *titin* gene by the polymerase chain reaction.

4. *The in vitro* method of claim 1, further comprising the step of sequencing *titin* nucleic acid molecules from said test subject.

5. The *in vitro* method of any of claims 1 to 3, wherein said test subject is a mammal.

6. *The in vitro* method of any of claims 1 to 3, wherein said test subject is a human.

7. A method for identifying a compound that can be used to treat or to prevent heart failure, said method comprising
a) contacting a non-human organism comprising a mutation in the N2-B exon of a *titin* gene resulting in the production of an abnormal titin polypeptide or in the production of abnormal amount of the *titin* polypeptide and having a phenotype characteristic of heart failure with said compound, and
b) determining the effect of said compound on said phenotype, wherein detection of an improvement in said phenotype indicates the identification of a compound that can be used to treat or to prevent heart failure.

8. The method of claim 7, wherein the abnormal *titin* polypeptide is a truncated titin polypeptide.

9. The method of claim 7 or 8, wherein said organism is a zebrafish.

10. A non-human animal comprising a mutation in the N2-B exon of a *titin* gene, wherein said non-human animal is a mouse.

11. The non-human animal of claim 10, wherein the mutation results in the presence of a stop codon in said *titin* gene.

## Patentansprüche

1. In-vitro-Verfahren zum Bestimmen, ob ein Testsubjekt eine Herzinsuffienz besitzt oder gefährdet ist, eine Herzinsuffienz zu entwickeln, wobei das Verfahren das Analysieren eines Nukleinsäuremoleküls oder eines Proteins einer Probe aus dem Testsubjekt umfasst, um zu bestimmen, ob das Testsubjekt eine Mutation in dem N2-B-Exon eines Titin-Gens besitzt, wobei die Mutation in der Herstellung eines abnormalen *Titin*-Polypeptids oder in der Herstellung einer abnormalen Menge des *Titin-*Polypeptids resultiert und wobei die Gegenwart der Mutation ein Zeichen ist, dass das Testsubjekt eine Herzinsuffienz besitzt oder gefährdet ist, eine Herzinsuffienz zu entwickeln.

2. *In-vitro-*Verfahren nach Anspruch 1, wobei das abnormale *Titin*-Polypeptid ein trunkiertes *Titin-*Polypeptid ist.

3. *In-vitro*-Verfahren nach Anspruch 1, zusätzlich umfassend den Schritt des Verwendens von Nukleinsäuremolekülprimem, die für das *Titin-Gen* fiir die Nukleinsäuremolekülamplifikation des *Titin-Gens* durch die Polymerasekettenreaktion spezifisch sind.

4. *In-vitro-*Verfahren nach Anspruch 1, zusätzlich umfassend den Schritt des Sequenzierens der *Titin*-Nukleinsäuremoleküle von dem Testsubjekt.

5. In-vitro-Verfahren nach einem beliebigen der Ansprüche 1 bis 3, wobei das Testsubjekt ein Säuger ist.

6. In-vitro-Verfahren nach einem beliebigen der Ansprüche 1 bis 3, wobei das Testsubjekt ein Mensch ist.

7. Verfahren zum Identifizieren einer Verbindung, die zum Behandeln von oder Vorbeugen vor einer Herzinsuffienz verwendet werden kann, wobei das Verfahren umfasst:
a) In-Kontakt-Bringen eines nichtmenschlichen Organismus', enthaltend eine Mutation in dem N2-B-Exon eines Titin-Gens, die in der Herstellung eines abnormalen *Titin*-Polypeptids oder in der Herstellung einer abnormalen Menge eines *Titin*-Polypeptids resultiert, und besitzend einen Herzinsuffienz-charakteristischen Phänotyp, mit der Verbindung und
b) Bestimmen der Wirkung der Verbindung auf den Phänotyp, wobei der Nachweis einer Verbesserung in dem Phänotyp die Identifizierung einer Verbindung anzeigt, die zur Behandlung von oder Vorbeugung vor einer Herzinsuffienz verwendet werden kann.

8. Verfahren nach Anspruch 7, wobei das abnormale *Titin*-Polypeptid ein trunkiertes *Titin*-Polypeptid ist.

9. Verfahren nach Anspruch 7 oder 8, wobei der Organismus ein Zebrafisch ist.

10. Nichtmenschliches Tier, enthaltend eine Mutation in dem N2-B-Exon eines *Titin-*Gens, wobei das nichtmenschliche Tier eine Maus ist.

11. Nichtmenschliches Tier von Anspruch 10, wobei die Mutation in dem Vorhandensein eines Stoppkodons in dem Titin-Gen resultiert.

## Revendications

1. Procédé in vivo de détermination qu'un sujet testé soit atteint d'une crise cardiaque, ou présente le risque de développer une crise cardiaque, ce procédé comprenant l'analyse d'une protéine ou molécule d'acide nucléique d'un échantillon issu du sujet testé pour déterminer si le sujet testé présente une mutation de l'exon N2-B d'un gène titine, dans lequel la mutation résulte en la production d'un polypeptide titine anormal ou en la production d'une quantité anormale de polypeptide titine et dans lequel la présence de ladite mutation est une indication que le sujet testé est atteint d'une crise cardiaque, ou présente le risque de développer une crise cardiaque.

2. Procédé in vivo selon la revendication 1, dans lequel le polypeptide titine anormal est un polypeptide titine tronqué.

3. Procédé in vivo selon la revendication 1, comprenant en outre l'étape d'utilisation d'amorces de molécules d'acide nucléique spécifiques du gène titine en vue de l'amplification en molécule d'acide nucléique du gène titine par la réaction de polymérisation en chaîne.

4. Procédé in vivo selon la revendication 1, comprenant en outre l'étape de séquençage des molécules d'acide nucléique titine dudit sujet testé.

5. Procédé in vivo selon l'une quelconque des revendications 1 à 3, dans lequel ledit sujet testé est un mammifère

6. Procédé in vivo selon l'une quelconque des revendications 1 à 3, dans lequel ledit sujet testé est humain.

7. Procédé pour identifier un composé susceptible d'être utilisé pour traiter ou prévenir une crise cardiaque, ce procédé comprenant :
a) la mise en contact d'un organisme non humain comprenant une mutation de l'exon N2-B d'un gène titine résultant en la production d'un polypeptide titine anormal ou en la production d'une quantité anormale de polypeptide titine et présentant avec ledit composé un phénotype caractéristique d'une crise cardiaque avec ledit composé, et
b) la détermination de l'effet dudit composé sur ledit phénotype, la détection d'une amélioration dans ledit phénotype constituant l'indication de l'identification d'un composé susceptible d'être utilisé pour traiter ou prévenir la crise cardiaque.

8. Procédé selon la revendication 7, dans lequel le polypeptide titine anormal est un polypeptide titine tronqué.

9. Procédé selon la revendication 7 ou 8, dans lequel ledit organisme est un poisson zèbre.

10. Animal non humain présentant une mutation de l'exon N2-B d'un gène titine, cet animal non humain étant une souris.

11. Animal non humain selon la revendication 10, chez lequel la mutation conduit à la présence d'un codon stop dans ledit gène titine.


### SEQUENCE LISTING

<110> The General Hospital Corporation
<120> Methods for Diagnosing and Treating Heart Disease
<130> 00786/381WO2
<150> US 60/175,787
   <151> 2000-01-12
<160> 11
<170> FastSEQ for Windows Version 4.0
<210> 1
   <211> 81940
   <212> DNA
   <213> Homo sapiens
<400> 1 
attagaggct caccgattca tgtcggagat ggtcagaaaa accaactctc cataggacgt 60
cgtttcagaa gcaaccttgg gcttagtccc acccttttta ggcactcttg agaaatcaga 120
gtgcctagaa agatgacaac tcaagcaccg acgtttacgc agccgttaca aagcgttgtg 180
gtactggagg gtagtaccgc aacctttgag gctcacatta gtggttttcc agttcctgag 240
gtgagctggt ttagggatgg ccaggtgatt tccacttcca ctctgcccgg cgtgcagatc 300
tcctttagcg atggccgcgc taaactgacg atccccgccg tgactaaagc caacagtgga 360
cgatattccc tgaaagccac caatggatct ggacaagcga ctagtactgc tgagcttctc 420
gtgaaagctg agacagcacc acccaacttc gttcaacgac tgcagagcat gaccgtgaga 480
caaggaagcc aagtgagact ccaagtgaga gtgactggaa tccctacacc tgtggtgaag 540
ttctaccggg atggagccga aatccagagc tcccttgatt tccaaatttc acaagaaggc 600
gacctctaca gcttactgat tgcagaagca taccctgagg actcagggac ctattcagta 660
aatgccacca atagcgttgg aagagctact tcgactgctg aattactggt tcaaggtgaa 720
gaagaagtac ctgctaaaaa gacaaagaca attgtttcga ctgctcagat ctcagaatca 780
agacaaaccc gaattgaaaa gaagattgaa gcccactttg atgccagatc aattgcaaca 840
gttgagatgg tcatagatgg tgccgctggg caacagctgc cacataaaac acctcccagg 900
attcctccga agccaaagtc aagatcccca acaccaccgt ctattgctgc caaagcacag 960
ctggctcggc agcagtcccc atcgcccata agacactccc cttccccggt cagacacgtg 1020
cgggcaccga ccccatctcc ggtcaggtcc gtgtctccag cagcaagaat ctccacatcc 1080
cccatcaggt ctgttaggtc tccattgctc atgcgtaaga ctcaggcatc caccgtggcc 1140
acaggtcctg aagtgcctcc cccttggaag caagagggct acgtggcctc ctcatctgag 1200
gctgagatga gagagacaac gctgacaacc tctactcaga tcaggacaga agagagatgg 1260
gaagggagat acggtgtcca ggagcaagtg accatcagtg gtgctgcggg tgctgccgcc 1320
agtgtgtcgg ccagtgctag ctacgcagca gaggctgttg ccactggtgc taaagaggtg 1380
aaacaagatg ctgacaaaag tgcagctgtt gcgactgttg ttgctgccgt tgatatggcc 1440
agagtgagag aaccagtgat cagcgctgta gagcagactg ctcagaggac aaccacgact 1500
gctgtgcaca tccaacctgc tcaagaacag gtaagaaagg aagcggagaa gactgctgta 1560
actaaggtag tagtggccgc cgataaagcc aaggaacaag aattaaaatc aagaaccaaa 1620
gaaataatta ccacaaagca agagcagatg cacgtaactc atgagcagat aagaaaagaa 1680
actgaaaaaa catttgtacc aaaggtagta atttccgcag ctaaagccaa agaacaagaa 1740
actagaattt ctgaagaaat tactaagaaa cagaaacaag taactcaaga agcaataatg 1800
aaggaaacta ggaaaacagt tgtacctaaa gtcatagttg ccacacccaa agtcaaagaa 1860
caagatttag tatcaagagg tagagaaggc attactacca aaagagaaca agtgcaaata 1920
actcaggaga agatgagaaa ggaagccgag aaaactgcct tgtctacaat agcagttgct 1980
actgctaaag ccaaagaaca agaaacaata ctgagaacta gagaaactat ggctactaga 2040
caagaacaaa tccaagttac ccatggaaag gtggacgttg gaaaaaaggc tgaagctgta 2100
gcaacagttg ttgctgcagt agaccaggcc cgagtcagag agcccagaga gcctgggcat 2160
cttgaagaat cctatgctca gcagaccact ttggagtacg gatataagga acgcatttcc 2220
gccgcaaagg tagctgagcc tccccaacgt ccagcctcag aaccccacgt tgtccctaaa 2280
gcagtcaagc ctagagtaat ccaggctcct tctgagactc atatcaaaac tactgatcaa 2340
 
aagggaatgc acatatcatc acagatcaag aaaactacag atctaacaac ggaaagatta 2400
gtccatgtgg ataaacgccc ccgcacagct agccctcact ttactgtttc aaaaatttct 2460
gttcctaaga cagaacatgg atatgaggca tcaatagccg gtagtgctat tgccacatta 2520
caaaaagagt tgtcagccac atcttctgct cagaagatca ccaaatcggt gaaggctcct 2580
actgtgaagc ccagtgagac tagagtaagg gcagagccca cacccttgcc acagttcccc 2640
ttcgctgaca caccagatac ttacaagagt gaagctggcg ttgaggtgaa aaaggaagta 2700
ggggtgagca tcactggcac caccgtccgt gaagagcgct ttgaagtact gcacggacgc 2760
gaagccaagg taacagaaac agcaagagta ccagcacctg ttgaaattcc tgttactcca 2820
ccaactttgg tctcgggctt aaaaaatgtg actgtcatag aaggtgaatc tgtcaccttg 2880
gagtgccaca tctctggata cccatccccg acagtgacat ggtacaggga agactaccaa 2940
atcgaaagtt ccattgactt ccagataacc ttccagagtg gaattgctcg tcttatgatt 3000
cgcgaagcat ttgcggaaga cagcgggcga tttacttgca gtgctgtaaa tgaggctgga 3060
accgtcagca catcctgcta tctggctgtg caggtgtcag aagaatttga aaaggaaacc 3120
acagccgtga ctgagaaatt tactacagaa gagaaacgct ttgttgagtc aagagatgtg 3180
gttatgactg atactagcct cacagaggaa caagcagggc ctggagaacc tgccgcgcct 3240
tactttatta caaaaccagt ggtccagaaa ctggtggaag gtgggagcgt ggtgtttgga 3300
tgccaagttg gcggcaaccc aaagccccat gtatactgga aaaaatctgg tgttcctcta 3360
accactggat acagatacaa agtgagttac aacaaacaaa ccggtgaatg caagctggtg 3420
atttctatga cttttgctga tgatgctgga gaatacacta ttgttgttcg caataagcat 3480
ggagaaactt ctgcatctgc ttccttgctt gaagaagctg attatgagtt actgatgaag 3540
tcccagcaag aaatgcttta tcagacacaa gtgactgcat ttgttcaaga acctgaagtt 3600
ggagaaacag cacctggatt tgtatactct gagtatgaaa aagagtatga aaaagaacaa 3660
gccttaatta ggaagaaaat ggccaaagat actgtagtgg tcagaactta tgtagaagat 3720
caggaattcc atatttcttc ctttgaagag agacttatta aagaaattga atatagaata 3780
ataaagacta cattagaaga acttcttgaa gaagatggag aagaaaagat ggcagttgac 3840
atttctgaat ctgaagctgt tgaatcagga tttgatttaa gaatcaagaa ttatagaatt 3900
cttgagggga tgggtgtcac ttttcattgc aagatgtctg gatatccatt accaaagatt 3960
gcttggtaca aagatggcaa gcgcatcaaa catggagaaa gataccaaat ggactttcta 4020
caagatggca gagctagtct gcgtatacct gttgttcttc cagaagatga aggaatctac 4080
actgcatttg ccagcaatat taaaggaaat gcaatttgct caggaaaatt gtatgtggag 4140
cctgctgcac cacttggagc tccgacttac attcccacac tagagccagt gagcagaatc 4200
agatctctct ctccacgttc agtgagcagg tctcctatac gcatgtctcc tgcacggatg 4260
tcacctgcaa ggatgtctcc tgcacggatg tcccctgcaa gaatgtcccc tggacgtagg 4320
ctggaggaga cagatgagtc acaacttgag agactatata aaccagtctt tgtgttaaaa 4380
cctgtttctt tcaaatgttt agaagggcaa actgccagat ttgacttaaa ggttgttggt 4440
agacctatgc cagagacgtt ctggtttcat gatggccagc aaattgtcaa tgactatacc 4500
cataaagtag tcattaaaga agatggtact caatcactaa ttattgtccc tgccacaccc 4560
agtgattctg gggaatggac tgtggttgcc caaaacaggg caggcagatc ttcaatttca 4620
gtgattttaa ctgtggaagc tgtggaacat caggtaaaac cgatgtttgt agaaaaactg 4680
aaaaatgtca atataaagga aggttcccga cttgaaatga aagtcagagc tacgggtaac 4740
cccaaccctg acattgtatg gttgaaaaac agtgacatca ttgtgcctca taaatatccc 4800
aaaatcagaa ttgaaggaac caagggagaa gctgccctta aaatcgattc cactgtcagc 4860
caagattctg cctggtatac tgcgactgct attaataaag ctggcagaga cactacaaga 4920
tgcaaagtaa atgttgaagt tgagtttgca gagcctgagc cagagagaaa gttaatcatc 4980
ccacggggga catatagagc aaaggagatt gcagccccag aactggagcc cctccatttg 5040
cgatatggcc aagagcaatg ggaagaaggt gatctctatg acaaagagaa acaacagaaa 5100
ccatttttca agaaaaaact cacttcctta agacttaagc gctttgggcc tgcccacttt 5160
gaatgcaggc taacacccat tagtgaccca acgatggtgg tggagtggct ccatgatgga 5220
aagccacttg aagcagccaa caggctccgt atgatcaatg aatttgggta ctgcagcctt 5280
gattatggcg ttgcatattc tagagacagt ggtatcatta cttgcagagc cactaacaaa 5340
tatggaacag atcacacatc tgctaccctt attgttaaag atgagaaaag tcttgtggaa 5400
gaatcccaat tgcctgaggg gaggaaaggc ttacagagaa ttgaagaatt agagagaatg 5460
gctcatgaag gtgcacttac aggtgtaaca acagatcaga aagaaaagca aaagccagac 5520
attgtcttgt acccagagcc agttagagta cttgaagggg agactgcaag gttccgctgc 5580
agggtaacag gctaccctca gcccaaagtc aactggtacc tcaatggaca gctcatccgc 5640
aaaagcaaaa ggttcagagt tcgctatgat ggtatccatt acctggacat cgtggactgc 5700
aaatcatatg acacaggtga agtgaaggtc accgcggaaa atcctgaagg tgtgatagag 5760
cataaagtga agcttgagat tcaacagagg gaagatttta ggtctgtcct taggagagct 5820
cctgaaccaa ggcctgagtt tcacgtacat gaaccaggaa agcttcagtt tgaagtacaa 5880
aaagtggata gacctgttga caccactgaa accaaagaag ttgtgaagtt gaaaagggct 5940
gaaagaatta cccatgaaaa agtgcctgaa gagtcggaag agctgcgcag taaattcaag 6000
cgcagaacag aagagggcta ttatgaagcc attaccgctg tggagctcaa gtctcgaaag 6060
 
aaggatgaat cctatgagga actcctcagg aagacaaaag atgaacttct ccactggacc 6120
aaagagttaa ctgaagagga aaagaaagct cttgccgaag aaggcaaaat cacgattcca 6180
acttttaaac ctgacaagat tgaactaagt cctagtatgg aggctccaaa aatcttcgaa 6240
agaatccaga gccaaacagt gggccaagga tctgatgcac acttccgggt cagagtcgtg 6300
gggaaaccag accccgaatg tgaatggtac aaaaatggtg tcaaaattga acggtctgac 6360
cggatctact ggtactggcc cgaagacaat gtttgtgaat tggtcataag agatgtgact 6420
gctgaggact ctgccagcat catggtaaaa gccatcaaca tagctggaga aacctccagt 6480
cacgcattct tacttgtcca agccaagcaa ttgatcactt tcacacagga attacaagat 6540
gttgttgcta aggaaaaaga cactatggca acctttgaat gtgaaacttc agaaccattt 6600
gtcaaagtga aatggtataa agatggtatg gaggttcatg agggagataa atacaggatg 6660
cactctgaca gaaaggttca cttcctctcc atactgacca ttgatacgtc tgatgctgaa 6720
gattacagct gtgtacttgt ggaagatgaa aatgtcaaaa cgactgctaa acttattgtt 6780
gaaggtgcag ttgttgagtt tgtgaaagaa cttcaggaca tagaagttcc agaatcatat 6840
tcaggagaat tagagtgcat tgtatcccca gaaaatatag aaggaaaatg gtatcataat 6900
gatgtggagc ttaaatccaa tggcaaatat acaattacat ctcgtcgtgg acgtcagaac 6960
ctcacggtca aggatgtaac caaggaggac cagggagaat acagctttgt catcgacggg 7020
aaaaagacaa cctgtaaatt aaagatgaaa ccccgcccca ttgctatcct acaaggactt 7080
agtgaccaaa aagtctgtga gggtgacatt gttcagcttg aagttaaagt ctccttggaa 7140
agtgtggaag gcgtctggat gaaagacggc caagaagtgc agcccagtga cagggttcac 7200
attgtgatag acaaacaatc tcatatgctg ctcattgaag acatgactaa ggaagatgct 7260
ggaaattact ctttcaccat tccagccctt ggcctctcca ccagtgggcg tgtctctgtc 7320
tatagtgtgg acgtgataac acctctaaaa gatgttaatg tgattgaagg caccaaggct 7380
gtgcttgaat gtaaggtgtc agtccctgat gtgacttctg ttaagtggta cttaaatgat 7440
gaacaaatca agcctgatga ccgtgtacag gccattgtga aaggtactaa acagcgacta 7500
gtcattaacc gaactcatgc ttcagacgaa ggaccttata agctgatagt tggcagagtt 7560
gaaaccaact gtaatctctc tgtagaaaaa attaaaatta tcagaggtct tcgtgacctt 7620
acctgtacag aaactcaaaa tgtggtgttt gaggttgagc tgtcccactc tggaattgat 7680
gtcctgtgga attttaagga caaggaaatc aagcccagtt ctaaatataa aattgaagca 7740
catggaaaaa tatataaatt gacagttcta aatatgatga aagatgatga aggaaaatac 7800
acattttacg cgggagaaaa tatgacatct ggaaaactta ctgtggcagg tggggccatc 7860
tccaagccac tcacagatca gaccgtagct gaatcccagg aagctgtgtt tgaatgtgaa 7920
gttgccaacc cagattccaa aggcgaatgg ttgagggatg gcaaacacct accactgact 7980
aacaacatca gaagtgagtc tgatggccac aaaaggagac ttatcattgc tgccaccaaa 8040
ttagatgaca ttggagaata tacctacaag gtggccacct ccaaaacatc tgccaaactc 8100
aaagttgaag ctgtcaaaat taagaagact ctgaagaacc tcacagtgac agaaacacag 8160
gatgctgttt tcactgtcga gcttacacac cctaatgtca aaggtgtcca gtggatcaaa 8220
aatggagttg tgctggaatc caatgaaaag tatgctatct ctgtcaaagg aacaatttac 8280
tctctgagga ttaaaaactg tgccatcgtg gatgagtctg tttatggctt caggcttgga 8340
aggcttggag ccagtgccag actgcacgtg gagactgtca agatcattaa aaagccaaag 8400
gatgtgacag ccttggaaaa tgccactgtt gcctttgaag ttagtgtttc ccatgacact 8460
gttccagtaa aatggttcca taagagtgtg gaaattaagc caagtgacaa acacagactg 8520
gtctcagaaa ggaaagtcca caagctgatg ctgcagaaca tctccccctc agatgctggg 8580
gaatacacag ctgtggtcgg gcaattggaa tgcaaagcaa aactgtttgt ggagacatta 8640
catattacaa aaaccatgaa aaatatcgag gtgcctgaga ccaaaactgc ctcttttgag 8700
tgtgaggtgt cccacttcaa tgtcccttcc atgtggctga agaatggtgt ggaaattgag 8760
atgagtgaaa agttcaagat agttgtgcag ggaaaactcc atcagctgat catcatgaac 8820
accagcacag aggactcggc agaatacaca tttgtctgtg gcaatgacca agtcagtgcc 8880
accctgacag tcactccaat catgattact tccatgctga aagacatcaa cgctgaagaa 8940
aaagacacta ttacttttga ggtgacagtg aactatgaag gcatctctta caaatggtta 9000
aagaatggtg tggaaatcaa atcaactgac aagtgccaga tgagaaccaa aaagctcaca 9060
cactcactga acatcaggaa tgttcacttt ggggatgctg ctgactacac ctttgtggct 9120
ggaaaagcaa catcaacagc cacactttat gtggaagctc gtcatataga atttaggaaa 9180
cacattaagg acattaaggt actggagaag aagcgagcca tgtttgaatg tgaagtttct 9240
gaacctgaca tcactgtaca gtggatgaaa gatgaccagg aactgcagat cacagacaga 9300
ataaagattc agaaggagaa atatgtccac cgccttctga tcccatccac ccggatgtct 9360
gatgctggga agtacacagt ggtggcagga ggcaacgtgt caactgcaaa actctttgta 9420
gaaggcagag atgttcgcat ccgaagtatt aaaaaggagg ttcaggtcat tgagaaacag 9480
cgtgctgttg ttgaatttga ggtcaatgaa gacgatgttg atgcccactg gtataaagat 9540
ggcattgaaa tcaatttcca agttcaagaa cgacacaaat atgtagtgga aagaagaatc 9600
caccgaatgt ttatctctga gaccagacag agcgatgcag gagaatacac ctttgtggca 9660
ggaaggaaca ggagttctgt cactctctat gtcaatgctc ctgaaccgcc ccaagttctg 9720
caggagctcc agcctgtcac tgtgcagtct ggcaagcctg cccgcttctg tgccatgata 9780
 
tccggaagac cacagcccaa aatttcctgg tacaaggaag agcagctgct ttccactggc 9840
ttcaagtgca aatttcttca tgatgggcaa gagtacacgc ttttgctaat tgaagccttc 9900
ccagaggatg cggcagtcta tacctgtgaa gccaagaatg actatggtgt tgccacaaca 9960
tcagcttcac tctcagtgga agttccagaa gttgtgtctc ctgatcagga aatgcctgtt 10020
tatccacctg ccatcatcac cccgcttcag gacactgtca cttctgaagg gcagccagcc 10080
cgttttcaat gccgggtttc tggaacagat ctaaaagtgt cgtggtacag caaagacaag 10140
aaaatcaagc catctcggtt ctttagaatg actcaatttg aagacactta tcaactggaa 10200
attgccgaag cttatccaga agatgaagga acttacacgt ttgttgctaa taatgctgta 10260
ggccaagtat caagcacagc caacctgagt ctggaagctc ctgaatcaat tttgcatgag 10320
aggattgaac aagagattga gatggaaatg aaagagtttt ctagttcttt tctgtctgcc 10380
gaggaagaag gacttcatag cgccgaactt caattatcta aaataaatga aacacttgaa 10440
cttttgtctg aatctccagt ttacccaact aaatttgatt ccgaaaagga aggcactggc 10500
ccaattttca tcaaagaagt gtcaaatgct gatataagca tgggggatgt ggctacactg 10560
tctgtaactg tcattggcat ccccaaacct aaaattcagt ggttctttaa tggagtgcta 10620
ttaacccctt ctgctgacta caaatttgtt tttgacggtg atgatcatag cctgatcatt 10680
ctgttcacca aattggagga tgagggagag tatacatgta tggccagtaa tgactatgga 10740
aagacaatat gtagtgccta tctaaaaatt aattccaaag gagagggtca caaagacact 10800
gaaacagaat cagcagtggc aaaatctctg gaaaagctgg gaggtccttg tcctcctcac 10860
ttccttaagg agttaaaacc aattcgctgt gctcaagggc ttcctgccat ctttgagtac 10920
acagtggttg gagagcctgc ccctactgtt acatggttca aagaaaacaa gcagctttgc 10980
accagtgttt attacactat cattcataac cctaatggct ctggaacttt cattgtcaat 11040
gaccctcaga gggaagacag tggcctctat atctgtaaag cagagaatat gttgggtgag 11100
tccacctgtg cagcagagct gcttgtgctt ctggaagaca cagacatgac tgataccccc 11160
tgcaaagcaa agtccacacc agaggctcct gaggattttc cacagacacc cttaaagggt 11220
cccgcagttg aagcacttga ctcggagcag gaaattgcaa cgtttgtaaa agacaccatt 11280
ttgaaagctg ctttaattac agaagaaaac cagcaactat cttatgagca tattgctaaa 11340
gccaatgaat tgagcagtca gcttcctttg ggagctcagg aattgcaatc cattttggag 11400
caagacaagc tcactcctga aagcaccagg gaatttcttt gcatcaatgg cagtattcac 11460
tttcagcctc tcaaggaacc atctcccaac ctacagctgc agattgtaca gtcccagaaa 11520
accttctcca aagaaggtat tctaatgcct gaagagcctg agacacaggc agttctatca 11580
gataccgaga aaatcttccc aagtgccatg tccatagaac aaattaattc attaacagtt 11640
gagcctctga aaactttatt agctgaacct gaagggaatt atccacagtc ttcaatagaa 11700
cctccaatgc attcttatct aacctctgtg gctgaggaag tactttcact aaaagaaaag 11760
acagtatctg acaccaacag agagcaaaga gtgactcttc aaaagcaaga ggcacaaagt 11820
gcgctcatct tgagtcagag cttagctgag ggacacgtgg agagtctcca gagtcctgat 11880
gtcatgatct ctcaggtaaa ctatgagccc ctagtccctt cagaacactc atgcacagaa 11940
ggaggtaaaa ttttgataga aagtgcaaat ccactggaaa atgcagggca agattctgcg 12000
gtcagaattg aggaaggcaa gtccttaaga tttccactag cacttgaaga aaagcaggta 12060
ctgctcaaag aagagcattc tgacaacgtg gtgatgcccc cagaccaaat cattgagtct 12120
aaaagagagc ccgtggcaat aaagaaagtg caggaggtac agggaaggga ccttctttct 12180
aaggaaagct tgctttctgg tattccagaa gagcagagat taaacctgaa aattcaaatc 12240
tgccgggctt tgcaagcagc cgtggccagc gagcagccag gtcttttctc tgagtggcta 12300
agaaatattg aaaaggtgga ggtcgaggct gtaaacatca cccaagagcc cagacacatc 12360
atgtgcatgt accttgttac ttcggcaaag tctgtaacag aagaagtaac catcattatt 12420
gaagatgttg atcctcaaat ggctaacctg aaaatggaac ttagggatgc tttgtgtgct 12480
attatatatg aggaaataga catcctaaca gctgagggtc ctagaattca gcaaggagcc 12540
aaaacaagtt tgcaagaaga aatggattct ttttcaggtt cacagaaggt tgaacccatt 12600
actgaaccag aagttgaatc taaatatctg atctcaactg aagaggtcag ttattttaac 12660
gtgcaaagta gggttaaata tttggatgcc acacctgtca ctaaaggggt tgcttcagct 12720
gttgtctctg acgaaaaaca agatgagagt ctgaaaccat cagaggaaaa agaggagtct 12780
tcctctgaaa gtggtactga ggaggttgct acagtaaaga tacaggaagc tgagggtggc 12840
ttaatcaaag aggatggccc catgatacat acacctttag tggacactgt ttctgaggaa 12900
ggtgatattg tacacctcac aacatccata acaaatgcta aagaggtgaa ttggtatttt 12960
gagaataaac tggtgccttc agatgaaaag ttcaagtgtt tacaagatca aaatacatat 13020
acgctagtca tcgacaaagt aaataccgaa gaccatcaag gagagtatgt ctgtgaggcc 13080
ttgaatgaca gcggaaaaac agcaacttca gccaaactca ctgtagtaaa aagagctgcc 13140
ccagtgatca agaggaaaat cgaacccctg gaagtagcac tgggccacct agccaaattc 13200
acctgtgaga tccaaagtgc tcccaatgtc cggttccagt ggtttaaagc tggccgagaa 13260
atttatgaga gtgacaagtg ttctattcga tcttcaaagt atatctccag ccttgaaatc 13320
ctgagaaccc aggtggttga ctgcggcgag tatacatgca aagcttccaa tgagtatggc 13380
agtgtcagct gtacagccac actaactgtg acagtgcctg gaggtgaaaa gaaagttcgc 13440
aaattacttc cggaacgtaa acctgaacca aaggaagaag ttgttctgaa aagcgttcta 13500
 
agaaaaagac ctgaagaaga agaacctaaa gtagaaccta aaaaactaga aaaagttaaa 13560
aaacctgcag taccagaacc accacctcca aaacctgttg aagaggttga agtacctact 13620
gttacaaaaa gggaaaggaa gattcctgaa ccaacaaaag tgcctgaaat caagccagca 13680
atacctctcc ctgcacctga accgaaacca aagcccgaag cagaagtgaa aacaatcaaa 13740
ccacctcctg tggaacctga accaaccccc atcgctgccc cagtaacagt gccagtggtt 13800
ggaaagaaag cagaagccaa agcacctaag gaagaggctg ccaagccaaa aggtcctatc 13860
aaaggtgtac ccaaaaagac tccttcacca atagaagccg aaaggagaaa gttaaggcca 13920
ggaagtggtg gagagaaacc tcctgatgaa gccccgttca cctaccagct aaaggctgtg 13980
ccactgaagt ttgtgaaaga aatcaaagac atcatcttga cagaatcaga gttcgttggc 14040
tcttcagcaa tctttgaatg tttggtctcc ccttccactg caattacaac ctggatgaaa 14100
gacggtagca atatccgtga gagtcccaag cacaggttta ttgcagatgg taaagacaga 14160
aagctgcaca tcattgatgt tcaactttcc gatgctggtg aatacacctg tgttttacgt 14220
ttgggaaaca aagaaaagac ctccacggct aaacttgttg tagaagaact tcctgtgcgt 14280
tttgtaaaaa cactggaaga ggaagtcaca gtggtcaaag gacagccatt gtacttgagc 14340
tgcgagttaa acaaagagcg tgacgtggtc tggaggaagg atggcaagat tgtggtggag 14400
aaacctggcc gaattgtgcc aggcgtcatt ggcttgatgc gggctctgac catcaacgat 14460
gcagatgaca cagatgctgg aacatacaca gttactgtgg aaaacgccaa caacctggag 14520
tgttcatctt gcgtaaaagt agtagaagtc attagagatt ggctggtgaa acctatacga 14580
gaccagcatg tgaaacccaa ggggacagct atttttgcct gtgatatagc aaaagatact 14640
ccaaacatta agtggttcaa aggatatgat gaaatccctg cggaaccaaa tgataagact 14700
gaaatactga gagatggaaa tcatctgtac ctcaaaatta agaatgctat gccagaagat 14760
attgctgagt atgcagtgga aattgaagga aaaagatacc ctgcaaagct gacacttgga 14820
gagcgtgaag ttgaactgct taaaccaata gaggacgtta ccatttatga gaaagaaagt 14880
gcaagctttg atgcagaaat ctcagaggca gacattcctg gacaatggaa actgaaagga 14940
gaacttctaa ggccctcacc tacttgtgaa atcaaagcag aaggtggaaa acgcttctta 15000
actttgcaca aagtcaaact ggaccaagct ggtgaagtcc tctaccaggc ccttaatgca 15060
attacaactg ccattttgac agtaaaagaa atcgaacttg actttgctgt gcccctgaag 15120
gatgtcactg ttccagaaag gcgacaggct cgattcgaat gtgtcctcac ccgagaggca 15180
aatgttatat ggtccaaagg acctgatata attaagtcat ctgacaaatt tgatatcatc 15240
gctgatggaa agaaacatat tcttgttatt aatgattctc aatttgatga tgaaggggtc 15300
tatactgctg aggtggaggg caagaagacc tcagctcggt tgtttgtcac aggtataaga 15360
ctgaaattca tgtcacctct tgaagatcaa acagtaaaag aaggtgaaac agcaactttt 15420
gtttgtgaac tttctcatga aaaaatgcat gtagtctggt tcaaaaatga tgccaaactc 15480
catacaagca gaacagtact catctcttct gagggcaaga ctcacaaatt ggaaatgaaa 15540
gaagtgacat tggatgatat atctcagata aaagctcaag tcaaggagct gagctccaca 15600
gcacagctga aggtcttaga ggccgatccc tacttcactg tgaaattaca tgacaaaact 15660
gcagtggaga aggatgagat tactttgaag tgtgaagtga gcaaagatgt accagtgaaa 15720
tggttcaaag atggtgaaga gattgtccct tcacccaaat attctatcaa ggcagatggc 15780
ctgcgccgca tcttaaaaat caaaaaggcg gaccttaaag ataaaggcga atatgtgtgt 15840
gactgtggca cagacaagac caaggcaaat gttactgttg aggctcgact aatagaagtg 15900
gaaaagcctc tgtacggagt agaggtgttt gttggtgaaa cagcccactt tgaaattgaa 15960
ctttctgaac ctgatgttca cggccagtgg aagctgaaag gacagccttt gacagcttcc 16020
cctgactgtg aaatcattga ggatggaaag aagcatattc tgatccttca taactgtcag 16080
ctgggtatga caggagaggt ttccttccag gctgctaatg ccaaatctgc agccaatctg 16140
aaagtgaaag aattgcctct tatcttcatc acacctctca gtgatgttaa agtcttcgag 16200
aaagatgagg ctaagtttga gtgtgaagta tccagggagc ccaaaacatt ccgttggcta 16260
aaaggaaccc aggaaatcac aggtgatgac agatttgagc ttataaagga tggcactaag 16320
cattcaatgg tgatcaagtc agctgctttt gaagatgaag caaaatacat gtttgaagct 16380
gaagataagc acacaagtgg caaactgatc attgaaggaa tccggctcaa attcctcacc 16440
cctctcaaag atgtaactgc caaagagaag gaaagtgctg tatttactgt ggagttatct 16500
catgataaca tccgagttaa atggttcaag aatgaccagc gcctacacac caccaggtcg 16560
gtctcaatgc aagacgaagg gaaaactcat tcgatcacat tcaaagacct gtctattgat 16620
gacacctccc aaattagagt agaagctatg gggatgagtt cagaagctaa actcactgtg 16680
cttgagggag acccatattt tacaggaaaa cttcaagatt atactggtgt agagaaagat 16740
gaagttattc tacagtgtga aattagcaaa gcagatgcac cagtgaaatg gtttaaggat 16800
gggaaggaaa taaagccatc caaaaatgct gttattaaga cagatggcaa gaaacgcatg 16860
ctaatcctaa agaaagcctt gaaatcagat attggacagt acacctgtga ctgtgggaca 16920
gataagacct caggaaaact tgacattgag gatcgggaaa ttaaactggt gcgacccctg 16980
cacagtgtgg aggtgatgga gactgagaca gcacgctttg aaaccgaaat ctctgaagat 17040
gatatccacg ccaactggaa actcaaggga gaggccctac tccaaacacc tgattgtgaa 17100
attaaggaag aaggcaaaat acactccctt gttttgcaca actgtcgcct ggaccagacg 17160
ggtggggtgg atttccaagc tgccaatgtt aaatctagtg cccacctccg agttaagcca 17220
 
cgagtaattg gtcttctgag gcctttaaag gatgtcaccg tgactgcagg ggaaacagcc 17280
accttcgact gcgagctctc ctacgaagat atcccagtgg aatggtatct caaagggaag 17340
aaactagagc ccagcgataa ggtggtccca cgttcagaag gaaaagttca tacacttact 17400
ctgagggatg taaagttaga agatgctggg gaagtccaac taacagcaaa agatttcaaa 17460
actcacgcca acctctttgt gaaagaaccc ccagttgaat tcactaagcc tcttgaggac 17520
cagacggtcg aagagggagc cactgcagtg ctggagtgtg aagtctccag agaaaatgct 17580
aaggtgaaat ggttcaaaaa tgggacagaa atcctcaaaa gcaagaagta tgaaattgtt 17640
gctgatggca gggtcagaaa acttgttata catgactgta ccccagagga tattaaaaca 17700
tacacttgtg atgctaagga ttttaagact tcctgtaacc tgaatgtcgt gcctcctcat 17760
gtggaattct taagaccact caccgacctt caagttagag aaaaagaaat ggctcgattt 17820
gagtgtgaac tttcccgaga aaatgctaag gttaagtggt ttaaagatgg tgctgaaatt 17880
aaaaagggca aaaaatatga catcatatcc aagggagcag tgcgcattct tgtcatcaac 17940
aaatgtctac tggatgatga agctgaatat tcctgtgaag taaggacagc gagaacttct 18000
ggcatgctga cagttctgga agaagaagct gtctttacca aaaatcttgc caacattgaa 18060
gttagtgaaa cagacactat aaaactggtt tgtgaagtct ccaaacctgg cgcagaagtg 18120
atttggtata aaggggatga ggagatcatt gaaacaggaa gatatgaaat actgactgaa 18180
ggacggaaga gaatcctggt cattcagaac gctcaccttg aggatgctgg caactacaac 18240
tgtcgactcc caagctctcg aaccgatggc aaagtcaaag tacatgaact ggctgctgaa 18300
tttatctcaa agcctcaaaa ccttgaaata cttgaaggag aaaaggctga atttgtctgc 18360
tctatatcaa aagaaagctt tccagtccag tggaagaggg atgataagac acttgaatct 18420
ggagataaat atgacgttat tgctgatggt aaaaagaggg tcctagttgt gaaagatgcc 18480
acattacaag atatgggcac ttacgttgtc atggtagggg ccgccagagc agcagctcac 18540
ttgacagtca ttgaaaaact caggatcgta gttcctctta aggacacccg ggtgaaggaa 18600
caacaggaag ttgtcttcaa ctgtgaagtc aatactgaag gtgccaaagc caaatggttc 18660
agaaatgaag aagctatatt tgatagttca aaatacatca ttctccaaaa agacctagtc 18720
tacaccctca gaattagaga tgcacactta gatgaccaag ccaactataa tgtgtctttg 18780
accaatcaca gaggtgaaaa tgttaaaagt gcagccaatc taatagtaga agaggaagac 18840
cttaggattg ttgagcctct taaagatatt gaaacaatgg agaagaaatc tgtcacattc 18900
tggtgcaagg tgaatcgtct caatgtaaca ctgaagtgga ccaaaaatgg tgaagaagtg 18960
ccttttgaca accgtgtctc atacagagtt gataagtaca agcacatgtt aaccattaaa 19020
gactgtggct tcccagatga aggtgaatac attgtcactg ctggacaaga taaatctgtt 19080
gctgagcttc tcatcataga agccccgaca gaatttgtgg aacacttgga agatcagaca 19140
gtcactgagt tcgatgacgc tgtcttctcc tgccagctct ccagagagaa agccaatgta 19200
aaatggtaca gaaatgggag agaaatcaaa gaaggcaaaa aatacaaatt tgaaaaagat 19260
ggaagtatac acagactcat tataaaagat tgcaggctgg atgatgagtg tgaatatgct 19320
tgcggggtag aagacaggaa gtctcgtgct agactttttg tggaagaaat tcctgttgag 19380
atcatcaggc ctccacaaga tattcttgaa gcccctggtg ctgatgttgt ctttttagca 19440
gaactcaata aagataaggt ggaagtccaa tggctaagaa ataacatggt tgttgtccag 19500
ggtgataaac accagatgat gagtgaagga aagatacatc gactacagat ttgtgatatt 19560
aagccccgtg accagggtga atacagattt attgccaaag acaaagaagc cagagctaag 19620
cttgaactgg cagctgcacc aaaaatcaag acagctgacc aagaccttgt ggttgatgtt 19680
ggcaagcctc tgacaatggt ggtgccatat gatgcctacc ccaaagcaga agctgaatgg 19740
tttaaagaaa atgaaccttt atctacaaaa accattgata ctacggctga acaaacttct 19800
ttcagaattt tagaagccaa gaaaggagac aaagggaggt ataaaattgt gcttcagaac 19860
aaacatggaa aagcagaagg attcatcaat ttaaaagtta ttgatgttcc tgggccagta 19920
cgtaacttag aagtgacaga aacatttgat ggtgaagtga gccttgcttg ggaagaacct 19980
ttaactgatg gtggaagcaa aatcataggt tacgttgttg aaagacgtga cattaagaga 20040
aagacctggg ttctggccac agaccgtgca gagagttgtg agtttactgt cactggtcta 20100
cagaaaggag gagttgagta cctattccgt gtgagtgcaa gaaacagagt tggcactggt 20160
gagccagtag aaactgacaa tcctgtagaa gcaaggagta aatatgatgt tccaggccct 20220
cctttgaatg taaccatcac tgatgtgaat cgatttggtg tctcactgac atgggaacca 20280
ccagagtatg atggaggtgc tgagatcaca aactacgtca ttgaattaag agacaagact 20340
tctatcaggt gggatactgc catgactgtg agagctgaag acctgtctgc aactgttact 20400
gatgtggtag aaggacagga gtacagtttc cgagtgagag cccaaaatcg aattggagtt 20460
ggaaaaccaa gtgcagccac acccttcgtc aaagttgctg atccaattga gagaccaagt 20520
cctcctgtaa acctaacttc ctcagatcag actcagtcat cagttcagct caaatgggaa 20580
cctcctctga aagatggagg aagcccaata ttaggctata taattgagcg atgcgaagaa 20640
ggaaaagata attggattcg ttgcaatatg aaacttgtcc ctgaactgac ttacaaggtt 20700
accggattgg aaaaaggaaa taaatattta tatagagtat ctgcagaaaa taaagctggt 20760
gtttcagatc catctgaaat tcttggtcct ctcaccgctg acgatgcatt tgttgaacca 20820
acaatggatt taagtgcatt taaagatggt ctggaagtta ttgtcccaaa tcctatcacg 20880
atcctggttc caagtacagg ctatccaagg ccaactgcaa cctggtgttt tggagataaa 20940
 
gtactagaaa caggggaccg ggtgaaaatg aagaccttgt ctgcctatgc cgaacttgtc 21000
atttctccaa gtgaacgttc agacaagggc atttatacac tgaaattaga aaaccgtgtg 21060
aaaacaattt ctgggaaaat tgatgtcaat gtaattgctc gcccaagtgc acccaaagaa 21120
ttgaaatttg gtgatataac caaggactca gtacatttga cttgggaacc acctgatgat 21180
gatggaggaa gtccgttaac tggatacgtt gttgaaaaac gagaagtcag ccggaaaaca 21240
tggactaaag ttatggactt tgtgactgat ctagaattca cagttcctga tcttgttcaa 21300
ggaaaagagt acttatttaa agtttgtgct cgtaacaaat gtggccctgg agaacctgca 21360
tatgttgatg aacctgtaaa tatgtcaact cctgcaacgg tacctgaccc accagagaat 21420
gttaaatgga gagatcgaac agccaatagc atcttcttaa catgggatcc acctaaaaat 21480
gatggtggtt cacgcatcaa aggatatata gttgaaagat gtccacgtgg ttctgataaa 21540
tgggttgcct gtggagaacc tgttgcagaa acaaaaatgg aagtgacagg tcttgaggaa 21600
ggcaaatggt atgcctaccg cgtgaagacc ttaaacaggc agggtgctag caaaccaagc 21660
agacccacag aggaaatcca ggctgtggac acacaagagg ccccagaaat cttcctcgat 21720
gtgaagctcc ttgctggtct cactgtaaaa gctgggacca agattgaact tcctgccacc 21780
gtaaccggaa aacctgaacc taaaataact tggacaaagg ctgatatgat tctgaagcag 21840
gacaaaagaa ttaccattga aaatgtccct aagaaatcca cagtgactat tgttgatagt 21900
aagagaagtg acactggcac atatatcatt gaggctgtga atgtgtgtgg ccgggccact 21960
gctgtggtgg aagtgaacgt cttagataaa cccggaccac cagctgcctt tgacatcaca 22020
gatgtaacca atgagtcatg tcttctaaca tggaacccac cacgcgatga tggtggatct 22080
aagatcacaa actatgttgt ggagagacga gcaactgata gtgaagtgtg gcacaagctc 22140
tcatccaccg tcaaggatac aaacttcaag gccaccaaat taatccccaa taaagagtac 22200
atcttcagag ttgctgcaga aaacatgtat ggtgctggtg aaccagttca ggcctctcca 22260
ataacagcca aatatcagtt tgatccacct ggtcctccaa ctcgcctaga accttctgat 22320
atcactaaag acgcagtgac tctcacatgg tgtgagccag atgatgatgg tggcagccca 22380
atcacaggat actgggttga aagactggat cctgatacag ataaatgggt tagatgcaat 22440
aagatgccag taaaggacac aacatacaga gtgaaaggtc tcactaataa gaaaaaatac 22500
agattccgtg tgttggctga aaatcttgct ggacctggaa aaccaagcaa atcaactgaa 22560
ccaatcttaa taaaggatcc catagatcct ccatggcccc ctggaaaacc aactgtaaaa 22620
gatgtaggca aaacatcagt aaggttgaat tggacaaaac cagaacatga tggaggtgca 22680
aagattgagt cttatgtcat tgaaatgctg aagactggaa cagatgagtg ggtcagagtg 22740
gcggaagggg ttcccaccac tcagcacttg ctcccagggc tcatggaagg acaggaatac 22800
tcattccgag ttagagctgt gaataaggct ggggaaagtg aacccagtga acccagtgac 22860
cctgtgcttt gccgggagaa gctatatcct ccatcaccac cacgctggct tgaagttatt 22920
aatatcacaa aaaatacagc agacctaaaa tggacagttc ctgagaaaga tggagggtcc 22980
cccatcacca actacattgt ggaaaagaga gacgtcaggc gaaaaggctg gcaaacagtg 23040
gataccactg tcaaggacac caagtgcaca gtcaccccac tgactgaggg ctctttatat 23100
gtgttccgag ttgctgcaga aaatgctata ggacaaagcg actacaccga aattgaggac 23160
tctgtgctgg ccaaagacac ctttaccact cctggaccac cctacgccct ggcagtggtt 23220
gatgtgacaa aacgacatgt tgacctaaag tgggagccac ctaaaaatga tggtggaaga 23280
ccaatacaga gatatgtcat tgagaagaaa gaaaggttag gtacccgttg ggtgaaagct 23340
ggaaagactg caggacctga ctgtaacttc agagtaactg atgtcatcga aggaacagag 23400
gtccagtttc aggttcgggc tgaaaatgaa gctggagttg gccacccaag tgaacccaca 23460
gaaatcctat ccattgaaga tccaacaagt cctccctcac caccccttga cctacatgtg 23520
actgatgctg ggagaaaaca cattgccatt gcttggaagc ctccagagaa aaatggtgga 23580
agtcctatca taggatacca tgttgaaatg tgtccagtag gcactgagaa atggatgaga 23640
gttaattctc gcccaataaa ggacttgaaa ttcaaggttg aagaaggtgt tgttcctgac 23700
aaagaatatg tcctgagagt gagagcagtc aatgctattg gtgtcagcga gccatctgaa 23760
atctctgaaa atgtggttgc caaagaccca gactgcaagc caacaattga cctggagact 23820
catgacatta ttgttattga aggtgaaaag ttaagcattc ctgttccctt cagagctgtc 23880
ccagttccaa ctgttagttg gcataaagat ggcaaagaag ttaaagcaag tgatagatta 23940
acaatgaaga atgatcacat ctctgcacac cttgaagttc ccaagagtgt ccgtgcagat 24000
gccggaattt ataccattac actggagaat aagctcggct cagcaacagc ctcaatcaat 24060
gtcaaagtca taggcctacc tggaccatgc aaagatatta aagcaagtga cattaccaag 24120
agttcttgta agttaacttg ggaacctcca gaatttgatg gtggaacccc aattcttcat 24180
tatgtcctgg agcgcagaga agctgggagg agaacatata taccagtcat gtctggtgag 24240
aacaaactgt catggactgt gaaggatctc ataccaaatg gtgaatactt cttccgtgtt 24300
aaagcagtca acaaggttgg tggaggagaa tatattgaac tgaaaaatcc agtcattgct 24360
caagatccaa agcaaccccc tgatccacct gtagatgtag aggttcataa tcctacagcg 24420
gaggcaatga ctattacatg gaagccacct ttgtatgatg gagggagcaa gataatgggc 24480
tacatcatag agaagattgc taagggtgaa gaaaggtgga agagatgcaa tgaacacctg 24540
gtaccaatcc tgacctatac agcaaaagga cttgaagagg ggaaagagta ccaattccgt 24600
gtgcgagcag agaacgccgc gggtattagt gaaccttctc gggctactcc tccaaccaaa 24660
 
gctgtagatc ccattgatgc ccccaaagtc attctgagaa caagcctaga agtgaaacga 24720
ggtgatgaaa tagcacttga tgcaagtatt tctggatcac cttacccaac tattacatgg 24780
ataaaggatg aaaatgttat tgtaccagag gaaattaaga agcgtgcagc acccttggtt 24840
aggagaagga agggtgaagt tcaagaagaa gaaccatttg tcctgcctct gacacagcgt 24900
ttgagtattg acaacagcaa aaagggagaa tctcagctac gcgtccgaga ttctctccga 24960
cctgaccatg gtctgtatat gatcaaagtt gaaaatgacc acggtattgc aaaagctcct 25020
tgtactgtca gtgtgttaga tacaccggga ccaccaatca actttgtatt tgaagatatc 25080
agaaagacct cagtcctttg taaatgggaa ccaccccttg atgatggtgg cagtgaaatc 25140
ataaactaca ctttggaaaa gaaagacaag acaaaacccg actcagaatg gattgttgtc 25200
acttcaacac ttagacattg caaatattca gtaacaaaac tgattgaagg aaaagagtac 25260
ctcttccgtg taagagctga aaacagattt gggccaggtc caccatgtgt ttcaaagcca 25320
cttgtggcta aagatccatt tggaccacct gatgcaccag ataagcccat tgtggaagat 25380
gttaccagca acagtatgct agtgaaatgg aatgaaccaa aagataatgg aagccccatt 25440
ttgggttact ggcttgaaaa acgtgaagtt aacagtacac attggtctcg tgtcaacaaa 25500
agccttctga atgccttgaa agccaatgta gatggcttat tagaaggact cacctatgtc 25560
ttcagagtat gtgctgaaaa tgcagctgga cctggaaagt tcagtccacc ttcagatccc 25620
aaaacagcac atgatccaat ctctcctcct gggccaccta tcccaagagt cactgacaca 25680
agctctacaa ctattgaact agaatgggaa cccccagctt tcaatggtgg tggggaaatt 25740
gttggctatt ttgttgataa gcagttggtt ggcacaaata aatggtcacg ctgcacagag 25800
aagatgatca aggtccgtca gtacaccgtc aaagaaatcc gagagggtgc tgattacaaa 25860
cttcgggtga gtgctgtcaa tgccgcaggg gaaggaccgc ctggagaaac acaacctgtt 25920
actgtggctg aaccacaaga gcctccagct gtggaactgg atgtttctgt caagggtgga 25980
atacaaataa tggctgggaa gactcttaga attccagctg tggtgactgg tcgccctgta 26040
cctacaaaag tatggaccaa agaagaaggg gagctggata aagaccgtgt tgtaatagac 26100
aacgttggaa ccaaatctga actaattatc aaggatgcac tgcgaaaaga ccatggcaga 26160
tatgtgatta cagctacaaa tagctgtggt tccaaatttg cagcagccag ggtagaagtt 26220
tttgatgtcc ctggtccagt tcttgactta aaacctgttg taacaaacag aaaaatgtgt 26280
ctacttaact ggtctgatcc agaagatgat ggaggaagtg aaataacagg ctttatcatt 26340
gaaagaaaag atgccaagat gcatacttgg agacaaccaa tagagactga gagatctaaa 26400
tgtgacatca caggtctgct tgagggacaa gaatataagt tccgtgttat tgccaagaac 26460
aagtttggct gtggccctcc tgttgaaata ggaccaattc ttgcagttga tccactaggt 26520
cctccaacat ctccagagag gctcacatac actgaaagac aaaggtccac tatcacactt 26580
gactggaaag agccccgcag taatggtggc agtcccatcc aaggatatat cattgaaaaa 26640
cggcgtcatg acaaacctga ctttgaaaga gttaacaagc gactctgccc aaccacatct 26700
tttctggttg aaaatcttga tgaacaccaa atgtatgagt tccgtgtcaa agctgtcaat 26760
gaaattggtg aaagtgaacc atccctacct cttaatgtag tcatacaaga tgatgaagtg 26820
cctccaacta ttaagttgcg tctgagtgtt cgaggagaca ctatcaaagt taaggcagga 26880
gagcctgtcc acatccctgc agatgtgaca ggccttccaa tgcctaagat tgaatggtcc 26940
aaaaatgaaa ctgtaattga aaaacccact gatgcacttc agataaccaa ggaagaggta 27000
tcccgaagtg aggcaaaaac tgagcttagc attcccaaag cggtccggga ggacaaaggc 27060
acttacacag ttactgcttc caatcgcctt ggctcagtgt tccgaaatgt tcacgttgaa 27120
gtatatgacc gcccatcccc accaagaaat cttgctgtta ctgacattaa agctgaatct 27180
tgctacttga catgggatgc ccctcttgat aatggtggca gtgaaatcac ccattatgtt 27240
attgacaaac gtgatgcaag taggaagaaa gcagaatggg aggaagtcac caacactgct 27300
gtagagaaaa gatatgggat ctggaaactt atccccaatg gtcagtatga gttccgagtc 27360
agggcagtga ataaatatgg aatcagtgat gagtgcaaat cagataaagt agtcattcaa 27420
gatccttatc gccttcctgg acctccagga aaaccaaaag ttttggcacg caccaaagga 27480
tcaatgctag tgagctggac tcctcctttg gacaatggtg gctctccaat tactggctac 27540
tggctggaga aaagagaaga gggaagtcct tattggtcac gtgttagccg agcaccaata 27600
accaaagtgg gattgaaagg cgtggaattt aatgttcctc gtttgcttga aggcgttaaa 27660
taccagttca gagccatggc aataaatgct gcaggaattg gtcctcccag tgaaccatca 27720
gatccagagg ttgcaggaga tcccatattt ccaccggggc caccttcttg cccagaagtt 27780
aaagataaaa cgaagtcaag catctcacta ggatggaaac ctccagccaa agatggtggc 27840
agcccaatca aaggatacat tgtagaaatg caagaagaag gtactactga ctggaaaaga 27900
gtaaatgaac cagacaaact tataactacc tgtgaatgtg tggtgcctaa tctgaaagag 27960
ctcaggaagt acagattcag agtgaaagct gtcaatgaag ctggtgaatc tgaaccaagt 28020
gatacaactg gggagatccc tgccactgat attcaagagg aaccagaagt tttcattgac 28080
attggagcac aggactgtct ggtttgtaaa gctggctcac agattaggat tcctgctgtc 28140
atcaagggac gcccaacacc aaaatcatct tgggaatttg atggaaaggc aaagaaagca 28200
atgaaggatg gagttcatga catacccgaa gatgcacagc tggagactgc tgaaaactcc 28260
tcagtaatta ttattccgga gtgtaaacga tctcatacag gcaaatacag catcacagcc 28320
aagaataaag caggacaaaa gactgcaaat tgcagagtta aagtcatgga tgtaccaggc 28380
 
ccacccaaag atctgaaagt cagtgatatc acaaggggta gttgcagact ttcatggaag 28440
atgccagacg acgatggagg agacaggatc aaaggctatg ttattgagaa gaggactatt 28500
gatggaaaag cctggaccaa agtcaatcca gactgtggaa gcaccacatt tgtagtgcct 28560
gatctcctct ctgaacagca atatttcttc cgtgtgcgag cagaaaaccg ttttggtatt 28620
ggcccacctg tggaaaccat tcagaggacc actgccagag atccgatata tcctcctgat 28680
cctcctatta aactcaagat tggcctcatc acaaagaaca cagtgcatct gtcatggaaa 28740
cccccgaaga atgatggggg ctcccctgtt acccactata ttgttgagtg ccttgcatgg 28800
gaccctactg ggacaaagaa agaagcctgg aggcagtgca ataagcgtga tgtggaagaa 28860
ctgcaattta ctgttgaaga cctagtagaa ggtggggaat atgaattccg agtcaaagct 28920
gtcaatgctg caggagtcag caagccttca gccactgttg gcccctgtga ctgtcaaaga 28980
ccagacatgc caccatcaat tgatctaaaa gaattcatgg aggttgaaga aggaaccaat 29040
gttaacattg tggccaaaat taaaggtgtg ccattcccga cactaacctg gtttaaagct 29100
cctccaaaga agcctgataa caaagaacct gttctctatg acacccatgt caacaaactg 29160
gtggtagatg atacttgcac tttagttatt ccgcagtctc gcaggagtga cactggctta 29220
tataccatca cagctgtaaa taatctggga acagcatcaa aggagatgag actgaatgtc 29280
ctgggtcgtc ctggccctcc agtgggaccc ataaaatttg aatctgtttc agcagatcaa 29340
atgacactat cttggtttcc acctaaagat gatggtgggt ctaagattac aaactatgta 29400
attgagaaaa gagaagctaa caggaagaca tgggtccatg tctccagtga acctaaggag 29460
tgcacgtaca cgattcccaa attgctagaa ggccatgaat atgtattccg aatcatggcc 29520
cagaataaat atggcattgg agaacctctt gacagtgaac ctgaaacagc aagaaacctc 29580
ttctctgtcc ctggagcacc agataaacca acagttagca gcgtgactcg taactccatg 29640
actgtcaact gggaagagcc agaatatgat ggaggctctc ctgtgacagg gtactggctg 29700
gaaatgaaag acaccacttc aaagagatgg aagagagtta accgagatcc tatcaaagcc 29760
atgactttgg gtgtttctta taaagtgact ggtcttattg aaggttccga ctatcaattc 29820
cgggtatatg caatcaatgc tgctggcgtg ggtccagcaa gtctgccatc agacccagcg 29880
actgctagag atccaattgc ccctcctggt cctccatttc ccaaagtgac agattggact 29940
aaatcatctg cagatctgga gtggtctccc ccactaaaag atggtggatc caaagtaact 30000
ggatacatcg ttgaatataa agaagaagga aaagaagaat gggaaaaggg taaagataaa 30060
gaagtgagag gaacaaagct cgttgtgaca ggattaaagg aaggagcatt ctacaaattt 30120
agagttagtg cagtcaacat tgctggcatt ggagaacctg gagaggtcac agatgtcatt 30180
gaaatgaagg acagacttgt ttcacctgac cttcagctag atgccagtgt cagagataga 30240
attgttgtcc atgctggagg ggtgatccga atcattgcct atgtgtctgg aaagcctcct 30300
ccaaccgtca cctggaacat gaatgaaaga accttacctc aagaagccac cattgagacc 30360
acagccatta gctcatccat ggtcatcaag aactgccaga ggagccatca aggcgtctat 30420
tctcttcttg ccaaaaatga agccggagaa agaaagaaga caattattgt tgatgtatta 30480
gatgttccag gtcccgttgg aacaccattc ctagctcaca acctaaccaa tgagtcctgc 30540
aaactgacat ggttttctcc agaagatgat ggaggctctc caatcaccaa ttatgtcatt 30600
gaaaagcgtg aatctgaccg cagagcatgg accccagtga catatacagt tacccgacaa 30660
aatgctactg tccagggtct cattcaagga aaagcctact ttttccgaat tgcggctgaa 30720
aatagtattg gcatgggtcc atttgttgag acatcagagg cacttgttat cagagagcca 30780
ataactgtac cagagcgtcc tgaagacctg gaagtcaaag aagttactaa aaatactgta 30840
actttgactt ggaatcctcc taagtatgat ggtgggtcag aaattattaa ctatgtccta 30900
gaaagtcggc tcattgggac tgagaagttc cacaaagtta caaatgacaa cttgcttagc 30960
agaaaataca ctgttaaagg cttaaaagaa ggtgatacct atgagtaccg tgtcagtgct 31020
gtcaacattg ttggacaagg caaaccatca ttttgcacca aaccaattac ttgcaaggat 31080
gagctggcac ccccaacgct tcacctcgac ttcagagata agctcacgat tcgagttggt 31140
gaagcttttg ccctcactgg ccgttactca ggcaaaccaa agcctaaggt ttcctggttc 31200
aaagatgaag ctgatgtgct ggaagatgat cgcactcata taaagactac accagcaaca 31260
cttgctttag agaagatcaa ggccaaacgt tcagattccg gcaaatactg tgtggttgtg 31320
gagaacagta caggctctag gaaaggtttc tgtcaagtta atgttgttga ccatcctgga 31380
ccaccagtag gaccagttag ttttgatgag gtgaccaaag attacatggt tatctcttgg 31440
aagcctcctt tagatgatgg aggcagtaaa atcaccaatt atattattga gaagaaggaa 31500
gtgggtaaag acgtctggat gccagtgaca tctgcaagtg ctaaaacaac atgcaaagtt 31560
tctaaactac ttgaaggaaa agattatatt ttccggatac atgctgaaaa tctgtatgga 31620
ataagtgatc ctctggtgtc tgattcaatg aaagccaaag atcgtttcag ggttcctgat 31680
gcacctgatc agccaattgt tacagaagtt accaaagact ctgcattagt aacctggaat 31740
aagccacatg atggaggaaa acccatcaca aactacatcc tggaaaagag agaaactatg 31800
tctaaacgat gggctagagt taccaaagat cctattcatc catacactaa atttagggtt 31860
cctgatcttc tagaaggatg tcagtatgaa ttccgggttt ctgcagaaaa tgaaattggt 31920
attggagatc caagcccacc atccaaacca gtctttgcta aagatccaat tgctaaacca 31980
agtccacctg ttaatcctga agcaatagat acaacatgca attcagtcga tctaacttgg 32040
cagccaccac gtcatgatgg tgggagcaag attctgggtt atattgttga gtaccagaaa 32100
 
gttggagatg aagagtggag aagagccaat cacacccctg agtcatgtcc tgaaactaaa 32160
tataaagtca ccggtcttcg ggacggtcaa acctataagt ttagagtgtt agcagtcaat 32220
gcagctggtg aatcagatcc agctcatgtt ccggagccag tcctagtaaa agacaggctt 32280
gaaccccctg agttgattct tgatgccaac atggcaagag aacaacacat taaagttggt 32340
gatactctaa gacttagtgc catcatcaaa ggagtgccat tcccaaaagt aacttggaaa 32400
aaagaagaca gagatgctcc aactaaagca agaattgatg tgactccagt tggtagcaag 32460
cttgaaattc gtaatgctgc ccatgaagat ggtggaattt attctttaac agtggagaat 32520
ccagctggtt caaaaactgt ctcagtaaaa gtacttgtat tagataaacc tgggccacct 32580
agagatctgg aagtcagtga aattaggaaa gattcatgtt accttacttg gaaagaacca 32640
ctagatgatg gtggttctgt tattaccaat tatgtggttg agaggagaga tgttgccagc 32700
gcccagtggt cacctctctc agctacatca aagaaaaaga gtcacttcgc taagcatctg 32760
aatgaaggca accagtacct cttccgagta gctgcggaga accagtatgg acgtggtcct 32820
tttgttgaaa caccaaaacc aatcaaggct ttggatcctc tccatccccc agggccaccc 32880
aaggacctgc accatgtaga tgttgacaag actgaagtct ccctagtctg gaataagccg 32940
gatcgtgatg gtggttctcc aatcactgga tatttggtag aatatcaaga agaaggcacc 33000
caggactgga ttaaatttaa gactgtgaca aacttagagt gtgtggttac tggactacaa 33060
caaggaaaga cctatagatt ccgtgtaaaa gctgaaaaca ttgtgggtct tggtctccct 33120
gacacaacta tcccgataga atgtcaagaa aaactagtgc ctccatccgt ggagctagat 33180
gtgaaattaa ttgaaggtct tgtggtaaag gctggaacca cagtcagatt ccctgctatt 33240
ataagaggtg tgcctgttcc tactgcaaag tggacaaccg atgggagtga gattaaaacc 33300
gatgagcact acacagttga aacagacaac ttctcatcag tacttaccat taagaactgc 33360
ttaaggagag acactgggga atatcaaatc acagtttcca atgcagccgg tagcaaaaca 33420
gtagccgtac atcttactgt tcttgatgtt cctgggccac caacaggtcc tattaatatt 33480
ctggatgtta ctcctgaaca catgactatc tcatggcagc cacctaagga tgatggagga 33540
agccctgtga taaattatat tgttgagaaa caagatacaa ggaaagacac gtggggtgtt 33600
gtctcttccg gaagcagtaa gacaaagctg aaaatcccac atctgcagaa gggctgtgaa 33660
tatgttttcc gagttagagc agagaataag ataggtgttg gtcctcccct tgactccaca 33720
cctactgttg ctaagcataa atttagtcct ccgtctcctc ctggtaaacc agtggttact 33780
gacattactg aaaatgcagc aacagtgtct tggaccctgc caaaatctga tggtggcagt 33840
ccaataactg gctactatat ggaacgtcga gaagtaactg gcaaatgggt gagggtcaac 33900
aaaacaccta tcgctgacct gaagttcaga gtgactggac tctatgaagg aaatacatat 33960
gagtttagag tttttgctga aaatcttgca ggactaagca aaccatcccc aagttctgat 34020
ccaataaaag cttgccggcc catcaaacca cctggaccac ctattaatcc taaactgaaa 34080
gacaagagca gagaaacagc tgatttggtg tggacaaagc ctctcagtga tggtggtagc 34140
cccattctag gatatgtagt ggaatgtcag aaacctggca cggcacaatg gaacaggatt 34200
aataaagatg aactcattag gcaatgtgcc tttagggtac ctggactaat tgaaggaaat 34260
gagtacagat tccgtataaa ggcagctaat attgtaggag agggtgagcc aagagaacta 34320
gcagaatctg tgattgcaaa agatatcctt catcctccag aagtagaact tgatgttact 34380
tgtcgtgatg ttattaccgt gagagtaggc caaactatcc gcattctagc tcgagtcaaa 34440
ggcagacctg aaccagacat aacttggact aaggaaggca aagtattggt ccgagaaaag 34500
agggtggacc ttattcagga tctacctcgt gttgagttac aaattaaaga agctgttaga 34560
gctgatcatg gcaagtatat catctcagct aagaacagca gtggacatgc ccaaggttca 34620
gccatcgtta acgtccttga cagacctggg ccttgccaga atttgaaggt taccaatgta 34680
accaaagaga actgtacaat ttcttgggaa aacccactag ataatggtgg ctcagaaata 34740
acaaacttca tagtagaata tcgcaaacca aaccagaaag gctggtcaat tgttgcatca 34800
gatgtcacta aacgattaat caaggccaac cttttagcca acaatgaata ctatttccga 34860
gtttgtgcag agaataaagt aggtgttggg ccaaccatcg aaacaaaaac tcccattctg 34920
gctattaacc ctattgacag accaggtgag cctgaaaacc ttcacattgc agataaagga 34980
aagacatttg tctacctaaa gtggcggagg cctgactatg atggtggcag tccaaatctg 35040
tcatatcatg ttgagagaag gcttaagggc tccgatgact gggaaagagt gcataaagga 35100
agcattaaag aaactcacta catggttgac agatgtgttg aaaaccagat ttatgagttc 35160
agagtgcaaa caaagaatga aggtggggaa agtgactggg tgaagacaga ggaagttgtt 35220
gtgaaagaag acttacaaaa accagtactt gatctgaaat taagtggggt cctaactgtc 35280
aaagcagggg acaccattag gcttgaggca ggggttagag gcaaaccatt cccagaagtt 35340
gcatggacca aggacaaaga cgctacagac ttaacaagat caccaagggt caagattgat 35400
acccgtgctg attcatctaa attttctctt actaaagcaa agcgaagtga tgggggtaaa 35460
tatgtagtta cggcaactaa cacggctggc agttttgtgg cctatgccac tgtcaatgtt 35520
ttagataagc ctggtcctgt gagaaatctg aaaattgttg atgtgtccag tgataggtgt 35580
actgtttgct gggatccacc agaagatgat ggtggctgtg aaatccaaaa ttatattcta 35640
gaaaaatgtg agacaaagcg aatggtttgg tctacctatt ctgctactgt cttgacacct 35700
ggtactacag taacacgtct catagaagga aatgaatata ttttcagagt ccgtgcagaa 35760
aataaaatag gcacaggccc tccaacagaa agtaaaccag tcatagccaa aaccaagtat 35820
 
gataaacctg gtcgccctga tcccccagaa gtcactaaag taagcaaaga agagatgact 35880
gtggtttgga atccacctga atatgatggt ggaaagtcta taactggata ctttttggag 35940
aaaaaggaaa agcattcaac acgatgggtc cctgtcaaca agagtgcaat ccctgagaga 36000
cgtatgaaag tacagaatct cctcccagac catgaatatc agttccgtgt caaggcagaa 36060
aatgaaattg gaattggaga accaagcttg ccttcaagac cggtggtggc aaaagacccc 36120
atagagccac ctggtccacc aaccaatttc agagtggttg atacaaccaa acattccata 36180
actcttgggt ggggaaaacc agtctatgat ggtggtgcac cgatcattgg atatgttgtg 36240
gaaatgagac caaaaatagc agatgcgtct cctgatgaag gctggaaacg gtgtaatgct 36300
gcagcacagc ttgtacgcaa ggaattcact gttaccagct tggatgaaaa ccaggaatat 36360
gagttcaggg tgtgtgccca aaaccaagtt ggtattgggc gccctgcaga gctaaaggaa 36420
gctatcaaac ctaaagaaat actagaacct ccggagattg atttggatgc cagcatgagg 36480
aaactggtca tagtgagagc aggatgccct attcgtctct ttgctatagt gagaggacga 36540
ccagccccta aagtcacttg gcgaaaagtt ggcattgata atgtggtcag aaaaggacaa 36600
gttgatctgg ttgacactat ggccttcctt gtcatcccca attctacccg tgatgactca 36660
ggaaaatatt ccttaacact tgtgaaccca gcaggagaaa aggctgtatt cgtaaatgtc 36720
agagtattag acactcctgg gcctgtgtct gatttaaaag tttcagatgt cactaaaaca 36780
tcatgccatg tgtcctgggc ccctcctgaa aacgacggtg ggagccaagt gacacattat 36840
atcgtggaga aacgtgaggc agacagaaag acatggtcga ccgttacccc agaagttaag 36900
aaaacaagct tccatgtaac caatcttgtc cctgggaatg agtattactt cagagtaact 36960
gctgtcaacg aatatggccc tggcgtccca acagatgtcc caaaaccagt gcttgcatca 37020
gatcctctaa gtgagccgga tcccccaagg aaattagaag cgactgaaat gaccaagaac 37080
agtgccacct tagcctggtt acctccccta cgtgatggag gtgctaaaat cgatggctac 37140
atcattagtt acagagaaga agagcagcct gcagatcgct ggacagagta ctcagtggta 37200
aaagatctga gccttgttgt cactggccta aaggaaggaa agaaatacaa atttagagta 37260
gcggccagaa atgctgttgg agtcagtttg ccaagagaag ctgaaggagt gtatgaagcc 37320
aaagagcaac tgttgccacc aaagatcctt atgccagagc aaataactat caaagctggg 37380
aaaaaactcc gaattgaagc ccatgtgtat ggaaagcctc atcccacctg taaatggaaa 37440
aaaggagaag atgaagttgt cacatccagc cacctggcag tgcataaagc agacagctct 37500
tcaattctga tcataaaaga tgtgactagg aaagacagtg gttactacag cctcacagca 37560
gagaacagtt ctgggacaga cactcagaaa atcaaagttg tagtcatgga tgcccccggc 37620
ccccctcagc ctccatttga catttctgat atagacgctg atgcttgctc cctgtcatgg 37680
cacatccctc tggaggacgg aggcagtaac atcaccaatt atatagtgga gaagtgtgat 37740
gtaagccgag gtgactgggt cacggctcta gcttcagtca caaaaacttc ctgcagggtt 37800
ggaaagctga tcccaggcca agagtacatc ttccgggtcc gtgctgaaaa ccgatttggc 37860
atttcagagc ctctcacatc tccaaagatg gttgcgcagt tcccatttgg tgttcctagt 37920
gaaccaaaga atgcacgagt caccaaagtc aacaaggact gtatttttgt tgcttgggac 37980
agaccagata gtgatggagg gagccccatt attggttatc tgattgaacg caaggaaaga 38040
aacagtttgc tgtgggtgaa agccaatgat actcttgtcc ggtcaactga atatccttgt 38100
gctggccttg tagaaggtct tgagtattca ttcagaatct atgccctaaa caaagctgga 38160
tccagcccac ccagcaaacc cacagaatat gtaactgcaa gaatgccagt tgatcctcct 38220
gggaaacctg aggttattga tgtcaccaag agtactgtat ctctgatctg ggctcgtcca 38280
aagcatgatg gaggcagtaa aattattggc tatttcgtag aagcttgcaa acttcctggt 38340
gataaatggg tacggtgcaa tactgcacct caccagattc cccaggaaga gtacacagct 38400
actggcctag aagagaaagc tcagtatcaa tttagagcta ttgccaggac cgcggtaaac 38460
attagcccac cttctgaacc ttctgatcca gtgactatcc tcgcagaaaa tgtccctccc 38520
aggatagacc tgagtgtggc tatgaaatct ttgcttactg tgaaagctgg aactaatgtc 38580
tgcttggatg ctactgtttt tggtaaaccg atgccaacag tttcttggaa aaaagatggc 38640
acactgctaa aaccagcaga aggcataaag atggccatgc agcggaatct gtgcaccttg 38700
gagctattca gcgtgaaccg gaaggactca ggagactata ccattactgc tgaaaattca 38760
agtggttcta aatcagccac cattaagctt aaagtgttag ataaaccggg tcctccagca 38820
tctgttaaaa tcaacaaaat gtattcagat cgtgctatgc tttcttggga accgcctctt 38880
gaagatggag gctcagaaat caccaactat attgttgaca aacgtgaaac aagcaggccc 38940
aactgggctc aagtctctgc aactgtgcct atcaccagct gcagcgtgga gaaacttata 39000
gagggccatg agtatcagtt ccgtatttgt gctgaaaata aatatggagt aggcgatcca 39060
gtcttcactg aaccagcaat tgccaaaaac ccatatgacc caccaggacg ctgtgatcct 39120
cctgttatta gcaacataac caaagatcac atgacagtca gctggaagcc accagcagat 39180
gatgggggct cacccatcac tggctatttg cttgaaaagc gggaaaccca ggctgttaac 39240
tggactaagg tcaacagaaa acctattata gaaagaacat taaaagcaac aggtcttcaa 39300
gaaggtaccg aatatgagtt ccgtgttaca gctataaata aagctggacc aggcaaaccc 39360
agtgacgcat ccaaggccgc ttatgctcgg gaccctcagt atcctcctgc gccaccggct 39420
ttccctaaag tatatgatac aactcgcagc tctgtgagtc tatcttgggg caagccagcc 39480
tatgacggcg gcagccctat cattggttat ctcgttgaag taaaacgggc tgactccgat 39540
 
aactgggtga ggtgcaactt accacagaat ctacagaaaa cccgctttga ggttactggc 39600
ctgatggaag acacacaata tcaattccgt gtgtatgccg ttaataagat tggatacagt 39660
gaccccagtg atgtgccaga taaacactat cccaaggaca tcttaattcc acctgaggga 39720
gaacatgatg cggacttaag gaagacactc atattacgtg ctggagttac tatgagacta 39780
tatgtaccag taaaaggacg cccacctcca aagattactt ggtctaaacc aaatgtcaat 39840
ctaagagaca ggattggact ggacataaag tcaactgact ttgacacttt cttgcgctgt 39900
gaaaatgtga acaaatatga tgcaggaaaa tatatcttaa ccctggagaa cagctgtggt 39960
aaaaaggaat ataccattgt tgtgaaagtg cttgatactc ctgggccacc tatcaatgtg 40020
actgttaagg aaatatccaa agactctgct tatgttacct gggagcctcc cattattgat 40080
ggcggaagcc ccatcataaa ctatgtggta caaaaacgtg atgcagagag gaaatcctgg 40140
tctacagtga caactgagtg ctccaaaaca agcttcagag tacctaattt ggaggaggga 40200
aaatcctact tcttccgagt gtttgctgaa aatgagtatg gcattggtga tcccggtgaa 40260
actcgtgatg ctgtcaaagc ttcccaaact cctggaccag ttgtggacct gaaagtgagg 40320
tctgtatcta agtcatcctg tagcattggc tggaaaaagc ctcacagtga tggtggaagt 40380
cggattattg gatatgtagt tgatttcctg actgaagaaa ataagtggca acgagttatg 40440
aaatccttaa gcctacagta ctctgcaaaa gatttgactg aagggaagga atataccttc 40500
agagtgagtg ctgagaatga aaatggagaa ggaaccccaa gcgaaatcac tgttgtggca 40560
agggatgatg ttgtggctcc tgatcttgac ttaaagggtc tacctgattt gtgctacttg 40620
gctaaagaaa acagcaactt ccggcttaag atccccataa aaggcaagcc agctccatca 40680
gtctcctgga agaaagggga agatcctcta gcaactgaca ctagagtcag tgttgagtca 40740
tctgcggtta acacaactct tatagtgtac gattgccaaa aatctgatgc tggaaaatac 40800
acaatcacac ttaagaatgt tgctggcacc aaggaaggaa ctatctccat aaaggttgtt 40860
ggcaagcctg gcatccccac tggaccaatc aaatttgatg aagtcacagc agaagccatg 40920
accttaaagt gggctcctcc aaaggatgat ggaggttctg aaatcaccaa ctatatccta 40980
gagaagaggg attctgtgaa caacaagtgg gtgacgtgcg cctcagctgt ccagaaaacc 41040
acctttagag taaccagact tcatgagggc atggaatata ccttcagggt cagtgccgaa 41100
aataaatatg gtgtagggga aggcctgaaa tcggagccaa ttgttgcgag acatccattt 41160
gatgtgcctg atgctccccc acctcccaat attgtggatg tcagacacga ttcagtatct 41220
ctaacttgga ctgaccccaa gaaaactggt ggttctccaa ttacagggta tcatctcgag 41280
ttcaaggaaa gaaacagcct tttgtggaag agagctaaca agactccgat aaggatgaga 41340
gactttaaag tgacaggatt aactgaaggt cttgaatatg aattccgagt tatggcaatc 41400
aatttagcag gtgtgggcaa gccaagccta ccatcagagc ctgttgtggc actggaccca 41460
attgatcctc ctggaaaacc tgaggttatt aacataacaa ggaattcagt gactctcatt 41520
tggactgaac ctaaatatga cggtggtcat aagttaactg gatatatagt ggagaagcga 41580
gatctacctt cgaagtcttg gatgaaagcc aaccatgtta atgtcccaga atgtgccttt 41640
actgtaactg accttgttga gggtggaaaa tatgaattca gaattagagc aaagaataca 41700
gcaggtgcta tcagtgctcc atcagaaagt acagaaacca ttatttgcaa ggatgaatac 41760
gaggcaccaa caattgtcct tgatcccaca ataaaagatg ggctaacaat taaagcaggg 41820
gataccattg ttttgaatgc cattagcatt cttggcaaac cccttccaaa atcaagttgg 41880
tccaaggcag gaaaagacat tagaccatca gatatcactc agataacttc aaccccaaca 41940
tcttccatgc ttactatcaa gtatgccact agaaaagatg cgggtgaata taccatcact 42000
gctaccaatc cttttggcac gaaggtggaa catgtgaagg taacagtcct tgatgtacct 42060
ggtcccccag gtcctgttga aatcagtaat gtttctgctg aaaaagcaac acttacatgg 42120
acacctccct tggaagatgg cggctcacca attaagtcct atatacttga aaagagagaa 42180
accagccgac ttttgtggac agtggtttct gaagatattc agtcttgcag gcatgtggca 42240
accaaactta tccaaggaaa tgagtacatc ttccgggtct cagctgtaaa ccactatggc 42300
aaaggagaac ctgtacagtc tgaacctgtc aaaatggtag acagatttgg tccccctggc 42360
cctcctgaaa aaccagaggt atcaaatgtc actaagaaca ctgccactgt cagctggaaa 42420
aggccagtgg atgatggtgg cagcgaaatt acaggatatc atgtagaaag gagagaaaag 42480
aaaagcctgc gatgggtgag agcaataaaa acaccagttt ccgatctcag gtgcaaagta 42540
acaggactgc aagaaggaag cacctacgaa ttccgtgtca gtgcagaaaa cagagcagga 42600
attggtccac ccagtgaggc ttcagattct gttctgatga aagatgcagc atatcctcca 42660
ggaccacctt caaatccgca tgtcactgat actaccaaga aatctgcttc tttggcatgg 42720
ggcaagcctc attatgatgg tggacttgaa atcactggct atgtcgtgga gcatcaaaaa 42780
gtaggagacg aggcctggat aaaagatacc acaggaaccg ccctcagaat cactcagttc 42840
gttgttcctg atcttcagac taaagaaaaa tacaacttca gaatcagtgc catcaacgat 42900
gcaggtgttg gggagccagc ggtgattcca gatgttgaaa tcgtagaacg ggagatggct 42960
cctgattttg aactagatgc cgagcttcga agaacacttg ttgttagagc aggactcagt 43020
attaggatat ttgtgccaat taaaggtcgt cctgctcctg aagtgacatg gaccaaagat 43080
aacatcaacc tgaaaaaccg agccaacatt gaaaatacgg aatcatttac tcttctgatt 43140
atcccagaat gtaacagata tgataccggt aaatttgtca tgaccattga aaacccggct 43200
gggaagaaaa gtggctttgt gaacgtcaga gtcttggaca cgccaggccc agtcctcaac 43260
 
ctgcggccta cagacatcac aaaggacagt gtcaccctgc actgggacct ccctctgata 43320
gatggaggct cacgtataac aaactacatt gtagagaaac gtgaagcaac acggaaatct 43380
tattccacag ccaccactaa gtgccataaa tgcacatata aagttaccgg cttgtctgaa 43440
gggtgtgaat atttcttcag agtgatggca gagaatgaat atggaattgg tgagccaaca 43500
gaaactacag agcccgtaaa agcctctgaa gcaccatctc caccagacag ccttaacatc 43560
atggacataa ctaagagcac cgtcagcctg gcatggccta agcccaaaca cgatggtggc 43620
agcaagatca ctggctatgt gattgaagcc caaagaaaag gctctgacca gtggacccac 43680
atcacaaccg tgaaagggtt agaatgtgtt gtgaggaatc taactgaagg agaggaatat 43740
accttccaag tgatggcagt gaacagcgcg ggagaagtgc cccctagaga aagcagaccc 43800
gtcattgtca aggagcagac aatgcttcca gagctggatc tccgtggcat ctatcagaaa 43860
ctggtcattg ccaaagctgg tgacaacatc aaagttgaaa ttccagtgct cggtcgaccg 43920
aagcccacag tgacatggaa aaaaggagac caaattctta aacagacaca gagagttaat 43980
tttgaaacca cagcgacttc aaccatttta aatatcaatg agtgtgtcag aagtgatagt 44040
gggccctatc cattaacagc aaggaacatt gtaggagagg ttggtgatgt catcaccatt 44100
caagtccatg atatcccagg gccacctact ggaccaatca aatttgatga agtttcatct 44160
gattttgtaa ccttctcttg ggacccacct gagaacgatg gtggtgtacc aataagcaac 44220
tatgtagtgg aaatgcggca gactgacagt actacctggg ttgagttagc aaccaccgtt 44280
atacgtacta cctataaagc cacccgcctt actactggat tagagtatca gttccgtgta 44340
aaagctcaga atagatatgg agttggacca ggcatcacat cagcatggat agttgccaac 44400
tatccattta aggttcctgg acctcctggt acccctcagg taactgcagt taccaaggat 44460
tcaatgacaa ttagctggca tgagccactt tctgatggtg gaagccccat tttaggatat 44520
catgttgaaa gaaaagaacg aaatggtatt ctctggcaga ctgtgagcaa agctttagta 44580
ccaggcaaca ttttcaaatc aagtggactt acagatggta ttgcttatga gttccgggtg 44640
attgcagaaa acatggcagg caaaagtaag ccaagcaagc catcagaacc tatgttggct 44700
ctggatccca ttgacccacc tggaaaacca gtacctctaa atattacaag acacacagta 44760
acacttaaat gggctaagcc tgaatatact gggggcttta aaattaccag ttatatcgtt 44820
gaaaagagag accttcctaa tggacggtgg ctgaaggcca acttcagcaa cattttggag 44880
aatgaattta cagtcagtgg cctaacagaa gatgctgcat atgaattccg tgtgatcgcc 44940
aaaaatgctg caggtgccat cagtccacca tctgagccat ctgatgctat cacttgcagg 45000
gatgatgttg aggcaccaaa gataaaggtg gatgttaaat ttaaggacac ggttatatta 45060
aaagcaggtg aagcattcag actggaagct gatgtttcag gccgcccacc tccaacaatg 45120
gaatggagca aagatggaaa agagctggaa ggcacagcaa agttagaaat aaaaattgca 45180
gatttctcta ctaatctggt aaacaaagat tcaacaagaa gggatagtgg tgcctatacc 45240
cttacagcga ctaatcctgg tggctttgct aaacacattt tcaatgtcaa agttcttgac 45300
agaccaggcc cacctgaagg acctttggct gtaactgaag tgacatcaga aaagtgtgta 45360
ctatcatggt tccctccact ggatgatgga ggtgccaaaa ttgatcatta catagtacag 45420
aaacgtgaaa ccagcagatt ggcatggaca aatgtagcct cagaagtcca agtaacaaag 45480
ctaaaggtca ctaaactctt gaaaggcaat gaatacatat tccgtgtcat ggctgtaaat 45540
aaatatggag tgggagagcc actggaatca gagcctgtgc ttgcagtgaa tccttatgga 45600
ccccctgatc cgcccaaaaa ccctgaagtg acaactatta ctaaagattc gatggttgtc 45660
tgctggggac atcctgattc tgatggtgga agtgaaatca tcaattatat tgtggaacgg 45720
cgtgataaag ctggccaacg ctggattaaa tgcaacaaaa aaactcttac tgatttaaga 45780
tataaagtgt ctggactgac agaaggacat gaatatgagt tcaggattat ggctgaaaat 45840
gctgctggaa ttagtgcacc aagtcctacc agtccatttt acaaggcttg tgacactgtg 45900
tttaaacctg gaccaccagg taacccacgt gttctggata caagcagatc atccatttca 45960
atcgcttgga ataaacctat ctatgatggt ggttcagaaa tcactgggta tatggttgag 46020
attgccctgc cagaggaaga tgaatggcag attgtcactc caccagcagg actcaaggca 46080
acttcgtata ctatcactgg cctcacagag aatcaggaat ataagatccg catctatgcc 46140
atgaattccg aaggacttgg ggaacctgcc cttgttcctg gaactccaaa ggctgaagac 46200
agaatgctgc ctccagaaat tgaactggat gctgacctgc gcaaagttgt tactataagg 46260
gcctgctgca ccctgagact tttgttcccc atcaaaggaa ggcctgaccc tgaggtgaag 46320
tgggcccggg accatggaga atctttagat aaagctagca tcgaatccgc aagctcttac 46380
accctgctta ttgttggaaa tgtaaacaga tttgacagtg gcaaatatat actaactgta 46440
gaaaatagtt caggcagcaa gtctgcattt gtcaatgtta gagttctcga tacaccaggc 46500
cccccacagg atctgaaggt aaaagaggtc actaagacat ctgtcacact cacatgggac 46560
ccacctctcc ttgatggagg ttcaaaaatc aagaactata ttgttgaaaa gcgggaatca 46620
acaagaaaag catattcaac tgttgcaaca aactgccaca agacttcctg gaaggtagac 46680
cagcttcaag aaggctgtag ctactatttc agggttctcg cagaaaatga atatggcatt 46740
gggctgcctg ctgaaaccgc agaatctgtg aaagcatcag aacgacctct tcctccagga 46800
aaaataactt tgatggatgt cacaagaaat agtgtgtcac tctcttggga gaaaccagag 46860
catgatggag gcagccgaat tctaggctac attgtggaga tgcagaccaa aggcagtgac 46920
aaatggccca cgtgtgccac agtcaaggtc actgaagcca ctatcactgg attaattcag 46980
 
ggtgaagaat actctttccg tgtttcagct cagaatgaaa agggcatcag tgatcctaga 47040
caactgagtg tgccagtgat cgccaaagat cttgtcattc caccagcctt caaactcctg 47100
ttcaatactt tcactgtact ggcaggtgaa gacctaaaag ttgatgttcc attcattggc 47160
cgccctaccc cagctgtaac ctggcataaa gataatgtac cactgaagca gacaactaga 47220
gtaaatgcag agagcacaga aaataattca ctactgacaa taaaggacgc ctgccgagaa 47280
gatgttggcc attatgtggt taaactgact aactcagctg gtgaagctat tgaaaccctt 47340
aatgttatcg ttcttgacaa accagggcct ccaactggac cagttaaaat ggatgaagtg 47400
acagctgata gtattactct ttcctggggc ccacccaagt atgatggcgg aagttctatc 47460
aataattaca ttgttgagaa acgggacact tccacaacca cctggcaaat tgtatcagct 47520
acagttgcaa ggacaacaat aaaggcttgc agactgaaga ctggatgtga atatcagttt 47580
agaattgcag ctgaaaacag atatgggaag agtacctacc tcaattcaga gcctactgta 47640
gcccaatatc cattcaaagt tcctggtcct cctggcactc cagttgtcac actgtcctcc 47700
agggacagca tggaagtaca atggaatgag ccaatcagtg atggaggaag tagagtcatt 47760
ggctatcatc tagaacgcaa ggaaagaaat agcatcctct gggttaagtt gaataaaaca 47820
cctattcctc aaaccaagtt taagacaact ggccttgaag aaggtgttga atatgaattt 47880
agagtctctg cagagaacat cgtgggcatt ggcaagccga gtaaagtatc agaatgttat 47940
gtggctcgtg acccatgtga tccaccagga cggccagagg caatcattgt cacaaggaat 48000
tctgtgactc ttcagtggaa gaaacccacc tatgacggtg gaagcaagat cactggttat 48060
attgttgaga agaaagaatt acctgagggc cgttggatga aagccagttt tacaaatatt 48120
attgacactc attttgaagt aactggccta gttgaagatc acagatatga gttccgggtt 48180
atagcccgaa atgccgcagg agtgtttagt gagccttcag aaagcacagg agcaataaca 48240
gctagagatg aggtagatcc accacgaata agtatggatc caaaatacaa agacacaatc 48300
gtggttcatg ctggtgaatc attcaaggtt gatgcagata tttatggcaa accaatacca 48360
accattcagt ggataaaagg tgatcaggag ctttcaaaca cagctcgatt agaaataaag 48420
agcaccgact ttgccaccag tctcagtgta aaagatgcag tacgtgtcga cagtggaaat 48480
tacatactga aggccaaaaa tgttgcagga gaaagatcag ttactgtgaa tgtcaaggtt 48540
cttgacagac cagggccacc tgaaggacct gttgttatct caggagttac agcagaaaaa 48600
tgcacactag cttggaaacc cccacttcag gatggtggga gtgacatcat aaattatatt 48660
gtggaaagga gagaaaccag ccgcttagtt tggactgtgg ttgatgccaa tgtgcagact 48720
ctcagctgca aggttactaa gcttcttgaa ggcaatgaat atactttccg tataatggca 48780
gtaaacaaat atggtgttgg tgaacctctt gaatctgagc cagtagttgc caagaatcca 48840
tttgtagtac cagatgcacc aaaagctcca gaagtcacaa cagtgaccaa ggactcaatg 48900
attgttgtat gggaaagacc agcatctgat ggtggtagtg aaattcttgg atatgttctt 48960
gagaaacggg ataaagaagg cattagatgg acaagatgcc ataagcgtct gattggagag 49020
ttgcgcctga gagtaactgg actcatagaa aatcacgatt atgagttcag agtttctgct 49080
gagaatgctg ctggacttag tgaaccaagc cctccttctg cttaccaaaa ggcttgtgat 49140
cctatttata aaccaggacc cccaaacaac cccaaagtca tagacataac cagatcttca 49200
gtattccttt cttggagcaa accaatatat gatggtggct gtgaaattca aggatacatt 49260
gttgaaaaat gtgatgtgaa tgttggtgaa tggacaatgt gcactccacc aacaggaatt 49320
aataaaacaa acatagaagt agagaagctg ttggaaaagc atgaatacaa cttccgtatc 49380
tgtgctatta ataaagctgg agttggagaa catgctgacg tccctggacc tattatagtt 49440
gaagaaaaat tagaagcacc agacattgat cttgacctag aactaaggaa aatcataaat 49500
ataagggcag gtggctcctt aaggttattt gttcctataa aaggtcgtcc tacaccagaa 49560
gttaaatggg gaaaggtgga tggtgaaatc cgagatgcag ctataattga tgtcactagc 49620
agtttcacct ctcttgttct tgacaatgtc aaccgatatg atagtggaaa atatacgctt 49680
acattagaaa acagcagtgg aacaaagtct gcctttgtta ctgtgagagt tctggacacg 49740
ccaagtccac ctgttaacct gaaagtcaca gaaatcacca aagactcagt atcaattaca 49800
tgggaacctc ctttgttgga tgggggatcc aaaataaaaa attacattgt tgagaaacgt 49860
gaagccacaa gaaaatcata tgctgctgtt gtaactaact gccataagaa ttcttggaaa 49920
atcgatcagc tccaagaagg ttgcagttat tactttagag tcacagctga gaatgagtat 49980
ggtattggcc ttcctgccca gactgctgat ccaattaagg ttgcagaagt gccacaacct 50040
cctggaaaaa taactgtgga tgatgtcacc agaaacagtg tctctctgag ttggacaaaa 50100
cctgaacatg atggtggcag taaaatcatt cagtatattg tggaaatgca agctaaacac 50160
agtgagaaat ggtcagagtg tgctcgagta aagtctcttc aggcagtaat taccaaccta 50220
actcaagggg aagaatatct ttttagagtt gttgctgtaa atgaaaaggg gagaagtgat 50280
cctcggtccc ttgcagttcc aatagttgcc aaagatctgg taattgagcc agatgtaaaa 50340
cctgcattca gtagttacag tgtacaggtt ggccaagatt tgaaaataga agtgccaatt 50400
tctggacgtc ctaagccaac cattacctgg actaaagatg gtctcccact gaagcagacc 50460
acaagaatca atgttaccga ttcactggat ctcaccacac tcagtattaa agaaactcat 50520
aaggatgatg gtggacaata tggaatcaca gttgccaatg ttgttggtca gaagacagca 50580
tccatcgaaa ttgtaactct agataaacct gatcctccaa aaggacctgt taaatttgat 50640
gacgtcagtg ctgaaagtat tacattatct tggaaccctc cattatatac agggggctgc 50700
 
caaatcacca actacattgt tcagaaaaga gatacaacca ccacagtatg ggatgttgtt 50760
tctgctactg ttgctagaac tacactcaaa gtgaccaaac tgaaaactgg tacagaatac 50820
caatttagaa tatttgccga aaacagatat ggacaaagct ttgccttaga gtctgatcca 50880
attgtagctc aatatcccta caaagaacca ggccctccag gtacaccatt tgccacagcc 50940
atttccaaag actccatggt catacagtgg catgaaccag tcaacaatgg tggaagcccc 51000
gtcataggtt accacctgga gagaaaagaa agaaacagta ttttgtggac aaaggtcaac 51060
aaaactatta ttcatgacac ccaattcaaa gcacagaatc ttgaagaagg cattgaatat 51120
gaattcagag tgtatgctga aaatattgtt ggtgtaggca aagcaagcaa gaattctgaa 51180
tgctatgtag ccagagatcc ctgtgaccca ccaggaaccc cagaaccaat aatggttaaa 51240
agaaatgaaa tcactttaca gtggaccaaa cctgtgtatg atggtggaag tatgattaca 51300
ggctacattg tagagaaacg tgatttgcct gatggtcgtt ggatgaaagc tagctttaca 51360
aatgtcattg aaactcaatt tactgtgtca ggtcttactg aagatcaaag atatgaattc 51420
agagtcattg caaagaatgc agctggtgca ataagtaaac cctctgacag tactggacca 51480
ataactgcca aggatgaggt tgaactccca agaatttcaa tggatccaaa attcagagac 51540
acaattgtgg taaatgctgg agaaacattc agacttgagg ctgatgtcca tggaaagccc 51600
ctacctacca ttgagtggtt aagaggagat aaggaaattg aagaatctgc tagatgtgaa 51660
ataaagaaca cagatttcaa ggctttactt attgtaaaag atgcaattag aattgatggt 51720
gggcagtata ttttaagagc ttccaatgtt gcaggttcta agtcattccc agtaaatgta 51780
aaagtattag atagaccagg acctccagaa gggccagtcc aggttactgg agtcacttct 51840
gaaaaatgct ctttaacatg gtctccacca cttcaagatg gtggcagtga catttctcac 51900
tatgttgttg aaaagcgaga aaccagtcga cttgcctgga ctgttgttgc ttcagaagtt 51960
gtgaccaatt ctctgaaagt taccaaactc ttagaaggta atgaatatgt tttccgtata 52020
atggctgtca acaaatatgg tgttggagag cctttggaat ctgcaccagt actaatgaaa 52080
aatccatttg tgcttcctgg accaccaaaa agcttggaag tcacaaatat tgccaaagac 52140
tccatgaccg tctgttggaa ccgtccagat agtgatggtg gaagtgagat tattggttac 52200
attgtagaga aaagagacag aagtggcatt cgatggataa aatgtaataa acgccgcatt 52260
acagatttgc gtctaagagt gacaggatta acagaagatc atgagtatga attcagggtc 52320
tctgcagaaa atgctgctgg agttggggaa ccaagtccag ctacagttta ttataaagcc 52380
tgtgatcctg tgttcaaacc tggcccacct accaatgcac acattgtaga caccactaaa 52440
aattcaatca cacttgcctg gggtaaaccc atctatgatg gcggcagtga gatcttggga 52500
tatgtagtag aaatctgtaa agcagatgaa gaagaatggc aaatagttac tccacagact 52560
ggcctgagag tcactcgatt tgaaatttca aaactcactg aacaccaaga gtataaaata 52620
cgagtctgtg ccctcaacaa agttggttta ggtgaggcta catcagttcc tggtactgtg 52680
aaaccagaag ataaacttga agcacctgaa cttgaccttg actccgaatt aagaaaagga 52740
attgttgtaa gagctggtgg atctgccaga attcacattc cattcaaagg tcgtccaatg 52800
cctgagatca cttggtctcg agaggaaggt gaattcacag ataaggtcca aattgaaaag 52860
ggagtaaact atacccaact atcaatagat aactgtgata gaaatgatgc tggaaaatac 52920
attcttaagt tggaaaacag cagtggatca aagtctgctt ttgtaactgt gaaagttctt 52980
gacactccag gaccaccaca gaatttggca gtcaaagaag tgagaaaaga ttctgccttc 53040
ctggtatggg agccacccat cattgatgga ggggcaaagg tcaagaacta tgtgattgac 53100
aaacgtgagt caaccagaaa agcgtatgct aatgtgagta gtaaatgcag caaaacaagt 53160
tttaaagtgg aaaaccttac agaaggagcc atttattact tcagagtcat ggctgaaaat 53220
gaatttggag ttggtgttcc agtggaaact gttgatgccg tgaaagctgc tgaacctcct 53280
tccccaccag gaaaggttac actcactgat gtgtcccaga ccagtgcatc acttatgtgg 53340
gagaaacctg aacatgatgg cggtagcaga gtcctggggt acgttgttga aatgcagccc 53400
aaaggaactg aaaaatggag cattgtggct gaatccaaag tctgtaatgc agttgttact 53460
ggtttgagtt ctggacaaga atatcagttc cgtgtcaagg cttataatga gaaaggaaaa 53520
agcgatccaa gagtgttggg tgttcctgtc atagccaagg acttgactat acagcctagt 53580
ttaaagttac catttaacac atatagtatc caagctggag aagatcttaa aatagaaatt 53640
ccagttatag gccgaccaag acctaacatt tcttgggtca aagatggtga gcctcttaaa 53700
cagacaacaa gagtaaacgt tgaagaaaca gctacctcaa ctgttttgca cattaaagaa 53760
ggtaacaaag atgactttgg aaaatacacc gtaacggcaa caaatagtgc aggcacagca 53820
acagaaaatc tcagtgttat cgttttagaa aagcctggac ctccagttgg cccagttcgg 53880
tttgatgaag ttagtgcaga ctttgtagtc atatcttggg aacctccagc ctatactggt 53940
ggctgccaaa taagcaacta cattgtagag aagcgagata caaccaccac cacttggcac 54000
atggtatcag caacagttgc aagaacaaca attaaaataa ccaaactgaa aacaggcacg 54060
gagtaccagt ttagaatttt tgctgaaaac aggtatggaa aaagtgcccc actggattct 54120
aaggcagtta ttgtacaata tccatttaaa gaacctggac cacctggaac tccttttgtg 54180
acatcaatct caaaagatca gatgcttgtg caatggcatg agccagtgaa tgatggaggc 54240
accaaaatta ttggctacca tcttgaacag aaagaaaaga acagtatttt atgggtcaag 54300
ttaaataaga cccccattca ggacaccaaa ttcaaaacaa ctgggcttga tgagggcctt 54360
gagtatgagt tcaaagtttc tgctgaaaat attgttggca ttggcaagcc tagcaaagtg 54420
 
tcagaatgct ttgttgctcg tgatccatgt gacccacctg gtcgccctga agccattgtt 54480
attacaagaa acaatgtcac actgaaatgg aagaaacctg cctatgatgg tggtagcaaa 54540
ataacaggtt atattgtaga aaagaaagat ctacctgatg gccgctggat gaaagccagc 54600
tttaccaacg tattagaaac tgaatttaca gtgagtggac ttgtagaaga ccaaagatat 54660
gaatttagag taattgcaag aaatgcagct ggaaacttta gtgaaccatc tgatagtagt 54720
ggtgccatta ctgcaagaga tgaaattgat gcaccaaatg cctctctgga tccaaaatat 54780
aaagatgtca tcgttgttca tgcaggagag acttttgttc ttgaagccga catccgtggc 54840
aaacctatac ctgatgttgt ttggtcaaaa gatggaaaag aacttgaaga aacagctgct 54900
agaatggaaa ttaaatctac tattcagaaa acaactcttg ttgtcaaaga ctgtatacgg 54960
actgatggag gacaatatat tctgaaactc agcaatgttg gtggtacaaa gtctataccc 55020
atcactgtaa aggtacttga caggccaggg tctcctgaag ggcctctgaa agttactgga 55080
gttactgcgg aaaaatgtta cctggcatgg aacccacctt tgcaagatgg tggtgctaat 55140
atttcacatt acatcattga aaagagggag acaagccgac tctcttggac ccaggtttca 55200
actgaggtac aggcccttaa ctacaaagtt actaaacttc ttcctggtaa tgagtacatt 55260
ttccgtgtca tggctgtgaa taaatatgga attggagagc ccttggaatc tgggcctgtt 55320
acggcctgta atccttataa gccaccaggt cctccctcaa cacctgaagt ctcagcaatc 55380
accaaagatt ctatggtagt aacatgggca cgcccagtag acgacggagg taccgaaatt 55440
gagggctaca ttcttgaaaa acgagataag gaaggcgtta gatggaccaa gtgcaacaag 55500
aaaacattaa cggatctgcg gctcagggta actggtctta ccgaaggcca ttcctatgaa 55560
ttcagagttg ctgctgaaaa tgcagctggt gtgggagaac ctagtgagcc atctgttttc 55620
taccgtgcgt gtgatgcctt gtatccacca ggtcccccaa gcaatccaaa agtgacggac 55680
acttccagat cttctgtctc cctggcatgg agtaagccaa tttatgatgg tggcgcacct 55740
gttaaaggct atgttgtaga ggtcaaagaa gctgctgcgg atgaatggac aacctgcact 55800
ccaccaacag gattacaagg aaagcagttc acagtgacca agcttaaaga aaacactgaa 55860
tataacttcc gtatttgtgc catcaattct gaaggtgtag gtgaacctgc aactctacct 55920
ggctcagtgg ttgctcagga gaggatagag ccaccagaaa tagaactcga tgctgatctc 55980
agaaaggtgg tcgttctgcg tgcaagtgct actttacgct tatttgtcac tatcaaaggt 56040
cgaccagaac ccgaagttaa atgggaaaag gcagaaggca ttctcactga cagggctcag 56100
atagaggtga ccagctcatt tacaatgttg gtgattgata atgttaccag atttgacagt 56160
ggtcggtata atctgacatt agaaaataat agtggctcca aaacagcttt tgttaacgtc 56220
agagttcttg actcaccaag tgcccctgtg aatttgacca taagagaagt gaagaaagac 56280
tcagtgacgt tgtcctggga accaccactt attgatggtg gagctaagat tacaaactac 56340
attgtcgaaa aacgagaaac tacaagaaaa gcctatgcta ccattacaaa taattgcact 56400
aaaactactt tcagaattga aaatctacaa gaaggatgtt cttactactt ccgagtcttg 56460
gcttccaatg aatatgggat tggtttgcca gctgaaacaa cagaacccgt taaagtgtct 56520
gaaccacccc tcccacctgg aagagtaact cttgttgatg tgacccgtaa tacagctaca 56580
attaagtggg agaaaccaga aagtgatggt ggcagcaaaa ttactggtta tgtggttgaa 56640
atgcagacta aagggagtga aaagtggagc acctgcacac aagttaagac tctagaagca 56700
actatatctg gcttaactgc aggagaagag tatgtcttca gggtagctgc agttaacgaa 56760
aagggaagaa gtgatccaag acaacttgga gtgccagtaa ttgcaaggga tattgaaata 56820
aagccttcag ttgagcttcc tttccatact ttcaatgtaa aggctagaga acaacttaag 56880
attgatgtgc cattcaaagg aagacctcaa gctactgtga actggagaaa agatggtcag 56940
actcttaaag agacaactag agtcaatgtt tcttcttcaa agactgtaac atcactatct 57000
attaaggaag cttcaaagga agatgttgga acttatgaat tatgtgtttc aaacagtgct 57060
ggatccataa cagttcctat tactataatt gtccttgaca gaccaggacc tccaggtcct 57120
atacgtattg atgaggttag ttgtgacagc ataaccattt cttggaatcc tccagaatat 57180
gatggtggct gccaaattag caattacatt gttgaaaaga aagaaaccac ctctacaaca 57240
tggcacatag tttcacaagc agttgcaaga acatccatta aaatagttcg cctgacaaca 57300
ggaagtgagt atcagttccg tgtttgtgca gaaaaccgct atggaaagag ctcctacagt 57360
gaatcttcag ctgttgttgc agagtatcca ttcagtcccc caggtcctcc tggtactcct 57420
aaagttgtgc atgccacaaa atctaccatg cttgtaacct ggcaagtgcc agttaatgat 57480
ggaggaagtc gagtaattgg ctatcatctt gagtataaag aaagaagcag cattctttgg 57540
tcaaaagcaa ataaaatcct cattgctgat actcaagtga aagtctccgg ccttgatgaa 57600
ggactgatgt atgagtatcg tgtatatgct gaaaatattg ctggaattgg taaatgcagt 57660
aaatcttgtg aaccagtccc tgcaagagat ccttgtgacc ctcctggaca acctgaagtc 57720
acaaatatca caagaaaatc agtgtcactt aaatggtcta aaccacatta tgatggtgga 57780
gctaagatca caggatacat tgttgaacgc agagaactac cagatggccg gtggctaaag 57840
tgcaattata ctaatataca agaaacatac tttgaagtaa ctgaacttac tgaagatcag 57900
cgttatgaat tccgggtttt tgcaaggaat gctgctgact cagttagtga gccatctgaa 57960
tccactgggc ctattatagt taaagatgat gttgagcctc caagagttat gatggatgtc 58020
aagttccgag acgttattgt tgtcaaagct ggagaggtcc ttaagataaa tgcagacatt 58080
gcagggcgac ctctgccagt aatttcctgg gccaaggatg gtatagaaat tgaagaaaga 58140
 
gcaagaacag aaatcatctc aacagacaat catactttgt taacagttaa agactgtata 58200
agacgagaca ctgggcaata tgtactaaca ctgaagaatg ttgccggcac tcggtctgtg 58260
gccgttaatt gcaaagtact tgataagcct ggtccaccag caggaccact tgaaataaat 58320
ggcctcactg ctgagaaatg ctctctttcc tggggacgtc cccaagaaga tggtggtgca 58380
gatatcgact attaccatcg taaaaaacgt gaaacaagcc accttgcatg gacaatatgt 58440
gaaggagagt tacagatgac atcctgtaaa gtaaccaagt tactcaaagg caatgaatat 58500
atatttagag taactggtgt taataaatat ggtgttggtg agcccctaga gagtgtagct 58560
ataaaggcac tagatccatt tacagttcca agtccaccca cgtctttgga aattacttct 58620
gtgaccaaag aatctatgac actttgctgg tcaagacccg agagtgatgg aggtagtgaa 58680
atatctggat atataattga aaggcgagag aaaaatagcc taagatgggt gcgtgtaaac 58740
aaaaaaccag tttatgatct aagagtgaaa tcaacaggac ttcgggaagg atgtgaatat 58800
gaatatcgtg tttatgcaga aaatgctgct ggcctaagtc ttccaagtga aacctctccc 58860
ttaattaggg cagaagatcc agtgttccta ccatctcctc catccaaacc caaaattgtg 58920
gactcaggca agacaactat aactattgcc tgggttaagc cgctgtttga tggtggggcc 58980
ccgataactg gatatactgt agaatacaaa aaatctgatg acactgactg gaaaacttcc 59040
attcagagct tacgagggac agaatataca ataagcggac taacaacagg agctgaatat 59100
gttttcagag taaaatctgt caataaggtt ggtgctagtg accccagtga tagctctgac 59160
cctcagatag caaaggaaag agaagaagaa cctttatttg atattgacag tgaaatgagg 59220
aagaccttga ttgtcaaggc tggtgcctca tttaccatga ctgtgccttt ccgaggaaga 59280
ccagtaccca atgtcttgtg gagtaagcca gacactgacc tccgtactag agcttatgtt 59340
gataccacag actctcgtac atcactgacc attgaaaatg ccaacagaaa tgactctgga 59400
aagtacacat taacaattca gaatgttttg agtgctgctt cactgacctt agttgtcaaa 59460
gttttagata ccccaggtcc tccaaccaac attactgtgc aagatgtaac caaagagtct 59520
gcagtgttat cctgggatgt tcctgaaaac gatggtggag caccagtgaa gaattaccac 59580
atagaaaaac gtgaggccag caagaaagca tgggtctctg tgaccaacaa ctgtaaccgc 59640
ctctcctaca aagttaccaa tttacaagaa ggagctatct attacttcag agtctctgga 59700
gaaaatgagt ttggtgttgg tataccagct gaaacaaagg aaggagtaaa aataacagaa 59760
aaaccaagcc cacctgaaaa acttggagta acaagtatat ccaaagacag tgtttccctg 59820
acctggctga agcctgaaca tgatggcgga agcagaattg tacactatgt cgttgaagca 59880
ctagaaaaag gacagaaaaa ctgggttaaa tgtgcagtgg caaagtcaac ccatcacgtt 59940
gtttccggtc tgagagagaa ttctgaatac tttttccgag tgtttgctga aaatcaagct 60000
ggcctgagtg acccgagaga gcttctgctt cctgttctta ttaaggagca actagaacca 60060
cctgaaattg atatgaagaa tttcccaagt cacactgtat atgttagagc tggttcaaac 60120
cttaaagttg acattccaat ctctggaaaa ccacttccca aagtgacctt atcaagagat 60180
ggtgtccccc ttaaggcaac catgagattt aataccgaaa ttactgctga gaacctgacc 60240
atcaatctca aagaaagtgt tacagctgac gctgggagat atgaaatcac tgctgccaac 60300
tccagtggta caaccaaagc tttcattaac attgttgtgc tagacaggcc tggtcctcca 60360
actggccctg ttgttattag tgatataact gaagaaagtg tgactctcaa atgggagcca 60420
cctaagtatg acggtggaag tcaagttacc aactacattc tactcaaaag agaaacaagt 60480
actgcagtgt ggactgaagt gtctgcaaca gttgcaagaa ccatgatgaa agtcatgaaa 60540
ctgaccacag gagaagaata ccaattccgc atcaaggcag aaaaccgctt tggcatcagt 60600
gatcatatag attcagcttg tgtgactgtc aaactaccat acacaacacc tggaccacca 60660
tctacaccat gggtcactaa tgttactcga gaaagcatca ctgtgggctg gcatgaacca 60720
gtgtcaaatg gaggcagtgc agtcgtaggc tatcacctgg aaatgaaaga cagaaacagt 60780
attttatggc aaaaagccaa caaactggtc atccgcacaa ctcacttcaa agtcacaaca 60840
atcagtgctg gacttattta tgaattcagg gtgtatgcag aaaatgctgc tggagttgga 60900
aaacctagcc atccttctga accagtcttg gcaattgatg cttgtgaacc cccaagaaat 60960
gttcgtatca ctgatatttc aaagaactct gtcagccttt catggcaaca accagctttc 61020
gatggaggta gcaagattac aggctacatt gttgagagac gtgaccttcc agatggcaga 61080
tggaccaagg ccagcttcac caatgttact gaaactcaat tcaccatctc tggcttgact 61140
cagaattccc agtatgaatt ccgtgtcttt gctaggaatg ctgttggttc cattagcaat 61200
ccatctgagg ttgtagggcc cattacttgc atcgattctt atggtggtcc tgtaattgat 61260
ttgcctctag aatatacaga agttgtcaaa tacagagcag gtacatctgt gaagctcaga 61320
gctggcattt ctggcaaacc tgcgcctact attgagtggt ataaagatga taaagaatta 61380
caaaccaatg cactggtgtg tgttgaaaat accacggacc tcgcatctat actcatcaaa 61440
gatgccgatc gccttaatag tggatgctat gaattaaaac taaggaatgc catggcgtca 61500
gcctcagcca ccatcagagt acagatcctt gacaaaccag gcccacctgg tggaccaatt 61560
gaatttaaga ctgtaactgc tgagaagatc acccttctct ggcggcctcc agctgatgat 61620
ggtggtgcaa aaatcactca ctacattgtg gaaaagcgtg agacaagccg cgttgtgtgg 61680
tctatggtgt ctgaacattt ggaagagtgc atcattacaa ccaccaaaat tatcaaagga 61740
aatgaataca tcttccgggt ccgagccgtg aacaaatatg gaattggcga gccactggaa 61800
tctgattccg ttgtagccaa gaacgcattt gttacacctg ggccaccagg cataccagaa 61860
 
gtgacaaaga ttaccaagaa ttcgatgact gttgtatgga gcaggccaat tgcagatggc 61920
ggtagtgata taagtggcta tttccttgaa aaacgagaca agaagagcct aggatggttt 61980
aaagtactaa aagagactat ccgtgacacc agacaaaaag taacaggact cacagaaaac 62040
agtgactatc aatacagagt ttgtgctgta aacgctgctg gacagggtcc attttctgaa 62100
ccatctgaat tctacaaagc tgctgatcct attgatcctc caggtccacc tgctaagata 62160
agaatcgcag attcaaccaa gtcatccatc acccttggct ggagtaagcc tgtctatgat 62220
gggggcagtg ctgttactgg gtatgttgtc gagataagac aaggagagga agaggaatgg 62280
actactgtct ctaccaaagg agaggtcaga actacagaat atgtggtatc caacctgaaa 62340
cctggagtca attactactt ccgggtatct gctgtaaact gtgctggaca aggagaacct 62400
atagaaatga atgaacctgt acaagctaaa gatatacttg aggcaccaga gattgacctg 62460
gatgtggctc tcagaacttc tgttattgcc aaagctggtg aagatgtaca agtgttgatt 62520
ccctttaaag gcagacctcc acctactgtc acatggagaa aagatgagaa gaatcttggc 62580
agtgatgcca gatacagcat tgaaaacact gattcatcct cattactcac cattcctcaa 62640
gttactcgca atgatacagg aaaatatatt ctcacaatag aaaatggagt tggtgaacct 62700
aagtcttcaa ctgtgagtgt taaagtgctt gacacaccag ctgcctgcca gaaactacag 62760
gttaaacatg tttctcgagg cacagtcact ttgctctggg atcctcctct cattgatgga 62820
ggatctccaa taattaatta tgtcattgaa aagagagatg ccaccaagag aacatggtct 62880
gtcgtgtcac acaaatgttc tagcacatcc ttcaagctaa tagatttgtc ggagaagact 62940
ccattcttct tcagagttct tgcagaaaat gaaattggaa ttggggaacc ctgtgaaact 63000
acagagccag tgaaggctgc tgaagtacca gctcctatac gtgatctctc aatgaaagac 63060
tcaacaaaga catctgtcat cctcagctgg accaaacctg actttgatgg tggtagcgtc 63120
atcacagaat atgttgtaga aaggaaaggt aaaggtgaac agacgtggtc ccacgctggc 63180
ataagtaaga catgtgaaat tgaggttagc caacttaagg agcagtcagt cctggagttc 63240
agagtgtttg ccaaaaatga gaaaggactg agtgatcctg tcactattgg gccaattaca 63300
gtgaaagaac ttattattac acctgaagtt gacctgtcag atatccctgg ggcacaagtc 63360
actgtgagaa ttgggcacaa tgtgcacctt gaattacctt ataagggaaa acccaaacca 63420
tccatcagtt ggctgaaaga tggcttgcca ctgaaagaaa gtgaatttgt tcgcttcagt 63480
aaaactgaaa acaaaattac tttgagtatt aagaatgcca agaaggagca tggaggaaaa 63540
tacactgtta ttcttgataa tgcagtgtgt agaattgcag tccccattac agtcatcacc 63600
cttggcccac catcaaagcc caaaggaccc attcgatttg atgaaatcaa ggctgatagt 63660
gtcatcctgt catgggatgt acctgaagat aatggaggag gagaaattac ttgttacagc 63720
atcgagaagc gggaaacttc acaaactaac tggaagatgg tgtgttcaag tgttgccaga 63780
acgactttca aagttcctaa tctagtcaaa gatgctgaat accagtttag agtgagagca 63840
gaaaacagat acggagtcag ccaaccactt gtctcaagca ttattgtggc aaaacaccag 63900
ttcaggattc ctggtccccc aggaaagcca gttatataca atgtgacttc tgatggcatg 63960
tcactaactt gggatgctcc agtttatgat ggtggttcag aagttactgg attccatgtt 64020
gaaaagaaag aaagaaatag catcctctgg caaaaagtta atacatcacc aatctctgga 64080
agagaatata gagccactgg actggtagaa ggtctggatt accaattccg tgtatatgct 64140
gaaaattctg ctggcctaag ctcacctagt gacccaagca aatttacctt agctgtttct 64200
ccagtagacc cacctggcac tcctgactac attgatgtca cccgggaaac catcacactt 64260
aaatggaacc caccattgcg tgatggaggc agtaagattg tgggctatag cattgagaaa 64320
cggcaaggaa atgaacgctg ggtgagatgc aactttactg acgtcagtga atgtcagtac 64380
acagttacag gactcagtcc tggggatcgc tatgagttca gaataattgc aagaaatgct 64440
gttggaacta taagcccgcc ctcacagtct tctggcatta ttatgacaag agatgaaaat 64500
gttccaccaa tagtagagtt tggccctgaa tactttgatg gtctcattat taagtccgga 64560
gagagcctta gaattaaagc tttggtacaa ggaagaccag tgcctcgagt aacttggttc 64620
aaagatggag tggaaatcga aaagaggatg aatatggaaa taaccaacgt acttggatcc 64680
accagcctat ttgttagaga tgctactcgg gaccatcgtg gtgtatacac agtggaagcc 64740
aaaaatgcat ctggttctgc aaaagcagaa attaaagtga aagtacaaga tacaccagga 64800
aaagtagttg ggccaataag attcaccaat attactgggg agaagatgac tctgtggtgg 64860
gatgccccac tcaatgacgg ttgtgctccc ataacccact acatcattga aaaacgggaa 64920
accagcagac ttgcctgggc actaattgag gataaatgtg aagcccaaag ttacactgcc 64980
attaaactaa taaacggcaa tgaataccaa ttccgtgttt ctgcagttaa caagtttggt 65040
gttggcaggc cacttgattc tgatccagtg gttgctcaaa tacaatatac tgttcctgat 65100
gcccctggca ttccagaacc tagcaacata acaggcaaca gcattaccct gacatgggca 65160
aggccagaat cagatggtgg cagtgaaatt caacagtata tccttgaaag aagagaaaag 65220
aaaagcacaa gatgggtaaa agtgatcagc aaacgaccaa tctctgaaac aagattcaaa 65280
gtcactggtc tgacagaagg caatgagtat gaattccatg tcatggctga aaatgctgca 65340
ggagttggac ctgcaagtgg catctcaaga ctcattaaat gtagagagcc cgtcaaccca 65400
ccaggtcctc ccacagtggt caaagtaaca gacacatcaa agacaactgt gagcttagaa 65460
tggtccaaac cagtgtttga tggtggcatg gaaataattg ggtatattat tgaaatgtgt 65520
aagaccgact taggagactg gcacaaggtg aatgcagagg catgtgtgaa aacaagatat 65580
 
acagtcactg atctacaagc aggtgaagaa tacaaattcc gagttagtgc tatcaatggt 65640
gctggaaaag gcgacagctg tgaagtgact ggcacaatta aagcagttga ccggttaaca 65700
gctcctgagt tagacataga tgcaaacttc aaacagactc atgttgttag agctggggcc 65760
agtattcgcc tcttcattgc ctaccaaggt agacctactc ctacagctgt gtggagcaaa 65820
ccagactcta accttagcct tcgggctgat atccatacaa cagattcctt cagcaccctc 65880
actgtggaaa actgcaacag aaatgatgca gggaaatata cccttactgt ggaaaacaac 65940
agtggtagta agtcaatcac attcaccgtg aaagtgctag acactccagg cccacctggc 66000
ccaattacct tcaaagatgt gacccgggga tctgctacat tgatgtggga tgcccctctt 66060
cttgacggtg gtgcccgaat ccatcattat gtggtagaga aacgagaggc aagtcgccgt 66120
agttggcagg ttatcagtga aaaatgcact cgtcagatct tcaaggtcaa tgacctggcc 66180
gaaggtgttc cgtactattt ccgtgtttct gcagtaaatg agtatggtgt tggtgagccc 66240
tatgaaatgc cagaaccaat tgtagccacg gaacagcctg ctccacctag gagacttgat 66300
gttgttgata ctagcaaatc ctccgcagtc ttagcttggc ttaaacctga ccacgatgga 66360
ggcagccgga tcactggcta cctgcttgaa atgagacaaa agggatctga cctctgggtt 66420
gaagctggtc acaccaaaca gctaactttc acagtagagc gtcttgttga gaaaactgaa 66480
tatgaattcc gtgtgaaggc caagaatgat gctggctata gtgaacccag agaagccttc 66540
tcttctgtca tcattaagga gcctcaaatc gagcccactg ctgacctcac tggaattacc 66600
aatcagctta taacttgcaa agcaggaagc ccatttacca ttgacgtacc aatcagtggt 66660
cgtcctgccc ccaaagtaac atggaaactg gaagaaatga gacttaaaga gacagatcga 66720
gtgagcatta caacaacaaa agacagaacc acactgactg taaaggacag catgagaggt 66780
gactctggaa gatacttctt gaccctggaa aatacagctg gtgttaaaac atttagcgtc 66840
acagttgtgg tcattggaag gccaggtcca gtaaccggcc ccattgaggt ctcatctgtc 66900
tcagctgaat cgtgtgtcct gtcatgggga gaacctaaag atggaggagg cactgaaatt 66960
actaattaca tagttgaaaa gcgtgaatcg ggtacaacag cttggcagct tgtcaattcc 67020
agtgtcaagc gcactcaaat taaagtcact catctcacaa aatacatgga atattctttc 67080
cgtgtcagtt cagagaacag atttggtgtc agcaaacctc tagaatcagc accaataatt 67140
gctgaacatc catttgtccc accaagcgct cctaccagac ctgaggtcta ccatgtgtct 67200
gccaatgcca tgtctattcg ttgggaagaa ccctaccacg atggtggcag taaaatcatt 67260
ggctactggg ttgagaagaa agaacgtaat acaattcttt gggtgaaaga aaacaaagtg 67320
ccatgcttag agtgcaacta caaagtaact ggtttagtag aaggactgga atatcagttc 67380
agaacttatg cactcaatgc tgcaggtgtt agcaaggcca gcgaagcttc aagacctata 67440
atggctcaaa atccagttga tgcaccaggc agaccagagg tgacagatgt cacaagatca 67500
acagtatcac tgatttggtc tgccccagcg tatgatggag gcagcaaggt tgtgggctac 67560
atcatagagc gtaagccagt cagtgaggta ggagatggtc gctggctgaa gtgcaactac 67620
accattgtat ctgacaattt cttcaccgtg actgctctca gtgaaggaga cacttatgag 67680
ttccgtgtgt tagccaagaa tgcagcaggc gtaattagca aagggtctga atctacaggc 67740
cctgtcactt gccgagatga atacgctcca cccaaagccg aactggatgc ccgattacac 67800
ggtgatctgg ttaccatcag agcaggttct gatcttgttc tggatgctgc agttggtggc 67860
aaacctgaac ccaaaattat ctggaccaaa ggagacaagg agctagatct ctgtgaaaaa 67920
gtctctttgc agtatactgg caaacgagca actgctgtga tcaagttctg tgacagaagt 67980
gacagtggaa aatacacttt aacagtgaaa aatgccagcg ggaccaaggc cgtgtctgtc 68040
atggtcaaag tgcttgattc ccctggccca tgtggaaagc tcaccgtcag cagagtaaca 68100
caggagaagt gcactttagc ctggagcctt ccgcaggaag acggaggagc agaaatcact 68160
cactacatcg tggaaagacg cgagactagc aggctcaact gggtgattgt tgaaggcgaa 68220
tgcccaaccc tatcctatgt cgttaccagg ctcatcaaga acaatgagta catattccga 68280
gtgagggcag taaacaaata tggccctggt gtgcctgttg aatcagagcc aattgtagcc 68340
agaaactcat tcactattcc atcaccaccc ggcatacctg aagaagttgg gactggcaaa 68400
gagcatatca tcattcagtg gacaaaacct gaatctgatg gtggcaatga aatcagcaac 68460
tacctagtag acaaacgtga gaaggagagc ctgcgctgga cacgtgtcaa caaagactat 68520
gtggtgtatg ataccaggct gaaggtgacc agcctgatgg agggttgtga ttaccagttc 68580
cgggtgaccg cagtgaatgc agctggtaac agtgagccca gcgaacgttc caacttcatc 68640
tcatgcagag aaccatcata tacccctgga ccaccttctg ctccaagagt tgtggatacc 68700
accaaacaca gcattagttt ggcatggacc aaacccatgt acgatggtgg tactgacatt 68760
gtaggatatg ttctggaaat gcaagagaag gacactgatc agtggtaccg agtgcatacc 68820
aatgccacaa taagaaatac tgaattcact gtgccagacc ttaaaatggg ccagaaatat 68880
tccttcagag ttgctgccgt gaacgtgaag ggtatgagcg aatacagcga atcaattgct 68940
gaaattgagc ccgtggaaag aatagaaata ccagatcttg agcttgcaga tgatctaaag 69000
aagactgtga ccatcagggc tggggcctcc ttgcgcttga tggtgtctgt atctggaaga 69060
ccacctcctg tcataacgtg gagcaagcag ggcattgacc ttgcaagccg ggcaattatt 69120
gacaccactg agagctactc attgctaata gtggacaaag ttaatcggta cgatgctgga 69180
aaatacacaa ttgaagctga aaaccaatct ggcaagaaat cagcaacagt ccttgttaaa 69240
gtctatgata ctcctggtcc ctgtccttca gtgaaagtta aggaagtatc aagagattct 69300
 
gtgactataa cttgggaaat tcccacgatt gatggtggag ctccaatcaa caattacatc 69360
gttgagaagc gtgaagctgc tatgagagca ttcaaaacag taactaccaa atgcagcaag 69420
acactttaca gaatttctgg acttgtagaa ggaaccatgc actatttcag agtgctgcca 69480
gaaaatattt atggcattgg agaaccttgt gaaacatctg atgcagtact ggtctcagaa 69540
gtgcctttgg tgcctgcaaa gctagaagtg gtcgatgtca ccaaatccac tgttaccctt 69600
gcctgggaaa aaccactcta cgatggtggt agccgactca ctggatatgt tctcgaggcc 69660
tgcaaagctg gcacagagag atggatgaag gttgtcacct taaaacccac agtcctagag 69720
cacactgtta cttccttaaa tgaaggtgaa caatacttat ttagaataag ggcacaaaat 69780
gagaaaggtg tgtcagaacc aagagagact gtcacagccg tgactgtaca agacctcaga 69840
gtgttgccaa caatcgatct ttctacaatg cctcagaaga ccatccatgt cccagctggc 69900
agaccagtag agctggtgat acctattgct ggccgtccac ctcctgctgc ttcctggttc 69960
tttgctggtt ctaaactgag agaatcagag cgtgtcacag ttgaaactca cactaaagta 70020
gctaaattaa ccatccgtga aaccactatc agagatactg gagaatacac acttgaattg 70080
aagaatgtta ccggaactac ttcagaaacc attaaagtta tcattcttga caagcctggt 70140
ccaccaacag gacctattaa gattgatgaa attgatgcta catcaattac catttcctgg 70200
gaaccacctg aattggacgg tggtgctcca ctgagtggtt atgtggtaga acaacgtgac 70260
gctcatcgtc caggatggct gcccgtttct gaatcagtga ctaggtccac gtttaagttt 70320
accagactca ccgaaggaaa tgagtatgtg ttccgtgtgg ctgcaacaaa ccgcttcggg 70380
attggctctt acttgcagtc tgaggtcata gagtgtcgca gcagcatccg tattcctgga 70440
cccccagaaa cattacagat atttgatgtt tcccgtgatg gcatgacact tacttggtac 70500
ccaccagagg atgacggtgg ctcccaagtg actggatata ttgtggagcg caaagaagtg 70560
agagcagatc gatgggtccg tgtaaataaa gtacctgtga caatgacacg gtaccgctcc 70620
actggcctta ctgaaggctt agaatatgaa caccgtgtca cagccattaa tgcaagaggg 70680
tctgggaaac caagtcgtcc ttccaaaccc atcgttgcca tggatccaat tgctcctcca 70740
ggaaagccac aaaacccaag agttactgat acaacaagga catcagtctc cctggcctgg 70800
agtgttccag aagatgaagg aggatctaaa gtcacaggct acttgattga aatgcaaaaa 70860
gtagatcaac atgaatggac caagtgtaac accactccaa ccaagattcg agagtatact 70920
ctaacacacc tacctcaggg tgcagaatac aggttccgcg tcctagcttg taatgctggt 70980
ggacctggtg agcctgctga ggtaccagga acagtcaaag tcactgaaat gcttgaatat 71040
cctgattatg aacttgatga aagataccaa gaaggtatct ttgtaaggca aggtggcgtc 71100
atcagactta ccataccaat caaaggaaaa ccattcccaa tatgtaaatg gaccaaggaa 71160
ggccaggata ttagtaagcg tgccatgatt gcaacatctg aaacacacac tgagcttgtg 71220
atcaaagaag cagacagggg tgattctggc acttatgacc tggttctgga aaataaatgt 71280
ggcaagaagg ctgtctacat caaggtcagg gtgataggaa gtcccaacag tccagaaggg 71340
ccactggaat atgatgacat ccaagtccgc tctgtgaggg tcagctggag acctcctgct 71400
gatgatggtg gtgctgacat cttaggctac atcctcgaga gacgagaagt gcctaaagcc 71460
gcctggtata ccattgattc cagagtccga ggtacatctc tggtggtaaa aggcctcaaa 71520
gagaatgtag aataccattt ccgtgtttca gcagaaaacc agtttggcat aagcaaaccc 71580
ttgaaatctg aggaaccagt cacaccaaaa acaccattga atcctccaga acctccaagc 71640
aatcctccag aagtactcga tgtaaccaag agttctgtta gcttgtcctg gtcccggccc 71700
aaagatgatg gtggttctag agtcacaggc tactacatcg aacgcaaaga gacatccact 71760
gacaagtggg tcagacacaa caagactcag atcaccacca caatgtacac tgtcacaggg 71820
cttgttcccg atgctgagta tcagttccgc atcatcgcac agaatgatgt tggcctgagt 71880
gagaccagcc ctgcttctga accagttgtt tgcaaagatc catttgataa accaagccaa 71940
ccaggagaac ttgagattct ttcaatatcc aaagatagtg tcactctaca gtgggagaaa 72000
cctgaatgtg atggtggtaa agaaattctt ggatactggg ttgaatatag acagtctgga 72060
gacagtgcct ggaagaagag caataaggaa cgtattaagg acaagcaatt cacaatagga 72120
ggtttgctgg aagctactga gtatgaattc agggtttttg ctgagaatga gactgggctg 72180
agcagacctc gcagaactgc tatgtctata aagactaaac tcacatctgg agaggcccca 72240
ggaatacgca aagaaatgaa ggatgttacc acaaaattgg gtgaagctgc tcaactctca 72300
tgccagattg ttggaaggcc tcttcctgac attaaatggt acagatttgg taaagagctc 72360
atacaaagcc ggaaatacaa aatgtcttca gatggacgca cacacactct tacagtaatg 72420
acagaggaac aggaagatga aggtgtttat acctgcatag ccaccaatga ggttggagaa 72480
gtagaaacca gtagtaagct tctcctgcaa gcaacaccgc agttccatcc tggttaccca 72540
ctgaaagaga aatattatgg agctgtgggt tccacacttc ggcttcatgt tatgtacatt 72600
ggtcgtccag tacctgccat gacttggttc catggtcaga aacttttgca aaactcagaa 72660
aacattacta ttgaaaacac tgagcactat actcatcttg tcatgaagaa tgtccaacgt 72720
aagactcatg ctgggaaata caaagtccag ctcagcaatg tttttggaac agttgatgcc 72780
atccttgatg tggaaataca agataaacca gacaaaccta caggaccaat tgtgatcgaa 72840
gctctattga agaactccgc agtgatcagc tggaaaccac ccgcagatga cggaggctcc 72900
tggatcacca actatgtggt ggaaaaatgt gaggccaagg agggggctga atggcaattg 72960
gtgtcttcag ccatctcagt gacaacctgt agaattgtga acctcacaga aaatgctggc 73020
 
tattacttcc gggtttcagc tcagaacact ttcggcatca gtgaccctct agaagtgtcc 73080
tcagttgtga tcattaagag tccatttgaa aagccaggtg ctcctggcaa accaactatt 73140
actgctgtca caaaagattc ttgtgttgtg gcctggaagc cacctgccag tgatggaggt 73200
gcaaagatta gaaattacta ccttgagaag cgtgagaaga agcagaataa atggatttct 73260
gtgacaacag aagaaattcg agaaactgtc ttttcagtga aaaaccttat tgaaggtctt 73320
gaatacgagt ttcgtgtgaa atgtgaaaat ctaggtgggg aaagtgaatg gagtgaaata 73380
tcagaaccca tcactcccaa atctgatgtc ccaattcagg caccacactt taaagaggaa 73440
ctgagaaatc taaatgtcag atatcagagc aatgctacct tggtctgcaa agtgactggt 73500
catccaaaac ctatcgtcaa atggtacaga caaggcaaag aaatcattgc agatggatta 73560
aaatatagga ttcaagaatt taagggtggc taccaccagc tcatcattgc aagtgtcaca 73620
gatgatgatg ccacagttta ccaagtcaga gctaccaacc aagggggatc tgtgtctggc 73680
actgcctcct tggaagtgga agttccagct aagatacact tacctaaaac tcttgaaggc 73740
atgggagcag ttcatgctct ccgaggtgaa gtggtcagca tcaagattcc tttcagtggc 73800
aaaccagatc ctgtgatcac ctggcagaaa ggacaagatc tcattgacaa taatggccac 73860
taccaagtta ttgtcacaag atccttcaca tcacttgttt tccccaatgg ggtagagaga 73920
aaagatgctg gtttctatgt ggtctgtgct aaaaacagat ttggaattga tcagaagaca 73980
gttgaactgg atgtggctga tgttcctgac ccacccagag gagtcaaagt tagtgatgcc 74040
tcacgagatt ctgtcaactt aacatggact gagccagcct ctgatggtgg cagcaaaatc 74100
accaactaca ttgttgaaaa atgtgcaact actgcagaaa gatggctccg tgtaggacag 74160
gcccgagaaa cacgttatac cgtgatcaac ttatttggaa aaacaagtta ccagttccgg 74220
gtaatagctg aaaataaatt tggtctgagc aagccttcag agccttcaga accaaccata 74280
accaaagaag ataagaccag agctatgaac tatgatgaag aggtagatga aaccagggaa 74340
gtctccatga ctaaagcatc tcactcttca accaaggaac tctatgagaa atatatgatt 74400
gctgaagatc ttgggcgtgg tgagtttgga attgtccatc gttgtgttga aacatcctca 74460
aagaagacat acatggccaa atttgttaaa gtcaaaggga ctgatcaggt tttggtaaag 74520
aaggaaattt ccattctgaa tattgctagg catagaaaca tcttacacct ccatgaatca 74580
tttgaaagca tggaagaatt agttatgatc tttgagttta tatcaggact tgacatattt 74640
gagcgcatta acacaagtgc ttttgaactt aatgaaagag aaattgtaag ttatgttcac 74700
caggtctgtg aagcacttca gtttttacac agtcataata ttggacactt tgacattaga 74760
ccagaaaata tcatttacca aaccagaaga agctctacca ttaaaatcat agaatttggt 74820
caagcccgtc agctgaaacc aggggacaac ttcaggcttc tattcactgc cccagaatac 74880
tatgcacctg aagtccacca gcatgatgtt gtcagcacag ccacagacat gtggtcactt 74940
ggaacactgg tatatgtgct attgagtggt atcaacccat tcctggctga aactaaccaa 75000
cagatcattg agaatatcat gaatgctgaa tatactttcg atgaggaagc attcaaagag 75060
attagcattg aagccatgga ttttgttgac cggttgttag tgaaagagag gaaatctcgc 75120
atgacagcat cggaggctct ccagcaccca tggttgaagc agaagataga aagagtcagt 75180
actaaagtta tcagaacatt aaaacaccgg cgttattacc acaccctgat caagaaagac 75240
ctcaacatgg ttgtgtcagc agcccggatc tcctgtggtg gtgcaattcg atctcagaag 75300
ggagtgagtg ttgctaaagt taaagtggca tccattgaaa ttggcccagt ttctgggcag 75360
ataatgcatg cagttggtga agaaggagga catgtcaaat atgtatgcaa aattgaaaat 75420
tatgatcagt ctacccaagt gacttggtac tttggtgtcc gacagctgga gaacagtgag 75480
aaatacgaaa tcacctacga agatggagtg gccatcctct atgtcaaaga cattaccaaa 75540
ttagatgatg gtacctacag atgcaaagta gtcaatgact atggtgaaga cagttcttat 75600
gcagagctat ttgttaaagg tgtgagagaa gtctatgact attactgccg tagaaccatg 75660
aagaaaatta agcgcagaac agacacaatg agactcctgg aaaggccacc agaatttacc 75720
ctgcctctct ataataagac agcttatgta ggtgaaaatg tccggtttgg agtaactata 75780
actgtccacc cagagcctca tgtaacatgg tataaatcag gtcagaaaat caaaccaggt 75840
gacaatgaca agaagtacac atttgagtca gacaagggtc tttaccaatt aacaatcaac 75900
agtgtcacta cagatgatga cgctgaatat actgttgtgg caaggaacaa atatggtgaa 75960
gacagctgta aagcaaagct gacagtaacc ctacacccac ctccaacaga tagtacctta 76020
agacccatgt tcaaaaggtt actggcaaat gcagaatgcc aagaaggcca aagtgtctgc 76080
tttgagatca gagtgtctgg catcccccca ccaacattaa aatgggagaa agatggtcag 76140
ccactgtccc tcgggcctaa cattgaaatt atccatgaag gcttggatta ttatgctctg 76200
cacatcaggg acactttgcc tgaagacacg ggttattata gagtcacagc cactaacaca 76260
gctgggtcca ccagctgcca ggctcaccta caagtggaac gcctgaggta caagaaacag 76320
gaattcaaga gtaaggagga gcatgagcga cacgtacaaa aacaaattga caaaaccctc 76380
agaatggctg aaattctttc tggaactgaa agtgtaccac tgacacaggt agctaaagag 76440
gctctgagag aagctgctgt cctttataaa ccggctgtaa gcaccaagac tgtaaaaggg 76500
gaattcagac ttgagataga agaaaagaag gaggagagaa aactccggat gccttatgat 76560
gtaccagagc cacgcaagta taagcagact accatagaag aagaccaacg catcaagcag 76620
ttcgtgccca tgtctgacat gaagtggtat aaaaagatac gtgatcagta tgaaatgcct 76680
gggaaacttg acagagttgt acagaaacga cccaagcgca tccgcctttc aagatgggaa 76740
 
cagttctatg tgatgcctct tccacgcatt acagatcaat acagacctaa atggcgtatt 76800
cctaaactgt cccaagatga tcttgagata gtgagaccag cccgccggcg tacaccttct 76860
cctgattatg acttttacta ccgacctaga agacgttctc ttggggacat ctctgatgaa 76920
gaattactcc tccccattga tgactactta gcaatgaaaa gaacagagga agagaggctg 76980
cgtcttgaag aagagcttga gttaggtttt tcagcttcac ccccaagtcg aagccctcca 77040
cactttgagc tttctagcct acgttactct tcaccacaag ctcatgtcaa ggtggaggaa 77100
acaagaaaaa acttcaggta ttcaacctat cacatcccaa cgaaggctga agctagtaca 77160
agttatgcag aactgaggga acggcatgcc caggctgcgt acagacagcc aaagcaacgg 77220
caaagaatca tggctgagag ggaggatgaa gagttgcttc gcccagttac gaccacccag 77280
catctctcag aatacaaaag cgaacttgac ttcatgtcaa aggaggaaaa gtctagaaag 77340
aaatcaaggc gacaaagaga agtgacagaa ataacagaaa ttgaggaaga atacgaaatc 77400
tcaaaacatg ctcaaagaga atcatcctca tctgcgtcta gactactgag acgacggcgc 77460
tccctgtctc caacttatat tgagttaatg aggccagtgt ctgagctgat ccggtcacgt 77520
ccacaaccgg ctgaggaata cgaagatgac acagaaagaa ggtcacctac tccagagaga 77580
actcgcccac gatcccccag ccctgtgtct agtgagagat cactctcgag atttgagagg 77640
tctgcaagat ttgatatctt ttccaggtat gagtccatga aagctgcttt aaaaactcag 77700
aagacatcag aaaggaagta tgaagttttg agtcagcagc ctttcacact ggaccatgcc 77760
cctcgaatca cactgagaat gcgctcgcac agggtaccat gtggccaaaa tacacgtttt 77820
attttaaatg ttcagtctaa gccaactgcc gaggttaaat ggtaccacaa tggtgtggaa 77880
ctccaagaaa gcagtaagat tcattacacc aacacgagtg gagtcctcac cctggaaatt 77940
ctggactgtc atactgatga cagtggaacc taccgtgctg tgtgcaccaa ctacaagggc 78000
gaagcttctg actatgcaac gttggacgtg acaggagggg attataccac ctatgcttcc 78060
caacgcagag atgaagaggt ccccagatct gttttccctg agctgacaag aacagaggcg 78120
tatgctgttc catcatttaa gaaaacatct gagatggaag cttcgtcttc tgtcagggaa 78180
gtgaaatcac agatgacgga gacaagggaa agtctctcct catatgaaca ctctgcatct 78240
gcagaaatga aaagtgctgc attagaagaa aagtcactgg aagaaaaatc cacaaccaga 78300
aagatcaaga cgactttggc agcaagaatt ctaacaaagc cacggtccat gaccgtctac 78360
gagggcgagt ctgcaaggtt ttcttgtgac accgatggtg agccggtacc aactgtgacc 78420
tggctgcgta aaggacaagt gctaagtact tctgcccgcc accaagtgac caccacaaag 78480
tacaaatcaa cctttgagat ctcttcagtc caggcttccg atgagggcaa ttacagcgtg 78540
gtggtagaaa acagtgaagg gaaacaagaa gcagagttca ctctgactat tcaaaaggcc 78600
agggtaactg aaaaggctgt gacatcacca ccaagagtca aatccccaga gcctcgggtg 78660
aaatccccag aagcagttaa gtctccaaaa cgagtgaaat ctccagaacc ttctcacccg 78720
aaagccgtat cacccacaga gacaaaacca acaccaagag agaaagttca gcacctccca 78780
gtctctgccc caccaaagat tactcagttc ctgaaagcag aagcttctaa agagattgca 78840
aaactgacct gtgtggttga aagcagtgta ttaagggcaa aagaggtcac ctggtataaa 78900
gatggcaaga aactgaagga aaatgggcat ttccagtttc attattcagc agatggtacc 78960
tatgagctca aaatcaataa cctcactgaa tctgatcaag gagaatatgt ttgtgagatt 79020
tctggtgaag gtggaacgtc taaaaccaac ttacaattta tggggcaagc ctttaagagt 79080
atccatgaga aggtatcaaa aatatcagaa actaagaaat cagatcagaa aaccactgag 79140
tcaacagtaa ccagaaaaac tgaaccaaaa gctcctgaac caatttcctc aaaaccagta 79200
attgttactg ggttgcagga tacaactgtt tcttcagaca gtgttgctaa atttgcagtt 79260
aaggctactg gagaaccccg gccaactgcc atctggacaa aagatggaaa ggccattaca 79320
caaggaggta aatataaact ctctgaagac aagggagggt tcttcttaga aattcataag 79380
actgatactt ctgacagtgg actttatact tgtacagtaa aaaattcagc tggatctgtg 79440
tcctctagct gcaaattaac aataaaagct ataaaagata ctgaggcaca gaaagtctct 79500
acacaaaaga cttctgaaat tacacctcag aagaaagctg ttgtccaaga ggaaatttcc 79560
caaaaagccc taaggtctga agaaattaag atgtcagagg caaaatctca agaaaagtta 79620
gccctcaaag aggaagcttc aaaggttctg atttctgaag aagtcaagaa atcagcagca 79680
acctccctgg aaaaatccat tgtccatgag gaaatcacta aaacatcaca ggcatcagaa 79740
gaagtcagaa ctcatgctga gattaaagca ttttctactc agatgagcat aaacgaaggt 79800
caaagactgg ttttaaaagc caacattgct ggtgccactg atgtgaaatg ggtactgaat 79860
ggcgtagagc ttaccaactc tgaggagtac cgatatggtg tctcaggcag cgatcagacc 79920
ctaaccatca agcaagccag tcacagagat gaaggaatcc tcacctgcat aagcaaaacc 79980
aaggaaggaa tcgtcaagtg tcagtatgat ttgacactga gcaaagaact ctcagatgct 80040
ccagccttca tctcacagcc tagatctcaa aatattaatg aaggacaaaa tgttctcttt 80100
acttgtgaaa tcagtggcga gccatcccct gaaatcgaat ggtttaaaaa caacctgcca 80160
atttctattt cttcaaatgt cagcataagc cgctccagaa atgtatactc ccttgaaatc 80220
cgaaatgcat cagtcagcga cagtggaaag tacacaatta aggccaaaaa tttccgtggc 80280
cagtgttcag ctacagcttc cttaatggtc cttcctctag ttgaagaacc ttccagagag 80340
gtagtattga gaacaagtgg tgacacaagc ttgcaaggaa gcttctcgtc tcagtcagtc 80400
caaatgtctg cctccaagca ggaggcctcc ttcagcagtt tcagcagcag cagtgctagc 80460
 
agcatgactg agatgaaatt tgcaagcatg tctgcccaaa gcatgtcctc catgcaagag 80520
tcctttgtag aaatgagttc cagcagcttt atgggaatat ctaatatgac acaactggaa 80580
agctcaacta gtaaaatgct taaagcaggc ataagaggaa ttccgcctaa aattgaagct 80640
cttccatctg atatcagcat tgatgaaggc aaagttctaa cagtagcctg tgctttcacg 80700
ggtgagccta ccccagaagt aacatggtcc tgtggtggaa gaaaaatcca cagtcaagaa 80760
caggggaggt tccacattga aaacacagat gacctgacaa ccctgatcat catggacgta 80820
cagaaacaag atggtggact ttataccctg agtttaggga atgaatttgg atctgactct 80880
gccactgtga atatacatat tcgatccatt taagagggcc tgtgccctta tactctacac 80940
tcattcttaa cttttcgcaa acgtttcaca cggactaatc tttctgaact gtaaatattt 81000
aaagaaaaaa agtagttttg tatcaaccta aatgagtcaa agttcaaaaa tattcatttc 81060
aatcttttca taattgttga cctaagaata taatacattt gctagtgaca tgtacatact 81120
gtatatagcc ggattaacgg ttataaagtt ttgtaccatt tattttatga cattttacaa 81180
tgtaagtttt gaaactaact gttggtagga gaaagtttct tatggaacga ataccctgct 81240
caacatttaa tcaatctttg tgcctcaaca tactgttgat gtctaagtat gcctcagtgg 81300
gttgagaaaa tccccattga agatgtcctg tccacctaaa agagaatgat gctgtgcata 81360
tcacttgata tgtgcaccaa tacctactga atcagaaatg taaggcattg gtgatgtttg 81420
catttaccct cctgtaagca acactttaac gtcttacatt ttctctgatg atgtcacaca 81480
aaattatcat gacaaatatt accagagcaa agtgtaacgg ccaacacttt gttcgctcat 81540
tttacgctgt ctctgacata aggagtgcct gaatagcttg gaaaagtaac atctcctggc 81600
catcccttca tttaaccaag ctattcaagt attcctatgc cagagcagtg ccaactcttg 81660
gaggtcccag agtgcagcca atgcctttgt gtggtagttc taaattttaa ttgcacctga 81720
aaaacctggg cacctaagca atgagccaca gcaaaaagta aagaacaaca acaaaataaa 81780
gctgttgtta aattttaaac aatattacta attgcccaaa atgtcaattt gatgtagttc 81840
ttttcatgca agtataaatt caattgttag ttataattgt tggacctcct tgagatagta 81900
acaacaaaat aaagcaagct atctgcacct caaaaaaaaa 81940
<210> 2
   <211> 26926
   <212> PRT
   <213> Homo sapiens
<400> 2 
Met Thr Thr Gln Ala Pro Thr Phe Thr Gln Pro Leu Gln Ser Val Val
1               5                   10                  15
Val Leu Glu Gly Ser Thr Ala Thr Phe Glu Ala His Ile Ser Gly Phe
            20                  25                  30
Pro Val Pro Glu Val Ser Trp Phe Arg Asp Gly Gln Val Ile Ser Thr
        35                  40                  45
Ser Thr Leu Pro Gly Val Gln Ile Ser Phe Ser Asp Gly Arg Ala Lys
    50                  55                  60
Leu Thr Ile Pro Ala Val Thr Lys Ala Asn Ser Gly Arg Tyr Ser Leu
65                  70                  75                  80
Lys Ala Thr Asn Gly Ser Gly Gln Ala Thr Ser Thr Ala Glu Leu Leu
                85                  90                  95
Val Lys Ala Glu Thr Ala Pro Pro Asn Phe Val Gln Arg Leu Gln Ser
            100                 105                 110
Met Thr Val Arg Gln Gly Ser Gln Val Arg Leu Gln Val Arg Val Thr
        115                 120                 125
Gly Ile Pro Thr Pro Val Val Lys Phe Tyr Arg Asp Gly Ala Glu Ile
    130                 135                 140
Gln Ser Ser Leu Asp Phe Gln Ile Ser Gln Glu Gly Asp Leu Tyr Ser
145                 150                 155                 160
Leu Leu Ile Ala Glu Ala Tyr Pro Glu Asp Ser Gly Thr Tyr Ser Val
                165                 170                 175
Asn Ala Thr Asn Ser Val Gly Arg Ala Thr Ser Thr Ala Glu Leu Leu
            180                 185                 190
Val Gln Gly Glu Glu Glu Val Pro Ala Lys Lys Thr Lys Thr Ile Val
        195                 200                 205
Ser Thr Ala Gln Ile Ser Glu Ser Arg Gln Thr Arg Ile Glu Lys Lys
    210                 215                 220
 
Ile Glu Ala His Phe Asp Ala Arg Ser Ile Ala Thr Val Glu Met Val
225                 230                 235                 240
Ile Asp Gly Ala Ala Gly Gln Gln Leu Pro His Lys Thr Pro Pro Arg
                245                 250                 255
Ile Pro Pro Lys Pro Lys Ser Arg Ser Pro Thr Pro Pro Ser Ile Ala
            260                 265                 270
Ala Lys Ala Gln Leu Ala Arg Gln Gln Ser Pro Ser Pro Ile Arg His
        275                 280                 285
Ser Pro Ser Pro Val Arg His Val Arg Ala Pro Thr Pro Ser Pro Val
    290                 295                 300
Arg Ser Val Ser Pro Ala Ala Arg Ile Ser Thr Ser Pro Ile Arg Ser
305                 310                 315                 320
Val Arg Ser Pro Leu Leu Met Arg Lys Thr Gln Ala Ser Thr Val Ala
                325                 330                 335
Thr Gly Pro Glu Val Pro Pro Pro Trp Lys Gln Glu Gly Tyr Val Ala
            340                 345                 350
Ser Ser Ser Glu Ala Glu Met Arg Glu Thr Thr Leu Thr Thr Ser Thr
        355                 360                 365
Gln Ile Arg Thr Glu Glu Arg Trp Glu Gly Arg Tyr Gly Val Gln Glu
    370                 375                 380
Gln Val Thr Ile Ser Gly Ala Ala Gly Ala Ala Ala Ser Val Ser Ala
385                 390                 395                 400
Ser Ala Ser Tyr Ala Ala Glu Ala Val Ala Thr Gly Ala Lys Glu Val
                405                 410                 415
Lys Gln Asp Ala Asp Lys Ser Ala Ala Val Ala Thr Val Val Ala Ala
            420                 425                 430
Val Asp Met Ala Arg Val Arg Glu Pro Val Ile Ser Ala Val Glu Gln
        435                 440                 445
Thr Ala Gln Arg Thr Thr Thr Thr Ala Val His Ile Gln Pro Ala Gln
    450                 455                 460
Glu Gln Val Arg Lys Glu Ala Glu Lys Thr Ala Val Thr Lys Val Val
465                 470                 475                 480
Val Ala Ala Asp Lys Ala Lys Glu Gln Glu Leu Lys Ser Arg Thr Lys
                485                 490                 495
Glu Ile Ile Thr Thr Lys Gln Glu Gln Met His Val Thr His Glu Gln
            500                 505                 510
Ile Arg Lys Glu Thr Glu Lys Thr Phe Val Pro Lys Val Val Ile Ser
        515                 520                 525
Ala Ala Lys Ala Lys Glu Gln Glu Thr Arg Ile Ser Glu Glu Ile Thr
    530                 535                 540
Lys Lys Gln Lys Gln Val Thr Gln Glu Ala Ile Met Lys Glu Thr Arg
545                 550                 555                 560
Lys Thr Val Val Pro Lys Val Ile Val Ala Thr Pro Lys Val Lys Glu
                565                 570                 575
Gln Asp Leu Val Ser Arg Gly Arg Glu Gly Ile Thr Thr Lys Arg Glu
            580                 585                 590
Gln Val Gln Ile Thr Gln Glu Lys Met Arg Lys Glu Ala Glu Lys Thr
        595                 600                 605
Ala Leu Ser Thr Ile Ala Val Ala Thr Ala Lys Ala Lys Glu Gln Glu
    610                 615                 620
Thr Ile Leu Arg Thr Arg Glu Thr Met Ala Thr Arg Gln Glu Gln Ile
625                 630                 635                 640
Gln Val Thr His Gly Lys Val Asp Val Gly Lys Lys Ala Glu Ala Val
                645                 650                 655
Ala Thr Val Val Ala Ala Val Asp Gln Ala Arg Val Arg Glu Pro Arg
            660                 665                 670
Glu Pro Gly His Leu Glu Glu Ser Tyr Ala Gln Gln Thr Thr Leu Glu
        675                 680                 685
Tyr Gly Tyr Lys Glu Arg Ile Ser Ala Ala Lys Val Ala Glu Pro Pro
    690                 695                 700
Gln Arg Pro Ala Ser Glu Pro His Val Val Pro Lys Ala Val Lys Pro
705                 710                 715                 720
 
Arg Val Ile Gln Ala Pro Ser Glu Thr His Ile Lys Thr Thr Asp Gln
                725                 730                 735
Lys Gly Met His Ile Ser Ser Gln Ile Lys Lys Thr Thr Asp Leu Thr
            740                 745                 750
Thr Glu Arg Leu Val His Val Asp Lys Arg Pro Arg Thr Ala Ser Pro
        755                 760                 765
His Phe Thr Val Ser Lys Ile Ser Val Pro Lys Thr Glu His Gly Tyr
    770                 775                 780
Glu Ala Ser Ile Ala Gly Ser Ala Ile Ala Thr Leu Gln Lys Glu Leu
785                 790                 795                 800
Ser Ala Thr Ser Ser Ala Gln Lys Ile Thr Lys Ser Val Lys Ala Pro
                805                 810                 815
Thr Val Lys Pro Ser Glu Thr Arg Val Arg Ala Glu Pro Thr Pro Leu
            820                 825                 830
Pro Gln Phe Pro Phe Ala Asp Thr Pro Asp Thr Tyr Lys Ser Glu Ala
        835                 840                 845
Gly Val Glu Val Lys Lys Glu Val Gly Val Ser Ile Thr Gly Thr Thr
    850                 855                 860
Val Arg Glu Glu Arg Phe Glu Val Leu His Gly Arg Glu Ala Lys Val
865                 870                 875                 880
Thr Glu Thr Ala Arg Val Pro Ala Pro Val Glu Ile Pro Val Thr Pro
                885                 890                 895
Pro Thr Leu Val Ser Gly Leu Lys Asn Val Thr Val Ile Glu Gly Glu
            900                 905                 910
Ser Val Thr Leu Glu Cys His Ile Ser Gly Tyr Pro Ser Pro Thr Val
        915                 920                 925
Thr Trp Tyr Arg Glu Asp Tyr Gln Ile Glu Ser Ser Ile Asp Phe Gln
    930                 935                 940
Ile Thr Phe Gln Ser Gly Ile Ala Arg Leu Met Ile Arg Glu Ala Phe
945                 950                 955                 960
Ala Glu Asp Ser Gly Arg Phe Thr Cys Ser Ala Val Asn Glu Ala Gly
                965                 970                 975
Thr Val Ser Thr Ser Cys Tyr Leu Ala Val Gln Val Ser Glu Glu Phe
            980                 985                 990
Glu Lys Glu Thr Thr Ala Val Thr Glu Lys Phe Thr Thr Glu Glu Lys
        995                 1000                1005
Arg Phe Val Glu Ser Arg Asp Val Val Met Thr Asp Thr Ser Leu Thr
    1010                1015                1020
Glu Glu Gln Ala Gly Pro Gly Glu Pro Ala Ala Pro Tyr Phe Ile Thr
1025                1030                1035                1040
Lys Pro Val Val Gln Lys Leu Val Glu Gly Gly Ser Val Val Phe Gly
                1045                1050                1055
Cys Gln Val Gly Gly Asn Pro Lys Pro His Val Tyr Trp Lys Lys Ser
            1060                1065                1070
Gly Val Pro Leu Thr Thr Gly Tyr Arg Tyr Lys Val Ser Tyr Asn Lys
        1075                1080                1085
Gln Thr Gly Glu Cys Lys Leu Val Ile Ser Met Thr Phe Ala Asp Asp
    1090                1095                1100
Ala Gly Glu Tyr Thr Ile Val Val Arg Asn Lys His Gly Glu Thr Ser
1105                1110                1115                1120
Ala Ser Ala Ser Leu Leu Glu Glu Ala Asp Tyr Glu Leu Leu Met Lys
                1125                1130                1135
Ser Gln Gln Glu Met Leu Tyr Gln Thr Gln Val Thr Ala Phe Val Gln
            1140                1145                1150
Glu Pro Glu Val Gly Glu Thr Ala Pro Gly Phe Val Tyr Ser Glu Tyr
        1155                1160                1165
Glu Lys Glu Tyr Glu Lys Glu Gln Ala Leu Ile Arg Lys Lys Met Ala
    1170                1175                1180
Lys Asp Thr Val Val Val Arg Thr Tyr Val Glu Asp Gln Glu Phe His
1185                1190                1195                1200
Ile Ser Ser Phe Glu Glu Arg Leu Ile Lys Glu Ile Glu Tyr Arg Ile
                1205                1210                1215
 
Ile Lys Thr Thr Leu Glu Glu Leu Leu Glu Glu Asp Gly Glu Glu Lys
            1220                1225                1230
Met Ala Val Asp Ile Ser Glu Ser Glu Ala Val Glu Ser Gly Phe Asp
        1235                1240                1245
Leu Arg Ile Lys Asn Tyr Arg Ile Leu Glu Gly Met Gly Val Thr Phe
    1250                1255                1260
His Cys Lys Met Ser Gly Tyr Pro Leu Pro Lys Ile Ala Trp Tyr Lys
1265                1270                1275                1280
Asp Gly Lys Arg Ile Lys His Gly Glu Arg Tyr Gln Met Asp Phe Leu
                1285                1290                1295
Gln Asp Gly Arg Ala Ser Leu Arg Ile Pro Val Val Leu Pro Glu Asp
            1300                1305                1310
Glu Gly Ile Tyr Thr Ala Phe Ala Ser Asn Ile Lys Gly Asn Ala Ile
        1315                1320                1325
Cys Ser Gly Lys Leu Tyr Val Glu Pro Ala Ala Pro Leu Gly Ala Pro
    1330                1335                1340
Thr Tyr Ile Pro Thr Leu Glu Pro Val Ser Arg Ile Arg Ser Leu Ser
1345                1350                1355                1360
Pro Arg Ser Val Ser Arg Ser Pro Ile Arg Met Ser Pro Ala Arg Met
                1365                1370                1375
Ser Pro Ala Arg Met Ser Pro Ala Arg Met Ser Pro Ala Arg Met Ser
            1380                1385                1390
Pro Gly Arg Arg Leu Glu Glu Thr Asp Glu Ser Gln Leu Glu Arg Leu
        1395                1400                1405
Tyr Lys Pro Val Phe Val Leu Lys Pro Val Ser Phe Lys Cys Leu Glu
    1410                1415                1420
Gly Gln Thr Ala Arg Phe Asp Leu Lys Val Val Gly Arg Pro Met Pro
1425                1430                1435                1440
Glu Thr Phe Trp Phe His Asp Gly Gln Gln Ile Val Asn Asp Tyr Thr
                1445                1450                1455
His Lys Val Val Ile Lys Glu Asp Gly Thr Gln Ser Leu Ile Ile Val
            1460                1465                1470
Pro Ala Thr Pro Ser Asp Ser Gly Glu Trp Thr Val Val Ala Gln Asn
        1475                1480                1485
Arg Ala Gly Arg Ser Ser Ile Ser Val Ile Leu Thr Val Glu Ala Val
    1490                1495                1500
Glu His Gln Val Lys Pro Met Phe Val Glu Lys Leu Lys Asn Val Asn
1505                1510                1515                1520
Ile Lys Glu Gly Ser Arg Leu Glu Met Lys Val Arg Ala Thr Gly Asn
                1525                1530                1535
Pro Asn Pro Asp Ile Val Trp Leu Lys Asn Ser Asp Ile Ile Val Pro
            1540                1545                1550
His Lys Tyr Pro Lys Ile Arg Ile Glu Gly Thr Lys Gly Glu Ala Ala
        1555                1560                1565
Leu Lys Ile Asp Ser Thr Val Ser Gln Asp Ser Ala Trp Tyr Thr Ala
    1570                1575                1580
Thr Ala Ile Asn Lys Ala Gly Arg Asp Thr Thr Arg Cys Lys Val Asn
1585                1590                1595                1600
Val Glu Val Glu Phe Ala Glu Pro Glu Pro Glu Arg Lys Leu Ile Ile
                1605                1610                1615
Pro Arg Gly Thr Tyr Arg Ala Lys Glu Ile Ala Ala Pro Glu Leu Glu
            1620                1625                1630
Pro Leu His Leu Arg Tyr Gly Gln Glu Gln Trp Glu Glu Gly Asp Leu
        1635                1640                1645
Tyr Asp Lys Glu Lys Gln Gln Lys Pro Phe Phe Lys Lys Lys Leu Thr
    1650                1655                1660
Ser Leu Arg Leu Lys Arg Phe Gly Pro Ala His Phe Glu Cys Arg Leu
1665                1670                1675                1680
Thr Pro Ile Ser Asp Pro Thr Met Val Val Glu Trp Leu His Asp Gly
                1685                1690                1695
Lys Pro Leu Glu Ala Ala Asn Arg Leu Arg Met Ile Asn Glu Phe Gly
            1700                1705                1710
 
Tyr Cys Ser Leu Asp Tyr Gly Val Ala Tyr Ser Arg Asp Ser Gly Ile
1715 . 1720 1725
Ile Thr Cys Arg Ala Thr Asn Lys Tyr Gly Thr Asp His Thr Ser Ala
1730 1735 1740
Thr Leu Ile Val Lys Asp Glu Lys Ser Leu Val Glu Glu Ser Gln Leu
1745 1750 1755 1760
Pro Glu Gly Arg Lys Gly Leu Gln Arg Ile Glu Glu Leu Glu Arg Met
1765 1770 1775
Ala His Glu Gly Ala Leu Thr Gly Val Thr Thr Asp Gln Lys Glu Lys
1780 1785 1790
Gln Lys Pro Asp Ile Val Leu Tyr Pro Glu Pro Val Arg Val Leu Glu
1795 1800 1805
Gly Glu Thr Ala Arg Phe Arg Cys Arg Val Thr Gly Tyr Pro Gln Pro
1810 1815 1820
Lys Val Asn Trp Tyr Leu Asn Gly Gln Leu Ile Arg Lys Ser Lys Arg
1825 1830 1835 1840
Phe Arg Val Arg Tyr Asp Gly Ile His Tyr Leu Asp Ile Val Asp Cys
1845 1850 1855
Lys Ser Tyr Asp Thr Gly Glu Val Lys Val Thr Ala Glu Asn Pro Glu
1860 1865 1870
Gly Val Ile Glu His Lys Val Lys Leu Glu Ile Gln Gln Arg Glu Asp
1875 1880 1885
Phe Arg Ser Val Leu Arg Arg Ala Pro Glu Pro Arg Pro Glu Phe His
1890 1895 1900
Val His Glu Pro Gly Lys Leu Gln Phe Glu Val Gln Lys Val Asp Arg
1905 1910 1915 1920
Pro Val Asp Thr Thr Glu Thr Lys Glu Val Val Lys Leu Lys Arg Ala
1925 1930 1935
Glu Arg Ile Thr His Glu Lys Val Pro Glu Glu Ser Glu Glu Leu Arg
1940 1945 1950
Ser Lys Phe Lys Arg Arg Thr Glu Glu Gly Tyr Tyr Glu Ala Ile Thr
1955 1960 1965
Ala Val Glu Leu Lys Ser Arg Lys Lys Asp Glu Ser Tyr Glu Glu Leu
1970 1975 1980
Leu Arg Lys Thr Lys Asp Glu Leu Leu His Trp Thr Lys Glu Leu Thr
1985 1990 1995 2000
Glu Glu Glu Lys Lys Ala Leu Ala Glu Glu Gly Lys Ile Thr Ile Pro
2005 2010 2015
Thr Phe Lys Pro Asp Lys Ile Glu Leu Ser Pro Ser Met Glu Ala Pro
2020 2025 2030
Lys Ile Phe Glu Arg Ile Gln Ser Gln Thr Val Gly Gln Gly Ser Asp
2035 2040 2045
Ala His Phe Arg Val Arg Val Val Gly Lys Pro Asp Pro Glu Cys Glu
2050 2055 2060
Trp Tyr Lys Asn Gly Val Lys Ile Glu Arg Ser Asp Arg Ile Tyr Trp
2065 2070 2075 2080
Tyr Trp Pro Glu Asp Asn Val Cys Glu Leu Val Ile Arg Asp Val Thr
2085 2090 2095
Ala Glu Asp Ser Ala Ser Ile Met Val Lys Ala Ile Asn Ile Ala Gly
2100 2105 2110
Glu Thr Ser Ser His Ala Phe Leu Leu Val Gln Ala Lys Gln Leu Ile
2115 2120 2125
Thr Phe Thr Gln Glu Leu Gln Asp Val Val Ala Lys Glu Lys Asp Thr
2130 2135 2140
Met Ala Thr Phe Glu Cys Glu Thr Ser Glu Pro Phe Val Lys Val Lys
2145 2150 2155 2160
Trp Tyr Lys Asp Gly Met Glu Val His Glu Gly Asp Lys Tyr Arg Met
2165 2170 2175
His Ser Asp Arg Lys Val His Phe Leu Ser Ile Leu Thr Ile Asp Thr
2180 2185 2190
Ser Asp Ala Glu Asp Tyr Ser Cys Val Leu Val Glu Asp Glu Asn Val
2195 2200 2205
 
Lys Thr Thr Ala Lys Leu Ile Val Glu Gly Ala Val Val Glu Phe Val
2210 2215 2220
Lys Glu Leu Gln Asp Ile Glu Val Pro Glu Ser Tyr Ser Gly Glu Leu
2225 2230 2235 2240
Glu Cys Ile Val Ser Pro Glu Asn Ile Glu Gly Lys Trp Tyr His Asn
2245 2250 2255
Asp Val Glu Leu Lys Ser Asn Gly Lys Tyr Thr Ile Thr Ser Arg Arg
2260 2265 2270
Gly Arg Gln Asn Leu Thr Val Lys Asp Val Thr Lys Glu Asp Gln Gly
2275 2280 2285
Glu Tyr Ser Phe Val Ile Asp Gly Lys Lys Thr Thr Cys Lys Leu Lys
2290 2295 2300
Met Lys Pro Arg Pro Ile Ala Ile Leu Gln Gly Leu Ser Asp Gln Lys
2305 2310 2315 2320
Val Cys Glu Gly Asp Ile Val Gln Leu Glu Val Lys Val Ser Leu Glu
2325 2330 2335
Ser Val Glu Gly Val Trp Met Lys Asp Gly Gln Glu Val Gln Pro Ser
2340 2345 2350
Asp Arg Val His Ile Val Ile Asp Lys Gln Ser His Met Leu Leu Ile
2355 2360 2365
Glu Asp Met Thr Lys Glu Asp Ala Gly Asn Tyr Ser Phe Thr Ile Pro
2370 2375 2380
Ala Leu Gly Leu Ser Thr Ser Gly Arg Val Ser Val Tyr Ser Val Asp
2385 2390 2395 2400
Val Ile Thr Pro Leu Lys Asp Val Asn Val Ile Glu Gly Thr Lys Ala
2405 2410 2415
Val Leu Glu Cys Lys Val Ser Val Pro Asp Val Thr Ser Val Lys Trp
2420 2425 2430
Tyr Leu Asn Asp Glu Gln Ile Lys Pro Asp Asp Arg Val Gln Ala Ile
2435 2440 2445
Val Lys Gly Thr Lys Gln Arg Leu Val Ile Asn Arg Thr His Ala Ser
2450 2455 2460
Asp Glu Gly Pro Tyr Lys Leu Ile Val Gly Arg Val Glu Thr Asn Cys
2465 2470 2475 2480
Asn Leu Ser Val Glu Lys Ile Lys Ile Ile Arg Gly Leu Arg Asp Leu
2485 2490 2495
Thr Cys Thr Glu Thr Gln Asn Val Val Phe Glu Val Glu Leu Ser His
2500 2505 2510
Ser Gly Ile Asp Val Leu Trp Asn Phe Lys Asp Lys Glu Ile Lys Pro
2515 2520 2525
Ser Ser Lys Tyr Lys Ile Glu Ala His Gly Lys Ile Tyr Lys Leu Thr
2530 2535 2540
Val Leu Asn Met Met Lys Asp Asp Glu Gly Lys Tyr Thr Phe Tyr Ala
2545 2550 2555 2560
Gly Glu Asn Met Thr Ser Gly Lys Leu Thr Val Ala Gly Gly Ala Ile
2565 2570 2575
Ser Lys Pro Leu Thr Asp Gln Thr Val Ala Glu Ser Gln Glu Ala Val
2580 2585 2590
Phe Glu Cys Glu Val Ala Asn Pro Asp Ser Lys Gly Glu Trp Leu Arg
2595 2600 2605
Asp Gly Lys His Leu Pro Leu Thr Asn Asn Ile Arg Ser Glu Ser Asp
2610 2615 2620
Gly His Lys Arg Arg Leu Ile Ile Ala Ala Thr Lys Leu Asp Asp Ile
2625 2630 2635 2640
Gly Glu Tyr Thr Tyr Lys Val Ala Thr Ser Lys Thr Ser Ala Lys Leu
2645 2650 2655
Lys Val Glu Ala Val Lys Ile Lys Lys Thr Leu Lys Asn Leu Thr Val
2660 2665 2670
Thr Glu Thr Gln Asp Ala Val Phe Thr Val Glu Leu Thr His Pro Asn
2675 2680 2685
Val Lys Gly Val Gln Trp Ile Lys Asn Gly Val Val Leu Glu Ser Asn
2690 2695 2700
 
Glu Lys Tyr Ala Ile Ser Val Lys Gly Thr Ile Tyr Ser Leu Arg Ile
2705                2710                2715                2720
Lys Asn Cys Ala Ile Val Asp Glu Ser Val Tyr Gly Phe Arg Leu Gly
                2725                2730                2735
Arg Leu Gly Ala Ser Ala Arg Leu His Val Glu Thr Val Lys Ile Ile
            2740                2745                2750
Lys Lys Pro Lys Asp Val Thr Ala Leu Glu Asn Ala Thr Val Ala Phe
        2755                2760                2765
Glu Val Ser Val Ser His Asp Thr Val Pro Val Lys Trp Phe His Lys
    2770                2775                2780
Ser Val Glu Ile Lys Pro Ser Asp Lys His Arg Leu Val Ser Glu Arg
2785                2790                2795                2800
Lys Val His Lys Leu Met Leu Gln Asn Ile Ser Pro Ser Asp Ala Gly
                2805                2810                2815
Glu Tyr Thr Ala Val Val Gly Gln Leu Glu Cys Lys Ala Lys Leu Phe
            2820                2825                2830
Val Glu Thr Leu His Ile Thr Lys Thr Met Lys Asn Ile Glu Val Pro
        2835                2840                2845
Glu Thr Lys Thr Ala Ser Phe Glu Cys Glu Val Ser His Phe Asn Val
    2850                2855                2860
Pro Ser Met Trp Leu Lys Asn Gly Val Glu Ile Glu Met Ser Glu Lys
2865                2870                2875                2880
Phe Lys Ile Val Val Gln Gly Lys Leu His Gln Leu Ile Ile Met Asn
                2885                2890                2895
Thr Ser Thr Glu Asp Ser Ala Glu Tyr Thr Phe Val Cys Gly Asn Asp
            2900                2905                2910
Gln Val Ser Ala Thr Leu Thr Val Thr Pro Ile Met Ile Thr Ser Met
        2915                2920                2925
Leu Lys Asp Ile Asn Ala Glu Glu Lys Asp Thr Ile Thr Phe Glu Val
    2930                2935                2940
Thr Val Asn Tyr Glu Gly Ile Ser Tyr Lys Trp Leu Lys Asn Gly Val
2945                2950                2955                2960
Glu Ile Lys Ser Thr Asp Lys Cys Gln Met Arg Thr Lys Lys Leu Thr
                2965                2970                2975
His Ser Leu Asn Ile Arg Asn Val His Phe Gly Asp Ala Ala Asp Tyr
            2980                2985                2990
Thr Phe Val Ala Gly Lys Ala Thr Ser Thr Ala Thr Leu Tyr Val Glu
        2995                3000                3005
Ala Arg His Ile Glu Phe Arg Lys His Ile Lys Asp Ile Lys Val Leu
    3010                3015                3020
Glu Lys Lys Arg Ala Met Phe Glu Cys Glu Val Ser Glu Pro Asp Ile
3025                3030                3035                3040
Thr Val Gln Trp Met Lys Asp Asp Gln Glu Leu Gln Ile Thr Asp Arg
                3045                3050                3055
Ile Lys Ile Gln Lys Glu Lys Tyr Val His Arg Leu Leu Ile Pro Ser
            3060                3065                3070
Thr Arg Met Ser Asp Ala Gly Lys Tyr Thr Val Val Ala Gly Gly Asn
        3075                3080                3085
Val Ser Thr Ala Lys Leu Phe Val Glu Gly Arg Asp Val Arg Ile Arg
    3090                3095                3100
Ser Ile Lys Lys Glu Val Gln Val Ile Glu Lys Gln Arg Ala Val Val
3105                3110                3115                3120
Glu Phe Glu Val Asn Glu Asp Asp Val Asp Ala His Trp Tyr Lys Asp
                3125                3130                3135
Gly Ile Glu Ile Asn Phe Gln Val Gln Glu Arg His Lys Tyr Val Val
            3140                3145                3150
Glu Arg Arg Ile His Arg Met Phe Ile Ser Glu Thr Arg Gln Ser Asp
        3155                3160                3165
Ala Gly Glu Tyr Thr Phe Val Ala Gly Arg Asn Arg Ser Ser Val Thr
    3170                3175                3180
Leu Tyr Val Asn Ala Pro Glu Pro Pro Gln Val Leu Gln Glu Leu Gln
3185                3190                3195                3200
 
Pro Val Thr Val Gln Ser Gly Lys Pro Ala Arg Phe Cys Ala Met Ile
3205 3210 3215
Ser Gly Arg Pro Gln Pro Lys Ile Ser Trp Tyr Lys Glu Glu Gln Leu
3220 3225 3230
Leu Ser Thr Gly Phe Lys Cys Lys Phe Leu His Asp Gly Gln Glu Tyr
3235 3240 3245
Thr Leu Leu Leu Ile Glu Ala Phe Pro Glu Asp Ala Ala Val Tyr Thr
3250 3255 3260
Cys Glu Ala Lys Asn Asp Tyr Gly Val Ala Thr Thr Ser Ala Ser Leu
3265 3270 3275 3280
Ser Val Glu Val Pro Glu Val Val Ser Pro Asp Gln Glu Met Pro Val
3285 3290 3295
Tyr Pro Pro Ala Ile Ile Thr Pro Leu Gln Asp Thr Val Thr Ser Glu
3300 3305 3310
Gly Gln Pro Ala Arg Phe Gln Cys Arg Val Ser Gly Thr Asp Leu Lys
3315 3320 3325
Val Ser Trp Tyr Ser Lys Asp Lys Lys Ile Lys Pro Ser Arg Phe Phe
3330 3335 3340
Arg Met Thr Gln Phe Glu Asp Thr Tyr Gln Leu Glu Ile Ala Glu Ala
3345 3350 3355 3360
Tyr Pro Glu Asp Glu Gly Thr Tyr Thr Phe Val Ala Asn Asn Ala Val
3365 3370 3375
Gly Gln Val Ser Ser Thr Ala Asn Leu Ser Leu Glu Ala Pro Glu Ser
3380 3385 3390
Ile Leu His Glu Arg Ile Glu Gln Glu Ile Glu Met Glu Met Lys Glu
3395 3400 3405
Phe Ser Ser Ser Phe Leu Ser Ala Glu Glu Glu Gly Leu His Ser Ala
3410 3415 3420
Glu Leu Gln Leu Ser Lys Ile Asn Glu Thr Leu Glu Leu Leu Ser Glu
3425 3430 3435 3440
Ser Pro Val Tyr Pro Thr Lys Phe Asp Ser Glu Lys Glu Gly Thr Gly
3445 3450 3455
Pro Ile Phe Ile Lys Glu Val Ser Asn Ala Asp Ile Ser Met Gly Asp
3460 3465 3470
Val Ala Thr Leu Ser Val Thr Val Ile Gly Ile Pro Lys Pro Lys Ile
3475 3480 3485
Gln Trp Phe Phe Asn Gly Val Leu Leu Thr Pro Ser Ala Asp Tyr Lys
3490 3495 3500
Phe Val Phe Asp Gly Asp Asp His Ser Leu Ile Ile Leu Phe Thr Lys
3505 3510 3515 3520
Leu Glu Asp Glu Gly Glu Tyr Thr Cys Met Ala Ser Asn Asp Tyr Gly
3525 3530 3535
Lys Thr Ile Cys Ser Ala Tyr Leu Lys Ile Asn Ser Lys Gly Glu Gly
3540 3545 3550
His Lys Asp Thr Glu Thr Glu Ser Ala Val Ala Lys Ser Leu Glu Lys
3555 3560 3565
Leu Gly Gly Pro Cys Pro Pro His Phe Leu Lys Glu Leu Lys Pro Ile
3570 3575 3580
Arg Cys Ala Gln Gly Leu Pro Ala Ile Phe Glu Tyr Thr Val Val Gly
3585 3590 3595 3600
Glu Pro Ala Pro Thr Val Thr Trp Phe Lys Glu Asn Lys Gln Leu Cys
3605 3610 3615
Thr Ser Val Tyr Tyr Thr Ile Ile His Asn Pro Asn Gly Ser Gly Thr
3620 3625 3630
Phe Ile Val Asn Asp Pro Gln Arg Glu Asp Ser Gly Leu Tyr Ile Cys
3635 3640 3645
Lys Ala Glu Asn Met Leu Gly Glu Ser Thr Cys Ala Ala Glu Leu Leu
3650 3655 3660
Val Leu Leu Glu Asp Thr Asp Met Thr Asp Thr Pro Cys Lys Ala Lys
3665 3670 3675 3680
Ser Thr Pro Glu Ala Pro Glu Asp Phe Pro Gln Thr Pro Leu Lys Gly
3685 3690 3695
 
Pro Ala Val Glu Ala Leu Asp Ser Glu Gln Glu Ile Ala Thr Phe Val
3700 3705 3710
Lys Asp Thr Ile Leu Lys Ala Ala Leu Ile Thr Glu Glu Asn Gln Gln
3715 3720 3725
Leu Ser Tyr Glu His Ile Ala Lys Ala Asn Glu Leu Ser Ser Gln Leu
3730 3735 3740
Pro Leu Gly Ala Gln Glu Leu Gln Ser Ile Leu Glu Gln Asp Lys Leu
3745 3750 3755 3760
Thr Pro Glu Ser Thr Arg Glu Phe Leu Cys Ile Asn Gly Ser Ile His
3765 3770 3775
Phe Gln Pro Leu Lys Glu Pro Ser Pro Asn Leu Gln Leu Gln Ile Val
3780 3785 3790
Gln Ser Gln Lys Thr Phe Ser Lys Glu Gly Ile Leu Met Pro Glu Glu
3795 3800 3805
Pro Glu Thr Gln Ala Val Leu Ser Asp Thr Glu Lys Ile Phe Pro Ser
3810 3815 3820
Ala Met Ser Ile Glu Gln Ile Asn Ser Leu Thr Val Glu Pro Leu Lys
3825 3830 3835 3840
Thr Leu Leu Ala Glu Pro Glu Gly Asn Tyr Pro Gln Ser Ser Ile Glu
3845 3850 3855
Pro Pro Met His Ser Tyr Leu Thr Ser Val Ala Glu Glu Val Leu Ser
3860 3865 3870
Leu Lys Glu Lys Thr Val Ser Asp Thr Asn Arg Glu Gln Arg Val Thr
3875 3880 3885
Leu Gln Lys Gln Glu Ala Gln Ser Ala Leu Ile Leu Ser Gln Ser Leu
3890 3895 3900
Ala Glu Gly His Val Glu Ser Leu Gln Ser Pro Asp Val Met Ile Ser
3905 3910 3915 3920
Gln Val Asn Tyr Glu Pro Leu Val Pro Ser Glu His Ser Cys Thr Glu
3925 3930 3935
Gly Gly Lys Ile Leu Ile Glu Ser Ala Asn Pro Leu Glu Asn Ala Gly
3940 3945 3950
Gln Asp Ser Ala Val Arg Ile Glu Glu Gly Lys Ser Leu Arg Phe Pro
3955 3960 3965
Leu Ala Leu Glu Glu Lys Gln Val Leu Leu Lys Glu Glu His Ser Asp
3970 3975 3980
Asn Val Val Met Pro Pro Asp Gln Ile Ile Glu Ser Lys Arg Glu Pro
3985 3990 3995 4000
Val Ala Ile Lys Lys Val Gln Glu Val Gln Gly Arg Asp Leu Leu Ser
4005 4010 4015
Lys Glu Ser Leu Leu Ser Gly Ile Pro Glu Glu Gln Arg Leu Asn Leu
4020 4025 4030
Lys Ile Gln Ile Cys Arg Ala Leu Gln Ala Ala Val Ala Ser Glu Gln
4035 4040 4045
Pro Gly Leu Phe Ser Glu Trp Leu Arg Asn Ile Glu Lys Val Glu Val
4050 4055 4060
Glu Ala Val Asn Ile Thr Gln Glu Pro Arg His Ile Met Cys Met Tyr
4065 4070 4075 4080
Leu Val Thr Ser Ala Lys Ser Val Thr Glu Glu Val Thr Ile Ile Ile
4085 4090 4095
Glu Asp Val Asp Pro Gln Met Ala Asn Leu Lys Met Glu Leu Arg Asp
4100 4105 4110
Ala Leu Cys Ala Ile Ile Tyr Glu Glu Ile Asp Ile Leu Thr Ala Glu
4115 4120 4125
Gly Pro Arg Ile Gln Gln Gly Ala Lys Thr Ser Leu Gln Glu Glu Met
4130 4135 4140
Asp Ser Phe Ser Gly Ser Gln Lys Val Glu Pro Ile Thr Glu Pro Glu
4145 4150 4155 4160
Val Glu Ser Lys Tyr Leu Ile Ser Thr Glu Glu Val Ser Tyr Phe Asn
4165 4170 4175
Val Gln Ser Arg Val Lys Tyr Leu Asp Ala Thr Pro Val Thr Lys Gly
4180 4185 4190
 
Val Ala Ser Ala Val Val Ser Asp Glu Lys Gln Asp Glu Ser Leu Lys
4195 4200 4205
Pro Ser Glu Glu Lys Glu Glu Ser Ser Ser Glu Ser Gly Thr Glu Glu
4210 4215 4220
Val Ala Thr Val Lys Ile Gln Glu Ala Glu Gly Gly Leu Ile Lys Glu
4225 4230 4235 4240
Asp Gly Pro Met Ile His Thr Pro Leu Val Asp Thr Val Ser Glu Glu
4245 4250 4255
Gly Asp Ile Val His Leu Thr Thr Ser Ile Thr Asn Ala Lys Glu Val
4260 4265 4270
Asn Trp Tyr Phe Glu Asn Lys Leu Val Pro Ser Asp Glu Lys Phe Lys
4275 4280 4285
Cys Leu Gln Asp Gln Asn Thr Tyr Thr Leu Val Ile Asp Lys Val Asn
4290 4295 4300
Thr Glu Asp His Gln Gly Glu Tyr Val Cys Glu Ala Leu Asn Asp Ser
4305 4310 4315 4320
Gly Lys Thr Ala Thr Ser Ala Lys Leu Thr Val Val Lys Arg Ala Ala
4325 4330 4335
Pro Val Ile Lys Arg Lys Ile Glu Pro Leu Glu Val Ala Leu Gly His
4340 4345 4350
Leu Ala Lys Phe Thr Cys Glu Ile Gln Ser Ala Pro Asn Val Arg Phe
4355 4360 4365
Gln Trp Phe Lys Ala Gly Arg Glu Ile Tyr Glu Ser Asp Lys Cys Ser
4370 4375 4380
Ile Arg Ser Ser Lys Tyr Ile Ser Ser Leu Glu Ile Leu Arg Thr Gln
4385 4390 4395 4400
Val Val Asp Cys Gly Glu Tyr Thr Cys Lys Ala Ser Asn Glu Tyr Gly
4405 4410 4415
Ser Val Ser Cys Thr Ala Thr Leu Thr Val Thr Val Pro Gly Gly Glu
4420 4425 4430
Lys Lys Val Arg Lys Leu Leu Pro Glu Arg Lys Pro Glu Pro Lys Glu
4435 4440 4445
Glu Val Val Leu Lys Ser Val Leu Arg Lys Arg Pro Glu Glu Glu Glu
4450 4455 4460
Pro Lys Val Glu Pro Lys Lys Leu Glu Lys Val Lys Lys Pro Ala Val
4465 4470 4475 4480
Pro Glu Pro Pro Pro Pro Lys Pro Val Glu Glu Val Glu Val Pro Thr
4485 4490 4495
Val Thr Lys Arg Glu Arg Lys Ile Pro Glu Pro Thr Lys Val Pro Glu
4500 4505 4510
Ile Lys Pro Ala Ile Pro Leu Pro Ala Pro Glu Pro Lys Pro Lys Pro
4515 4520 4525
Glu Ala Glu Val Lys Thr Ile Lys Pro Pro Pro Val Glu Pro Glu Pro
4530 4535 4540
Thr Pro Ile Ala Ala Pro Val Thr Val Pro Val Val Gly Lys Lys Ala
4545 4550 4555 4560
Glu Ala Lys Ala Pro Lys Glu Glu Ala Ala Lys Pro Lys Gly Pro Ile
4565 4570 4575
Lys Gly Val Pro Lys Lys Thr Pro Ser Pro Ile Glu Ala Glu Arg Arg
4580 4585 4590
Lys Leu Arg Pro Gly Ser Gly Gly Glu Lys Pro Pro Asp Glu Ala Pro
4595 4600 4605
Phe Thr Tyr Gln Leu Lys Ala Val Pro Leu Lys Phe Val Lys Glu Ile
4610 4615 4620
Lys Asp Ile Ile Leu Thr Glu Ser Glu Phe Val Gly Ser Ser Ala Ile
4625 4630 4635 4640
Phe Glu Cys Leu Val Ser Pro Ser Thr Ala Ile Thr Thr Trp Met Lys
4645 4650 4655
Asp Gly Ser Asn Ile Arg Glu Ser Pro Lys His Arg Phe Ile Ala Asp
4660 4665 4670
Gly Lys Asp Arg Lys Leu His Ile Ile Asp Val Gln Leu Ser Asp Ala
4675 4680 4685
 
Gly Glu Tyr Thr Cys Val Leu Arg Leu Gly Asn Lys Glu Lys Thr Ser
    4690                4695                4700
Thr Ala Lys Leu Val Val Glu Glu Leu Pro Val Arg Phe Val Lys Thr
4705                4710                4715                4720
Leu Glu Glu Glu Val Thr Val Val Lys Gly Gln Pro Leu Tyr Leu Ser
                4725                4730                4735
Cys Glu Leu Asn Lys Glu Arg Asp Val Val Trp Arg Lys Asp Gly Lys
            4740                4745                4750
Ile Val Val Glu Lys Pro Gly Arg Ile Val Pro Gly Val Ile Gly Leu
        4755                4760                4765
Met Arg Ala Leu Thr Ile Asn Asp Ala Asp Asp Thr Asp Ala Gly Thr
    4770                4775                4780
Tyr Thr Val Thr Val Glu Asn Ala Asn Asn Leu Glu Cys Ser Ser Cys
4785                4790                4795                4800
Val Lys Val Val Glu Val Ile Arg Asp Trp Leu Val Lys Pro Ile Arg
                4805                4810                4815
Asp Gln His Val Lys Pro Lys Gly Thr Ala Ile Phe Ala Cys Asp Ile
            4820                4825                4830
Ala Lys Asp Thr Pro Asn Ile Lys Trp Phe Lys Gly Tyr Asp Glu Ile
        4835                4840                4845
Pro Ala Glu Pro Asn Asp Lys Thr Glu Ile Leu Arg Asp Gly Asn His
    4850                4855                4860
Leu Tyr Leu Lys Ile Lys Asn Ala Met Pro Glu Asp Ile Ala Glu Tyr
4865                4870                4875                4880
Ala Val Glu Ile Glu Gly Lys Arg Tyr Pro Ala Lys Leu Thr Leu Gly
                4885                4890                4895
Glu Arg Glu Val Glu Leu Leu Lys Pro Ile Glu Asp Val Thr Ile Tyr
            4900                4905                4910
Glu Lys Glu Ser Ala Ser Phe Asp Ala Glu Ile Ser Glu Ala Asp Ile
        4915                4920                4925
Pro Gly Gln Trp Lys Leu Lys Gly Glu Leu Leu Arg Pro Ser Pro Thr
    4930                4935                4940
Cys Glu Ile Lys Ala Glu Gly Gly Lys Arg Phe Leu Thr Leu His Lys
4945                4950                4955                4960
Val Lys Leu Asp Gln Ala Gly Glu Val Leu Tyr Gln Ala Leu Asn Ala
                4965                4970                4975
Ile Thr Thr Ala Ile Leu Thr Val Lys Glu Ile Glu Leu Asp Phe Ala
            4980                4985                4990
Val Pro Leu Lys Asp Val Thr Val Pro Glu Arg Arg Gln Ala Arg Phe
        4995                5000                5005
Glu Cys Val Leu Thr Arg Glu Ala Asn Val Ile Trp Ser Lys Gly Pro
    5010                5015                5020
Asp Ile Ile Lys Ser Ser Asp Lys Phe Asp Ile Ile Ala Asp Gly Lys
5025                5030                5035                5040
Lys His Ile Leu Val Ile Asn Asp Ser Gln Phe Asp Asp Glu Gly Val
                5045                5050                5055
Tyr Thr Ala Glu Val Glu Gly Lys Lys Thr Ser Ala Arg Leu Phe Val
            5060                5065                5070
Thr Gly Ile Arg Leu Lys Phe Met Ser Pro Leu Glu Asp Gln Thr Val
        5075                5080                5085
Lys Glu Gly Glu Thr Ala Thr Phe Val Cys Glu Leu Ser His Glu Lys
    5090                5095                5100
Met His Val Val Trp Phe Lys Asn Asp Ala Lys Leu His Thr Ser Arg
5105                5110                5115                5120
Thr Val Leu Ile Ser Ser Glu Gly Lys Thr His Lys Leu Glu Met Lys
                5125                5130                5135
Glu Val Thr Leu Asp Asp Ile Ser Gln Ile Lys Ala Gln Val Lys Glu
            5140                5145                5150
Leu Ser Ser Thr Ala Gln Leu Lys Val Leu Glu Ala Asp Pro Tyr Phe
        5155                5160                5165
Thr Val Lys Leu His Asp Lys Thr Ala Val Glu Lys Asp Glu Ile Thr
    5170                5175                5180
 
Leu Lys Cys Glu Val Ser Lys Asp Val Pro Val Lys Trp Phe Lys Asp
5185                5190                5195                5200
Gly Glu Glu Ile Val Pro Ser Pro Lys Tyr Ser Ile Lys Ala Asp Gly
                5205                5210                5215
Leu Arg Arg Ile Leu Lys Ile Lys Lys Ala Asp Leu Lys Asp Lys Gly
            5220                5225                5230
Glu Tyr Val Cys Asp Cys Gly Thr Asp Lys Thr Lys Ala Asn Val Thr
        5235                5240                5245
Val Glu Ala Arg Leu Ile Glu Val Glu Lys Pro Leu Tyr Gly Val Glu
    5250                5255                5260
Val Phe Val Gly Glu Thr Ala His Phe Glu Ile Glu Leu Ser Glu Pro
5265                5270                5275                5280
Asp Val His Gly Gln Trp Lys Leu Lys Gly Gln Pro Leu Thr Ala Ser
                5285                5290                5295
Pro Asp Cys Glu Ile Ile Glu Asp Gly Lys Lys His Ile Leu Ile Leu
            5300                5305                5310
His Asn Cys Gln Leu Gly Met Thr Gly Glu Val Ser Phe Gln Ala Ala
        5315                5320                5325
Asn Ala Lys Ser Ala Ala Asn Leu Lys Val Lys Glu Leu Pro Leu Ile
    5330                5335                5340
Phe Ile Thr Pro Leu Ser Asp Val Lys Val Phe Glu Lys Asp Glu Ala
5345                5350                5355                5360
Lys Phe Glu Cys Glu Val Ser Arg Glu Pro Lys Thr Phe Arg Trp Leu
                5365                5370                5375
Lys Gly Thr Gln Glu Ile Thr Gly Asp Asp Arg Phe Glu Leu Ile Lys
            5380                5385                5390
Asp Gly Thr Lys His Ser Met Val Ile Lys Ser Ala Ala Phe Glu Asp
        5395                5400                5405
Glu Ala Lys Tyr Met Phe Glu Ala Glu Asp Lys His Thr Ser Gly Lys
    5410                5415                5420
Leu Ile Ile Glu Gly Ile Arg Leu Lys Phe Leu Thr Pro Leu Lys Asp
5425                5430                5435                5440
Val Thr Ala Lys Glu Lys Glu Ser Ala Val Phe Thr Val Glu Leu Ser
                5445                5450                5455
His Asp Asn Ile Arg Val Lys Trp Phe Lys Asn Asp Gln Arg Leu His
            5460                5465                5470
Thr Thr Arg Ser Val Ser Met Gln Asp Glu Gly Lys Thr His Ser Ile
        5475                5480                5485
Thr Phe Lys Asp Leu Ser Ile Asp Asp Thr Ser Gln Ile Arg Val Glu
    5490                5495                5500
Ala Met Gly Met Ser Ser Glu Ala Lys Leu Thr Val Leu Glu Gly Asp
5505                5510                5515                5520
Pro Tyr Phe Thr Gly Lys Leu Gln Asp Tyr Thr Gly Val Glu Lys Asp
                5525                5530                5535
Glu Val Ile Leu Gln Cys Glu Ile Ser Lys Ala Asp Ala Pro Val Lys
            5540                5545                5550
Trp Phe Lys Asp Gly Lys Glu Ile Lys Pro Ser Lys Asn Ala Val Ile
        5555                5560                5565
Lys Thr Asp Gly Lys Lys Arg Met Leu Ile Leu Lys Lys Ala Leu Lys
    5570                5575                5580
Ser Asp Ile Gly Gln Tyr Thr Cys Asp Cys Gly Thr Asp Lys Thr Ser
5585                5590                5595                5600
Gly Lys Leu Asp Ile Glu Asp Arg Glu Ile Lys Leu Val Arg Pro Leu
                5605                5610                5615
His Ser Val Glu Val Met Glu Thr Glu Thr Ala Arg Phe Glu Thr Glu
            5620                5625                5630
Ile Ser Glu Asp Asp Ile His Ala Asn Trp Lys Leu Lys Gly Glu Ala
        5635                5640                5645
Leu Leu Gln Thr Pro Asp Cys Glu Ile Lys Glu Glu Gly Lys Ile His
    5650                5655                5660
Ser Leu Val Leu His Asn Cys Arg Leu Asp Gln Thr Gly Gly Val Asp
5665                5670                5675                5680
 
Phe Gln Ala Ala Asn Val Lys Ser Ser Ala His Leu Arg Val Lys Pro
5685 5690 5695
Arg Val Ile Gly Leu Leu Arg Pro Leu Lys Asp Val Thr Val Thr Ala
5700 5705 5710
Gly Glu Thr Ala Thr Phe Asp Cys Glu Leu Ser Tyr Glu Asp Ile Pro
5715 5720 5725
Val Glu Trp Tyr Leu Lys Gly Lys Lys Leu Glu Pro Ser Asp Lys Val
5730 5735 5740
Val Pro Arg Ser Glu Gly Lys Val His Thr Leu Thr Leu Arg Asp Val
5745 5750 5755 5760
Lys Leu Glu Asp Ala Gly Glu Val Gln Leu Thr Ala Lys Asp Phe Lys
5765 5770 5775
Thr His Ala Asn Leu Phe Val Lys Glu Pro Pro Val Glu Phe Thr Lys
5780 5785 5790
Pro Leu Glu Asp Gln Thr Val Glu Glu Gly Ala Thr Ala Val Leu Glu
5795 5800 5805
Cys Glu Val Ser Arg Glu Asn Ala Lys Val Lys Trp Phe Lys Asn Gly
5810 5815 5820
Thr Glu Ile Leu Lys Ser Lys Lys Tyr Glu Ile Val Ala Asp Gly Arg
5825 5830 5835 5840
Val Arg Lys Leu Val Ile His Asp Cys Thr Pro Glu Asp Ile Lys Thr
5845 5850 5855
Tyr Thr Cys Asp Ala Lys Asp Phe Lys Thr Ser Cys Asn Leu Asn Val
5860 5865 5870
Val Pro Pro His Val Glu Phe Leu Arg Pro Leu Thr Asp Leu Gln Val
5875 5880 5885
Arg Glu Lys Glu Met Ala Arg Phe Glu Cys Glu Leu Ser Arg Glu Asn
5890 5895 5900
Ala Lys Val Lys Trp Phe Lys Asp Gly Ala Glu Ile Lys Lys Gly Lys
5905 5910 5915 5920
Lys Tyr Asp Ile Ile Ser Lys Gly Ala Val Arg Ile Leu Val Ile Asn
5925 5930 5935
Lys Cys Leu Leu Asp Asp Glu Ala Glu Tyr Ser Cys Glu Val Arg Thr
5940 5945 5950
Ala Arg Thr Ser Gly Met Leu Thr Val Leu Glu Glu Glu Ala Val Phe
5955 5960 5965
Thr Lys Asn Leu Ala Asn Ile Glu Val Ser Glu Thr Asp Thr Ile Lys
5970 5975 5980
Leu Val Cys Glu Val Ser Lys Pro Gly Ala Glu Val Ile Trp Tyr Lys
5985 5990 5995 6000
Gly Asp Glu Glu Ile Ile Glu Thr Gly Arg Tyr Glu Ile Leu Thr Glu
6005 6010 6015
Gly Arg Lys Arg Ile Leu Val Ile Gln Asn Ala His Leu Glu Asp Ala
6020 6025 6030
Gly Asn Tyr Asn Cys Arg Leu Pro Ser Ser Arg Thr Asp Gly Lys Val
6035 6040 6045
Lys Val His Glu Leu Ala Ala Glu Phe Ile Ser Lys Pro Gln Asn Leu
6050 6055 6060
Glu Ile Leu Glu Gly Glu Lys Ala Glu Phe Val Cys Ser Ile Ser Lys
6065 6070 6075 6080
Glu Ser Phe Pro Val Gln Trp Lys Arg Asp Asp Lys Thr Leu Glu Ser
6085 6090 6095
Gly Asp Lys Tyr Asp Val Ile Ala Asp Gly Lys Lys Arg Val Leu Val
6100 6105 6110
Val Lys Asp Ala Thr Leu Gln Asp Met Gly Thr Tyr Val Val Met Val
6115 6120 6125
Gly Ala Ala Arg Ala Ala Ala His Leu Thr Val Ile Glu Lys Leu Arg
6130 6135 6140
Ile Val Val Pro Leu Lys Asp Thr Arg Val Lys Glu Gln Gln Glu Val
6145 6150 6155 6160
Val Phe Asn Cys Glu Val Asn Thr Glu Gly Ala Lys Ala Lys Trp Phe
6165 6170 6175
 
Arg Asn Glu Glu Ala Ile Phe Asp Ser Ser Lys Tyr Ile Ile Leu Gln
6180 6185 6190
Lys Asp Leu Val Tyr Thr Leu Arg Ile Arg Asp Ala His Leu Asp Asp
6195 6200 6205
Gln Ala Asn Tyr Asn Val Ser Leu Thr Asn His Arg Gly Glu Asn Val
6210 6215 6220
Lys Ser Ala Ala Asn Leu Ile Val Glu Glu Glu Asp Leu Arg Ile Val
6225 6230 6235 6240
Glu Pro Leu Lys Asp Ile Glu Thr Met Glu Lys Lys Ser Val Thr Phe
6245 6250 6255
Trp Cys Lys Val Asn Arg Leu Asn Val Thr Leu Lys Trp Thr Lys Asn
6260 6265 6270
Gly Glu Glu Val Pro Phe Asp Asn Arg Val Ser Tyr Arg Val Asp Lys
6275 6280 6285
Tyr Lys His Met Leu Thr Ile Lys Asp Cys Gly Phe Pro Asp Glu Gly
6290 6295 6300
Glu Tyr Ile Val Thr Ala Gly Gln Asp Lys Ser Val Ala Glu Leu Leu
6305 6310 6315 6320
Ile Ile Glu Ala Pro Thr Glu Phe Val Glu His Leu Glu Asp Gln Thr
6325 6330 6335
Val Thr Glu Phe Asp Asp Ala Val Phe Ser Cys Gln Leu Ser Arg Glu
6340 6345 6350
Lys Ala Asn Val Lys Trp Tyr Arg Asn Gly Arg Glu Ile Lys Glu Gly
6355 6360 6365
Lys Lys Tyr Lys Phe Glu Lys Asp Gly Ser Ile His Arg Leu Ile Ile
6370 6375 6380
Lys Asp Cys Arg Leu Asp Asp Glu Cys Glu Tyr Ala Cys Gly Val Glu
6385 6390 6395 6400
Asp Arg Lys Ser Arg Ala Arg Leu Phe Val Glu Glu Ile Pro Val Glu
6405 6410 6415
Ile Ile Arg Pro Pro Gln Asp Ile Leu Glu Ala Pro Gly Ala Asp Val
6420 6425 6430
Val Phe Leu Ala Glu Leu Asn Lys Asp Lys Val Glu Val Gln Trp Leu
6435 6440 6445
Arg Asn Asn Met Val Val Val Gln Gly Asp Lys His Gln Met Met Ser
6450 6455 6460
Glu Gly Lys Ile His Arg Leu Gln Ile Cys Asp Ile Lys Pro Arg Asp
6465 6470 6475 6480
Gln Gly Glu Tyr Arg Phe Ile Ala Lys Asp Lys Glu Ala Arg Ala Lys
6485 6490 6495
Leu Glu Leu Ala Ala Ala Pro Lys Ile Lys Thr Ala Asp Gln Asp Leu
6500 6505 6510
Val Val Asp Val Gly Lys Pro Leu Thr Met Val Val Pro Tyr Asp Ala
6515 6520 6525
Tyr Pro Lys Ala Glu Ala Glu Trp Phe Lys Glu Asn Glu Pro Leu Ser
6530 6535 6540
Thr Lys Thr Ile Asp Thr Thr Ala Glu Gln Thr Ser Phe Arg Ile Leu
6545 6550 6555 6560
Glu Ala Lys Lys Gly Asp Lys Gly Arg Tyr Lys Ile Val Leu Gln Asn
6565 6570 6575
Lys His Gly Lys Ala Glu Gly Phe Ile Asn Leu Lys Val Ile Asp Val
6580 6585 6590
Pro Gly Pro Val Arg Asn Leu Glu Val Thr Glu Thr Phe Asp Gly Glu
6595 6600 6605
Val Ser Leu Ala Trp Glu Glu Pro Leu Thr Asp Gly Gly Ser Lys Ile
6610 6615 6620
Ile Gly Tyr Val Val Glu Arg Arg Asp Ile Lys Arg Lys Thr Trp Val
6625 6630 6635 6640
Leu Ala Thr Asp Arg Ala Glu Ser Cys Glu Phe Thr Val Thr Gly Leu
6645 6650 6655
Gln Lys Gly Gly Val Glu Tyr Leu Phe Arg Val Ser Ala Arg Asn Arg
6660 6665 6670
 
Val Gly Thr Gly Glu Pro Val Glu Thr Asp Asn Pro Val Glu Ala Arg
6675 6680 6685
Ser Lys Tyr Asp Val Pro Gly Pro Pro Leu Asn Val Thr Ile Thr Asp
6690 6695 6700
Val Asn Arg Phe Gly Val Ser Leu Thr Trp Glu Pro Pro Glu Tyr Asp
6705 6710 6715 6720
Gly Gly Ala Glu Ile Thr Asn Tyr Val Ile Glu Leu Arg Asp Lys Thr
6725 6730 6735
Ser Ile Arg Trp Asp Thr Ala Met Thr Val Arg Ala Glu Asp Leu Ser
6740 6745 6750
Ala Thr Val Thr Asp Val Val Glu Gly Gln Glu Tyr Ser Phe Arg Val
6755 6760 6765
Arg Ala Gln Asn Arg Ile Gly Val Gly Lys Pro Ser Ala Ala Thr Pro
6770 6775 6780
Phe Val Lys Val Ala Asp Pro Ile Glu Arg Pro Ser Pro Pro Val Asn
6785 6790 6795 6800
Leu Thr Ser Ser Asp Gln Thr Gln Ser Ser Val Gln Leu Lys Trp Glu
6805 6810 6815
Pro Pro Leu Lys Asp Gly Gly Ser Pro Ile Leu Gly Tyr Ile Ile Glu
6820 6825 6830
Arg Cys Glu Glu Gly Lys Asp Asn Trp Ile Arg Cys Asn Met Lys Leu
6835 6840 6845
Val Pro Glu Leu Thr Tyr Lys Val Thr Gly Leu Glu Lys Gly Asn Lys
6850 6855 6860
Tyr Leu Tyr Arg Val Ser Ala Glu Asn Lys Ala Gly Val Ser Asp Pro
6865 6870 6875 6880
Ser Glu Ile Leu Gly Pro Leu Thr Ala Asp Asp Ala Phe Val Glu Pro
6885 6890 6895
Thr Met Asp Leu Ser Ala Phe Lys Asp Gly Leu Glu Val Ile Val Pro
6900 6905 6910
Asn Pro Ile Thr Ile Leu Val Pro Ser Thr Gly Tyr Pro Arg Pro Thr
6915 6920 6925
Ala Thr Trp Cys Phe Gly Asp Lys Val Leu Glu Thr Gly Asp Arg Val
6930 6935 6940
Lys Met Lys Thr Leu Ser Ala Tyr Ala Glu Leu Val Ile Ser Pro Ser
6945 6950 6955 6960
Glu Arg Ser Asp Lys Gly Ile Tyr Thr Leu Lys Leu Glu Asn Arg Val
6965 6970 6975
Lys Thr Ile Ser Gly Glu Ile Asp Val Asn Val Ile Ala Arg Pro Ser
6980 6985 6990
Ala Pro Lys Glu Leu Lys Phe Gly Asp Ile Thr Lys Asp Ser Val His
6995 7000 7005
Leu Thr Trp Glu Pro Pro Asp Asp Asp Gly Gly Ser Pro Leu Thr Gly
7010 7015 7020
Tyr Val Val Glu Lys Arg Glu Val Ser Arg Lys Thr Trp Thr Lys Val
7025 7030 7035 7040
Met Asp Phe Val Thr Asp Leu Glu Phe Thr Val Pro Asp Leu Val Gln
7045 7050 7055
Gly Lys Glu Tyr Leu Phe Lys Val Cys Ala Arg Asn Lys Cys Gly Pro
7060 7065 7070
Gly Glu Pro Ala Tyr Val Asp Glu Pro Val Asn Met Ser Thr Pro Ala
7075 7080 7085
Thr Val Pro Asp Pro Pro Glu Asn Val Lys Trp Arg Asp Arg Thr Ala
7090 7095 7100
Asn Ser Ile Phe Leu Thr Trp Asp Pro Pro Lys Asn Asp Gly Gly Ser
7105 7110 7115 7120
Arg Ile Lys Gly Tyr Ile Val Glu Arg Cys Pro Arg Gly Ser Asp Lys
7125 7130 7135
Trp Val Ala Cys Gly Glu Pro Val Ala Glu Thr Lys Met Glu Val Thr
7140 7145 7150
Gly Leu Glu Glu Gly Lys Trp Tyr Ala Tyr Arg Val Lys Thr Leu Asn
7155 7160 7165
 
Arg Gln Gly Ala Ser Lys Pro Ser Arg Pro Thr Glu Glu Ile Gln Ala
7170 7175 7180
Val Asp Thr Gln Glu Ala Pro Glu Ile Phe Leu Asp Val Lys Leu Leu
7185 7190 7195 7200
Ala Gly Leu Thr Val Lys Ala Gly Thr Lys Ile Glu Leu Pro Ala Thr
7205 7210 7215
Val Thr Gly Lys Pro Glu Pro Lys Ile Thr Trp Thr Lys Ala Asp Met
7220 7225 7230
Ile Leu Lys Gln Asp Lys Arg Ile Thr Ile Glu Asn Val Pro Lys Lys
7235 7240 7245
Ser Thr Val Thr Ile Val Asp Ser Lys Arg Ser Asp Thr Gly Thr Tyr
7250 7255 7260
Ile Ile Glu Ala Val Asn Val Cys Gly Arg Ala Thr Ala Val Val Glu
7265 7270 7275 7280
Val Asn Val Leu Asp Lys Pro Gly Pro Pro Ala Ala Phe Asp Ile Thr
7285 7290 7295
Asp Val Thr Asn Glu Ser Cys Leu Leu Thr Trp Asn Pro Pro Arg Asp
7300 7305 7310
Asp Gly Gly Ser Lys Ile Thr Asn Tyr Val Val Glu Arg Arg Ala Thr
7315 7320 7325
Asp Ser Glu Val Trp His Lys Leu Ser Ser Thr Val Lys Asp Thr Asn
7330 7335 7340
Phe Lys Ala Thr Lys Leu Ile Pro Asn Lys Glu Tyr Ile Phe Arg Val
7345 7350 7355 7360
Ala Ala Glu Asn Met Tyr Gly Ala Gly Glu Pro Val Gln Ala Ser Pro
7365 7370 7375
Ile Thr Ala Lys Tyr Gln Phe Asp Pro Pro Gly Pro Pro Thr Arg Leu
7380 7385 7390
Glu Pro Ser Asp Ile Thr Lys Asp Ala Val Thr Leu Thr Trp Cys Glu
7395 7400 7405
Pro Asp Asp Asp Gly Gly Ser Pro Ile Thr Gly Tyr Trp Val Glu Arg
7410 7415 7420
Leu Asp Pro Asp Thr Asp Lys Trp Val Arg Cys Asn Lys Met Pro Val
7425 7430 7435 7440
Lys Asp Thr Thr Tyr Arg Val Lys Gly Leu Thr Asn Lys Lys Lys Tyr
7445 7450 7455
Arg Phe Arg Val Leu Ala Glu Asn Leu Ala Gly Pro Gly Lys Pro Ser
7460 7465 7470
Lys Ser Thr Glu Pro Ile Leu Ile Lys Asp Pro Ile Asp Pro Pro Trp
7475 7480 7485
Pro Pro Gly Lys Pro Thr Val Lys Asp Val Gly Lys Thr Ser Val Arg
7490 7495 7500
Leu Asn Trp Thr Lys Pro Glu His Asp Gly Gly Ala Lys Ile Glu Ser
7505 7510 7515 7520
Tyr Val Ile Glu Met Leu Lys Thr Gly Thr Asp Glu Trp Val Arg Val
7525 7530 7535
Ala Glu Gly Val Pro Thr Thr Gln His Leu Leu Pro Gly Leu Met Glu
7540 7545 7550
Gly Gln Glu Tyr Ser Phe Arg Val Arg Ala Val Asn Lys Ala Gly Glu
7555 7560 7565
Ser Glu Pro Ser Glu Pro Ser Asp Pro Val Leu Cys Arg Glu Lys Leu
7570 7575 7580
Tyr Pro Pro Ser Pro Pro Arg Trp Leu Glu Val Ile Asn Ile Thr Lys
7585 7590 7595 7600
Asn Thr Ala Asp Leu Lys Trp Thr Val Pro Glu Lys Asp Gly Gly Ser
7605 7610 7615
Pro Ile Thr Asn Tyr Ile Val Glu Lys Arg Asp Val Arg Arg Lys Gly
7620 7625 7630
Trp Gln Thr Val Asp Thr Thr Val Lys Asp Thr Lys Cys Thr Val Thr
7635 7640 7645
Pro Leu Thr Glu Gly Ser Leu Tyr Val Phe Arg Val Ala Ala Glu Asn
7650 7655 7660
 
Ala Ile Gly Gln Ser Asp Tyr Thr Glu Ile Glu Asp Ser Val Leu Ala
7665                7670                7675                7680
Lys Asp Thr Phe Thr Thr Pro Gly Pro Pro Tyr Ala Leu Ala Val Val
                7685                7690                7695
Asp Val Thr Lys Arg His Val Asp Leu Lys Trp Glu Pro Pro Lys Asn
            7700                7705                7710
Asp Gly Gly Arg Pro Ile Gln Arg Tyr Val Ile Glu Lys Lys Glu Arg
        7715                7720                7725
Leu Gly Thr Arg Trp Val Lys Ala Gly Lys Thr Ala Gly Pro Asp Cys
    7730                7735                7740
Asn Phe Arg Val Thr Asp Val Ile Glu Gly Thr Glu Val Gln Phe Gln
7745                7750                7755                7760
Val Arg Ala Glu Asn Glu Ala Gly Val Gly His Pro Ser Glu Pro Thr
                7765                7770                7775
Glu Ile Leu Ser Ile Glu Asp Pro Thr Ser Pro Pro Ser Pro Pro Leu
            7780                7785                7790
Asp Leu His Val Thr Asp Ala Gly Arg Lys His Ile Ala Ile Ala Trp
        7795                7800                7805
Lys Pro Pro Glu Lys Asn Gly Gly Ser Pro Ile Ile Gly Tyr His Val
    7810                7815                7820
Glu Met Cys Pro Val Gly Thr Glu Lys Trp Met Arg Val Asn Ser Arg
7825                7830                7835                7840
Pro Ile Lys Asp Leu Lys Phe Lys Val Glu Glu Gly Val Val Pro Asp
                7845                7850                7855
Lys Glu Tyr Val Leu Arg Val Arg Ala Val Asn Ala Ile Gly Val Ser
            7860                7865                7870
Glu Pro Ser Glu Ile Ser Glu Asn Val Val Ala Lys Asp Pro Asp Cys
        7875                7880                7885
Lys Pro Thr Ile Asp Leu Glu Thr His Asp Ile Ile Val Ile Glu Gly
    7890                7895                7900
Glu Lys Leu Ser Ile Pro Val Pro Phe Arg Ala Val Pro Val Pro Thr
7905                7910                7915                7920
Val Ser Trp His Lys Asp Gly Lys Glu Val Lys Ala Ser Asp Arg Leu
                7925                7930                7935
Thr Met Lys Asn Asp His Ile Ser Ala His Leu Glu Val Pro Lys Ser
            7940                7945                7950
Val Arg Ala Asp Ala Gly Ile Tyr Thr Ile Thr Leu Glu Asn Lys Leu
        7955                7960                7965
Gly Ser Ala Thr Ala Ser Ile Asn Val Lys Val Ile Gly Leu Pro Gly
    7970                7975                7980
Pro Cys Lys Asp Ile Lys Ala Ser Asp Ile Thr Lys Ser Ser Cys Lys
7985                7990                7995                8000
Leu Thr Trp Glu Pro Pro Glu Phe Asp Gly Gly Thr Pro Ile Leu His
                8005                8010                8015
Tyr Val Leu Glu Arg Arg Glu Ala Gly Arg Arg Thr Tyr Ile Pro Val
            8020                8025                8030
Met Ser Gly Glu Asn Lys Leu Ser Trp Thr Val Lys Asp Leu Ile Pro
        8035                8040                8045
Asn Gly Glu Tyr Phe Phe Arg Val Lys Ala Val Asn Lys Val Gly Gly
    8050                8055                8060
Gly Glu Tyr Ile Glu Leu Lys Asn Pro Val Ile Ala Gln Asp Pro Lys
8065                8070                8075                8080
Gln Pro Pro Asp Pro Pro Val Asp Val Glu Val His Asn Pro Thr Ala
                8085                8090                8095
Glu Ala Met Thr Ile Thr Trp Lys Pro Pro Leu Tyr Asp Gly Gly Ser
            8100                8105                8110
Lys Ile Met Gly Tyr Ile Ile Glu Lys Ile Ala Lys Gly Glu Glu Arg
        8115                8120                8125
Trp Lys Arg Cys Asn Glu His Leu Val Pro Ile Leu Thr Tyr Thr Ala
    8130                8135                8140
Lys Gly Leu Glu Glu Gly Lys Glu Tyr Gln Phe Arg Val Arg Ala Glu
8145                8150                8155                8160
 
Asn Ala Ala Gly Ile Ser Glu Pro Ser Arg Ala Thr Pro Pro Thr Lys
8165 8170 8175
Ala Val Asp Pro Ile Asp Ala Pro Lys Val Ile Leu Arg Thr Ser Leu
8180 8185 8190
Glu Val Lys Arg Gly Asp Glu Ile Ala Leu Asp Ala Ser Ile Ser Gly
8195 8200 8205
Ser Pro Tyr Pro Thr Ile Thr Trp Ile Lys Asp Glu Asn Val Ile Val
8210 8215 8220
Pro Glu Glu Ile Lys Lys Arg Ala Ala Pro Leu Val Arg Arg Arg Lys
8225 8230 8235 8240
Gly Glu Val Gln Glu Glu Glu Pro Phe Val Leu Pro Leu Thr Gln Arg
8245 8250 8255
Leu Ser Ile Asp Asn Ser Lys Lys Gly Glu Ser Gln Leu Arg Val Arg
8260 8265 8270
Asp Ser Leu Arg Pro Asp His Gly Leu Tyr Met Ile Lys Val Glu Asn
8275 8280 8285
Asp His Gly Ile Ala Lys Ala Pro Cys Thr Val Ser Val Leu Asp Thr
8290 8295 8300
Pro Gly Pro Pro Ile Asn Phe Val Phe Glu Asp Ile Arg Lys Thr Ser
8305 8310 8315 8320
Val Leu Cys Lys Trp Glu Pro Pro Leu Asp Asp Gly Gly Ser Glu Ile
8325 8330 8335
Ile Asn Tyr Thr Leu Glu Lys Lys Asp Lys Thr Lys Pro Asp Ser Glu
8340 8345 8350
Trp Ile Val Val Thr Ser Thr Leu Arg His Cys Lys Tyr Ser Val Thr
8355 8360 8365
Lys Leu Ile Glu Gly Lys Glu Tyr Leu Phe Arg Val Arg Ala Glu Asn
8370 8375 8380
Arg Phe Gly Pro Gly Pro Pro Cys Val Ser Lys Pro Leu Val Ala Lys
8385 8390 8395 8400
Asp Pro Phe Gly Pro Pro Asp Ala Pro Asp Lys Pro Ile Val Glu Asp
8405 8410 8415
Val Thr Ser Asn Ser Met Leu Val Lys Trp Asn Glu Pro Lys Asp Asn
8420 8425 8430
Gly Ser Pro Ile Leu Gly Tyr Trp Leu Glu Lys Arg Glu Val Asn Ser
8435 8440 8445
Thr His Trp Ser Arg Val Asn Lys Ser Leu Leu Asn Ala Leu Lys Ala
8450 8455 8460
Asn Val Asp Gly Leu Leu Glu Gly Leu Thr Tyr Val Phe Arg Val Cys
8465 8470 8475 8480
Ala Glu Asn Ala Ala Gly Pro Gly Lys Phe Ser Pro Pro Ser Asp Pro
8485 8490 8495
Lys Thr Ala His Asp Pro Ile Ser Pro Pro Gly Pro Pro Ile Pro Arg
8500 8505 8510
Val Thr Asp Thr Ser Ser Thr Thr Ile Glu Leu Glu Trp Glu Pro Pro
8515 8520 8525
Ala Phe Asn Gly Gly Gly Glu Ile Val Gly Tyr Phe Val Asp Lys Gln
8530 8535 8540
Leu Val Gly Thr Asn Lys Trp Ser Arg Cys Thr Glu Lys Met Ile Lys
8545 8550 8555 8560
Val Arg Gln Tyr Thr Val Lys Glu Ile Arg Glu Gly Ala Asp Tyr Lys
8565 8570 8575
Leu Arg Val Ser Ala Val Asn Ala Ala Gly Glu Gly Pro Pro Gly Glu
8580 8585 8590
Thr Gln Pro Val Thr Val Ala Glu Pro Gln Glu Pro Pro Ala Val Glu
8595 8600 8605
Leu Asp Val Ser Val Lys Gly Gly Ile Gln Ile Met Ala Gly Lys Thr
8610 8615 8620
Leu Arg Ile Pro Ala Val Val Thr Gly Arg Pro Val Pro Thr Lys Val
8625 8630 8635 8640
Trp Thr Lys Glu Glu Gly Glu Leu Asp Lys Asp Arg Val Val Ile Asp
8645 8650 8655
 
Asn Val Gly Thr Lys Ser Glu Leu Ile Ile Lys Asp Ala Leu Arg Lys
            8660                8665                8670
Asp His Gly Arg Tyr Val Ile Thr Ala Thr Asn Ser Cys Gly Ser Lys
        8675                8680                8685
Phe Ala Ala Ala Arg Val Glu Val Phe Asp Val Pro Gly Pro Val Leu
    8690                8695                8700
Asp Leu Lys Pro Val Val Thr Asn Arg Lys Met Cys Leu Leu Asn Trp
8705                8710                8715                8720
Ser Asp Pro Glu Asp Asp Gly Gly Ser Glu Ile Thr Gly Phe Ile Ile
                8725                8730                8735
Glu Arg Lys Asp Ala Lys Met His Thr Trp Arg Gln Pro Ile Glu Thr
            8740                8745                8750
Glu Arg Ser Lys Cys Asp Ile Thr Gly Leu Leu Glu Gly Gln Glu Tyr
        8755                8760                8765
Lys Phe Arg Val Ile Ala Lys Asn Lys Phe Gly Cys Gly Pro Pro Val
    8770                8775                8780
Glu Ile Gly Pro Ile Leu Ala Val Asp Pro Leu Gly Pro Pro Thr Ser
8785                8790                8795                8800
Pro Glu Arg Leu Thr Tyr Thr Glu Arg Gln Arg Ser Thr Ile Thr Leu
                8805                8810                8815
Asp Trp Lys Glu Pro Arg Ser Asn Gly Gly Ser Pro Ile Gln Gly Tyr
            8820                8825                8830
Ile Ile Glu Lys Arg Arg His Asp Lys Pro Asp Phe Glu Arg Val Asn
        8835                8840                8845
Lys Arg Leu Cys Pro Thr Thr Ser Phe Leu Val Glu Asn Leu Asp Glu
    8850                8855                8860
His Gln Met Tyr Glu Phe Arg Val Lys Ala Val Asn Glu Ile Gly Glu
8865                8870                8875                8880
Ser Glu Pro Ser Leu Pro Leu Asn Val Val Ile Gln Asp Asp Glu Val
                8885                8890                8895
Pro Pro Thr Ile Lys Leu Arg Leu Ser Val Arg Gly Asp Thr Ile Lys
            8900                8905                8910
Val Lys Ala Gly Glu Pro Val His Ile Pro Ala Asp Val Thr Gly Leu
        8915                8920                8925
Pro Met Pro Lys Ile Glu Trp Ser Lys Asn Glu Thr Val Ile Glu Lys
    8930                8935                8940
Pro Thr Asp Ala Leu Gln Ile Thr Lys Glu Glu Val Ser Arg Ser Glu
8945                8950                8955                8960
Ala Lys Thr Glu Leu Ser Ile Pro Lys Ala Val Arg Glu Asp Lys Gly
                8965                8970                8975
Thr Tyr Thr Val Thr Ala Ser Asn Arg Leu Gly Ser Val Phe Arg Asn
            8980                8985                8990
Val His Val Glu Val Tyr Asp Arg Pro Ser Pro Pro Arg Asn Leu Ala
        8995                9000                9005
Val Thr Asp Ile Lys Ala Glu Ser Cys Tyr Leu Thr Trp Asp Ala Pro
    9010                9015                9020
Leu Asp Asn Gly Gly Ser Glu Ile Thr His Tyr Val Ile Asp Lys Arg
9025                9030                9035                9040
Asp Ala Ser Arg Lys Lys Ala Glu Trp Glu Glu Val Thr Asn Thr Ala
                9045                9050                9055
Val Glu Lys Arg Tyr Gly Ile Trp Lys Leu Ile Pro Asn Gly Gln Tyr
            9060                9065                9070
Glu Phe Arg Val Arg Ala Val Asn Lys Tyr Gly Ile Ser Asp Glu Cys
        9075                9080                9085
Lys Ser Asp Lys Val Val Ile Gln Asp Pro Tyr Arg Leu Pro Gly Pro
    9090                9095                9100
Pro Gly Lys Pro Lys Val Leu Ala Arg Thr Lys Gly Ser Met Leu Val
9105                9110                9115                9120
Ser Trp Thr Pro Pro Leu Asp Asn Gly Gly Ser Pro Ile Thr Gly Tyr
                9125                9130                9135
Trp Leu Glu Lys Arg Glu Glu Gly Ser Pro Tyr Trp Ser Arg Val Ser
            9140                9145                9150
 
Arg Ala Pro Ile Thr Lys Val Gly Leu Lys Gly Val Glu Phe Asn Val
9155 9160 9165
Pro Arg Leu Leu Glu Gly Val Lys Tyr Gln Phe Arg Ala Met Ala Ile
9170 9175 9180
Asn Ala Ala Gly Ile Gly Pro Pro Ser Glu Pro Ser Asp Pro Glu Val
9185 9190 9195 9200
Ala Gly Asp Pro Ile Phe Pro Pro Gly Pro Pro Ser Cys Pro Glu Val
9205 9210 9215
Lys Asp Lys Thr Lys Ser Ser Ile Ser Leu Gly Trp Lys Pro Pro Ala
9220 9225 9230
Lys Asp Gly Gly Ser Pro Ile Lys Gly Tyr Ile Val Glu Met Gln Glu
9235 9240 9245
Glu Gly Thr Thr Asp Trp Lys Arg Val Asn Glu Pro Asp Lys Leu Ile
9250 9255 9260
Thr Thr Cys Glu Cys Val Val Pro Asn Leu Lys Glu Leu Arg Lys Tyr
9265 9270 9275 9280
Arg Phe Arg Val Lys Ala Val Asn Glu Ala Gly Glu Ser Glu Pro Ser
9285 9290 9295
Asp Thr Thr Gly Glu Ile Pro Ala Thr Asp Ile Gln Glu Glu Pro Glu
9300 9305 9310
Val Phe Ile Asp Ile Gly Ala Gln Asp Cys Leu Val Cys Lys Ala Gly
9315 9320 9325
Ser Gln Ile Arg Ile Pro Ala Val Ile Lys Gly Arg Pro Thr Pro Lys
9330 9335 9340
Ser Ser Trp Glu Phe Asp Gly Lys Ala Lys Lys Ala Met Lys Asp Gly
9345 9350 9355 9360
Val His Asp Ile Pro Glu Asp Ala Gln Leu Glu Thr Ala Glu Asn Ser
9365 9370 9375
Ser Val Ile Ile Ile Pro Glu Cys Lys Arg Ser His Thr Gly Lys Tyr
9380 9385 9390
Ser Ile Thr Ala Lys Asn Lys Ala Gly Gln Lys Thr Ala Asn Cys Arg
9395 9400 9405
Val Lys Val Met Asp Val Pro Gly Pro Pro Lys Asp Leu Lys Val Ser
9410 9415 9420
Asp Ile Thr Arg Gly Ser Cys Arg Leu Ser Trp Lys Met Pro Asp Asp
9425 9430 9435 9440
Asp Gly Gly Asp Arg Ile Lys Gly Tyr Val Ile Glu Lys Arg Thr Ile
9445 9450 9455
Asp Gly Lys Ala Trp Thr Lys Val Asn Pro Asp Cys Gly Ser Thr Thr
9460 9465 9470
Phe Val Val Pro Asp Leu Leu Ser Glu Gln Gln Tyr Phe Phe Arg Val
9475 9480 9485
Arg Ala Glu Asn Arg Phe Gly Ile Gly Pro Pro Val Glu Thr Ile Gln
9490 9495 9500
Arg Thr Thr Ala Arg Asp Pro Ile Tyr Pro Pro Asp Pro Pro Ile Lys
9505 9510 9515 9520
Leu Lys Ile Gly Leu Ile Thr Lys Asn Thr Val His Leu Ser Trp Lys
9525 9530 9535
Pro Pro Lys Asn Asp Gly Gly Ser Pro Val Thr His Tyr Ile Val Glu
9540 9545 9550
Cys Leu Ala Trp Asp Pro Thr Gly Thr Lys Lys Glu Ala Trp Arg Gln
9555 9560 9565
Cys Asn Lys Arg Asp Val Glu Glu Leu Gln Phe Thr Val Glu Asp Leu
9570 9575 9580
Val Glu Gly Gly Glu Tyr Glu Phe Arg Val Lys Ala Val Asn Ala Ala
9585 9590 9595 9600
Gly Val Ser Lys Pro Ser Ala Thr Val Gly Pro Cys Asp Cys Gln Arg
9605 9610 9615
Pro Asp Met Pro Pro Ser Ile Asp Leu Lys Glu Phe Met Glu Val Glu
9620 9625 9630
Glu Gly Thr Asn Val Asn Ile Val Ala Lys Ile Lys Gly Val Pro Phe
9635 9640 9645
 
Pro Thr Leu Thr Trp Phe Lys Ala Pro Pro Lys Lys Pro Asp Asn Lys
9650 9655 9660
Glu Pro Val Leu Tyr Asp Thr His Val Asn Lys Leu Val Val Asp Asp
9665 9670 9675 9680
Thr Cys Thr Leu Val Ile Pro Gln Ser Arg Arg Ser Asp Thr Gly Leu
9685 9690 9695
Tyr Thr Ile Thr Ala Val Asn Asn Leu Gly Thr Ala Ser Lys Glu Met
9700 9705 9710
Arg Leu Asn Val Leu Gly Arg Pro Gly Pro Pro Val Gly Pro Ile Lys
9715 9720 9725
Phe Glu Ser Val Ser Ala Asp Gln Met Thr Leu Ser Trp Phe Pro Pro
9730 9735 9740
Lys Asp Asp Gly Gly Ser Lys Ile Thr Asn Tyr Val Ile Glu Lys Arg
9745 9750 9755 9760
Glu Ala Asn Arg Lys Thr Trp Val His Val Ser Ser Glu Pro Lys Glu
9765 9770 9775
Cys Thr Tyr Thr Ile Pro Lys Leu Leu Glu Gly His Glu Tyr Val Phe
9780 9785 9790
Arg Ile Met Ala Gln Asn Lys Tyr Gly Ile Gly Glu Pro Leu Asp Ser
9795 9800 9805
Glu Pro Glu Thr Ala Arg Asn Leu Phe Ser Val Pro Gly Ala Pro Asp
9810 9815 9820
Lys Pro Thr Val Ser Ser Val Thr Arg Asn Ser Met Thr Val Asn Trp
9825 9830 9835 9840
Glu Glu Pro Glu Tyr Asp Gly Gly Ser Pro Val Thr Gly Tyr Trp Leu
9845 9850 9855
Glu Met Lys Asp Thr Thr Ser Lys Arg Trp Lys Arg Val Asn Arg Asp
9860 9865 9870
Pro Ile Lys Ala Met Thr Leu Gly Val Ser Tyr Lys Val Thr Gly Leu
9875 9880 9885
Ile Glu Gly Ser Asp Tyr Gln Phe Arg Val Tyr Ala Ile Asn Ala Ala
9890 9895 9900
Gly Val Gly Pro Ala Ser Leu Pro Ser Asp Pro Ala Thr Ala Arg Asp
9905 9910 9915 9920
Pro Ile Ala Pro Pro Gly Pro Pro Phe Pro Lys Val Thr Asp Trp Thr
9925 9930 9935
Lys Ser Ser Ala Asp Leu Glu Trp Ser Pro Pro Leu Lys Asp Gly Gly
9940 9945 9950
Ser Lys Val Thr Gly Tyr Ile Val Glu Tyr Lys Glu Glu Gly Lys Glu
9955 9960 9965
Glu Trp Glu Lys Gly Lys Asp Lys Glu Val Arg Gly Thr Lys Leu Val
9970 9975 9980
Val Thr Gly Leu Lys Glu Gly Ala Phe Tyr Lys Phe Arg Val Ser Ala
9985 9990 9995 10000
Val Asn Ile Ala Gly Ile Gly Glu Pro Gly Glu Val Thr Asp Val Ile
10005 10010 10015
Glu Met Lys Asp Arg Leu Val Ser Pro Asp Leu Gln Leu Asp Ala Ser
10020 10025 10030
Val Arg Asp Arg Ile Val Val His Ala Gly Gly Val Ile Arg Ile Ile
10035 10040 10045
Ala Tyr Val Ser Gly Lys Pro Pro Pro Thr Val Thr Trp Asn Met Asn
10050 10055 10060 1
Glu Arg Thr Leu Pro Gln Glu Ala Thr Ile Glu Thr Thr Ala Ile Ser
0065 10070 10075 10080
Ser Ser Met Val Ile Lys Asn Cys Gln Arg Ser His Gln Gly Val Tyr
10085 10090 10095
Ser Leu Leu Ala Lys Asn Glu Ala Gly Glu Arg Lys Lys Thr Ile Ile
10100 10105 10110
Val Asp Val Leu Asp Val Pro Gly Pro Val Gly Thr Pro Phe Leu Ala
10115 10120 10125
His Asn Leu Thr Asn Glu Ser Cys Lys Leu Thr Trp Phe Ser Pro Glu
10130 10135 10140 1
 
Asp Asp Gly Gly Ser Pro Ile Thr Asn Tyr Val Ile Glu Lys Arg Glu
0145                10150               10155               10160
Ser Asp Arg Arg Ala Trp Thr Pro Val Thr Tyr Thr Val Thr Arg Gln
                10165               10170               10175
Asn Ala Thr Val Gln Gly Leu Ile Gln Gly Lys Ala Tyr Phe Phe Arg
            10180               10185               10190
Ile Ala Ala Glu Asn Ser Ile Gly Met Gly Pro Phe Val Glu Thr Ser
        10195               10200               10205
Glu Ala Leu Val Ile Arg Glu Pro Ile Thr Val Pro Glu Arg Pro Glu
    10210               10215               10220               1
Asp Leu Glu Val Lys Glu Val Thr Lys Asn Thr Val Thr Leu Thr Trp
0225                10230               10235               10240
Asn Pro Pro Lys Tyr Asp Gly Gly Ser Glu Ile Ile Asn Tyr Val Leu
                10245               10250               10255
Glu Ser Arg Leu Ile Gly Thr Glu Lys Phe His Lys Val Thr Asn Asp
            10260               10265               10270
Asn Leu Leu Ser Arg Lys Tyr Thr Val Lys Gly Leu Lys Glu Gly Asp
        10275               10280               10285
Thr Tyr Glu Tyr Arg Val Ser Ala Val Asn Ile Val Gly Gln Gly Lys
    10290               10295               10300               1
Pro Ser Phe Cys Thr Lys Pro Ile Thr Cys Lys Asp Glu Leu Ala Pro
0305                10310               10315               10320
Pro Thr Leu His Leu Asp Phe Arg Asp Lys Leu Thr Ile Arg Val Gly
                10325               10330               10335
Glu Ala Phe Ala Leu Thr Gly Arg Tyr Ser Gly Lys Pro Lys Pro Lys
            10340               10345               10350
Val Ser Trp Phe Lys Asp Glu Ala Asp Val Leu Glu Asp Asp Arg Thr
        10355               10360               10365
His Ile Lys Thr Thr Pro Ala Thr Leu Ala Leu Glu Lys Ile Lys Ala
    10370               10375               10380               1
Lys Arg Ser Asp Ser Gly Lys Tyr Cys Val Val Val Glu Asn Ser Thr
0385                10390               10395               10400
Gly Ser Arg Lys Gly Phe Cys Gln Val Asn Val Val Asp His Pro Gly
                10405               10410               10415
Pro Pro Val Gly Pro Val Ser Phe Asp Glu Val Thr Lys Asp Tyr Met
            10420               10425               10430
Val Ile Ser Trp Lys Pro Pro Leu Asp Asp Gly Gly Ser Lys Ile Thr
        10435               10440               10445
Asn Tyr Ile Ile Glu Lys Lys Glu Val Gly Lys Asp Val Trp Met Pro
    10450               10455               10460               1
Val Thr Ser Ala Ser Ala Lys Thr Thr Cys Lys Val Ser Lys Leu Leu
0465                10470               10475               10480
Glu Gly Lys Asp Tyr Ile Phe Arg Ile His Ala Glu Asn Leu Tyr Gly
                10485               10490               10495
Ile Ser Asp Pro Leu Val Ser Asp Ser Met Lys Ala Lys Asp Arg Phe
            10500               10505               10510
Arg Val Pro Asp Ala Pro Asp Gln Pro Ile Val Thr Glu Val Thr Lys
        10515               10520               10525
Asp Ser Ala Leu Val Thr Trp Asn Lys Pro His Asp Gly Gly Lys Pro
    10530               10535               10540               1
Ile Thr Asn Tyr Ile Leu Glu Lys Arg Glu Thr Met Ser Lys Arg Trp
0545                10550               10555               10560
Ala Arg Val Thr Lys Asp Pro Ile His Pro Tyr Thr Lys Phe Arg Val
                10565               10570               10575
Pro Asp Leu Leu Glu Gly Cys Gln Tyr Glu Phe Arg Val Ser Ala Glu
            10580               10585               10590
Asn Glu Ile Gly Ile Gly Asp Pro Ser Pro Pro Ser Lys Pro Val Phe
        10595               10600               10605
Ala Lys Asp Pro Ile Ala Lys Pro Ser Pro Pro Val Asn Pro Glu Ala
    10610               10615               10620               1
Ile Asp Thr Thr Cys Asn Ser Val Asp Leu Thr Trp Gln Pro Pro Arg
0625                10630               10635               10640
 
His Asp Gly Gly Ser Lys Ile Leu Gly Tyr Ile Val Glu Tyr Gln Lys
10645 10650 10655
Val Gly Asp Glu Glu Trp Arg Arg Ala Asn His Thr Pro Glu Ser Cys
10660 10665 10670
Pro Glu Thr Lys Tyr Lys Val Thr Gly Leu Arg Asp Gly Gln Thr Tyr
10675 10680 10685
Lys Phe Arg Val Leu Ala Val Asn Ala Ala Gly Glu Ser Asp Pro Ala
10690 10695 10700 1
His Val Pro Glu Pro Val Leu Val Lys Asp Arg Leu Glu Pro Pro Glu
0705 10710 10715 10720
Leu Ile Leu Asp Ala Asn Met Ala Arg Glu Gln His Ile Lys Val Gly
10725 10730 10735
Asp Thr Leu Arg Leu Ser Ala Ile Ile Lys Gly Val Pro Phe Pro Lys
10740 10745 10750
Val Thr Trp Lys Lys Glu Asp Arg Asp Ala Pro Thr Lys Ala Arg Ile
10755 10760 10765
Asp Val Thr Pro Val Gly Ser Lys Leu Glu Ile Arg Asn Ala Ala His
10770 10775 10780 1
Glu Asp Gly Gly Ile Tyr Ser Leu Thr Val Glu Asn Pro Ala Gly Ser
0785 10790 10795 10800
Lys Thr Val Ser Val Lys Val Leu Val Leu Asp Lys Pro Gly Pro Pro
10805 10810 10815
Arg Asp Leu Glu Val Ser Glu Ile Arg Lys Asp Ser Cys Tyr Leu Thr
10820 10825 10830
Trp Lys Glu Pro Leu Asp Asp Gly Gly Ser Val Ile Thr Asn Tyr Val
10835 10840 10845
Val Glu Arg Arg Asp Val Ala Ser Ala Gln Trp Ser Pro Leu Ser Ala
10850 10855 10860 1
Thr Ser Lys Lys Lys Ser His Phe Ala Lys His Leu Asn Glu Gly Asn
0865 10870 10875 10880
Gln Tyr Leu Phe Arg Val Ala Ala Glu Asn Gln Tyr Gly Arg Gly Pro
10885 10890 10895
Phe Val Glu Thr Pro Lys Pro Ile Lys Ala Leu Asp Pro Leu His Pro
10900 10905 10910
Pro Gly Pro Pro Lys Asp Leu His His Val Asp Val Asp Lys Thr Glu
10915 10920 10925
Val Ser Leu Val Trp Asn Lys Pro Asp Arg Asp Gly Gly Ser Pro Ile
10930 10935 10940 1
Thr Gly Tyr Leu Val Glu Tyr Gln Glu Glu Gly Thr Gln Asp Trp Ile
0945 10950 10955 10960
Lys Phe Lys Thr Val Thr Asn Leu Glu Cys Val Val Thr Gly Leu Gln
10965 10970 10975
Gln Gly Lys Thr Tyr Arg Phe Arg Val Lys Ala Glu Asn Ile Val Gly
10980 10985 10990
Leu Gly Leu Pro Asp Thr Thr Ile Pro Ile Glu Cys Gln Glu Lys Leu
10995 11000 11005
Val Pro Pro Ser Val Glu Leu Asp Val Lys Leu Ile Glu Gly Leu Val
11010 11015 11020 1
Val Lys Ala Gly Thr Thr Val Arg Phe Pro Ala Ile Ile Arg Gly Val
1025 11030 11035 11040
Pro Val Pro Thr Ala Lys Trp Thr Thr Asp Gly Ser Glu Ile Lys Thr
11045 11050 11055
Asp Glu His Tyr Thr Val Glu Thr Asp Asn Phe Ser Ser Val Leu Thr
11060 11065 11070
Ile Lys Asn Cys Leu Arg Arg Asp Thr Gly Glu Tyr Gln Ile Thr Val
11075 11080 11085
Ser Asn Ala Ala Gly Ser Lys Thr Val Ala Val His Leu Thr Val Leu
11090 11095 11100 1
Asp Val Pro Gly Pro Pro Thr Gly Pro Ile Asn Ile Leu Asp Val Thr
1105 11110 11115 11120
Pro Glu His Met Thr Ile Ser Trp Gln Pro Pro Lys Asp Asp Gly Gly
11125 11130 11135
 
Ser Pro Val Ile Asn Tyr Ile Val Glu Lys Gln Asp Thr Arg Lys Asp
            11140               11145               11150
Thr Trp Gly Val Val Ser Ser Gly Ser Ser Lys Thr Lys Leu Lys Ile
        11155               11160               11165
Pro His Leu Gln Lys Gly Cys Glu Tyr Val Phe Arg Val Arg Ala Glu
    11170               11175               11180               1
Asn Lys Ile Gly Val Gly Pro Pro Leu Asp Ser Thr Pro Thr Val Ala
1185                11190               11195               11200
Lys His Lys Phe Ser Pro Pro Ser Pro Pro Gly Lys Pro Val Val Thr
                11205               11210               11215
Asp Ile Thr Glu Asn Ala Ala Thr Val Ser Trp Thr Leu Pro Lys Ser
            11220               11225               11230
Asp Gly Gly Ser Pro Ile Thr Gly Tyr Tyr Met Glu Arg Arg Glu Val
        11235               11240               11245
Thr Gly Lys Trp Val Arg Val Asn Lys Thr Pro Ile Ala Asp Leu Lys
    11250               11255               11260               1
Phe Arg Val Thr Gly Leu Tyr Glu Gly Asn Thr Tyr Glu Phe Arg Val
1265                11270               11275               11280
Phe Ala Glu Asn Leu Ala Gly Leu Ser Lys Pro Ser Pro Ser Ser Asp
                11285               11290               11295
Pro Ile Lys Ala Cys Arg Pro Ile Lys Pro Pro Gly Pro Pro Ile Asn
            11300               11305               11310
Pro Lys Leu Lys Asp Lys Ser Arg Glu Thr Ala Asp Leu Val Trp Thr
        11315               11320               11325
Lys Pro Leu Ser Asp Gly Gly Ser Pro Ile Leu Gly Tyr Val Val Glu
    11330               11335               11340               1
Cys Gln Lys Pro Gly Thr Ala Gln Trp Asn Arg Ile Asn Lys Asp Glu
1345                11350               11355               11360
Leu Ile Arg Gln Cys Ala Phe Arg Val Pro Gly Leu Ile Glu Gly Asn
                11365               11370               11375
Glu Tyr Arg Phe Arg Ile Lys Ala Ala Asn Ile Val Gly Glu Gly Glu
            11380               11385               11390
Pro Arg Glu Leu Ala Glu Ser Val Ile Ala Lys Asp Ile Leu His Pro
        11395               11400               11405
Pro Glu Val Glu Leu Asp Val Thr Cys Arg Asp Val Ile Thr Val Arg
    11410               11415               11420               1
Val Gly Gln Thr Ile Arg Ile Leu Ala Arg Val Lys Gly Arg Pro Glu
1425                11430               11435               11440
Pro Asp Ile Thr Trp Thr Lys Glu Gly Lys Val Leu Val Arg Glu Lys
                11445               11450               11455
Arg Val Asp Leu Ile Gln Asp Leu Pro Arg Val Glu Leu Gln Ile Lys
            11460               11465               11470
Glu Ala Val Arg Ala Asp His Gly Lys Tyr Ile Ile Ser Ala Lys Asn
        11475               11480               11485
Ser Ser Gly His Ala Gln Gly Ser Ala Ile Val Asn Val Leu Asp Arg
    11490               11495               11500               1
Pro Gly Pro Cys Gln Asn Leu Lys Val Thr Asn Val Thr Lys Glu Asn
1505                11510               11515               11520
Cys Thr Ile Ser Trp Glu Asn Pro Leu Asp Asn Gly Gly Ser Glu Ile
                11525               11530               11535
Thr Asn Phe Ile Val Glu Tyr Arg Lys Pro Asn Gln Lys Gly Trp Ser
            11540               11545               11550
Ile Val Ala Ser Asp Val Thr Lys Arg Leu Ile Lys Ala Asn Leu Leu
        11555               11560               11565
Ala Asn Asn Glu Tyr Tyr Phe Arg Val Cys Ala Glu Asn Lys Val Gly
    11570               11575               11580               1
Val Gly Pro Thr Ile Glu Thr Lys Thr Pro Ile Leu Ala Ile Asn Pro
1585                11590               11595               11600
Ile Asp Arg Pro Gly Glu Pro Glu Asn Leu His Ile Ala Asp Lys Gly
                11605               11610               11615
Lys Thr Phe Val Tyr Leu Lys Trp Arg Arg Pro Asp Tyr Asp Gly Gly
            11620               11625               11630
 
Ser Pro Asn Leu Ser Tyr His Val Glu Arg Arg Leu Lys Gly Ser Asp
        11635               11640               11645
Asp Trp Glu Arg Val His Lys Gly Ser Ile Lys Glu Thr His Tyr Met
    11650               11655               11660               1
Val Asp Arg Cys Val Glu Asn Gln Ile Tyr Glu Phe Arg Val Gln Thr
1665                11670               11675               11680
Lys Asn Glu Gly Gly Glu Ser Asp Trp Val Lys Thr Glu Glu Val Val
                11685               11690               11695
Val Lys Glu Asp Leu Gln Lys Pro Val Leu Asp Leu Lys Leu Ser Gly
            11700               11705               11710
Val Leu Thr Val Lys Ala Gly Asp Thr Ile Arg Leu Glu Ala Gly Val
        11715               11720               11725
Arg Gly Lys Pro Phe Pro Glu Val Ala Trp Thr Lys Asp Lys Asp Ala
    11730               11735               11740               1
Thr Asp Leu Thr Arg Ser Pro Arg Val Lys Ile Asp Thr Arg Ala Asp
1745                11750               11755               11760
Ser Ser Lys Phe Ser Leu Thr Lys Ala Lys Arg Ser Asp Gly Gly Lys
                11765               11770               11775
Tyr Val Val Thr Ala Thr Asn Thr Ala Gly Ser Phe Val Ala Tyr Ala
            11780               11785               11790
Thr Val Asn Val Leu Asp Lys Pro Gly Pro Val Arg Asn Leu Lys Ile
        11795               11800               11805
Val Asp Val Ser Ser Asp Arg Cys Thr Val Cys Trp Asp Pro Pro Glu
    11810               11815               11820               1
Asp Asp Gly Gly Cys Glu Ile Gln Asn Tyr Ile Leu Glu Lys Cys Glu
1825                11830               11835               11840
Thr Lys Arg Met Val Trp Ser Thr Tyr Ser Ala Thr Val Leu Thr Pro
                11845               11850               11855
Gly Thr Thr Val Thr Arg Leu Ile Glu Gly Asn Glu Tyr Ile Phe Arg
            11860               11865               11870
Val Arg Ala Glu Asn Lys Ile Gly Thr Gly Pro Pro Thr Glu Ser Lys
        11875               11880               11885
Pro Val Ile Ala Lys Thr Lys Tyr Asp Lys Pro Gly Arg Pro Asp Pro
    11890               11895               11900               1
Pro Glu Val Thr Lys Val Ser Lys Glu Glu Met Thr Val Val Trp Asn
1905                11910               11915               11920
Pro Pro Glu Tyr Asp Gly Gly Lys Ser Ile Thr Gly Tyr Phe Leu Glu
                11925               11930               11935
Lys Lys Glu Lys His Ser Thr Arg Trp Val Pro Val Asn Lys Ser Ala
            11940               11945               11950
Ile Pro Glu Arg Arg Met Lys Val Gln Asn Leu Leu Pro Asp His Glu
        11955               11960               11965
Tyr Gln Phe Arg Val Lys Ala Glu Asn Glu Ile Gly Ile Gly Glu Pro
    11970               11975               11980               1
Ser Leu Pro Ser Arg Pro Val Val Ala Lys Asp Pro Ile Glu Pro Pro
1985                11990               11995               12000
Gly Pro Pro Thr Asn Phe Arg Val Val Asp Thr Thr Lys His Ser Ile
                12005               12010               12015
Thr Leu Gly Trp Gly Lys Pro Val Tyr Asp Gly Gly Ala Pro Ile Ile
            12020               12025               12030
Gly Tyr Val Val Glu Met Arg Pro Lys Ile Ala Asp Ala Ser Pro Asp
        12035               12040               12045
Glu Gly Trp Lys Arg Cys Asn Ala Ala Ala Gln Leu Val Arg Lys Glu
    12050               12055               12060               1
Phe Thr Val Thr Ser Leu Asp Glu Asn Gln Glu Tyr Glu Phe Arg Val
2065                12070               12075               12080
Cys Ala Gln Asn Gln Val Gly Ile Gly Arg Pro Ala Glu Leu Lys Glu
                12085               12090               12095
Ala Ile Lys Pro Lys Glu Ile Leu Glu Pro Pro Glu Ile Asp Leu Asp
            12100               12105               12110
Ala Ser Met Arg Lys Leu Val Ile Val Arg Ala Gly Cys Pro Ile Arg
        12115               12120               12125
 
Leu Phe Ala Ile Val Arg Gly Arg Pro Ala Pro Lys Val Thr Trp Arg
12130 12135 12140 1
Lys Val Gly Ile Asp Asn Val Val Arg Lys Gly Gln Val Asp Leu Val
2145 12150 12155 12160
Asp Thr Met Ala Phe Leu Val Ile Pro Asn Ser Thr Arg Asp Asp Ser
12165 12170 12175
Gly Lys Tyr Ser Leu Thr Leu Val Asn Pro Ala Gly Glu Lys Ala Val
12180 12185 12190
Phe Val Asn Val Arg Val Leu Asp Thr Pro Gly Pro Val Ser Asp Leu
12195 12200 12205
Lys Val Ser Asp Val Thr Lys Thr Ser Cys His Val Ser Trp Ala Pro
12210 12215 12220 1
Pro Glu Asn Asp Gly Gly Ser Gln Val Thr His Tyr Ile Val Glu Lys
2225 12230 12235 12240
Arg Glu Ala Asp Arg Lys Thr Trp Ser Thr Val Thr Pro Glu Val Lys
12245 12250 12255
Lys Thr Ser Phe His Val Thr Asn Leu Val Pro Gly Asn Glu Tyr Tyr
12260 12265 12270
Phe Arg Val Thr Ala Val Asn Glu Tyr Gly Pro Gly Val Pro Thr Asp
12275 12280 12285
Val Pro Lys Pro Val Leu Ala Ser Asp Pro Leu Ser Glu Pro Asp Pro
12290 12295 12300 1
Pro Arg Lys Leu Glu Ala Thr Glu Met Thr Lys Asn Ser Ala Thr Leu
2305 12310 12315 12320
Ala Trp Leu Pro Pro Leu Arg Asp Gly Gly Ala Lys Ile Asp Gly Tyr
12325 12330 12335
Ile Ile Ser Tyr Arg Glu Glu Glu Gln Pro Ala Asp Arg Trp Thr Glu
12340 12345 12350
Tyr Ser Val Val Lys Asp Leu Ser Leu Val Val Thr Gly Leu Lys Glu
12355 12360 12365
Gly Lys Lys Tyr Lys Phe Arg Val Ala Ala Arg Asn Ala Val Gly Val
12370 12375 12380 1
Ser Leu Pro Arg Glu Ala Glu Gly Val Tyr Glu Ala Lys Glu Gln Leu
2385 12390 12395 12400
Leu Pro Pro Lys Ile Leu Met Pro Glu Gln Ile Thr Ile Lys Ala Gly
12405 12410 12415
Lys Lys Leu Arg Ile Glu Ala His Val Tyr Gly Lys Pro His Pro Thr
12420 12425 12430
Cys Lys Trp Lys Lys Gly Glu Asp Glu Val Val Thr Ser Ser His Leu
12435 12440 12445
Ala Val His Lys Ala Asp Ser Ser Ser Ile Leu Ile Ile Lys Asp Val
12450 12455 12460 1
Thr Arg Lys Asp Ser Gly Tyr Tyr Ser Leu Thr Ala Glu Asn Ser Ser
2465 12470 12475 12480
Gly Thr Asp Thr Gln Lys Ile Lys Val Val Val Met Asp Ala Pro Gly
12485 12490 12495
Pro Pro Gln Pro Pro Phe Asp Ile Ser Asp Ile Asp Ala Asp Ala Cys
12500 12505 12510
Ser Leu Ser Trp His Ile Pro Leu Glu Asp Gly Gly Ser Asn Ile Thr
12515 12520 12525
Asn Tyr Ile Val Glu Lys Cys Asp Val Ser Arg Gly Asp Trp Val Thr
12530 12535 12540 1
Ala Leu Ala Ser Val Thr Lys Thr Ser Cys Arg Val Gly Lys Leu Ile
2545 12550 12555 12560
Pro Gly Gln Glu Tyr Ile Phe Arg Val Arg Ala Glu Asn Arg Phe Gly
12565 12570 12575
Ile Ser Glu Pro Leu Thr Ser Pro Lys Met Val Ala Gln Phe Pro Phe
12580 12585 12590
Gly Val Pro Ser Glu Pro Lys Asn Ala Arg Val Thr Lys Val Asn Lys
12595 12600 12605
Asp Cys Ile Phe Val Ala Trp Asp Arg Pro Asp Ser Asp Gly Gly Ser
12610 12615 12620 1
 
Pro Ile Ile Gly Tyr Leu Ile Glu Arg Lys Glu Arg Asn Ser Leu Leu
2625                12630               12635               12640
Trp Val Lys Ala Asn Asp Thr Leu Val Arg Ser Thr Glu Tyr Pro Cys
            12645               12650               12655
Ala Gly Leu Val Glu Gly Leu Glu Tyr Ser Phe Arg Ile Tyr Ala Leu
        12660               12665               12670
Asn Lys Ala Gly Ser Ser Pro Pro Ser Lys Pro Thr Glu Tyr Val Thr
    12675               12680               12685
Ala Arg Met Pro Val Asp Pro Pro Gly Lys Pro Glu Val Ile Asp Val
12690               12695               12700               1
Thr Lys Ser Thr Val Ser Leu Ile Trp Ala Arg Pro Lys His Asp Gly
2705                12710               12715               12720
Gly Ser Lys Ile Ile Gly Tyr Phe Val Glu Ala Cys Lys Leu Pro Gly
            12725               12730               12735
Asp Lys Trp Val Arg Cys Asn Thr Ala Pro His Gln Ile Pro Gln Glu
        12740               12745               12750
Glu Tyr Thr Ala Thr Gly Leu Glu Glu Lys Ala Gln Tyr Gln Phe Arg
    12755               12760               12765
Ala Ile Ala Arg Thr Ala Val Asn Ile Ser Pro Pro Ser Glu Pro Ser
12770               12775               12780               1
Asp Pro Val Thr Ile Leu Ala Glu Asn Val Pro Pro Arg Ile Asp Leu
2785                12790               12795               12800
Ser Val Ala Met Lys Ser Leu Leu Thr Val Lys Ala Gly Thr Asn Val
            12805               12810               12815
Cys Leu Asp Ala Thr Val Phe Gly Lys Pro Met Pro Thr Val Ser Trp
        12820               12825               12830
Lys Lys Asp Gly Thr Leu Leu Lys Pro Ala Glu Gly Ile Lys Met Ala
    12835               12840               12845
Met Gln Arg Asn Leu Cys Thr Leu Glu Leu Phe Ser Val Asn Arg Lys
12850               12855               12860               1
Asp Ser Gly Asp Tyr Thr Ile Thr Ala Glu Asn Ser Ser Gly Ser Lys
2865                12870               12875               12880
Ser Ala Thr Ile Lys Leu Lys Val Leu Asp Lys Pro Gly Pro Pro Ala
            12885               12890               12895
Ser Val Lys Ile Asn Lys Met Tyr Ser Asp Arg Ala Met Leu Ser Trp
        12900               12905               12910
Glu Pro Pro Leu Glu Asp Gly Gly Ser Glu Ile Thr Asn Tyr Ile Val
    12915               12920               12925
Asp Lys Arg Glu Thr Ser Arg Pro Asn Trp Ala Gln Val Ser Ala Thr
12930               12935               12940               1
Val Pro Ile Thr Ser Cys Ser Val Glu Lys Leu Ile Glu Gly His Glu
2945                12950               12955               12960
Tyr Gln Phe Arg Ile Cys Ala Glu Asn Lys Tyr Gly Val Gly Asp Pro
            12965               12970               12975
Val Phe Thr Glu Pro Ala Ile Ala Lys Asn Pro Tyr Asp Pro Pro Gly
        12980               12985               12990
Arg Cys Asp Pro Pro Val Ile Ser Asn Ile Thr Lys Asp His Met Thr
    12995               13000               13005
Val Ser Trp Lys Pro Pro Ala Asp Asp Gly Gly Ser Pro Ile Thr Gly
13010               13015               13020               1
Tyr Leu Leu Glu Lys Arg Glu Thr Gln Ala Val Asn Trp Thr Lys Val
3025                13030               13035               13040
Asn Arg Lys Pro Ile Ile Glu Arg Thr Leu Lys Ala Thr Gly Leu Gln
            13045               13050               13055
Glu Gly Thr Glu Tyr Glu Phe Arg Val Thr Ala Ile Asn Lys Ala Gly
        13060               13065               13070
Pro Gly Lys Pro Ser Asp Ala Ser Lys Ala Ala Tyr Ala Arg Asp Pro
    13075               13080               13085
Gln Tyr Pro Pro Ala Pro Pro Ala Phe Pro Lys Val Tyr Asp Thr Thr
13090               13095               13100               1
Arg Ser Ser Val Ser Leu Ser Trp Gly Lys Pro Ala Tyr Asp Gly Gly
3105                13110               13115               13120
 
Ser Pro Ile Ile Gly Tyr Leu Val Glu Val Lys Arg Ala Asp Ser Asp
13125 13130 13135
Asn Trp Val Arg Cys Asn Leu Pro Gln Asn Leu Gln Lys Thr Arg Phe
13140 13145 13150
Glu Val Thr Gly Leu Met Glu Asp Thr Gln Tyr Gln Phe Arg Val Tyr
13155 13160 13165
Ala Val Asn Lys Ile Gly Tyr Ser Asp Pro Ser Asp Val Pro Asp Lys
13170 13175 13180 1
His Tyr Pro Lys Asp Ile Leu Ile Pro Pro Glu Gly Glu His Asp Ala
3185 13190 13195 13200
Asp Leu Arg Lys Thr Leu Ile Leu Arg Ala Gly Val Thr Met Arg Leu
13205 13210 13215
Tyr Val Pro Val Lys Gly Arg Pro Pro Pro Lys Ile Thr Trp Ser Lys
13220 13225 13230
Pro Asn Val Asn Leu Arg Asp Arg Ile Gly Leu Asp Ile Lys Ser Thr
13235 13240 13245
Asp Phe Asp Thr Phe Leu Arg Cys Glu Asn Val Asn Lys Tyr Asp Ala
13250 13255 13260 1
Gly Lys Tyr Ile Leu Thr Leu Glu Asn Ser Cys Gly Lys Lys Glu Tyr
3265 13270 13275 13280
Thr Ile Val Val Lys Val Leu Asp Thr Pro Gly Pro Pro Ile Asn Val
13285 13290 13295
Thr Val Lys Glu Ile Ser Lys Asp Ser Ala Tyr Val Thr Trp Glu Pro
13300 13305 13310
Pro Ile Ile Asp Gly Gly Ser Pro Ile Ile Asn Tyr Val Val Gln Lys
13315 13320 13325
Arg Asp Ala Glu Arg Lys Ser Trp Ser Thr Val Thr Thr Glu Cys Ser
13330 13335 13340 1
Lys Thr Ser Phe Arg Val Pro Asn Leu Glu Glu Gly Lys Ser Tyr Phe
3345 13350 13355 13360
Phe Arg Val Phe Ala Glu Asn Glu Tyr Gly Ile Gly Asp Pro Gly Glu
13365 13370 13375
Thr Arg Asp Ala Val Lys Ala Ser Gln Thr Pro Gly Pro Val Val Asp
13380 13385 13390
Leu Lys Val Arg Ser Val Ser Lys Ser Ser Cys Ser Ile Gly Trp Lys
13395 13400 13405
Lys Pro His Ser Asp Gly Gly Ser Arg Ile Ile Gly Tyr Val Val Asp
13410 13415 13420 1
Phe Leu Thr Glu Glu Asn Lys Trp Gln Arg Val Met Lys Ser Leu Ser
3425 13430 13435 13440
Leu Gln Tyr Ser Ala Lys Asp Leu Thr Glu Gly Lys Glu Tyr Thr Phe
13445 13450 13455
Arg Val Ser Ala Glu Asn Glu Asn Gly Glu Gly Thr Pro Ser Glu Ile
13460 13465 13470
Thr Val Val Ala Arg Asp Asp Val Val Ala Pro Asp Leu Asp Leu Lys
13475 13480 13485
Gly Leu Pro Asp Leu Cys Tyr Leu Ala Lys Glu Asn Ser Asn Phe Arg
13490 13495 13500 1
Leu Lys Ile Pro Ile Lys Gly Lys Pro Ala Pro Ser Val Ser Trp Lys
3505 13510 13515 13520
Lys Gly Glu Asp Pro Leu Ala Thr Asp Thr Arg Val Ser Val Glu Ser
13525 13530 13535
Ser Ala Val Asn Thr Thr Leu Ile Val Tyr Asp Cys Gln Lys Ser Asp
13540 13545 13550
Ala Gly Lys Tyr Thr Ile Thr Leu Lys Asn Val Ala Gly Thr Lys Glu
13555 13560 13565
Gly Thr Ile Ser Ile Lys Val Val Gly Lys Pro Gly Ile Pro Thr Gly
13570 13575 13580 1
Pro Ile Lys Phe Asp Glu Val Thr Ala Glu Ala Met Thr Leu Lys Trp
3585 13590 13595 13600
Ala Pro Pro Lys Asp Asp Gly Gly Ser Glu Ile Thr Asn Tyr Ile Leu
13605 13610 13615
 
Glu Lys Arg Asp Ser Val Asn Asn Lys Trp Val Thr Cys Ala Ser Ala
            13620                   13625                   13630
Val Gln Lys Thr Thr Phe Arg Val Thr Arg Leu His Glu Gly Met Glu
        13635                   13640                   13645
Tyr Thr Phe Arg Val Ser Ala Glu Asn Lys Tyr Gly Val Gly Glu Gly
    13650                   13655                   13660                   1
Leu Lys Ser Glu Pro Ile Val Ala Arg His Pro Phe Asp Val Pro Asp
3665                    13670                   13675                   13680
Ala Pro Pro Pro Pro Asn Ile Val Asp Val Arg His Asp Ser Val Ser
                13685                   13690                   13695
Leu Thr Trp Thr Asp Pro Lys Lys Thr Gly Gly Ser Pro Ile Thr Gly
            13700                   13705                   13710
Tyr His Leu Glu Phe Lys Glu Arg Asn Ser Leu Leu Trp Lys Arg Ala
        13715                   13720                   13725
Asn Lys Thr Pro Ile Arg Met Arg Asp Phe Lys Val Thr Gly Leu Thr
    13730                   13735                   13740                   1
Glu Gly Leu Glu Tyr Glu Phe Arg Val Met Ala Ile Asn Leu Ala Gly
3745                    13750                   13755                   13760
Val Gly Lys Pro Ser Leu Pro Ser Glu Pro Val Val Ala Leu Asp Pro
                13765                   13770                   13775
Ile Asp Pro Pro Gly Lys Pro Glu Val Ile Asn Ile Thr Arg Asn Ser
            13780                   13785                   13790
Val Thr Leu Ile Trp Thr Glu Pro Lys Tyr Asp Gly Gly His Lys Leu
        13795                   13800                   13805
Thr Gly Tyr Ile Val Glu Lys Arg Asp Leu Pro Ser Lys Ser Trp Met
    13810                   13815                   13820                   1
Lys Ala Asn His Val Asn Val Pro Glu Cys Ala Phe Thr Val Thr Asp
3825                    13830                   13835                   13840
Leu Val Glu Gly Gly Lys Tyr Glu Phe Arg Ile Arg Ala Lys Asn Thr
                13845                   13850                   13855
Ala Gly Ala Ile Ser Ala Pro Ser Glu Ser Thr Glu Thr Ile Ile Cys
            13860                   13865                   13870
Lys Asp Glu Tyr Glu Ala Pro Thr Ile Val Leu Asp Pro Thr Ile Lys
        13875                   13880                   13885
Asp Gly Leu Thr Ile Lys Ala Gly Asp Thr Ile Val Leu Asn Ala Ile
    13890                   13895                   13900                   1
Ser Ile Leu Gly Lys Pro Leu Pro Lys Ser Ser Trp Ser Lys Ala Gly
3905                    13910                   13915                   13920
Lys Asp Ile Arg Pro Ser Asp Ile Thr Gln Ile Thr Ser Thr Pro Thr
                13925                   13930                   13935
Ser Ser Met Leu Thr Ile Lys Tyr Ala Thr Arg Lys Asp Ala Gly Glu
            13940                   13945                   13950
Tyr Thr Ile Thr Ala Thr Asn Pro Phe Gly Thr Lys Val Glu His Val
        13955                   13960                   13965
Lys Val Thr Val Leu Asp Val Pro Gly Pro Pro Gly Pro Val Glu Ile
    13970                   13975                   13980                   1
Ser Asn Val Ser Ala Glu Lys Ala Thr Leu Thr Trp Thr Pro Pro Leu
3985                    13990                   13995                   14000
Glu Asp Gly Gly Ser Pro Ile Lys Ser Tyr Ile Leu Glu Lys Arg Glu
                14005                   14010                   14015
Thr Ser Arg Leu Leu Trp Thr Val Val Ser Glu Asp Ile Gln Ser Cys
            14020                   14025                   14030
Arg His Val Ala Thr Lys Leu Ile Gln Gly Asn Glu Tyr Ile Phe Arg
        14035                   14040                   14045
Val Ser Ala Val Asn His Tyr Gly Lys Gly Glu Pro Val Gln Ser Glu
    14050                   14055                   14060                   1
Pro Val Lys Met Val Asp Arg Phe Gly Pro Pro Gly Pro Pro Glu Lys
4065                    14070                   14075                   14080
Pro Glu Val Ser Asn Val Thr Lys Asn Thr Ala Thr Val Ser Trp Lys
                14085                   14090                   14095
Arg Pro Val Asp Asp Gly Gly Ser Glu Ile Thr Gly Tyr His Val Glu
            14100                   14105                   14110
 
Arg Arg Glu Lys Lys Ser Leu Arg Trp Val Arg Ala Ile Lys Thr Pro
14115 14120 14125
Val Ser Asp Leu Arg Cys Lys Val Thr Gly Leu Gln Glu Gly Ser Thr
14130 14135 14140 1
Tyr Glu Phe Arg Val Ser Ala Glu Asn Arg Ala Gly Ile Gly Pro Pro
4145 14150 14155 14160
Ser Glu Ala Ser Asp Ser Val Leu Met Lys Asp Ala Ala Tyr Pro Pro
14165 14170 14175
Gly Pro Pro Ser Asn Pro His Val Thr Asp Thr Thr Lys Lys Ser Ala
14180 14185 14190
Ser Leu Ala Trp Gly Lys Pro His Tyr Asp Gly Gly Leu Glu Ile Thr
14195 14200 14205
Gly Tyr Val Val Glu His Gln Lys Val Gly Asp Glu Ala Trp Ile Lys
14210 14215 14220 1
Asp Thr Thr Gly Thr Ala Leu Arg Ile Thr Gln Phe Val Val Pro Asp
4225 14230 14235 14240
Leu Gln Thr Lys Glu Lys Tyr Asn Phe Arg Ile Ser Ala Ile Asn Asp
14245 14250 14255
Ala Gly Val Gly Glu Pro Ala Val Ile Pro Asp Val Glu Ile Val Glu
14260 14265 14270
Arg Glu Met Ala Pro Asp Phe Glu Leu Asp Ala Glu Leu Arg Arg Thr
14275 14280 14285
Leu Val Val Arg Ala Gly Leu Ser Ile Arg Ile Phe Val Pro Ile Lys
14290 14295 14300 1
Gly Arg Pro Ala Pro Glu Val Thr Trp Thr Lys Asp Asn Ile Asn Leu
4305 14310 14315 14320
Lys Asn Arg Ala Asn Ile Glu Asn Thr Glu Ser Phe Thr Leu Leu Ile
14325 14330 14335
Ile Pro Glu Cys Asn Arg Tyr Asp Thr Gly Lys Phe Val Met Thr Ile
14340 14345 14350
Glu Asn Pro Ala Gly Lys Lys Ser Gly Phe Val Asn Val Arg Val Leu
14355 14360 14365
Asp Thr Pro Gly Pro Val Leu Asn Leu Arg Pro Thr Asp Ile Thr Lys
14370 14375 14380 1
Asp Ser Val Thr Leu His Trp Asp Leu Pro Leu Ile Asp Gly Gly Ser
4385 14390 14395 14400
Arg Ile Thr Asn Tyr Ile Val Glu Lys Arg Glu Ala Thr Arg Lys Ser
14405 14410 14415
Tyr Ser Thr Ala Thr Thr Lys Cys His Lys Cys Thr Tyr Lys Val Thr
14420 14425 14430
Gly Leu Ser Glu Gly Cys Glu Tyr Phe Phe Arg Val Met Ala Glu Asn
14435 14440 14445
Glu Tyr Gly Ile Gly Glu Pro Thr Glu Thr Thr Glu Pro Val Lys Ala
14450 14455 14460 1
Ser Glu Ala Pro Ser Pro Pro Asp Ser Leu Asn Ile Met Asp Ile Thr
4465 14470 14475 14480
Lys Ser Thr Val Ser Leu Ala Trp Pro Lys Pro Lys His Asp Gly Gly
14485 14490 14495
Ser Lys Ile Thr Gly Tyr Val Ile Glu Ala Gln Arg Lys Gly Ser Asp
14500 14505 14510
Gln Trp Thr His Ile Thr Thr Val Lys Gly Leu Glu Cys Val Val Arg
14515 14520 14525
Asn Leu Thr Glu Gly Glu Glu Tyr Thr Phe Gln Val Met Ala Val Asn
14530 14535 14540 1
Ser Ala Gly Arg Ser Ala Pro Arg Glu Ser Arg Pro Val Ile Val Lys
4545 14550 14555 14560
Glu Gln Thr Met Leu Pro Glu Leu Asp Leu Arg Gly Ile Tyr Gln Lys
14565 14570 14575
Leu Val Ile Ala Lys Ala Gly Asp Asn Ile Lys Val Glu Ile Pro Val
14580 14585 14590
Leu Gly Arg Pro Lys Pro Thr Val Thr Trp Lys Lys Gly Asp Gln Ile
14595 14600 14605
 
Leu Lys Gln Thr Gln Arg Val Asn Phe Glu Thr Thr Ala Thr Ser Thr
14610 14615 14620 1
Ile Leu Asn Ile Asn Glu Cys Val Arg Ser Asp Ser Gly Pro Tyr Pro
4625 14630 14635 14640
Leu Thr Ala Arg Asn Ile Val Gly Glu Val Gly Asp Val Ile Thr Ile
14645 14650 14655
Gln Val His Asp Ile Pro Gly Pro Pro Thr Gly Pro Ile Lys Phe Asp
14660 14665 14670
Glu Val Ser Ser Asp Phe Val Thr Phe Ser Trp Asp Pro Pro Glu Asn
14675 14680 14685
Asp Gly Gly Val Pro Ile Ser Asn Tyr Val Val Glu Met Arg Gln Thr
14690 14695 14700 1
Asp Ser Thr Thr Trp Val Glu Leu Ala Thr Thr Val Ile Arg Thr Thr
4705 14710 14715 14720
Tyr Lys Ala Thr Arg Leu Thr Thr Gly Leu Glu Tyr Gln Phe Arg Val
14725 14730 14735
Lys Ala Gln Asn Arg Tyr Gly Val Gly Pro Gly Ile Thr Ser Ala Trp
14740 14745 14750
Ile Val Ala Asn Tyr Pro Phe Lys Val Pro Gly Pro Pro Gly Thr Pro
14755 14760 14765
Gln Val Thr Ala Val Thr Lys Asp Ser Met Thr Ile Ser Trp His Glu
14770 14775 14780 1
Pro Leu Ser Asp Gly Gly Ser Pro Ile Leu Gly Tyr His Val Glu Arg
4785 14790 14795 14800
Lys Glu Arg Asn Gly Ile Leu Trp Gln Thr Val Ser Lys Ala Leu Val
14805 14810 14815
Pro Gly Asn Ile Phe Lys Ser Ser Gly Leu Thr Asp Gly Ile Ala Tyr
14820 14825 14830
Glu Phe Arg Val Ile Ala Glu Asn Met Ala Gly Lys Ser Lys Pro Ser
14835 14840 14845
Lys Pro Ser Glu Pro Met Leu Ala Leu Asp Pro Ile Asp Pro Pro Gly
14850 14855 14860 1
Lys Pro Val Pro Leu Asn Ile Thr Arg His Thr Val Thr Leu Lys Trp
4865 14870 14875 14880
Ala Lys Pro Glu Tyr Thr Gly Gly Phe Lys Ile Thr Ser Tyr Ile Val
14885 14890 14895
Glu Lys Arg Asp Leu Pro Asn Gly Arg Trp Leu Lys Ala Asn Phe Ser
14900 14905 14910
Asn Ile Leu Glu Asn Glu Phe Thr Val Ser Gly Leu Thr Glu Asp Ala
14915 14920 14925
Ala Tyr Glu Phe Arg Val Ile Ala Lys Asn Ala Ala Gly Ala Ile Ser
14930 14935 14940 1
Pro Pro Ser Glu Pro Ser Asp Ala Ile Thr Cys Arg Asp Asp Val Glu
4945 14950 14955 14960
Ala Pro Lys Ile Lys Val Asp Val Lys Phe Lys Asp Thr Val Ile Leu
14965 14970 14975
Lys Ala Gly Glu Ala Phe Arg Leu Glu Ala Asp Val Ser Gly Arg Pro
14980 14985 14990
Pro Pro Thr Met Glu Trp Ser Lys Asp Gly Lys Glu Leu Glu Gly Thr
14995 15000 15005
Ala Lys Leu Glu Ile Lys Ile Ala Asp Phe Ser Thr Asn Leu Val Asn
15010 15015 15020 1
Lys Asp Ser Thr Arg Arg Asp Ser Gly Ala Tyr Thr Leu Thr Ala Thr
5025 15030 15035 15040
Asn Pro Gly Gly Phe Ala Lys His Ile Phe Asn Val Lys Val Leu Asp
15045 15050 15055
Arg Pro Gly Pro Pro Glu Gly Pro Leu Ala Val Thr Glu Val Thr Ser
15060 15065 15070
Glu Lys Cys Val Leu Ser Trp Phe Pro Pro Leu Asp Asp Gly Gly Ala
15075 15080 15085
Lys Ile Asp His Tyr Ile Val Gln Lys Arg Glu Thr Ser Arg Leu Ala
15090 15095 15100 1
 
Trp Thr Asn Val Ala Ser Glu Val Gln Val Thr Lys Leu Lys Val Thr
5105                15110               15115               15120
Lys Leu Leu Lys Gly Asn Glu Tyr Ile Phe Arg Val Met Ala Val Asn
                15125               15130               15135
Lys Tyr Gly Val Gly Glu Pro Leu Glu Ser Glu Pro Val Leu Ala Val
            15140               15145               15150
Asn Pro Tyr Gly Pro Pro Asp Pro Pro Lys Asn Pro Glu Val Thr Thr
        15155               15160               15165
Ile Thr Lys Asp Ser Met Val Val Cys Trp Gly His Pro Asp Ser Asp
    15170               15175               15180               1
Gly Gly Ser Glu Ile Ile Asn Tyr Ile Val Glu Arg Arg Asp Lys Ala
5185                15190               15195               15200
Gly Gln Arg Trp Ile Lys Cys Asn Lys Lys Thr Leu Thr Asp Leu Arg
                15205               15210               15215
Tyr Lys Val Ser Gly Leu Thr Glu Gly His Glu Tyr Glu Phe Arg Ile
            15220               15225               15230
Met Ala Glu Asn Ala Ala Gly Ile Ser Ala Pro Ser Pro Thr Ser Pro
        15235               15240               15245
Phe Tyr Lys Ala Cys Asp Thr Val Phe Lys Pro Gly Pro Pro Gly Asn
    15250               15255               15260               1
Pro Arg Val Leu Asp Thr Ser Arg Ser Ser Ile Ser Ile Ala Trp Asn
5265                15270               15275               15280
Lys Pro Ile Tyr Asp Gly Gly Ser Glu Ile Thr Gly Tyr Met Val Glu
                15285               15290               15295
Ile Ala Leu Pro Glu Glu Asp Glu Trp Gln Ile Val Thr Pro Pro Ala
            15300               15305               15310
Gly Leu Lys Ala Thr Ser Tyr Thr Ile Thr Gly Leu Thr Glu Asn Gln
        15315               15320               15325
Glu Tyr Lys Ile Arg Ile Tyr Ala Met Asn Ser Glu Gly Leu Gly Glu
    15330               15335               15340               1
Pro Ala Leu Val Pro Gly Thr Pro Lys Ala Glu Asp Arg Met Leu Pro
5345                15350               15355               15360
Pro Glu Ile Glu Leu Asp Ala Asp Leu Arg Lys Val Val Thr Ile Arg
                15365               15370               15375
Ala Cys Cys Thr Leu Arg Leu Phe Val Pro Ile Lys Gly Arg Pro Asp
            15380               15385               15390
Pro Glu Val Lys Trp Ala Arg Asp His Gly Glu Ser Leu Asp Lys Ala
        15395               15400               15405
Ser Ile Glu Ser Ala Ser Ser Tyr Thr Leu Leu Ile Val Gly Asn Val
    15410               15415               15420               1
Asn Arg Phe Asp Ser Gly Lys Tyr Ile Leu Thr Val Glu Asn Ser Ser
5425                15430               15435               15440
Gly Ser Lys Ser Ala Phe Val Asn Val Arg Val Leu Asp Thr Pro Gly
                15445               15450               15455
Pro Pro Gln Asp Leu Lys Val Lys Glu Val Thr Lys Thr Ser Val Thr
            15460               15465               15470
Leu Thr Trp Asp Pro Pro Leu Leu Asp Gly Gly Ser Lys Ile Lys Asn
        15475               15480               15485
Tyr Ile Val Glu Lys Arg Glu Ser Thr Arg Lys Ala Tyr Ser Thr Val
    15490               15495               15500               1
Ala Thr Asn Cys His Lys Thr Ser Trp Lys Val Asp Gln Leu Gln Glu
5505                15510               15515               15520
Gly Cys Ser Tyr Tyr Phe Arg Val Leu Ala Glu Asn Glu Tyr Gly Ile
                15525               15530               15535
Gly Leu Pro Ala Glu Thr Ala Glu Ser Val Lys Ala Ser Glu Arg Pro
            15540               15545               15550
Leu Pro Pro Gly Lys Ile Thr Leu Met Asp Val Thr Arg Asn Ser Val
        15555               15560               15565
Ser Leu Ser Trp Glu Lys Pro Glu His Asp Gly Gly Ser Arg Ile Leu
    15570               15575               15580               1
Gly Tyr Ile Val Glu Met Gln Thr Lys Gly Ser Asp Lys Trp Ala Thr
5585                15590               15595               15600
 
Cys Ala Thr Val Lys Val Thr Glu Ala Thr Ile Thr Gly Leu Ile Gln
                15605               15610               15615
Gly Glu Glu Tyr Ser Phe Arg Val Ser Ala Gln Asn Glu Lys Gly Ile
            15620               15625               15630
Ser Asp Pro Arg Gln Leu Ser Val Pro Val Ile Ala Lys Asp Leu Val
        15635               15640               15645
Ile Pro Pro Ala Phe Lys Leu Leu Phe Asn Thr Phe Thr Val Leu Ala
    15650               15655               15660               1
5665
Gly Glu Asp Leu Lys Val Asp Val Pro Phe Ile Gly Arg Pro Thr Pro
5665                15670               15675               15680
Ala Val Thr Trp His Lys Asp Asn Val Pro Leu Lys Gln Thr Thr Arg
                15685               15690               15695
Val Asn Ala Glu Ser Thr Glu Asn Asn Ser Leu Leu Thr Ile Lys Asp
            15700               15705               15710
Ala Cys Arg Glu Asp Val Gly His Tyr Val Val Lys Leu Thr Asn Ser
        15715               15720               15725
Ala Gly Glu Ala Ile Glu Thr Leu Asn Val Ile Val Leu Asp Lys Pro
    15730               15735               15740               1
Gly Pro Pro Thr Gly Pro Val Lys Met Asp Glu Val Thr Ala Asp Ser
5745                15750               15755               15760
Ile Thr Leu Ser Trp Gly Pro Pro Lys Tyr Asp Gly Gly Ser Ser Ile
                15765               15770               15775
Asn Asn Tyr Ile Val Glu Lys Arg Asp Thr Ser Thr Thr Thr Trp Gln
            15780               15785               15790
Ile Val Ser Ala Thr Val Ala Arg Thr Thr Ile Lys Ala Cys Arg Leu
        15795               15800               15805
Lys Thr Gly Cys Glu Tyr Gln Phe Arg Ile Ala Ala Glu Asn Arg Tyr
    15810               15815               15820               1
Gly Lys Ser Thr Tyr Leu Asn Ser Glu Pro Thr Val Ala Gln Tyr Pro
5825                15830               15835               15840
Phe Lys Val Pro Gly Pro Pro Gly Thr Pro Val Val Thr Leu Ser Ser
                15845               15850               15855
Arg Asp Ser Met Glu Val Gln Trp Asn Glu Pro Ile Ser Asp Gly Gly
            15860               15865               15870
Ser Arg Val Ile Gly Tyr His Leu Glu Arg Lys Glu Arg Asn Ser Ile
        15875               15880               15885
Leu Trp Val Lys Leu Asn Lys Thr Pro Ile Pro Gln Thr Lys Phe Lys
    15890               15895               15900               1
Thr Thr Gly Leu Glu Glu Gly Val Glu Tyr Glu Phe Arg Val Ser Ala
5905                15910               15915               15920
Glu Asn Ile Val Gly Ile Gly Lys Pro Ser Lys Val Ser Glu Cys Tyr
                15925               15930               15935
Val Ala Arg Asp Pro Cys Asp Pro Pro Gly Arg Pro Glu Ala Ile Ile
            15940               15945               15950
Val Thr Arg Asn Ser Val Thr Leu Gln Trp Lys Lys Pro Thr Tyr Asp
        15955               15960               15965
Gly Gly Ser Lys Ile Thr Gly Tyr Ile Val Glu Lys Lys Glu Leu Pro
    15970               15975               15980               1
Glu Gly Arg Trp Met Lys Ala Ser Phe Thr Asn Ile Ile Asp Thr His
5985                15990               15995               16000
Phe Glu Val Thr Gly Leu Val Glu Asp His Arg Tyr Glu Phe Arg Val
                16005               16010               16015
Ile Ala Arg Asn Ala Ala Gly Val Phe Ser Glu Pro Ser Glu Ser Thr
            16020               16025               16030
Gly Ala Ile Thr Ala Arg Asp Glu Val Asp Pro Pro Arg Ile Ser Met
        16035               16040               16045
Asp Pro Lys Tyr Lys Asp Thr Ile Val Val His Ala Gly Glu Ser Phe
    16050               16055               16060               1
Lys Val Asp Ala Asp Ile Tyr Gly Lys Pro Ile Pro Thr Ile Gln Trp
6065                16070               16075               16080
Ile Lys Gly Asp Gln Glu Leu Ser Asn Thr Ala Arg Leu Glu Ile Lys
                16085               16090               16095
 
Ser Thr Asp Phe Ala Thr Ser Leu Ser Val Lys Asp Ala Val Arg Val
16100 16105 16110
Asp Ser Gly Asn Tyr Ile Leu Lys Ala Lys Asn Val Ala Gly Glu Arg
16115 16120 16125
Ser Val Thr Val Asn Val Lys Val Leu Asp Arg Pro Gly Pro Pro Glu
16130 16135 16140 1
Gly Pro Val Val Ile Ser Gly Val Thr Ala Glu Lys Cys Thr Leu Ala
6145 16150 16155 16160
Trp Lys Pro Pro Leu Gln Asp Gly Gly Ser Asp Ile Ile Asn Tyr Ile
16165 16170 16175
Val Glu Arg Arg Glu Thr Ser Arg Leu Val Trp Thr Val Val Asp Ala
16180 16185 16190
Asn Val Gln Thr Leu Ser Cys Lys Val Thr Lys Leu Leu Glu Gly Asn
16195 16200 16205
Glu Tyr Thr Phe Arg Ile Met Ala Val Asn Lys Tyr Gly Val Gly Glu
16210 16215 16220 1
Pro Leu Glu Ser Glu Pro Val Val Ala Lys Asn Pro Phe Val Val Pro
6225 16230 16235 16240
Asp Ala Pro Lys Ala Pro Glu Val Thr Thr Val Thr Lys Asp Ser Met
16245 16250 16255
Ile Val Val Trp Glu Arg Pro Ala Ser Asp Gly Gly Ser Glu Ile Leu
16260 16265 16270
Gly Tyr Val Leu Glu Lys Arg Asp Lys Glu Gly Ile Arg Trp Thr Arg
16275 16280 16285
Cys His Lys Arg Leu Ile Gly Glu Leu Arg Leu Arg Val Thr Gly Leu
16290 16295 16300 1
Ile Glu Asn His Asp Tyr Glu Phe Arg Val Ser Ala Glu Asn Ala Ala
6305 16310 16315 16320
Gly Leu Ser Glu Pro Ser Pro Pro Ser Ala Tyr Gln Lys Ala Cys Asp
16325 16330 16335
Pro Ile Tyr Lys Pro Gly Pro Pro Asn Asn Pro Lys Val Ile Asp Ile
16340 16345 16350
Thr Arg Ser Ser Val Phe Leu Ser Trp Ser Lys Pro Ile Tyr Asp Gly
16355 16360 16365
Gly Cys Glu Ile Gln Gly Tyr Ile Val Glu Lys Cys Asp Val Asn Val
16370 16375 16380 1
Gly Glu Trp Thr Met Cys Thr Pro Pro Thr Gly Ile Asn Lys Thr Asn
6385 16390 16395 16400
Ile Glu Val Glu Lys Leu Leu Glu Lys His Glu Tyr Asn Phe Arg Ile
16405 16410 16415
Cys Ala Ile Asn Lys Ala Gly Val Gly Glu His Ala Asp Val Pro Gly
16420 16425 16430
Pro Ile Ile Val Glu Glu Lys Leu Glu Ala Pro Asp Ile Asp Leu Asp
16435 16440 16445
Leu Glu Leu Arg Lys Ile Ile Asn Ile Arg Ala Gly Gly Ser Leu Arg
16450 16455 16460 1
Leu Phe Val Pro Ile Lys Gly Arg Pro Thr Pro Glu Val Lys Trp Gly
6465 16470 16475 16480
Lys Val Asp Gly Glu Ile Arg Asp Ala Ala Ile Ile Asp Val Thr Ser
16485 16490 16495
Ser Phe Thr Ser Leu Val Leu Asp Asn Val Asn Arg Tyr Asp Ser Gly
16500 16505 16510
Lys Tyr Thr Leu Thr Leu Glu Asn Ser Ser Gly Thr Lys Ser Ala Phe
16515 16520 16525
Val Thr Val Arg Val Leu Asp Thr Pro Ser Pro Pro Val Asn Leu Lys
16530 16535 16540 1
Val Thr Glu Ile Thr Lys Asp Ser Val Ser Ile Thr Trp Glu Pro Pro
6545 16550 16555 16560
Leu Leu Asp Gly Gly Ser Lys Ile Lys Asn Tyr Ile Val Glu Lys Arg
16565 16570 16575
Glu Ala Thr Arg Lys Ser Tyr Ala Ala Val Val Thr Asn Cys His Lys
16580 16585 16590
 
Asn Ser Trp Lys Ile Asp Gln Leu Gln Glu Gly Cys Ser Tyr Tyr Phe
16595 16600 16605
Arg Val Thr Ala Glu Asn Glu Tyr Gly Ile Gly Leu Pro Ala Gln Thr
16610 16615 16620 1
Ala Asp Pro Ile Lys Val Ala Glu Val Pro Gln Pro Pro Gly Lys Ile
6625 16630 16635 16640
Thr Val Asp Asp Val Thr Arg Asn Ser Val Ser Leu Ser Trp Thr Lys
16645 16650 16655
Pro Glu His Asp Gly Gly Ser Lys Ile Ile Gln Tyr Ile Val Glu Met
16660 16665 16670
Gln Ala Lys His Ser Glu Lys Trp Ser Glu Cys Ala Arg Val Lys Ser
16675 16680 16685
Leu Gln Ala Val Ile Thr Asn Leu Thr Gln Gly Glu Glu Tyr Leu Phe
16690 16695 16700 1
Arg Val Val Ala Val Asn Glu Lys Gly Arg Ser Asp Pro Arg Ser Leu
6705 16710 16715 16720
Ala Val Pro Ile Val Ala Lys Asp Leu Val Ile Glu Pro Asp Val Lys
16725 16730 16735
Pro Ala Phe Ser Ser Tyr Ser Val Gln Val Gly Gln Asp Leu Lys Ile
16740 16745 16750
Glu Val Pro Ile Ser Gly Arg Pro Lys Pro Thr Ile Thr Trp Thr Lys
16755 16760 16765
Asp Gly Leu Pro Leu Lys Gln Thr Thr Arg Ile Asn Val Thr Asp Ser
16770 16775 16780 1
Leu Asp Leu Thr Thr Leu Ser Ile Lys Glu Thr His Lys Asp Asp Gly
6785 16790 16795 16800
Gly Gln Tyr Gly Ile Thr Val Ala Asn Val Val Gly Gln Lys Thr Ala
16805 16810 16815
Ser Ile Glu Ile Val Thr Leu Asp Lys Pro Asp Pro Pro Lys Gly Pro
16820 16825 16830
Val Lys Phe Asp Asp Val Ser Ala Glu Ser Ile Thr Leu Ser Trp Asn
16835 16840 16845
Pro Pro Leu Tyr Thr Gly Gly Cys Gln Ile Thr Asn Tyr Ile Val Gln
16850 16855 16860 1
Lys Arg Asp Thr Thr Thr Thr Val Trp Asp Val Val Ser Ala Thr Val
6865 16870 16875 16880
Ala Arg Thr Thr Leu Lys Val Thr Lys Leu Lys Thr Gly Thr Glu Tyr
16885 16890 16895
Gln Phe Arg Ile Phe Ala Glu Asn Arg Tyr Gly Gln Ser Phe Ala Leu
16900 16905 16910
Glu Ser Asp Pro Ile Val Ala Gln Tyr Pro Tyr Lys Glu Pro Gly Pro
16915 16920 16925
Pro Gly Thr Pro Phe Ala Thr Ala Ile Ser Lys Asp Ser Met Val Ile
16930 16935 16940 1
Gln Trp His Glu Pro Val Asn Asn Gly Gly Ser Pro Val Ile Gly Tyr
6945 16950 16955 16960
His Leu Glu Arg Lys Glu Arg Asn Ser Ile Leu Trp Thr Lys Val Asn
16965 16970 16975
Lys Thr Ile Ile His Asp Thr Gln Phe Lys Ala Gln Asn Leu Glu Glu
16980 16985 16990
Gly Ile Glu Tyr Glu Phe Arg Val Tyr Ala Glu Asn Ile Val Gly Val
16995 17000 17005
Gly Lys Ala Ser Lys Asn Ser Glu Cys Tyr Val Ala Arg Asp Pro Cys
17010 17015 17020 1
Asp Pro Pro Gly Thr Pro Glu Pro Ile Met Val Lys Arg Asn Glu Ile
7025 17030 17035 17040
Thr Leu Gln Trp Thr Lys Pro Val Tyr Asp Gly Gly Ser Met Ile Thr
17045 17050 17055
Gly Tyr Ile Val Glu Lys Arg Asp Leu Pro Asp Gly Arg Trp Met Lys
17060 17065 17070
Ala Ser Phe Thr Asn Val Ile Glu Thr Gln Phe Thr Val Ser Gly Leu
17075 17080 17085
 
Thr Glu Asp Gln Arg Tyr Glu Phe Arg Val Ile Ala Lys Asn Ala Ala
17090 17095 17100 1
Gly Ala Ile Ser Lys Pro Ser Asp Ser Thr Gly Pro Ile Thr Ala Lys
7105 17110 17115 17120
Asp Glu Val Glu Leu Pro Arg Ile Ser Met Asp Pro Lys Phe Arg Asp
17125 17130 17135
Thr Ile Val Val Asn Ala Gly Glu Thr Phe Arg Leu Glu Ala Asp Val
17140 17145 17150
His Gly Lys Pro Leu Pro Thr Ile Glu Trp Leu Arg Gly Asp Lys Glu
17155 17160 17165
Ile Glu Glu Ser Ala Arg Cys Glu Ile Lys Asn Thr Asp Phe Lys Ala
17170 17175 17180 1
Leu Leu Ile Val Lys Asp Ala Ile Arg Ile Asp Gly Gly Gln Tyr Ile
7185 17190 17195 17200
Leu Arg Ala Ser Asn Val Ala Gly Ser Lys Ser Phe Pro Val Asn Val
17205 17210 17215
Lys Val Leu Asp Arg Pro Gly Pro Pro Glu Gly Pro Val Gln Val Thr
17220 17225 17230
Gly Val Thr Ser Glu Lys Cys Ser Leu Thr Trp Ser Pro Pro Leu Gln
17235 17240 17245
Asp Gly Gly Ser Asp Ile Ser His Tyr Val Val Glu Lys Arg Glu Thr
17250 17255 17260 1
Ser Arg Leu Ala Trp Thr Val Val Ala Ser Glu Val Val Thr Asn Ser
7265 17270 17275 17280
Leu Lys Val Thr Lys Leu Leu Glu Gly Asn Glu Tyr Val Phe Arg Ile
17285 17290 17295
Met Ala Val Asn Lys Tyr Gly Val Gly Glu Pro Leu Glu Ser Ala Pro
17300 17305 17310
Val Leu Met Lys Asn Pro Phe Val Leu Pro Gly Pro Pro Lys Ser Leu
17315 17320 17325
Glu Val Thr Asn Ile Ala Lys Asp Ser Met Thr Val Cys Trp Asn Arg
17330 17335 17340 1
Pro Asp Ser Asp Gly Gly Ser Glu Ile Ile Gly Tyr Ile Val Glu Lys
7345 17350 17355 17360
Arg Asp Arg Ser Gly Ile Arg Trp Ile Lys Cys Asn Lys Arg Arg Ile
17365 17370 17375
Thr Asp Leu Arg Leu Arg Val Thr Gly Leu Thr Glu Asp His Glu Tyr
17380 17385 17390
Glu Phe Arg Val Ser Ala Glu Asn Ala Ala Gly Val Gly Glu Pro Ser
17395 17400 17405
Pro Ala Thr Val Tyr Tyr Lys Ala Cys Asp Pro Val Phe Lys Pro Gly
17410 17415 17420 1
Pro Pro Thr Asn Ala His Ile Val Asp Thr Thr Lys Asn Ser Ile Thr
7425 17430 17435 17440
Leu Ala Trp Gly Lys Pro Ile Tyr Asp Gly Gly Ser Glu Ile Leu Gly
17445 17450 17455
Tyr Val Val Glu Ile Cys Lys Ala Asp Glu Glu Glu Trp Gln Ile Val
17460 17465 17470
Thr Pro Gln Thr Gly Leu Arg Val Thr Arg Phe Glu Ile Ser Lys Leu
17475 17480 17485
Thr Glu His Gln Glu Tyr Lys Ile Arg Val Cys Ala Leu Asn Lys Val
17490 17495 17500 1
Gly Leu Gly Glu Ala Thr Ser Val Pro Gly Thr Val Lys Pro Glu Asp
7505 17510 17515 17520
Lys Leu Glu Ala Pro Glu Leu Asp Leu Asp Ser Glu Leu Arg Lys Gly
17525 17530 17535
Ile Val Val Arg Ala Gly Gly Ser Ala Arg Ile His Ile Pro Phe Lys
17540 17545 17550
Gly Arg Pro Met Pro Glu Ile Thr Trp Ser Arg Glu Glu Gly Glu Phe
17555 17560 17565
Thr Asp Lys Val Gln Ile Glu Lys Gly Val Asn Tyr Thr Gln Leu Ser
17570 17575 17580 1
 
Ile Asp Asn Cys Asp Arg Asn Asp Ala Gly Lys Tyr Ile Leu Lys Leu
7585                17590               17595               17600
Glu Asn Ser Ser Gly Ser Lys Ser Ala Phe Val Thr Val Lys Val Leu
                17605               17610               17615
Asp Thr Pro Gly Pro Pro Gln Asn Leu Ala Val Lys Glu Val Arg Lys
            17620               17625               17630
Asp Ser Ala Phe Leu Val Trp Glu Pro Pro Ile Ile Asp Gly Gly Ala
        17635               17640               17645
Lys Val Lys Asn Tyr Val Ile Asp Lys Arg Glu Ser Thr Arg Lys Ala
    17650               17655               17660               1
Tyr Ala Asn Val Ser Ser Lys Cys Ser Lys Thr Ser Phe Lys Val Glu
7665                17670               17675               17680
Asn Leu Thr Glu Gly Ala Ile Tyr Tyr Phe Arg Val Met Ala Glu Asn
                17685               17690               17695
Glu Phe Gly Val Gly Val Pro Val Glu Thr Val Asp Ala Val Lys Ala
            17700               17705               17710
Ala Glu Pro Pro Ser Pro Pro Gly Lys Val Thr Leu Thr Asp Val Ser
        17715               17720               17725
Gln Thr Ser Ala Ser Leu Met Trp Glu Lys Pro Glu His Asp Gly Gly
    17730               17735               17740               1
Ser Arg Val Leu Gly Tyr Val Val Glu Met Gln Pro Lys Gly Thr Glu
7745                17750               17755               17760
Lys Trp Ser Ile Val Ala Glu Ser Lys Val Cys Asn Ala Val Val Thr
                17765               17770               17775
Gly Leu Ser Ser Gly Gln Glu Tyr Gln Phe Arg Val Lys Ala Tyr Asn
            17780               17785               17790
Glu Lys Gly Lys Ser Asp Pro Arg Val Leu Gly Val Pro Val Ile Ala
        17795               17800               17805
Lys Asp Leu Thr Ile Gln Pro Ser Leu Lys Leu Pro Phe Asn Thr Tyr
    17810               17815               17820               1
Ser Ile Gln Ala Gly Glu Asp Leu Lys Ile Glu Ile Pro Val Ile Gly
7825                17830               17835               17840
Arg Pro Arg Pro Asn Ile Ser Trp Val Lys Asp Gly Glu Pro Leu Lys
                17845               17850               17855
Gln Thr Thr Arg Val Asn Val Glu Glu Thr Ala Thr Ser Thr Val Leu
            17860               17865               17870
His Ile Lys Glu Gly Asn Lys Asp Asp Phe Gly Lys Tyr Thr Val Thr
        17875               17880               17885
Ala Thr Asn Ser Ala Gly Thr Ala Thr Glu Asn Leu Ser Val Ile Val
    17890               17895               17900               1
Leu Glu Lys Pro Gly Pro Pro Val Gly Pro Val Arg Phe Asp Glu Val
7905                17910               17915               17920
Ser Ala Asp Phe Val Val Ile Ser Trp Glu Pro Pro Ala Tyr Thr Gly
                17925               17930               17935
Gly Cys Gln Ile Ser Asn Tyr Ile Val Glu Lys Arg Asp Thr Thr Thr
            17940               17945               17950
Thr Thr Trp His Met Val Ser Ala Thr Val Ala Arg Thr Thr Ile Lys
        17955               17960               17965
Ile Thr Lys Leu Lys Thr Gly Thr Glu Tyr Gln Phe Arg Ile Phe Ala
    17970               17975               17980               1
Glu Asn Arg Tyr Gly Lys Ser Ala Pro Leu Asp Ser Lys Ala Val Ile
7985                17990               17995               18000
Val Gln Tyr Pro Phe Lys Glu Pro Gly Pro Pro Gly Thr Pro Phe Val
                18005               18010               18015
Thr Ser Ile Ser Lys Asp Gln Met Leu Val Gln Trp His Glu Pro Val
            18020               18025               18030
Asn Asp Gly Gly Thr Lys Ile Ile Gly Tyr His Leu Glu Gln Lys Glu
        18035               18040               18045
Lys Asn Ser Ile Leu Trp Val Lys Leu Asn Lys Thr Pro Ile Gln Asp
    18050               18055               18060               1
Thr Lys Phe Lys Thr Thr Gly Leu Asp Glu Gly Leu Glu Tyr Glu Phe
8065                18070               18075               18080
 
Lys Val Ser Ala Glu Asn Ile Val Gly Ile Gly Lys Pro Ser Lys Val
18085 18090 18095
Ser Glu Cys Phe Val Ala Arg Asp Pro Cys Asp Pro Pro Gly Arg Pro
18100 18105 18110
Glu Ala Ile Val Ile Thr Arg Asn Asn Val Thr Leu Lys Trp Lys Lys
18115 18120 18125
Pro Ala Tyr Asp Gly Gly Ser Lys Ile Thr Gly Tyr Ile Val Glu Lys
18130 18135 18140 1
Lys Asp Leu Pro Asp Gly Arg Trp Met Lys Ala Ser Phe Thr Asn Val
8145 18150 18155 18160
Leu Glu Thr Glu Phe Thr Val Ser Gly Leu Val Glu Asp Gln Arg Tyr
18165 18170 18175
Glu Phe Arg Val Ile Ala Arg Asn Ala Ala Gly Asn Phe Ser Glu Pro
18180 18185 18190
Ser Asp Ser Ser Gly Ala Ile Thr Ala Arg Asp Glu Ile Asp Ala Pro
18195 18200 18205
Asn Ala Ser Leu Asp Pro Lys Tyr Lys Asp Val Ile Val Val His Ala
18210 18215 18220 1
Gly Glu Thr Phe Val Leu Glu Ala Asp Ile Arg Gly Lys Pro Ile Pro
8225 18230 18235 18240
Asp Val Val Trp Ser Lys Asp Gly Lys Glu Leu Glu Glu Thr Ala Ala
18245 18250 18255
Arg Met Glu Ile Lys Ser Thr Ile Gln Lys Thr Thr Leu Val Val Lys
18260 18265 18270
Asp Cys Ile Arg Thr Asp Gly Gly Gln Tyr Ile Leu Lys Leu Ser Asn
18275 18280 18285
Val Gly Gly Thr Lys Ser Ile Pro Ile Thr Val Lys Val Leu Asp Arg
18290 18295 18300 1
Pro Gly Ser Pro Glu Gly Pro Leu Lys Val Thr Gly Val Thr Ala Glu
8305 18310 18315 18320
Lys Cys Tyr Leu Ala Trp Asn Pro Pro Leu Gln Asp Gly Gly Ala Asn
18325 18330 18335
Ile Ser His Tyr Ile Ile Glu Lys Arg Glu Thr Ser Arg Leu Ser Trp
18340 18345 18350
Thr Gln Val Ser Thr Glu Val Gln Ala Leu Asn Tyr Lys Val Thr Lys
18355 18360 18365
Leu Leu Pro Gly Asn Glu Tyr Ile Phe Arg Val Met Ala Val Asn Lys
18370 18375 18380 1
Tyr Gly Ile Gly Glu Pro Leu Glu Ser Gly Pro Val Thr Ala Cys Asn
8385 18390 18395 18400
Pro Tyr Lys Pro Pro Gly Pro Pro Ser Thr Pro Glu Val Ser Ala Ile
18405 18410 18415
Thr Lys Asp Ser Met Val Val Thr Trp Ala Arg Pro Val Asp Asp Gly
18420 18425 18430
Gly Thr Glu Ile Glu Gly Tyr Ile Leu Glu Lys Arg Asp Lys Glu Gly
18435 18440 18445
Val Arg Trp Thr Lys Cys Asn Lys Lys Thr Leu Thr Asp Leu Arg Leu
18450 18455 18460 1
Arg Val Thr Gly Leu Thr Glu Gly His Ser Tyr Glu Phe Arg Val Ala
8465 18470 18475 18480
Ala Glu Asn Ala Ala Gly Val Gly Glu Pro Ser Glu Pro Ser Val Phe
18485 18490 18495
Tyr Arg Ala Cys Asp Ala Leu Tyr Pro Pro Gly Pro Pro Ser Asn Pro
18500 18505 18510
Lys Val Thr Asp Thr Ser Arg Ser Ser Val Ser Leu Ala Trp Ser Lys
18515 18520 18525
Pro Ile Tyr Asp Gly Gly Ala Pro Val Lys Gly Tyr Val Val Glu Val
18530 18535 18540 1
Lys Glu Ala Ala Ala Asp Glu Trp Thr Thr Cys Thr Pro Pro Thr Gly
8545 18550 18555 18560
Leu Gln Gly Lys Gln Phe Thr Val Thr Lys Leu Lys Glu Asn Thr Glu
18565 18570 18575
 
Tyr Asn Phe Arg Ile Cys Ala Ile Asn Ser Glu Gly Val Gly Glu Pro
18580 18585 18590
Ala Thr Leu Pro Gly Ser Val Val Ala Gln Glu Arg Ile Glu Pro Pro
18595 18600 18605
Glu Ile Glu Leu Asp Ala Asp Leu Arg Lys Val Val Val Leu Arg Ala
18610 18615 18620 1
Ser Ala Thr Leu Arg Leu Phe Val Thr Ile Lys Gly Arg Pro Glu Pro
8625 18630 18635 18640
Glu Val Lys Trp Glu Lys Ala Glu Gly Ile Leu Thr Asp Arg Ala Gln
18645 18650 18655
Ile Glu Val Thr Ser Ser Phe Thr Met Leu Val Ile Asp Asn Val Thr
18660 18665 18670
Arg Phe Asp Ser Gly Arg Tyr Asn Leu Thr Leu Glu Asn Asn Ser Gly
18675 18680 18685
Ser Lys Thr Ala Phe Val Asn Val Arg Val Leu Asp Ser Pro Ser Ala
18690 18695 18700 1
Pro Val Asn Leu Thr Ile Arg Glu Val Lys Lys Asp Ser Val Thr Leu
8705 18710 18715 18720
Ser Trp Glu Pro Pro Leu Ile Asp Gly Gly Ala Lys Ile Thr Asn Tyr
18725 18730 18735
Ile Val Glu Lys Arg Glu Thr Thr Arg Lys Ala Tyr Ala Thr Ile Thr
18740 18745 18750
Asn Asn Cys Thr Lys Thr Thr Phe Arg Ile Glu Asn Leu Gln Glu Gly
18755 18760 18765
Cys Ser Tyr Tyr Phe Arg Val Leu Ala Ser Asn Glu Tyr Gly Ile Gly
18770 18775 18780 1
Leu Pro Ala Glu Thr Thr Glu Pro Val Lys Val Ser Glu Pro Pro Leu
8785 18790 18795 18800
Pro Pro Gly Arg Val Thr Leu Val Asp Val Thr Arg Asn Thr Ala Thr
18805 18810 18815
Ile Lys Trp Glu Lys Pro Glu Ser Asp Gly Gly Ser Lys Ile Thr Gly
18820 18825 18830
Tyr Val Val Glu Met Gln Thr Lys Gly Ser Glu Lys Trp Ser Thr Cys
18835 18840 18845
Thr Gln Val Lys Thr Leu Glu Ala Thr Ile Ser Gly Leu Thr Ala Gly
18850 18855 18860 1
Glu Glu Tyr Val Phe Arg Val Ala Ala Val Asn Glu Lys Gly Arg Ser
8865 18870 18875 18880
Asp Pro Arg Gln Leu Gly Val Pro Val Ile Ala Arg Asp Ile Glu Ile
18885 18890 18895
Lys Pro Ser Val Glu Leu Pro Phe His Thr Phe Asn Val Lys Ala Arg
18900 18905 18910
Glu Gln Leu Lys Ile Asp Val Pro Phe Lys Gly Arg Pro Gln Ala Thr
18915 18920 18925
Val Asn Trp Arg Lys Asp Gly Gln Thr Leu Lys Glu Thr Thr Arg Val
18930 18935 18940 1
Asn Val Ser Ser Ser Lys Thr Val Thr Ser Leu Ser Ile Lys Glu Ala
8945 18950 18955 18960
Ser Lys Glu Asp Val Gly Thr Tyr Glu Leu Cys Val Ser Asn Ser Ala
18965 18970 18975
Gly Ser Ile Thr Val Pro Ile Thr Ile Ile Val Leu Asp Arg Pro Gly
18980 18985 18990
Pro Pro Gly Pro Ile Arg Ile Asp Glu Val Ser Cys Asp Ser Ile Thr
18995 19000 19005
Ile Ser Trp Asn Pro Pro Glu Tyr Asp Gly Gly Cys Gln Ile Ser Asn
19010 19015 19020 1
Tyr Ile Val Glu Lys Lys Glu Thr Thr Ser Thr Thr Trp His Ile Val
9025 19030 19035 19040
Ser Gln Ala Val Ala Arg Thr Ser Ile Lys Ile Val Arg Leu Thr Thr
19045 19050 19055
Gly Ser Glu Tyr Gln Phe Arg Val Cys Ala Glu Asn Arg Tyr Gly Lys
19060 19065 19070
 
Ser Ser Tyr Ser Glu Ser Ser Ala Val Val Ala Glu Tyr Pro Phe Ser
19075 19080 19085
Pro Pro Gly Pro Pro Gly Thr Pro Lys Val Val His Ala Thr Lys Ser
19090 19095 19100 1
Thr Met Leu Val Thr Trp Gln Val Pro Val Asn Asp Gly Gly Ser Arg
9105 19110 19115 19120
Val Ile Gly Tyr His Leu Glu Tyr Lys Glu Arg Ser Ser Ile Leu Trp
19125 19130 19135
Ser Lys Ala Asn Lys Ile Leu Ile Ala Asp Thr Gln Val Lys Val Ser
19140 19145 19150
Gly Leu Asp Glu Gly Leu Met Tyr Glu Tyr Arg Val Tyr Ala Glu Asn
19155 19160 19165
Ile Ala Gly Ile Gly Lys Cys Ser Lys Ser Cys Glu Pro Val Pro Ala
19170 19175 19180 1
Arg Asp Pro Cys Asp Pro Pro Gly Gln Pro Glu Val Thr Asn Ile Thr
9185 19190 19195 19200
Arg Lys Ser Val Ser Leu Lys Trp Ser Lys Pro His Tyr Asp Gly Gly
19205 19210 19215
Ala Lys Ile Thr Gly Tyr Ile Val Glu Arg Arg Glu Leu Pro Asp Gly
19220 19225 19230
Arg Trp Leu Lys Cys Asn Tyr Thr Asn Ile Gln Glu Thr Tyr Phe Glu
19235 19240 19245
Val Thr Glu Leu Thr Glu Asp Gln Arg Tyr Glu Phe Arg Val Phe Ala
19250 19255 19260 1
Arg Asn Ala Ala Asp Ser Val Ser Glu Pro Ser Glu Ser Thr Gly Pro
9265 19270 19275 19280
Ile Ile Val Lys Asp Asp Val Glu Pro Pro Arg Val Met Met Asp Val
19285 19290 19295
Lys Phe Arg Asp Val Ile Val Val Lys Ala Gly Glu Val Leu Lys Ile
19300 19305 19310
Asn Ala Asp Ile Ala Gly Arg Pro Leu Pro Val Ile Ser Trp Ala Lys
19315 19320 19325
Asp Gly Ile Glu Ile Glu Glu Arg Ala Arg Thr Glu Ile Ile Ser Thr
19330 19335 19340 1
Asp Asn His Thr Leu Leu Thr Val Lys Asp Cys Ile Arg Arg Asp Thr
9345 19350 19355 19360
Gly Gln Tyr Val Leu Thr Leu Lys Asn Val Ala Gly Thr Arg Ser Val
19365 19370 19375
Ala Val Asn Cys Lys Val Leu Asp Lys Pro Gly Pro Pro Ala Gly Pro
19380 19385 19390
Leu Glu Ile Asn Gly Leu Thr Ala Glu Lys Cys Ser Leu Ser Trp Gly
19395 19400 19405
Arg Pro Gln Glu Asp Gly Gly Ala Asp Ile Asp Tyr Tyr His Arg Lys
19410 19415 19420 1
Lys Arg Glu Thr Ser His Leu Ala Trp Thr Ile Cys Glu Gly Glu Leu
9425 19430 19435 19440
Gln Met Thr Ser Cys Lys Val Thr Lys Leu Leu Lys Gly Asn Glu Tyr
19445 19450 19455
Ile Phe Arg Val Thr Gly Val Asn Lys Tyr Gly Val Gly Glu Pro Leu
19460 19465 19470
Glu Ser Val Ala Ile Lys Ala Leu Asp Pro Phe Thr Val Pro Ser Pro
19475 19480 19485
Pro Thr Ser Leu Glu Ile Thr Ser Val Thr Lys Glu Ser Met Thr Leu
19490 19495 19500 1
Cys Trp Ser Arg Pro Glu Ser Asp Gly Gly Ser Glu Ile Ser Gly Tyr
9505 19510 19515 19520
Ile Ile Glu Arg Arg Glu Lys Asn Ser Leu Arg Trp Val Arg Val Asn
19525 19530 19535
Lys Lys Pro Val Tyr Asp Leu Arg Val Lys Ser Thr Gly Leu Arg Glu
19540 19545 19550
Gly Cys Glu Tyr Glu Tyr Arg Val Tyr Ala Glu Asn Ala Ala Gly Leu
19555 19560 19565
 
Ser Leu Pro Ser Glu Thr Ser Pro Leu Ile Arg Ala Glu Asp Pro Val
19570 19575 19580 1
Phe Leu Pro Ser Pro Pro Ser Lys Pro Lys Ile Val Asp Ser Gly Lys
9585 19590 19595 19600
Thr Thr Ile Thr Ile Ala Trp Val Lys Pro Leu Phe Asp Gly Gly Ala
19605 19610 19615
Pro Ile Thr Gly Tyr Thr Val Glu Tyr Lys Lys Ser Asp Asp Thr Asp
19620 19625 19630
Trp Lys Thr Ser Ile Gln Ser Leu Arg Gly Thr Glu Tyr Thr Ile Ser
19635 19640 19645
Gly Leu Thr Thr Gly Ala Glu Tyr Val Phe Arg Val Lys Ser Val Asn
19650 19655 19660 1
Lys Val Gly Ala Ser Asp Pro Ser Asp Ser Ser Asp Pro Gln Ile Ala
9665 19670 19675 19680
Lys Glu Arg Glu Glu Glu Pro Leu Phe Asp Ile Asp Ser Glu Met Arg
19685 19690 19695
Lys Thr Leu Ile Val Lys Ala Gly Ala Ser Phe Thr Met Thr Val Pro
19700 19705 19710
Phe Arg Gly Arg Pro Val Pro Asn Val Leu Trp Ser Lys Pro Asp Thr
19715 19720 19725
Asp Leu Arg Thr Arg Ala Tyr Val Asp Thr Thr Asp Ser Arg Thr Ser
19730 19735 19740 1
Leu Thr Ile Glu Asn Ala Asn Arg Asn Asp Ser Gly Lys Tyr Thr Leu
9745 19750 19755 19760
Thr Ile Gln Asn Val Leu Ser Ala Ala Ser Leu Thr Leu Val Val Lys
19765 19770 19775
Val Leu Asp Thr Pro Gly Pro Pro Thr Asn Ile Thr Val Gln Asp Val
19780 19785 19790
Thr Lys Glu Ser Ala Val Leu Ser Trp Asp Val Pro Glu Asn Asp Gly
19795 19800 19805
Gly Ala Pro Val Lys Asn Tyr His Ile Glu Lys Arg Glu Ala Ser Lys
19810 19815 19820 1
Lys Ala Trp Val Ser Val Thr Asn Asn Cys Asn Arg Leu Ser Tyr Lys
9825 19830 19835 19840
Val Thr Asn Leu Gln Glu Gly Ala Ile Tyr Tyr Phe Arg Val Ser Gly
19845 19850 19855
Glu Asn Glu Phe Gly Val Gly Ile Pro Ala Glu Thr Lys Glu Gly Val
19860 19865 19870
Lys Ile Thr Glu Lys Pro Ser Pro Pro Glu Lys Leu Gly Val Thr Ser
19875 19880 19885
Ile Ser Lys Asp Ser Val Ser Leu Thr Trp Leu Lys Pro Glu His Asp
19890 19895 19900 1
Gly Gly Ser Arg Ile Val His Tyr Val Val Glu Ala Leu Glu Lys Gly
9905 19910 19915 19920
Gln Lys Asn Trp Val Lys Cys Ala Val Ala Lys Ser Thr His His Val
19925 19930 19935
Val Ser Gly Leu Arg Glu Asn Ser Glu Tyr Phe Phe Arg Val Phe Ala
19940 19945 19950
Glu Asn Gln Ala Gly Leu Ser Asp Pro Arg Glu Leu Leu Leu Pro Val
19955 19960 19965
Leu Ile Lys Glu Gln Leu Glu Pro Pro Glu Ile Asp Met Lys Asn Phe
19970 19975 19980 1
Pro Ser His Thr Val Tyr Val Arg Ala Gly Ser Asn Leu Lys Val Asp
9985 19990 19995 20000
Ile Pro Ile Ser Gly Lys Pro Leu Pro Lys Val Thr Leu Ser Arg Asp
20005 20010 20015
Gly Val Pro Leu Lys Ala Thr Met Arg Phe Asn Thr Glu Ile Thr Ala
20020 20025 20030
Glu Asn Leu Thr Ile Asn Leu Lys Glu Ser Val Thr Ala Asp Ala Gly
20035 20040 20045
Arg Tyr Glu Ile Thr Ala Ala Asn Ser Ser Gly Thr Thr Lys Ala Phe
20050 20055 20060 2
 
Ile Asn Ile Val Val Leu Asp Arg Pro Gly Pro Pro Thr Gly Pro Val
0065                20070               20075               20080
Val Ile Ser Asp Ile Thr Glu Glu Ser Val Thr Leu Lys Trp Glu Pro
                20085               20090               20095
Pro Lys Tyr Asp Gly Gly Ser Gln Val Thr Asn Tyr Ile Leu Leu Lys
            20100               20105               20110
Arg Glu Thr Ser Thr Ala Val Trp Thr Glu Val Ser Ala Thr Val Ala
        20115               20120               20125
Arg Thr Met Met Lys Val Met Lys Leu Thr Thr Gly Glu Glu Tyr Gln
    20130               20135               20140               2
Phe Arg Ile Lys Ala Glu Asn Arg Phe Gly Ile Ser Asp His Ile Asp
0145                20150               20155               20160
Ser Ala Cys Val Thr Val Lys Leu Pro Tyr Thr Thr Pro Gly Pro Pro
                20165               20170               20175
Ser Thr Pro Trp Val Thr Asn Val Thr Arg Glu Ser Ile Thr Val Gly
            20180               20185               20190
Trp His Glu Pro Val Ser Asn Gly Gly Ser Ala Val Val Gly Tyr His
        20195               20200               20205
Leu Glu Met Lys Asp Arg Asn Ser Ile Leu Trp Gln Lys Ala Asn Lys
    20210               20215               20220               2
Leu Val Ile Arg Thr Thr His Phe Lys Val Thr Thr Ile Ser Ala Gly
0225                20230               20235               20240
Leu Ile Tyr Glu Phe Arg Val Tyr Ala Glu Asn Ala Ala Gly Val Gly
                20245               20250               20255
Lys Pro Ser His Pro Ser Glu Pro Val Leu Ala Ile Asp Ala Cys Glu
            20260               20265               20270
Pro Pro Arg Asn Val Arg Ile Thr Asp Ile Ser Lys Asn Ser Val Ser
        20275               20280               20285
Leu Ser Trp Gln Gln Pro Ala Phe Asp Gly Gly Ser Lys Ile Thr Gly
    20290               20295               20300               2
Tyr Ile Val Glu Arg Arg Asp Leu Pro Asp Gly Arg Trp Thr Lys Ala
0305                20310               20315               20320
Ser Phe Thr Asn Val Thr Glu Thr Gln Phe Thr Ile Ser Gly Leu Thr
                20325               20330               20335
Gln Asn Ser Gln Tyr Glu Phe Arg Val Phe Ala Arg Asn Ala Val Gly
            20340               20345               20350
Ser Ile Ser Asn Pro Ser Glu Val Val Gly Pro Ile Thr Cys Ile Asp
        20355               20360               20365
Ser Tyr Gly Gly Pro Val Ile Asp Leu Pro Leu Glu Tyr Thr Glu Val
    20370               20375               20380               2
Val Lys Tyr Arg Ala Gly Thr Ser Val Lys Leu Arg Ala Gly Ile Ser
0385                20390               20395               20400
Gly Lys Pro Ala Pro Thr Ile Glu Trp Tyr Lys Asp Asp Lys Glu Leu
                20405               20410               20415
Gln Thr Asn Ala Leu Val Cys Val Glu Asn Thr Thr Asp Leu Ala Ser
            20420               20425               20430
Ile Leu Ile Lys Asp Ala Asp Arg Leu Asn Ser Gly Cys Tyr Glu Leu
        20435               20440               20445
Lys Leu Arg Asn Ala Met Ala Ser Ala Ser Ala Thr Ile Arg Val Gln
    20450               20455               20460               2
Ile Leu Asp Lys Pro Gly Pro Pro Gly Gly Pro Ile Glu Phe Lys Thr
0465                20470               20475               20480
Val Thr Ala Glu Lys Ile Thr Leu Leu Trp Arg Pro Pro Ala Asp Asp
                20485               20490               20495
Gly Gly Ala Lys Ile Thr His Tyr Ile Val Glu Lys Arg Glu Thr Ser
            20500               20505               20510
Arg Val Val Trp Ser Met Val Ser Glu His Leu Glu Glu Cys Ile Ile
        20515               20520               20525
Thr Thr Thr Lys Ile Ile Lys Gly Asn Glu Tyr Ile Phe Arg Val Arg
    20530               20535               20540               2
Ala Val Asn Lys Tyr Gly Ile Gly Glu Pro Leu Glu Ser Asp Ser Val
0545                20550               20555               20560
 
Val Ala Lys Asn Ala Phe Val Thr Pro Gly Pro Pro Gly Ile Pro Glu
20565 20570 20575
Val Thr Lys Ile Thr Lys Asn Ser Met Thr Val Val Trp Ser Arg Pro
20580 20585 20590
Ile Ala Asp Gly Gly Ser Asp Ile Ser Gly Tyr Phe Leu Glu Lys Arg
20595 20600 20605
Asp Lys Lys Ser Leu Gly Trp Phe Lys Val Leu Lys Glu Thr Ile Arg
20610 20615 20620 2
Asp Thr Arg Gln Lys Val Thr Gly Leu Thr Glu Asn Ser Asp Tyr Gln
0625 20630 20635 20640
Tyr Arg Val Cys Ala Val Asn Ala Ala Gly Gln Gly Pro Phe Ser Glu
20645 20650 20655
Pro Ser Glu Phe Tyr Lys Ala Ala Asp Pro Ile Asp Pro Pro Gly Pro
20660 20665 20670
Pro Ala Lys Ile Arg Ile Ala Asp Ser Thr Lys Ser Ser Ile Thr Leu
20675 20680 20685
Gly Trp Ser Lys Pro Val Tyr Asp Gly Gly Ser Ala Val Thr Gly Tyr
20690 20695 20700 2
Val Val Glu Ile Arg Gln Gly Glu Glu Glu Glu Trp Thr Thr Val Ser
0705 20710 20715 20720
Thr Lys Gly Glu Val Arg Thr Thr Glu Tyr Val Val Ser Asn Leu Lys
20725 20730 20735
Pro Gly Val Asn Tyr Tyr Phe Arg Val Ser Ala Val Asn Cys Ala Gly
20740 20745 20750
Gln Gly Glu Pro Ile Glu Met Asn Glu Pro Val Gln Ala Lys Asp Ile
20755 20760 20765
Leu Glu Ala Pro Glu Ile Asp Leu Asp Val Ala Leu Arg Thr Ser Val
20770 20775 20780 2
Ile Ala Lys Ala Gly Glu Asp Val Gln Val Leu Ile Pro Phe Lys Gly
0785 20790 20795 20800
Arg Pro Pro Pro Thr Val Thr Trp Arg Lys Asp Glu Lys Asn Leu Gly
20805 20810 20815
Ser Asp Ala Arg Tyr Ser Ile Glu Asn Thr Asp Ser Ser Ser Leu Leu
20820 20825 20830
Thr Ile Pro Gln Val Thr Arg Asn Asp Thr Gly Lys Tyr Ile Leu Thr
20835 20840 20845
Ile Glu Asn Gly Val Gly Glu Pro Lys Ser Ser Thr Val Ser Val Lys
20850 20855 20860 2
Val Leu Asp Thr Pro Ala Ala Cys Gln Lys Leu Gln Val Lys His Val
0865 20870 20875 20880
Ser Arg Gly Thr Val Thr Leu Leu Trp Asp Pro Pro Leu Ile Asp Gly
20885 20890 20895
Gly Ser Pro Ile Ile Asn Tyr Val Ile Glu Lys Arg Asp Ala Thr Lys
20900 20905 20910
Arg Thr Trp Ser Val Val Ser His Lys Cys Ser Ser Thr Ser Phe Lys
20915 20920 20925
Leu Ile Asp Leu Ser Glu Lys Thr Pro Phe Phe Phe Arg Val Leu Ala
20930 20935 20940 2
Glu Asn Glu Ile Gly Ile Gly Glu Pro Cys Glu Thr Thr Glu Pro Val
0945 20950 20955 20960
Lys Ala Ala Glu Val Pro Ala Pro Ile Arg Asp Leu Ser Met Lys Asp
20965 20970 20975
Ser Thr Lys Thr Ser Val Ile Leu Ser Trp Thr Lys Pro Asp Phe Asp
20980 20985 20990
Gly Gly Ser Val Ile Thr Glu Tyr Val Val Glu Arg Lys Gly Lys Gly
20995 21000 21005
Glu Gln Thr Trp Ser His Ala Gly Ile Ser Lys Thr Cys Glu Ile Glu
21010 21015 21020 2
Val Ser Gln Leu Lys Glu Gln Ser Val Leu Glu Phe Arg Val Phe Ala
1025 21030 21035 21040
Lys Asn Glu Lys Gly Leu Ser Asp Pro Val Thr Ile Gly Pro Ile Thr
21045 21050 21055
 
Val Lys Glu Leu Ile Ile Thr Pro Glu Val Asp Leu Ser Asp Ile Pro
21060 21065 21070
Gly Ala Gln Val Thr Val Arg Ile Gly His Asn Val His Leu Glu Leu
21075 21080 21085
Pro Tyr Lys Gly Lys Pro Lys Pro Ser Ile Ser Trp Leu Lys Asp Gly
21090 21095 21100 2
Leu Pro Leu Lys Glu Ser Glu Phe Val Arg Phe Ser Lys Thr Glu Asn
1105 21110 21115 21120
Lys Ile Thr Leu Ser Ile Lys Asn Ala Lys Lys Glu His Gly Gly Lys
21125 21130 21135
Tyr Thr Val Ile Leu Asp Asn Ala Val Cys Arg Ile Ala Val Pro Ile
21140 21145 21150
Thr Val Ile Thr Leu Gly Pro Pro Ser Lys Pro Lys Gly Pro Ile Arg
21155 21160 21165
Phe Asp Glu Ile Lys Ala Asp Ser Val Ile Leu Ser Trp Asp Val Pro
21170 21175 21180 2
Glu Asp Asn Gly Gly Gly Glu Ile Thr Cys Tyr Ser Ile Glu Lys Arg
1185 21190 21195 21200
Glu Thr Ser Gln Thr Asn Trp Lys Met Val Cys Ser Ser Val Ala Arg
21205 21210 21215
Thr Thr Phe Lys Val Pro Asn Leu Val Lys Asp Ala Glu Tyr Gln Phe
21220 21225 21230
Arg Val Arg Ala Glu Asn Arg Tyr Gly Val Ser Gln Pro Leu Val Ser
21235 21240 21245
Ser Ile Ile Val Ala Lys His Gln Phe Arg Ile Pro Gly Pro Pro Gly
21250 21255 21260 2
Lys Pro Val Ile Tyr Asn Val Thr Ser Asp Gly Met Ser Leu Thr Trp
1265 21270 21275 21280
Asp Ala Pro Val Tyr Asp Gly Gly Ser Glu Val Thr Gly Phe His Val
21285 21290 21295
Glu Lys Lys Glu Arg Asn Ser Ile Leu Trp Gln Lys Val Asn Thr Ser
21300 21305 21310
Pro Ile Ser Gly Arg Glu Tyr Arg Ala Thr Gly Leu Val Glu Gly Leu
21315 21320 21325
Asp Tyr Gln Phe Arg Val Tyr Ala Glu Asn Ser Ala Gly Leu Ser Ser
21330 21335 21340 2
Pro Ser Asp Pro Ser Lys Phe Thr Leu Ala Val Ser Pro Val Asp Pro
1345 21350 21355 21360
Pro Gly Thr Pro Asp Tyr Ile Asp Val Thr Arg Glu Thr Ile Thr Leu
21365 21370 21375
Lys Trp Asn Pro Pro Leu Arg Asp Gly Gly Ser Lys Ile Val Gly Tyr
21380 21385 21390
Ser Ile Glu Lys Arg Gln Gly Asn Glu Arg Trp Val Arg Cys Asn Phe
21395 21400 21405
Thr Asp Val Ser Glu Cys Gln Tyr Thr Val Thr Gly Leu Ser Pro Gly
21410 21415 21420 2
Asp Arg Tyr Glu Phe Arg Ile Ile Ala Arg Asn Ala Val Gly Thr Ile
1425 21430 21435 21440
Ser Pro Pro Ser Gln Ser Ser Gly Ile Ile Met Thr Arg Asp Glu Asn
21445 21450 21455
Val Pro Pro Ile Val Glu Phe Gly Pro Glu Tyr Phe Asp Gly Leu Ile
21460 21465 21470
Ile Lys Ser Gly Glu Ser Leu Arg Ile Lys Ala Leu Val Gln Gly Arg
21475 21480 21485
Pro Val Pro Arg Val Thr Trp Phe Lys Asp Gly Val Glu Ile Glu Lys
21490 21495 21500 2
Arg Met Asn Met Glu Ile Thr Asn Val Leu Gly Ser Thr Ser Leu Phe
1505 21510 21515 21520
Val Arg Asp Ala Thr Arg Asp His Arg Gly Val Tyr Thr Val Glu Ala
21525 21530 21535
Lys Asn Ala Ser Gly Ser Ala Lys Ala Glu Ile Lys Val Lys Val Gln
21540 21545 21550
 
Asp Thr Pro Gly Lys Val Val Gly Pro Ile Arg Phe Thr Asn Ile Thr
21555 21560 21565
Gly Glu Lys Met Thr Leu Trp Trp Asp Ala Pro Leu Asn Asp Gly Cys
21570 21575 21580 2
Ala Pro Ile Thr His Tyr Ile Ile Glu Lys Arg Glu Thr Ser Arg Leu
1585 21590 21595 21600
Ala Trp Ala Leu Ile Glu Asp Lys Cys Glu Ala Gln Ser Tyr Thr Ala
21605 21610 21615
Ile Lys Leu Ile Asn Gly Asn Glu Tyr Gln Phe Arg Val Ser Ala Val
21620 21625 21630
Asn Lys Phe Gly Val Gly Arg Pro Leu Asp Ser Asp Pro Val Val Ala
21635 21640 21645
Gln Ile Gln Tyr Thr Val Pro Asp Ala Pro Gly Ile Pro Glu Pro Ser
21650 21655 21660 2
Asn Ile Thr Gly Asn Ser Ile Thr Leu Thr Trp Ala Arg Pro Glu Ser
1665 21670 21675 21680
Asp Gly Gly Ser Glu Ile Gln Gln Tyr Ile Leu Glu Arg Arg Glu Lys
21685 21690 21695
Lys Ser Thr Arg Trp Val Lys Val Ile Ser Lys Arg Pro Ile Ser Glu
21700 21705 21710
Thr Arg Phe Lys Val Thr Gly Leu Thr Glu Gly Asn Glu Tyr Glu Phe
21715 21720 21725
His Val Met Ala Glu Asn Ala Ala Gly Val Gly Pro Ala Ser Gly Ile
21730 21735 21740 2
Ser Arg Leu Ile Lys Cys Arg Glu Pro Val Asn Pro Pro Gly Pro Pro
1745 21750 21755 21760
Thr Val Val Lys Val Thr Asp Thr Ser Lys Thr Thr Val Ser Leu Glu
21765 21770 21775
Trp Ser Lys Pro Val Phe Asp Gly Gly Met Glu Ile Ile Gly Tyr Ile
21780 21785 21790
Ile Glu Met Cys Lys Thr Asp Leu Gly Asp Trp His Lys Val Asn Ala
21795 21800 21805
Glu Ala Cys Val Lys Thr Arg Tyr Thr Val Thr Asp Leu Gln Ala Gly
21810 21815 21820 2
Glu Glu Tyr Lys Phe Arg Val Ser Ala Ile Asn Gly Ala Gly Lys Gly
1825 21830 21835 21840
Asp Ser Cys Glu Val Thr Gly Thr Ile Lys Ala Val Asp Arg Leu Thr
21845 21850 21855
Ala Pro Glu Leu Asp Ile Asp Ala Asn Phe Lys Gln Thr His Val Val
21860 21865 21870
Arg Ala Gly Ala Ser Ile Arg Leu Phe Ile Ala Tyr Gln Gly Arg Pro
21875 21880 21885
Thr Pro Thr Ala Val Trp Ser Lys Pro Asp Ser Asn Leu Ser Leu Arg
21890 21895 21900 2
Ala Asp Ile His Thr Thr Asp Ser Phe Ser Thr Leu Thr Val Glu Asn
1905 21910 21915 21920
Cys Asn Arg Asn Asp Ala Gly Lys Tyr Thr Leu Thr Val Glu Asn Asn
21925 21930 21935
Ser Gly Ser Lys Ser Ile Thr Phe Thr Val Lys Val Leu Asp Thr Pro
21940 21945 21950
Gly Pro Pro Gly Pro Ile Thr Phe Lys Asp Val Thr Arg Gly Ser Ala
21955 21960 21965
Thr Leu Met Trp Asp Ala Pro Leu Leu Asp Gly Gly Ala Arg Ile His
21970 21975 21980 2
His Tyr Val Val Glu Lys Arg Glu Ala Ser Arg Arg Ser Trp Gln Val
1985 21990 21995 22000
Ile Ser Glu Lys Cys Thr Arg Gln Ile Phe Lys Val Asn Asp Leu Ala
22005 22010 22015
Glu Gly Val Pro Tyr Tyr Phe Arg Val Ser Ala Val Asn Glu Tyr Gly
22020 22025 22030
Val Gly Glu Pro Tyr Glu Met Pro Glu Pro Ile Val Ala Thr Glu Gln
22035 22040 22045
 
Pro Ala Pro Pro Arg Arg Leu Asp Val Val Asp Thr Ser Lys Ser Ser
22050 22055 22060 2
Ala Val Leu Ala Trp Leu Lys Pro Asp His Asp Gly Gly Ser Arg Ile
2065 22070 22075 22080
Thr Gly Tyr Leu Leu Glu Met Arg Gln Lys Gly Ser Asp Leu Trp Val
22085 22090 22095
Glu Ala Gly His Thr Lys Gln Leu Thr Phe Thr Val Glu Arg Leu Val
22100 22105 22110
Glu Lys Thr Glu Tyr Glu Phe Arg Val Lys Ala Lys Asn Asp Ala Gly
22115 22120 22125
Tyr Ser Glu Pro Arg Glu Ala Phe Ser Ser Val Ile Ile Lys Glu Pro
22130 22135 22140 2
Gln Ile Glu Pro Thr Ala Asp Leu Thr Gly Ile Thr Asn Gln Leu Ile
2145 22150 22155 22160
Thr Cys Lys Ala Gly Ser Pro Phe Thr Ile Asp Val Pro Ile Ser Gly
22165 22170 22175
Arg Pro Ala Pro Lys Val Thr Trp Lys Leu Glu Glu Met Arg Leu Lys
22180 22185 22190
Glu Thr Asp Arg Val Ser Ile Thr Thr Thr Lys Asp Arg Thr Thr Leu
22195 22200 22205
Thr Val Lys Asp Ser Met Arg Gly Asp Ser Gly Arg Tyr Phe Leu Thr
22210 22215 22220 2
Leu Glu Asn Thr Ala Gly Val Lys Thr Phe Ser Val Thr Val Val Val
2225 22230 22235 22240
Ile Gly Arg Pro Gly Pro Val Thr Gly Pro Ile Glu Val Ser Ser Val
22245 22250 22255
Ser Ala Glu Ser Cys Val Leu Ser Trp Gly Glu Pro Lys Asp Gly Gly
22260 22265 22270
Gly Thr Glu Ile Thr Asn Tyr Ile Val Glu Lys Arg Glu Ser Gly Thr
22275 22280 22285
Thr Ala Trp Gln Leu Val Asn Ser Ser Val Lys Arg Thr Gln Ile Lys
22290 22295 22300 2
Val Thr His Leu Thr Lys Tyr Met Glu Tyr Ser Phe Arg Val Ser Ser
2305 22310 22315 22320
Glu Asn Arg Phe Gly Val Ser Lys Pro Leu Glu Ser Ala Pro Ile Ile
22325 22330 22335
Ala Glu His Pro Phe Val Pro Pro Ser Ala Pro Thr Arg Pro Glu Val
22340 22345 22350
Tyr His Val Ser Ala Asn Ala Met Ser Ile Arg Trp Glu Glu Pro Tyr
22355 22360 22365
His Asp Gly Gly Ser Lys Ile Ile Gly Tyr Trp Val Glu Lys Lys Glu
22370 22375 22380 2
Arg Asn Thr Ile Leu Trp Val Lys Glu Asn Lys Val Pro Cys Leu Glu
2385 22390 22395 22400
Cys Asn Tyr Lys Val Thr Gly Leu Val Glu Gly Leu Glu Tyr Gln Phe
22405 22410 22415
Arg Thr Tyr Ala Leu Asn Ala Ala Gly Val Ser Lys Ala Ser Glu Ala
22420 22425 22430
Ser Arg Pro Ile Met Ala Gln Asn Pro Val Asp Ala Pro Gly Arg Pro
22435 22440 22445
Glu Val Thr Asp Val Thr Arg Ser Thr Val Ser Leu Ile Trp Ser Ala
22450 22455 22460 2
Pro Ala Tyr Asp Gly Gly Ser Lys Val Val Gly Tyr Ile Ile Glu Arg
2465 22470 22475 22480
Lys Pro Val Ser Glu Val Gly Asp Gly Arg Trp Leu Lys Cys Asn Tyr
22485 22490 22495
Thr Ile Val Ser Asp Asn Phe Phe Thr Val Thr Ala Leu Ser Glu Gly
22500 22505 22510
Asp Thr Tyr Glu Phe Arg Val Leu Ala Lys Asn Ala Ala Gly Val Ile
22515 22520 22525
Ser Lys Gly Ser Glu Ser Thr Gly Pro Val Thr Cys Arg Asp Glu Tyr
22530 22535 22540 2
 
Ala Pro Pro Lys Ala Glu Leu Asp Ala Arg Leu His Gly Asp Leu Val
2545                22550               22555               22560
Thr Ile Arg Ala Gly Ser Asp Leu Val Leu Asp Ala Ala Val Gly Gly
                22565               22570               22575
Lys Pro Glu Pro Lys Ile Ile Trp Thr Lys Gly Asp Lys Glu Leu Asp
            22580               22585               22590
Leu Cys Glu Lys Val Ser Leu Gln Tyr Thr Gly Lys Arg Ala Thr Ala
        22595               22600               22605
Val Ile Lys Phe Cys Asp Arg Ser Asp Ser Gly Lys Tyr Thr Leu Thr
    22610               22615               22620               2
Val Lys Asn Ala Ser Gly Thr Lys Ala Val Ser Val Met Val Lys Val
2625                22630               22635               22640
Leu Asp Ser Pro Gly Pro Cys Gly Lys Leu Thr Val Ser Arg Val Thr
                22645               22650               22655
Gln Glu Lys Cys Thr Leu Ala Trp Ser Leu Pro Gln Glu Asp Gly Gly
            22660               22665               22670
Ala Glu Ile Thr His Tyr Ile Val Glu Arg Arg Glu Thr Ser Arg Leu
        22675               22680               22685
Asn Trp Val Ile Val Glu Gly Glu Cys Pro Thr Leu Ser Tyr Val Val
    22690               22695               22700               2
Thr Arg Leu Ile Lys Asn Asn Glu Tyr Ile Phe Arg Val Arg Ala Val
2705                22710               22715               22720
Asn Lys Tyr Gly Pro Gly Val Pro Val Glu Ser Glu Pro Ile Val Ala
                22725               22730               22735
Arg Asn Ser Phe Thr Ile Pro Ser Pro Pro Gly Ile Pro Glu Glu Val
            22740               22745               22750
Gly Thr Gly Lys Glu His Ile Ile Ile Gln Trp Thr Lys Pro Glu Ser
        22755               22760               22765
Asp Gly Gly Asn Glu Ile Ser Asn Tyr Leu Val Asp Lys Arg Glu Lys
    22770               22775               22780               2
Glu Ser Leu Arg Trp Thr Arg Val Asn Lys Asp Tyr Val Val Tyr Asp
2785                22790               22795               22800
Thr Arg Leu Lys Val Thr Ser Leu Met Glu Gly Cys Asp Tyr Gln Phe
                22805               22810               22815
Arg Val Thr Ala Val Asn Ala Ala Gly Asn Ser Glu Pro Ser Glu Arg
            22820               22825               22830
Ser Asn Phe Ile Ser Cys Arg Glu Pro Ser Tyr Thr Pro Gly Pro Pro
        22835               22840               22845
Ser Ala Pro Arg Val Val Asp Thr Thr Lys His Ser Ile Ser Leu Ala
    22850               22855               22860               2
Trp Thr Lys Pro Met Tyr Asp Gly Gly Thr Asp Ile Val Gly Tyr Val
2865                22870               22875               22880
Leu Glu Met Gln Glu Lys Asp Thr Asp Gln Trp Tyr Arg Val His Thr
                22885               22890               22895
Asn Ala Thr Ile Arg Asn Thr Glu Phe Thr Val Pro Asp Leu Lys Met
            22900               22905               22910
Gly Gln Lys Tyr Ser Phe Arg Val Ala Ala Val Asn Val Lys Gly Met
        22915               22920               22925
Ser Glu Tyr Ser Glu Ser Ile Ala Glu Ile Glu Pro Val Glu Arg Ile
    22930               22935               22940               2
Glu Ile Pro Asp Leu Glu Leu Ala Asp Asp Leu Lys Lys Thr Val Thr
2945                22950               22955               22960
Ile Arg Ala Gly Ala Ser Leu Arg Leu Met Val Ser Val Ser Gly Arg
                22965               22970               22975
Pro Pro Pro Val Ile Thr Trp Ser Lys Gln Gly Ile Asp Leu Ala Ser
            22980               22985               22990
Arg Ala Ile Ile Asp Thr Thr Glu Ser Tyr Ser Leu Leu Ile Val Asp
        22995               23000               23005
Lys Val Asn Arg Tyr Asp Ala Gly Lys Tyr Thr Ile Glu Ala Glu Asn
    23010               23015               23020               2
Gln Ser Gly Lys Lys Ser Ala Thr Val Leu Val Lys Val Tyr Asp Thr
3025                23030               23035               23040
 
Pro Gly Pro Cys Pro Ser Val Lys Val Lys Glu Val Ser Arg Asp Ser
23045 23050 23055
Val Thr Ile Thr Trp Glu Ile Pro Thr Ile Asp Gly Gly Ala Pro Ile
23060 23065 23070
Asn Asn Tyr Ile Val Glu Lys Arg Glu Ala Ala Met Arg Ala Phe Lys
23075 23080 23085
Thr Val Thr Thr Lys Cys Ser Lys Thr Leu Tyr Arg Ile Ser Gly Leu
23090 23095 23100 2
Val Glu Gly Thr Met His Tyr Phe Arg Val Leu Pro Glu Asn Ile Tyr
3105 23110 23115 23120
Gly Ile Gly Glu Pro Cys Glu Thr Ser Asp Ala Val Leu Val Ser Glu
23125 23130 23135
Val Pro Leu Val Pro Ala Lys Leu Glu Val Val Asp Val Thr Lys Ser
23140 23145 23150
Thr Val Thr Leu Ala Trp Glu Lys Pro Leu Tyr Asp Gly Gly Ser Arg
23155 23160 23165
Leu Thr Gly Tyr Val Leu Glu Ala Cys Lys Ala Gly Thr Glu Arg Trp
23170 23175 23180 2
Met Lys Val Val Thr Leu Lys Pro Thr Val Leu Glu His Thr Val Thr
3185 23190 23195 23200
Ser Leu Asn Glu Gly Glu Gln Tyr Leu Phe Arg Ile Arg Ala Gln Asn
23205 23210 23215
Glu Lys Gly Val Ser Glu Pro Arg Glu Thr Val Thr Ala Val Thr Val
23220 23225 23230
Gln Asp Leu Arg Val Leu Pro Thr Ile Asp Leu Ser Thr Met Pro Gln
23235 23240 23245
Lys Thr Ile His Val Pro Ala Gly Arg Pro Val Glu Leu Val Ile Pro
23250 23255 23260 2
Ile Ala Gly Arg Pro Pro Pro Ala Ala Ser Trp Phe Phe Ala Gly Ser
3265 23270 23275 23280
Lys Leu Arg Glu Ser Glu Arg Val Thr Val Glu Thr His Thr Lys Val
23285 23290 23295
Ala Lys Leu Thr Ile Arg Glu Thr Thr Ile Arg Asp Thr Gly Glu Tyr
23300 23305 23310
Thr Leu Glu Leu Lys Asn Val Thr Gly Thr Thr Ser Glu Thr Ile Lys
23315 23320 23325
Val Ile Ile Leu Asp Lys Pro Gly Pro Pro Thr Gly Pro Ile Lys Ile
23330 23335 23340 2
Asp Glu Ile Asp Ala Thr Ser Ile Thr Ile Ser Trp Glu Pro Pro Glu
3345 23350 23355 23360
Leu Asp Gly Gly Ala Pro Leu Ser Gly Tyr Val Val Glu Gln Arg Asp
23365 23370 23375
Ala His Arg Pro Gly Trp Leu Pro Val Ser Glu Ser Val Thr Arg Ser
23380 23385 23390
Thr Phe Lys Phe Thr Arg Leu Thr Glu Gly Asn Glu Tyr Val Phe Arg
23395 23400 23405
Val Ala Ala Thr Asn Arg Phe Gly Ile Gly Ser Tyr Leu Gln Ser Glu
23410 23415 23420 2
Val Ile Glu Cys Arg Ser Ser Ile Arg Ile Pro Gly Pro Pro Glu Thr
3425 23430 23435 23440
Leu Gln Ile Phe Asp Val Ser Arg Asp Gly Met Thr Leu Thr Trp Tyr
23445 23450 23455
Pro Pro Glu Asp Asp Gly Gly Ser Gln Val Thr Gly Tyr Ile Val Glu
23460 23465 23470
Arg Lys Glu Val Arg Ala Asp Arg Trp Val Arg Val Asn Lys Val Pro
23475 23480 23485
Val Thr Met Thr Arg Tyr Arg Ser Thr Gly Leu Thr Glu Gly Leu Glu
23490 23495 23500 2
Tyr Glu His Arg Val Thr Ala Ile Asn Ala Arg Gly Ser Gly Lys Pro
3505 23510 23515 23520
Ser Arg Pro Ser Lys Pro Ile Val Ala Met Asp Pro Ile Ala Pro Pro
23525 23530 23535
 
Gly Lys Pro Gln Asn Pro Arg Val Thr Asp Thr Thr Arg Thr Ser Val
            23540               23545               23550
Ser Leu Ala Trp Ser Val Pro Glu Asp Glu Gly Gly Ser Lys Val Thr
        23555               23560               23565
Gly Tyr Leu Ile Glu Met Gln Lys Val Asp Gln His Glu Trp Thr Lys
    23570               23575               23580               2
Cys Asn Thr Thr Pro Thr Lys Ile Arg Glu Tyr Thr Leu Thr His Leu
3585                23590               23595               23600
Pro Gln Gly Ala Glu Tyr Arg Phe Arg Val Leu Ala Cys Asn Ala Gly
                23605               23610               23615
Gly Pro Gly Glu Pro Ala Glu Val Pro Gly Thr Val Lys Val Thr Glu
            23620               23625               23630
Met Leu Glu Tyr Pro Asp Tyr Glu Leu Asp Glu Arg Tyr Gln Glu Gly
        23635               23640               23645
Ile Phe Val Arg Gln Gly Gly Val Ile Arg Leu Thr Ile Pro Ile Lys
    23650               23655               23660               2
Gly Lys Pro Phe Pro Ile Cys Lys Trp Thr Lys Glu Gly Gln Asp Ile
3665                23670               23675               23680
Ser Lys Arg Ala Met Ile Ala Thr Ser Glu Thr His Thr Glu Leu Val
                23685               23690               23695
Ile Lys Glu Ala Asp Arg Gly Asp Ser Gly Thr Tyr Asp Leu Val Leu
            23700               23705               23710
Glu Asn Lys Cys Gly Lys Lys Ala Val Tyr Ile Lys Val Arg Val Ile
        23715               23720               23725
Gly Ser Pro Asn Ser Pro Glu Gly Pro Leu Glu Tyr Asp Asp Ile Gln
    23730               23735               23740               2
Val Arg Ser Val Arg Val Ser Trp Arg Pro Pro Ala Asp Asp Gly Gly
3745                23750               23755               23760
Ala Asp Ile Leu Gly Tyr Ile Leu Glu Arg Arg Glu Val Pro Lys Ala
                23765               23770               23775
Ala Trp Tyr Thr Ile Asp Ser Arg Val Arg Gly Thr Ser Leu Val Val
            23780               23785               23790
Lys Gly Leu Lys Glu Asn Val Glu Tyr His Phe Arg Val Ser Ala Glu
        23795               23800               23805
Asn Gln Phe Gly Ile Ser Lys Pro Leu Lys Ser Glu Glu Pro Val Thr
    23810               23815               23820               2
Pro Lys Thr Pro Leu Asn Pro Pro Glu Pro Pro Ser Asn Pro Pro Glu
3825                23830               23835               23840
Val Leu Asp Val Thr Lys Ser Ser Val Ser Leu Ser Trp Ser Arg Pro
                23845               23850               23855
Lys Asp Asp Gly Gly Ser Arg Val Thr Gly Tyr Tyr Ile Glu Arg Lys
            23860               23865               23870
Glu Thr Ser Thr Asp Lys Trp Val Arg His Asn Lys Thr Gln Ile Thr
        23875               23880               23885
Thr Thr Met Tyr Thr Val Thr Gly Leu Val Pro Asp Ala Glu Tyr Gln
    23890               23895               23900               2
Phe Arg Ile Ile Ala Gln Asn Asp Val Gly Leu Ser Glu Thr Ser Pro
3905                23910               23915               23920
Ala Ser Glu Pro Val Val Cys Lys Asp Pro Phe Asp Lys Pro Ser Gln
                23925               23930               23935
Pro Gly Glu Leu Glu Ile Leu Ser Ile Ser Lys Asp Ser Val Thr Leu
            23940               23945               23950
Gln Trp Glu Lys Pro Glu Cys Asp Gly Gly Lys Glu Ile Leu Gly Tyr
        23955               23960               23965
Trp Val Glu Tyr Arg Gln Ser Gly Asp Ser Ala Trp Lys Lys Ser Asn
    23970               23975               23980               2
Lys Glu Arg Ile Lys Asp Lys Gln Phe Thr Ile Gly Gly Leu Leu Glu
3985                23990               23995               24000
Ala Thr Glu Tyr Glu Phe Arg Val Phe Ala Glu Asn Glu Thr Gly Leu
                24005               24010               24015
Ser Arg Pro Arg Arg Thr Ala Met Ser Ile Lys Thr Lys Leu Thr Ser
            24020               24025               24030
 
Gly Glu Ala Pro Gly Ile Arg Lys Glu Met Lys Asp Val Thr Thr Lys
24035 24040 24045
Leu Gly Glu Ala Ala Gln Leu Ser Cys Gln Ile Val Gly Arg Pro Leu
24050 24055 24060 2
Pro Asp Ile Lys Trp Tyr Arg Phe Gly Lys Glu Leu Ile Gln Ser Arg
4065 24070 24075 24080
Lys Tyr Lys Met Ser Ser Asp Gly Arg Thr His Thr Leu Thr Val Met
24085 24090 24095
Thr Glu Glu Gln Glu Asp Glu Gly Val Tyr Thr Cys Ile Ala Thr Asn
24100 24105 24110
Glu Val Gly Glu Val Glu Thr Ser Ser Lys Leu Leu Leu Gln Ala Thr
24115 24120 24125
Pro Gln Phe His Pro Gly Tyr Pro Leu Lys Glu Lys Tyr Tyr Gly Ala
24130 24135 24140 2
Val Gly Ser Thr Leu Arg Leu His Val Met Tyr Ile Gly Arg Pro Val
4145 24150 24155 24160
Pro Ala Met Thr Trp Phe His Gly Gln Lys Leu Leu Gln Asn Ser Glu
24165 24170 24175
Asn Ile Thr Ile Glu Asn Thr Glu His Tyr Thr His Leu Val Met Lys
24180 24185 24190
Asn Val Gln Arg Lys Thr His Ala Gly Lys Tyr Lys Val Gln Leu Ser
24195 24200 24205
Asn Val Phe Gly Thr Val Asp Ala Ile Leu Asp Val Glu Ile Gln Asp
24210 24215 24220 2
Lys Pro Asp Lys Pro Thr Gly Pro Ile Val Ile Glu Ala Leu Leu Lys
4225 24230 24235 24240
Asn Ser Ala Val Ile Ser Trp Lys Pro Pro Ala Asp Asp Gly Gly Ser
24245 24250 24255
Trp Ile Thr Asn Tyr Val Val Glu Lys Cys Glu Ala Lys Glu Gly Ala
24260 24265 24270
Glu Trp Gln Leu Val Ser Ser Ala Ile Ser Val Thr Thr Cys Arg Ile
24275 24280 24285
Val Asn Leu Thr Glu Asn Ala Gly Tyr Tyr Phe Arg Val Ser Ala Gln
24290 24295 24300 2
Asn Thr Phe Gly Ile Ser Asp Pro Leu Glu Val Ser Ser Val Val Ile
4305 24310 24315 24320
Ile Lys Ser Pro Phe Glu Lys Pro Gly Ala Pro Gly Lys Pro Thr Ile
24325 24330 24335
Thr Ala Val Thr Lys Asp Ser Cys Val Val Ala Trp Lys Pro Pro Ala
24340 24345 24350
Ser Asp Gly Gly Ala Lys Ile Arg Asn Tyr Tyr Leu Glu Lys Arg Glu
24355 24360 24365
Lys Lys Gln Asn Lys Trp Ile Ser Val Thr Thr Glu Glu Ile Arg Glu
24370 24375 24380 2
Thr Val Phe Ser Val Lys Asn Leu Ile Glu Gly Leu Glu Tyr Glu Phe
4385 24390 24395 24400
Arg Val Lys Cys Glu Asn Leu Gly Gly Glu Ser Glu Trp Ser Glu Ile
24405 24410 24415
Ser Glu Pro Ile Thr Pro Lys Ser Asp Val Pro Ile Gln Ala Pro His
24420 24425 24430
Phe Lys Glu Glu Leu Arg Asn Leu Asn Val Arg Tyr Gln Ser Asn Ala
24435 24440 24445
Thr Leu Val Cys Lys Val Thr Gly His Pro Lys Pro Ile Val Lys Trp
24450 24455 24460 2
Tyr Arg Gln Gly Lys Glu Ile Ile Ala Asp Gly Leu Lys Tyr Arg Ile
4465 24470 24475 24480
Gln Glu Phe Lys Gly Gly Tyr His Gln Leu Ile Ile Ala Ser Val Thr
24485 24490 24495
Asp Asp Asp Ala Thr Val Tyr Gln Val Arg Ala Thr Asn Gln Gly Gly
24500 24505 24510
Ser Val Ser Gly Thr Ala Ser Leu Glu Val Glu Val Pro Ala Lys Ile
24515 24520 24525
 
His Leu Pro Lys Thr Leu Glu Gly Met Gly Ala Val His Ala Leu Arg
24530 24535 24540 2
Gly Glu Val Val Ser Ile Lys Ile Pro Phe Ser Gly Lys Pro Asp Pro
4545 24550 24555 24560
Val Ile Thr Trp Gln Lys Gly Gln Asp Leu Ile Asp Asn Asn Gly His
24565 24570 24575
Tyr Gln Val Ile Val Thr Arg Ser Phe Thr Ser Leu Val Phe Pro Asn
24580 24585 24590
Gly Val Glu Arg Lys Asp Ala Gly Phe Tyr Val Val Cys Ala Lys Asn
24595 24600 24605
Arg Phe Gly Ile Asp Gln Lys Thr Val Glu Leu Asp Val Ala Asp Val
24610 24615 24620 2
Pro Asp Pro Pro Arg Gly Val Lys Val Ser Asp Ala Ser Arg Asp Ser
4625 24630 24635 24640
Val Asn Leu Thr Trp Thr Glu Pro Ala Ser Asp Gly Gly Ser Lys Ile
24645 24650 24655
Thr Asn Tyr Ile Val Glu Lys Cys Ala Thr Thr Ala Glu Arg Trp Leu
24660 24665 24670
Arg Val Gly Gln Ala Arg Glu Thr Arg Tyr Thr Val Ile Asn Leu Phe
24675 24680 24685
Gly Lys Thr Ser Tyr Gln Phe Arg Val Ile Ala Glu Asn Lys Phe Gly
24690 24695 24700 2
Leu Ser Lys Pro Ser Glu Pro Ser Glu Pro Thr Ile Thr Lys Glu Asp
4705 24710 24715 24720
Lys Thr Arg Ala Met Asn Tyr Asp Glu Glu Val Asp Glu Thr Arg Glu
24725 24730 24735
Val Ser Met Thr Lys Ala Ser His Ser Ser Thr Lys Glu Leu Tyr Glu
24740 24745 24750
Lys Tyr Met Ile Ala Glu Asp Leu Gly Arg Gly Glu Phe Gly Ile Val
24755 24760 24765
His Arg Cys Val Glu Thr Ser Ser Lys Lys Thr Tyr Met Ala Lys Phe
24770 24775 24780 2
Val Lys Val Lys Gly Thr Asp Gln Val Leu Val Lys Lys Glu Ile Ser
4785 24790 24795 24800
Ile Leu Asn Ile Ala Arg His Arg Asn Ile Leu His Leu His Glu Ser
24805 24810 24815
Phe Glu Ser Met Glu Glu Leu Val Met Ile Phe Glu Phe Ile Ser Gly
24820 24825 24830
Leu Asp Ile Phe Glu Arg Ile Asn Thr Ser Ala Phe Glu Leu Asn Glu
24835 24840 24845
Arg Glu Ile Val Ser Tyr Val His Gln Val Cys Glu Ala Leu Gln Phe
24850 24855 24860 2
Leu His Ser His Asn Ile Gly His Phe Asp Ile Arg Pro Glu Asn Ile
4865 24870 24875 24880
Ile Tyr Gln Thr Arg Arg Ser Ser Thr Ile Lys Ile Ile Glu Phe Gly
24885 24890 24895
Gln Ala Arg Gln Leu Lys Pro Gly Asp Asn Phe Arg Leu Leu Phe Thr
24900 24905 24910
Ala Pro Glu Tyr Tyr Ala Pro Glu Val His Gln His Asp Val Val Ser
24915 24920 24925
Thr Ala Thr Asp Met Trp Ser Leu Gly Thr Leu Val Tyr Val Leu Leu
24930 24935 24940 2
Ser Gly Ile Asn Pro Phe Leu Ala Glu Thr Asn Gln Gln Ile Ile Glu
4945 24950 24955 24960
Asn Ile Met Asn Ala Glu Tyr Thr Phe Asp Glu Glu Ala Phe Lys Glu
24965 24970 24975
Ile Ser Ile Glu Ala Met Asp Phe Val Asp Arg Leu Leu Val Lys Glu
24980 24985 24990
Arg Lys Ser Arg Met Thr Ala Ser Glu Ala Leu Gln His Pro Trp Leu
24995 25000 25005
Lys Gln Lys Ile Glu Arg Val Ser Thr Lys Val Ile Arg Thr Leu Lys
25010 25015 25020 2
 
His Arg Arg Tyr Tyr His Thr Leu Ile Lys Lys Asp Leu Asn Met Val
5025                25030               25035               25040
Val Ser Ala Ala Arg Ile Ser Cys Gly Gly Ala Ile Arg Ser Gln Lys
                25045               25050               25055
Gly Val Ser Val Ala Lys Val Lys Val Ala Ser Ile Glu Ile Gly Pro
            25060               25065               25070
Val Ser Gly Gln Ile Met His Ala Val Gly Glu Glu Gly Gly His Val
        25075               25080               25085
Lys Tyr Val Cys Lys Ile Glu Asn Tyr Asp Gln Ser Thr Gln Val Thr
    25090               25095               25100               2
Trp Tyr Phe Gly Val Arg Gln Leu Glu Asn Ser Glu Lys Tyr Glu Ile
5105                25110               25115               25120
Thr Tyr Glu Asp Gly Val Ala Ile Leu Tyr Val Lys Asp Ile Thr Lys
                25125               25130               25135
Leu Asp Asp Gly Thr Tyr Arg Cys Lys Val Val Asn Asp Tyr Gly Glu
            25140               25145               25150
Asp Ser Ser Tyr Ala Glu Leu Phe Val Lys Gly Val Arg Glu Val Tyr
        25155               25160               25165
Asp Tyr Tyr Cys Arg Arg Thr Met Lys Lys Ile Lys Arg Arg Thr Asp
    25170               25175               25180               2
Thr Met Arg Leu Leu Glu Arg Pro Pro Glu Phe Thr Leu Pro Leu Tyr
5185                25190               25195               25200
Asn Lys Thr Ala Tyr Val Gly Glu Asn Val Arg Phe Gly Val Thr Ile
                25205               25210               25215
Thr Val His Pro Glu Pro His Val Thr Trp Tyr Lys Ser Gly Gln Lys
            25220               25225               25230
Ile Lys Pro Gly Asp Asn Asp Lys Lys Tyr Thr Phe Glu Ser Asp Lys
        25235               25240               25245
Gly Leu Tyr Gln Leu Thr Ile Asn Ser Val Thr Thr Asp Asp Asp Ala
    25250               25255               25260               2
Glu Tyr Thr Val Val Ala Arg Asn Lys Tyr Gly Glu Asp Ser Cys Lys
5265                25270               25275               25280
Ala Lys Leu Thr Val Thr Leu His Pro Pro Pro Thr Asp Ser Thr Leu
                25285               25290               25295
Arg Pro Met Phe Lys Arg Leu Leu Ala Asn Ala Glu Cys Gln Glu Gly
            25300               25305               25310
Gln Ser Val Cys Phe Glu Ile Arg Val Ser Gly Ile Pro Pro Pro Thr
        25315               25320               25325
Leu Lys Trp Glu Lys Asp Gly Gln Pro Leu Ser Leu Gly Pro Asn Ile
    25330               25335               25340               2
Glu Ile Ile His Glu Gly Leu Asp Tyr Tyr Ala Leu His Ile Arg Asp
5345                25350               25355               25360
Thr Leu Pro Glu Asp Thr Gly Tyr Tyr Arg Val Thr Ala Thr Asn Thr
                25365               25370               25375
Ala Gly Ser Thr Ser Cys Gln Ala His Leu Gln Val Glu Arg Leu Arg
            25380               25385               25390
Tyr Lys Lys Gln Glu Phe Lys Ser Lys Glu Glu His Glu Arg His Val
        25395               25400               25405
Gln Lys Gln Ile Asp Lys Thr Leu Arg Met Ala Glu Ile Leu Ser Gly
    25410               25415               25420               2
Thr Glu Ser Val Pro Leu Thr Gln Val Ala Lys Glu Ala Leu Arg Glu
5425                25430               25435               25440
Ala Ala Val Leu Tyr Lys Pro Ala Val Ser Thr Lys Thr Val Lys Gly
                25445               25450               25455
Glu Phe Arg Leu Glu Ile Glu Glu Lys Lys Glu Glu Arg Lys Leu Arg
            25460               25465               25470
Met Pro Tyr Asp Val Pro Glu Pro Arg Lys Tyr Lys Gln Thr Thr Ile
        25475               25480               25485
Glu Glu Asp Gln Arg Ile Lys Gln Phe Val Pro Met Ser Asp Met Lys
    25490               25495               25500               2
Trp Tyr Lys Lys Ile Arg Asp Gln Tyr Glu Met Pro Gly Lys Leu Asp
5505                25510               25515               25520
 
Arg Val Val Gln Lys Arg Pro Lys Arg Ile Arg Leu Ser Arg Trp Glu
25525 25530 25535
Gln Phe Tyr Val Met Pro Leu Pro Arg Ile Thr Asp Gln Tyr Arg Pro
25540 25545 25550
Lys Trp Arg Ile Pro Lys Leu Ser Gln Asp Asp Leu Glu Ile Val Arg
25555 25560 25565
Pro Ala Arg Arg Arg Thr Pro Ser Pro Asp Tyr Asp Phe Tyr Tyr Arg
25570 25575 25580 2
Pro Arg Arg Arg Ser Leu Gly Asp Ile Ser Asp Glu Glu Leu Leu Leu
5585 25590 25595 25600
Pro Ile Asp Asp Tyr Leu Ala Met Lys Arg Thr Glu Glu Glu Arg Leu
25605 25610 25615
Arg Leu Glu Glu Glu Leu Glu Leu Gly Phe Ser Ala Ser Pro Pro Ser
25620 25625 25630
Arg Ser Pro Pro His Phe Glu Leu Ser Ser Leu Arg Tyr Ser Ser Pro
25635 25640 25645
Gln Ala His Val Lys Val Glu Glu Thr Arg Lys Asn Phe Arg Tyr Ser
25650 25655 25660 2
Thr Tyr His Ile Pro Thr Lys Ala Glu Ala Ser Thr Ser Tyr Ala Glu
5665 25670 25675 25680
Leu Arg Glu Arg His Ala Gln Ala Ala Tyr Arg Gln Pro Lys Gln Arg
25685 25690 25695
Gln Arg Ile Met Ala Glu Arg Glu Asp Glu Glu Leu Leu Arg Pro Val
25700 25705 25710
Thr Thr Thr Gln His Leu Ser Glu Tyr Lys Ser Glu Leu Asp Phe Met
25715 25720 25725
Ser Lys Glu Glu Lys Ser Arg Lys Lys Ser Arg Arg Gln Arg Glu Val
25730 25735 25740 2
Thr Glu Ile Thr Glu Ile Glu Glu Glu Tyr Glu Ile Ser Lys His Ala
5745 25750 25755 25760
Gln Arg Glu Ser Ser Ser Ser Ala Ser Arg Leu Leu Arg Arg Arg Arg
25765 25770 25775
Ser Leu Ser Pro Thr Tyr Ile Glu Leu Met Arg Pro Val Ser Glu Leu
25780 25785 25790
Ile Arg Ser Arg Pro Gln Pro Ala Glu Glu Tyr Glu Asp Asp Thr Glu
25795 25800 25805
Arg Arg Ser Pro Thr Pro Glu Arg Thr Arg Pro Arg Ser Pro Ser Pro
25810 25815 25820 2
Val Ser Ser Glu Arg Ser Leu Ser Arg Phe Glu Arg Ser Ala Arg Phe
5825 25830 25835 25840
Asp Ile Phe Ser Arg Tyr Glu Ser Met Lys Ala Ala Leu Lys Thr Gln
25845 25850 25855
Lys Thr Ser Glu Arg Lys Tyr Glu Val Leu Ser Gln Gln Pro Phe Thr
25860 25865 25870
Leu Asp His Ala Pro Arg Ile Thr Leu Arg Met Arg Ser His Arg Val
25875 25880 25885
Pro Cys Gly Gln Asn Thr Arg Phe Ile Leu Asn Val Gln Ser Lys Pro
25890 25895 25900 2
Thr Ala Glu Val Lys Trp Tyr His Asn Gly Val Glu Leu Gln Glu Ser
5905 25910 25915 25920
Ser Lys Ile His Tyr Thr Asn Thr Ser Gly Val Leu Thr Leu Glu Ile
25925 25930 25935
Leu Asp Cys His Thr Asp Asp Ser Gly Thr Tyr Arg Ala Val Cys Thr
25940 25945 25950
Asn Tyr Lys Gly Glu Ala Ser Asp Tyr Ala Thr Leu Asp Val Thr Gly
25955 25960 25965
Gly Asp Tyr Thr Thr Tyr Ala Ser Gln Arg Arg Asp Glu Glu Val Pro
25970 25975 25980 2
Arg Ser Val Phe Pro Glu Leu Thr Arg Thr Glu Ala Tyr Ala Val Pro
5985 25990 25995 26000
Ser Phe Lys Lys Thr Ser Glu Met Glu Ala Ser Ser Ser Val Arg Glu
26005 26010 26015
 
Val Lys Ser Gln Met Thr Glu Thr Arg Glu Ser Leu Ser Ser Tyr Glu
26020 26025 26030
His Ser Ala Ser Ala Glu Met Lys Ser Ala Ala Leu Glu Glu Lys Ser
26035 26040 26045
Leu Glu Glu Lys Ser Thr Thr Arg Lys Ile Lys Thr Thr Leu Ala Ala
26050 26055 26060 2
Arg Ile Leu Thr Lys Pro Arg Ser Met Thr Val Tyr Glu Gly Glu Ser
6065 26070 26075 26080
Ala Arg Phe Ser Cys Asp Thr Asp Gly Glu Pro Val Pro Thr Val Thr
26085 26090 26095
Trp Leu Arg Lys Gly Gln Val Leu Ser Thr Ser Ala Arg His Gln Val
26100 26105 26110
Thr Thr Thr Lys Tyr Lys Ser Thr Phe Glu Ile Ser Ser Val Gln Ala
26115 26120 26125
Ser Asp Glu Gly Asn Tyr Ser Val Val Val Glu Asn Ser Glu Gly Lys
26130 26135 26140 2
Gln Glu Ala Glu Phe Thr Leu Thr Ile Gln Lys Ala Arg Val Thr Glu
6145 26150 26155 26160
Lys Ala Val Thr Ser Pro Pro Arg Val Lys Ser Pro Glu Pro Arg Val
26165 26170 26175
Lys Ser Pro Glu Ala Val Lys Ser Pro Lys Arg Val Lys Ser Pro Glu
26180 26185 26190
Pro Ser His Pro Lys Ala Val Ser Pro Thr Glu Thr Lys Pro Thr Pro
26195 26200 26205
Arg Glu Lys Val Gln His Leu Pro Val Ser Ala Pro Pro Lys Ile Thr
26210 26215 26220 2
Gln Phe Leu Lys Ala Glu Ala Ser Lys Glu Ile Ala Lys Leu Thr Cys
6225 26230 26235 26240
Val Val Glu Ser Ser Val Leu Arg Ala Lys Glu Val Thr Trp Tyr Lys
26245 26250 26255
Asp Gly Lys Lys Leu Lys Glu Asn Gly His Phe Gln Phe His Tyr Ser
26260 26265 26270
Ala Asp Gly Thr Tyr Glu Leu Lys Ile Asn Asn Leu Thr Glu Ser Asp
26275 26280 26285
Gln Gly Glu Tyr Val Cys Glu Ile Ser Gly Glu Gly Gly Thr Ser Lys
26290 26295 26300 2
Thr Asn Leu Gln Phe Met Gly Gln Ala Phe Lys Ser Ile His Glu Lys
6305 26310 26315 26320
Val Ser Lys Ile Ser Glu Thr Lys Lys Ser Asp Gln Lys Thr Thr Glu
26325 26330 26335
Ser Thr Val Thr Arg Lys Thr Glu Pro Lys Ala Pro Glu Pro Ile Ser
26340 26345 26350
Ser Lys Pro Val Ile Val Thr Gly Leu Gln Asp Thr Thr Val Ser Ser
26355 26360 26365
Asp Ser Val Ala Lys Phe Ala Val Lys Ala Thr Gly Glu Pro Arg Pro
26370 26375 26380 2
Thr Ala Ile Trp Thr Lys Asp Gly Lys Ala Ile Thr Gln Gly Gly Lys
6385 26390 26395 26400
Tyr Lys Leu Ser Glu Asp Lys Gly Gly Phe Phe Leu Glu Ile His Lys
26405 26410 26415
Thr Asp Thr Ser Asp Ser Gly Leu Tyr Thr Cys Thr Val Lys Asn Ser
26420 26425 26430
Ala Gly Ser Val Ser Ser Ser Cys Lys Leu Thr Ile Lys Ala Ile Lys
26435 26440 26445
Asp Thr Glu Ala Gln Lys Val Ser Thr Gln Lys Thr Ser Glu Ile Thr
26450 26455 26460 2
Pro Gln Lys Lys Ala Val Val Gln Glu Glu Ile Ser Gln Lys Ala Leu
6465 26470 26475 26480
Arg Ser Glu Glu Ile Lys Met Ser Glu Ala Lys Ser Gln Glu Lys Leu
26485 26490 26495
Ala Leu Lys Glu Glu Ala Ser Lys Val Leu Ile Ser Glu Glu Val Lys
26500 26505 26510
 
Lys Ser Ala Ala Thr Ser Leu Glu Lys Ser Ile Val His Glu Glu Ile
26515 26520 26525
Thr Lys Thr Ser Gln Ala Ser Glu Glu Val Arg Thr His Ala Glu Ile
26530 26535 26540 2
Lys Ala Phe Ser Thr Gln Met Ser Ile Asn Glu Gly Gln Arg Leu Val
6545 26550 26555 26560
Leu Lys Ala Asn Ile Ala Gly Ala Thr Asp Val Lys Trp Val Leu Asn
26565 26570 26575
Gly Val Glu Leu Thr Asn Ser Glu Glu Tyr Arg Tyr Gly Val Ser Gly
26580 26585 26590
Ser Asp Gln Thr Leu Thr Ile Lys Gln Ala Ser His Arg Asp Glu Gly
26595 26600 26605
Ile Leu Thr Cys Ile Ser Lys Thr Lys Glu Gly Ile Val Lys Cys Gln
26610 26615 26620 2
Tyr Asp Leu Thr Leu Ser Lys Glu Leu Ser Asp Ala Pro Ala Phe Ile
6625 26630 26635 26640
Ser Gln Pro Arg Ser Gln Asn Ile Asn Glu Gly Gln Asn Val Leu Phe
26645 26650 26655
Thr Cys Glu Ile Ser Gly Glu Pro Ser Pro Glu Ile Glu Trp Phe Lys
26660 26665 26670
Asn Asn Leu Pro Ile Ser Ile Ser Ser Asn Val Ser Ile Ser Arg Ser
26675 26680 26685
Arg Asn Val Tyr Ser Leu Glu Ile Arg Asn Ala Ser Val Ser Asp Ser
26690 26695 26700 2
Gly Lys Tyr Thr Ile Lys Ala Lys Asn Phe Arg Gly Gln Cys Ser Ala
6705 26710 26715 26720
Thr Ala Ser Leu Met Val Leu Pro Leu Val Glu Glu Pro Ser Arg Glu
26725 26730 26735
Val Val Leu Arg Thr Ser Gly Asp Thr Ser Leu Gln Gly Ser Phe Ser
26740 26745 26750
Ser Gln Ser Val Gln Met Ser Ala Ser Lys Gln Glu Ala Ser Phe Ser
26755 26760 26765
Ser Phe Ser Ser Ser Ser Ala Ser Ser Met Thr Glu Met Lys Phe Ala
26770 26775 26780 2
Ser Met Ser Ala Gln Ser Met Ser Ser Met Gln Glu Ser Phe Val Glu
6785 26790 26795 26800
Met Ser Ser Ser Ser Phe Met Gly Ile Ser Asn Met Thr Gln Leu Glu
26805 26810 26815
Ser Ser Thr Ser Lys Met Leu Lys Ala Gly Ile Arg Gly Ile Pro Pro
26820 26825 26830
Lys Ile Glu Ala Leu Pro Ser Asp Ile Ser Ile Asp Glu Gly Lys Val
26835 26840 26845
Leu Thr Val Ala Cys Ala Phe Thr Gly Glu Pro Thr Pro Glu Val Thr
26850 26855 26860 2
Trp Ser Cys Gly Gly Arg Lys Ile His Ser Gln Glu Gln Gly Arg Phe
6865 26870 26875 26880
His Ile Glu Asn Thr Asp Asp Leu Thr Thr Leu Ile Ile Met Asp Val
26885 26890 26895
Gln Lys Gln Asp Gly Gly Leu Tyr Thr Leu Ser Leu Gly Asn Glu Phe
26900 26905 26910
Gly Ser Asp Ser Ala Thr Val Asn Ile His Ile Arg Ser Ile
26915 26920 26925
<210> 3
   <211> 21
   <212> DNA
   <213> Danio rerio
<400> 3
   agggacactc agagaccata g 21
<210> 4
   <211> 40
   <212> DNA
   <213> Danio rerio
<400>
   taatacgact cactataggg gtctgaggat actcgccttc 40
<210> 5
   <211> 27
   <212> DNA
   <213> Danio rerio
<400> 5
   tttgaaccac ttgaaggtca caccagg 27
<210> 6
   <211> 30
   <212> DNA
   <213> Danio rerio
<400> 6
   gctaagaatg actatggagt tgccacaagc 30
<210> 7
   <211> 27
   <212> DNA
   <213> Danio rerio
<400> 7
   tgaaccactt gaaggtcaca ccaggag 27
<210> 8
   <211> 41
   <212> DNA
   <213> Danio rerio
<400> 8
   taatacgact cactataggg agggacactc agagaccata g 41
<210> 9
   <211> 40
   <212> DNA
   <213> Danio rerio
<400> 9
   taatacgact cactataggg gtctgaggat actcgccttc 40
<210> 10
   <211> 21
   <212> DNA
   <213> Danio rerio
<400> 10
   agggacactc agagaccata g 21
<210> 11
   <211> 22
   <212> DNA
   <213> Danio rerio
<400> 11
   ggcaatgtta ctctctgttg ag 22